# EUROPEAN PATENT SPECIFICATION

(11) **EP 3 551 767 B1**
(45) Date of publication and mention of the grant of the patent: **18.06.2025**
(21) Application number: 17877986.4
(22) Date of filing: 11.12.2017
(51) Int. Cl.: C12Q 1/6886, C12Q 1/6827

(54) **IMAGE DIFFERENTIATED MULTIPLEX ASSAYS FOR MULTIPLEX DETECTION OF DNA MUTATIONS**
BILDDIFFERENZIERTE MULTIPLEX-ASSAYS ZUR MULTIPLEX-DETEKTION VON DNA-MUTATIONEN
DOSAGES MULTIPLEX DIFFÉRENCIÉS PAR IMAGE POUR LA DÉTECTION MULTIPLEX DE MUTATIONS DE L'ADN

(30) Priority: 09.12.2016 US 201662432534 P; 08.12.2017 US 201715836809
(43) Date of publication of application: 16.10.2019
(73) Proprietor: Plexbio Co., Ltd., Taipei 11491 (TW)
(72) Inventor: TSAO, Dean, Hillsborough, CA 94010 (US); HUANG, Chin-shiou, Taipei 11491 (TW); HU, Shian, Pin, Taipei 11491 (TW)
(74) Representative: J A Kemp LLP
(86) International application number: PCT/US2017/065656
(87) International publication number: WO 2018/107183

(56) References cited:
- EP-A1- 2 774 997
- WO-A1-2014/144016
- WO-A1-2016/198954
- US-A1- 2007 172 823
- MANDY SCHNEIDER ET AL: "Detection of up to 65% of Precancerous Lesions of the Human Colon and Rectum by Mutation Analysis of APC, K-Ras, B-Raf and CTNNB1", CANCERS, vol. 3, no. 1, 29 December 2010 (2010-12-29), pages 91 - 105, XP055708735, DOI: 10.3390/cancers3010091
- LI GUOQIANG ET AL: "A novel liquidchip platform for simultaneous detection of 70 alleles of DNA somatic mutations on EGFR, KRAS, BRAF and PIK3CA from formalin-fixed and paraffin-embedded slides containing tumor tissue", CLINICAL CHEMISTRY AND LABORATORY MEDICINE, DE GRUYTER, DE, vol. 49, no. 2, 1 February 2011 (2011-02-01), pages 191 - 195, XP009162631, ISSN: 1434-6621, DOI: 10.1515/CCLM.2011.040
- DANIEL O. HERZIG ET AL: "Molecular markers for colon diagnosis, prognosis and targeted therapy : Molecular Markers for Colon Diagnosis", JOURNAL OF SURGICAL ONCOLOGY, vol. 111, no. 1, 8 October 2014 (2014-10-08), US, pages 96 - 102, XP055708728, ISSN: 0022-4790, DOI: 10.1002/jso.23806
- STEPHEN ANGELONI ET AL: "xMAP Cookbook", 15 January 2016 (2016-01-15), XP055708390, Retrieved from the Internet <URL:http://www.komabiotech.co.kr/www/techniques/pdf/Luminex_cookbook_3rd.pdf> [retrieved on 20200624]
- MILENA I IVANOVA ET AL: "Novel multiplex bead-based assay with LNA-modified probes for detection ofexon 10 mutations", LEUKEMIA RESEARCH, NEW YORK,NY, US, vol. 35, no. 8, 12 April 2011 (2011-04-12), pages 1120 - 1123, XP028245709, ISSN: 0145-2126, [retrieved on 20110418], DOI: 10.1016/J.LEUKRES.2011.04.012
- STEFAAN DERVEAUX ET AL: "Layer-by-Layer Coated Digitally Encoded Microcarriers for Quantification of Proteins in Serum and Plasma", ANALYTICAL CHEMISTRY, vol. 80, no. 1, 1 January 2008 (2008-01-01), US, pages 85 - 94, XP055334621, ISSN: 0003-2700, DOI: 10.1021/ac071212i
- BRAECKMANS KEVIN ET AL: "Encoding microcarriers by spatial selective photobleaching", NATURE MATERIALS, NATURE PUB. GROUP, LONDON, vol. 2, no. 3, 1 March 2003 (2003-03-01), pages 169 - 173, XP002495700, ISSN: 1476-1122, [retrieved on 20030202], DOI: 10.1038/NMAT828
- VOORHAM, Q.J.M ET AL.: "Comprehensive Mutation Analysis in Colorectal Flat Adenomas", PLOS ONE, vol. 7, 2012, pages e41963, XP055491198
- AMASON, T. ET AL.: "Morphology, tumor genetics, and outcomes in five cases of biliary adenofibroma", LABORATORY INVESTIGATION, vol. 94, no. 1, pages 417A, XP9514984
- FRIEDMAN, K. ET AL.: "A rapid multiplex mass spectrometry assay for mutational profiling of colorectal cancer", LABORATORY INVESTIGATION, vol. 95, no. 1, 2015, pages 514A, XP9514985

## Description

### FIELD

Provided herein are methods for multiplex detection of DNA mutations in a sample using microcarriers, as well as kits related thereto. The microcarriers are encoded with an identifier and include a probe for detection of a DNA mutation of interest.

### BACKGROUND

Early detection is a critically important factor in reducing the number of deaths attributable to cancer, since growth and metastasis of more advanced tumors are associated with increased mortality. Most cases of colorectal cancer are sporadic, rather than hereditary, but several genes and even types of specific mutations are commonly seen in these sporadic cases. For example, the *KRAS* gene is thought to be mutated in 30-50% of colorectal cancers (Mundade, R. et al. (2014) Oncoscience 1:400-6). While existing techniques such as colonoscopy and sigmoidoscopy are effective in detecting many types of early colorectal cancers, they suffer from low patient acceptance due to their invasive nature. Alternatives such as fecal DNA tests have been tested (Carethers, J.M. (2014) Clin. Gastroenterol. Hepatol. 12:377-81), but there is a need for comprehensive tests that examine multiple genes simultaneously with a high level of accuracy, rather than individual gene testing.

Immunological and molecular diagnostic assays play a critical role both in the research and clinical fields. Often it is necessary to perform assays for a panel of multiple targets to gain a meaningful or bird's-eye view of results to facilitate research or clinical decision-making. This is particularly true in the era of genomics and proteomics, where an abundance of genetic markers and/or biomarkers are thought to influence or be predictive of particular disease states. In theory, assays of multiple targets can be accomplished by testing each target separately in parallel or sequentially in different reaction vessels (*i.e.,* multiple singleplexing). However, not only are assays adopting a singleplexing strategy often cumbersome, but they also typically required large sample volumes, especially when the targets to be analyzed are large in number.

A multiplex assay simultaneously measures multiple analytes (two or more) in a single assay. Multiplex assays are commonly used in high-throughput screening settings, where many specimens can be analyzed at once. It is the ability to assay many analytes simultaneously and many specimens in parallel that is the hallmark of multiplex assays and is the reason that such assays have become a powerful tool in fields ranging from drug discovery to functional genomics to clinical diagnostics. In contrast to singleplexing, by combining all targets in the same reaction vessel, the assay is much less cumbersome and much easier to perform, since only one reaction vessel is handled per sample. The required test samples can thus be dramatically reduced in volume, which is especially important when samples (*e.g*., tumor tissues, cerebral spinal fluid, or bone marrow) are difficult and/or invasive to retrieve in large quantities. Equally important is the fact that the reagent cost can be decreased and assay throughput increased drastically.

Many assays of complex macromolecule samples are composed of two steps. In the first step, agents capable of specifically capturing the target macromolecules are attached to a solid phase surface. These immobilized molecules may be used to capture the target macromolecules from a complex sample by various means, such as hybridization (*e.g*., in DNA, RNA based assays). In the second step, detection molecules are incubated with and bind to the complex of capture molecule and the target, emitting signals such as fluorescence or other electromagnetic signals. The amount of the target is then quantified by the intensity of those signals.

Multiplex assays may be carried out by utilizing multiple capture agents, each specific for a different target macromolecule. In chip-based array multiplex assays, each type of capture agent (*e.g.,* a single-stranded oligonucleotide probe) is attached to a pre-defined position on the chip. The amount of multiplex targets in a complex sample is determined by measuring the signal of the detection molecule at each position corresponding to a type of capture agent. In suspension array multiplex assays, microparticles or microcarriers are suspended in the assay solution. These microparticles or microcarriers contain an identification element, which may be embedded, printed, or otherwise generated by one or more elements of the microparticle/microcarrier. Each type of capture agent is immobilized to particles with the same ID, and the signals emitted from the detection molecules on the surface of the particles with a particular ID reflect the amount of the corresponding target.

One application for which multiplex assays are particularly well-suited is detection of DNA mutations. In particular, detecting mutations associated with cancer can aid in early diagnosis of cancer and in detection of tumors that are small and/or in locations not easily found through conventional diagnostic tools, such as colonoscopies. *See, e.g.,* U.S. Patent No. 7,833,757. However, existing diagnostic techniques are often expensive or time-consuming. Methods for detecting multiple gene mutations using serial, individual assays are time consuming and suffer from lack of uniformity if carried out using different assay types (*see* Schneider, M. et al. (2011) Cancers 3:91-105). Applying multiplex assay technologies such as analog-encoded microcarriers to this problem can provide cheaper, quicker assays with more accurate results while enabling multiplex screening for many mutations known to be correlated with tumorigenesis in a single assay.
Mandy Schneider et al. (2010) describes use of mutation analysis of APC, K-Ras, B-Raf and CTNNB1 to detect precancerous lesions of the human colon and rectum.
Li Guoqiang et al. (2011) describes the detection of DNA somatic mutations on EGFR, KRAS, BRAF and PIK2CA from formalin-fixed and paraffin-embedded slides using a liquidchip platform.
Stephen Angeloni et al. (2016) describes the analysis of mutations using PCR and subsequent analysis of the PCR products by encoded microbeads.
WO 2014/144016 A1 describes compositions of beads encoded with information storing identifiers and methods for use in multiplex chemical and biological assays.

Therefore, a need exists for applying a robust and sensitive multiplex assay system to the problem of screening for DNA mutations. This provides a mechanism for multiplex detection of many DNA mutations in a single assay.

### BRIEF SUMMARY

To meet this need, the invention provides a method for detecting the presence of DNA mutations in the *KRAS, BRAF, CTNNB1,* and *APC* genes, the method comprising: (a) isolating DNA from a sample; (b) amplifying the isolated DNA by polymerase chain reaction (PCR) using primer pairs specific for the loci of one or more DNA mutations in each of the *KRAS, BRAF, CTNNB1*, and *APC* genes, wherein the isolated DNA is amplified in the presence of at least four blocking nucleic acids, wherein each of said at least four blocking nucleic acids hybridizes with a wild-type DNA locus corresponding with one of the DNA mutations in the KRAS, BRAF, CTNNB1, or APC genes and prevents amplification of the wild-type DNA locus, wherein optionally the *KRAS, BRAF, CTNNB1,* and *APC* genes are human genes; (c) hybridizing the amplified DNA with at least four probes, said at least four probes comprising one or more probes specific for a DNA mutation in each of the *KRAS, BRAF, CTNNB1*, and *APC* genes, wherein each of said at least four probes is coupled to a microcarrier, and wherein each of the microcarriers comprises an identifier corresponding to the probe coupled thereto; (d) detecting presence or absence of hybridization of the amplified DNA with said at least four probes, wherein hybridization between the amplified DNA and one of the probes indicates the presence of the DNA mutation corresponding to the probe; (e) detecting the identifiers of the microcarriers; and (f) correlating the detected identifiers of the microcarriers with the detected presence or absence of hybridization of the amplified DNA to the corresponding probes of the microcarriers.
The invention also provides a kit comprising (a) at least four microcarriers, wherein each of said at least four microcarriers comprises: (i) a probe coupled to the microcarrier, wherein the probe is specific for a DNA mutation in the *KRAS, BRAF, CTNNB1*, or *APC* gene, wherein optionally the KRAS, BRAF, *CTNNB1*, and APC genes are human genes; and (ii) an identifier corresponding to the probe coupled thereto; wherein the kit comprises at least one microcarrier comprising a probe specific for a DNA mutation in the *KRAS* gene, at least one microcarrier comprising a probe specific for a DNA mutation in the *BRAF* gene, at least one microcarrier comprising a probe specific for a DNA mutation in the *CTNNB1* gene, and at least one microcarrier comprising a probe specific for a DNA mutation in the *APC* gene, and (b) at least four blocking nucleic acids, wherein each of said at least four blocking nucleic acids hybridizes with a wild-type DNA locus corresponding with one of the DNA mutations in the KRAS, BRAF, CTNNB1, or APC genes.
The methods and kits disclosed herein may find use, *e.g.,* in monitoring colorectal cancer, monitoring response to treatment of colorectal cancer, and/or early screening/detection of colorectal cancer.

In some embodiments, the *KRAS, BRAF, CTNNB1*, and *APC* genes are human genes. In some embodiments, each of said at least four blocking nucleic acids comprises: a single-stranded oligonucleotide that hybridizes with the corresponding wild-type DNA locus; and a 3' terminal moiety that blocks extension from the single-stranded oligonucleotide. In some embodiments, the 3' terminal moiety comprises one or more inverted deoxythymidines. In some embodiments, each of said at least four blocking nucleic acids comprises one or more modified nucleotides selected from the group consisting of locked nucleic acids (LNAs), peptide nucleic acids (PNAs), hexose nucleic acids (HNAs), threose nucleic acids (TNAs), glycol nucleic acids (GNAs), and cyclohexenyl nucleic acids (CeNAs). In some embodiments, the one or more DNA mutations in the *KRAS* gene comprise one or more DNA mutations encoding a G12D, G12V, G12S, or G13D mutated KRAS protein. In some embodiments, at least one of the at least four blocking nucleic acids comprises the sequence TACGCCACCAGCT(invdT)*ₙ*, wherein *n* is 1, 2, or 3 (SEQ ID NO:3), TT*GG*A*G*CT*GGTGGC*GTA(invdT)*ₙ*, wherein *n* is 1, 2, or 3 (SEQ ID NO: 142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n* is 1, 2, or 3 (SEQ ID NO: 144), or TT*GG*A*G*CT*GG*T*GG*C*G*T(invdT)*ₙ*, wherein *n* is 1, 2, or 3 (SEQ ID NO: 145), with italicized nucleic acids representing locked nucleic acids. In some embodiments, the one or more DNA mutations in the *BRAF* gene comprise one or more DNA mutations encoding a V600E mutated BRAF protein. In some embodiments, at least one of the at least four blocking nucleic acids comprises the sequence G*AGAT*T*TCAC*T*GTAGC*(invdT)*ₙ,* wherein *n* is 1, 2, or 3 (SEQ ID NO:10), G*AGAT*T*TCAC*T*GTAGC*(invdT)*ₙ,* wherein *n is* 1, 2, or 3 (SEQ ID NO: 146), G*AGAT*T*TCAC*T*GTAGC*(invdT)*ₙ,* wherein *n is* 1, 2, or 3 (SEQ ID NO: 147), G*AGAT*T*TCAC*T*GTAGC*(invdT)*ₙ,* wherein *n* is 1, 2, or 3 (SEQ ID NO:148), or G*AGAT*T*TCAC*T*GTAGC*(invdT)*ₙ,* wherein *n* is 1, 2, or 3 (SEQ ID NO: 149), with italicized nucleic acids representing locked nucleic acids. In some embodiments, the one or more DNA mutations in the *CTNNB1* gene comprise at least a first *CTNNB1* mutation encoding a T41A or T41I mutated CTNNB1 protein. In some embodiments, at least one of the at least four blocking nucleic acids comprises the sequence GC*CACTACCACAG*CT(invdT)*ₙ,* wherein *n* is 1, 2, or 3 (SEQ ID NO:15), T*G*C*CA*CT*ACCA*C*AG*(invdT)*ₙ,* wherein *n* is 1, 2, or 3 (SEQ ID NO:150), C*AC*T*ACCA*C*AGC*T*C*C(invdT)*ₙ,* wherein *n* is 1, 2, or 3 (SEQ ID NO:151), GC*CACTACCACAG*CT(invdT)*ₙ,* wherein *n* is 1, 2, or 3 (SEQ ID NO: 152), or GC*CACTACCACAG*CT(invdT)*ₙ,* wherein *n* is 1, 2, or 3 (SEQ ID NO: 153), with italicized nucleic acids representing locked nucleic acids. In some embodiments, the one or more DNA mutations in the *CTNNB1* gene comprise at least a first *CTNNB1* mutation encoding an S45F or S45P mutated CTNNB1 protein. In some embodiments, at least one of the at least four blocking nucleic acids comprises the sequence GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein *n* is 1, 2, or 3 (SEQ ID NO:174), G*C*T*CC*T*TC*TC*TGA*GT(invdT)n, wherein n is 1, 2, or 3 (SEQ ID NO: 175), T*CC*TT*CT*CT*GAG*T*G*G(invdT)n, wherein n is 1, 2, or 3 (SEQ ID NO: 176), or G*C*T*CC*TT*CTCTGAG*T(invdT)n, wherein n is 1, 2, or 3 (SEQ ID NO: 177), with italicized nucleic acids representing locked nucleic acids. In some embodiments, the one or more DNA mutations in the *APC* gene comprise at least a first *APC* mutation encoding a Q1367* mutated APC protein. In some embodiments, at least one of the at least four blocking nucleic acids comprises the sequence GTG*CTCA*G*ACAC*C(invdT)*ₙ,* wherein *n is* 1, 2, or 3 (SEQ ID NO:33), G*TGC*T*CA*G*A*C*ACC*(invdT)n, wherein n is 1, 2, or 3 (SEQ ID NO: 158), AGTGGTGCTCAGACACCCA(invdT)n, wherein n is 1, 2, or 3 (SEQ ID NO:159), *AGTGGTGCTCAGACACCCA(invdT)n,* wherein n is 1, 2, or 3 (SEQ ID NO:160), or AGTGGTGCTCAGACACCCA(invdT)n, wherein n is 1, 2, or 3 (SEQ ID NO:161), with italicized nucleic acids representing locked nucleic acids. In some embodiments, the one or more DNA mutations in the *APC* gene comprise at least a first *APC* mutation encoding an R1450* mutated APC protein. In some embodiments, at least one of the at least four blocking nucleic acids comprises the sequence C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO:162), *CTTCTCGCTTGGTT(invdT)n,* wherein n is 1, 2, or 3 (SEQ ID NO:163), GT*ACT*T*CTCG*CT*TGG*T(invdT)n, wherein n is 1, 2, or 3 (SEQ ID NO:164), or GT*ACTTCTCGC*TT*GG*T(invdT)n, wherein n is 1, 2, or 3 (SEQ ID NO:165), with italicized nucleic acids representing locked nucleic acids. In some embodiments, the one or more DNA mutations in the *APC* gene comprise at least a first *APC* mutation encoding an E1309 frameshift mutated APC protein. In some embodiments, at least one of the at least four blocking nucleic acids comprises the sequence C*TTTTCTTTTAT*TT*C*T*GC*(invdT)*ₙ,* wherein *n* is 1, 2, or 3 (SEQ ID NO:29), *CTTTTCTTTTATTTCTGC(invdT)n,* wherein n is 1, 2, or 3 (SEQ ID NO:154), *CTTTTCTTTTATTTCTGC(invdT)n,* wherein n is 1, 2, or 3 (SEQ ID NO:155), C*TTT*TCT*TTT*A*T*T*TC*T*GC*(invdT)n, wherein n is 1, 2, or 3 (SEQ ID NO:156), or *CTTTTCTTTTATTTCTGC(invdT)n,* wherein n is 1, 2, or 3 (SEQ ID NO:157), with italicized nucleic acids representing locked nucleic acids. In some embodiments, the one or more DNA mutations in the *APC* gene comprise at least a first *APC* mutation encoding an S1465 frameshift mutated APC protein. In some embodiments, at least one of the at least four blocking nucleic acids comprises the sequence *CCA*C*TC*TCTCT*C*T*TT*T*CAGC*(invdT)*ₙ,* wherein *n* is 1, 2, or 3 (SEQ ID NO:25), TA*GG*T*CC*ACTCTCTCTCT*TT*T*C*A*GC*A(invdT)n, wherein n is 1, 2, or 3 (SEQ ID NO:166), *TAGGTCCACTCTCTCTCTTTTCAGCA* (invdT)n, wherein n is 1, 2, or 3 (SEQ ID NO:167), *CCACTCTCTCTCTTTTCAGC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO:169), with italicized nucleic acids representing locked nucleic acids. In some embodiments, the one or more DNA mutations in the *APC* gene comprise at least a first *APC* mutation encoding a T1556 frameshift mutated APC protein. In some embodiments, at least one of the at least four blocking nucleic acids comprises the sequence *C*A*ATAG*T*TTTT*T*CTGC*C(invdT)*ₙ,* wherein *n* is 1, 2, or 3 (SEQ ID NO:41), *GAATCAATAGTTTTTTCTGCCTC* (invdT)n, wherein n is 1, 2, or 3 (SEQ ID NO:170), *TCAGAATCAATAGTTTTTTCTG* (invdT)n, wherein n is 1, 2, or 3 (SEQ ID NO:171), *GAATCAATAGATTTTACTGCCTC* (invdT)n, wherein n is 1, 2, or 3 (SEQ ID NO:172), or *AATCAATAGTTTTTTCTGCCTC* (invdT)n, wherein n is 1, 2, or 3 (SEQ ID NO:173), with italicized nucleic acids representing locked nucleic acids. In some embodiments, each of the primer pairs comprises a primer coupled to a detection reagent. In some embodiments, the detection reagent comprises a fluorescent detection reagent, and wherein detecting the presence or absence of hybridization of the amplified DNA with said at least four probes in step (d) comprises fluorescence imaging of the fluorescent detection reagent. In some embodiments, the detection reagent comprises biotin, and wherein detecting the presence or absence of hybridization of the amplified DNA with said at least four probes in step (d) comprises: (1) after hybridization in step (c), contacting the microcarriers with streptavidin conjugated to a signal-emitting entity; and (2) detecting a signal from the signal-emitting entity in association with the microcarriers. In some embodiments, the signal-emitting entity comprises phycoerythrin (PE). In some embodiments, detecting the identifiers of the microcarriers in step (e) comprises bright field imaging of the identifiers.

In some embodiments, the one or more DNA mutations in the *KRAS* gene comprise *KRAS* mutations encoding G12D, G12V, G12S, and G13D mutated KRAS proteins. In some embodiments, the probes specific for one or more DNA mutations in the *KRAS* gene comprise four probes comprising the sequences GGAGCTGATGG (SEQ ID NO:4), GGAGCTGTTGG (SEQ ID NO:5), TGGAGCTAGTGG (SEQ ID NO:6), and TGGAGCTGGTGACGT (SEQ ID NO:7), and wherein each of the four probes is coupled to a microcarrier with a different identifier. In some embodiments, each of the four probes further comprises eight nucleotides at the 5' end, wherein the eight nucleotides at the 5' end are adenine or thymine nucleotides, and wherein each of the four probes comprises at least 24 total nucleotides. In some embodiments, the four probes comprise: (1) a first probe comprising a sequence selected from the group consisting of GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO: 180), and GCTGATGGCGTA (SEQ ID NO:181); (2) a second probe comprising a sequence selected from the group consisting of GGAGCTGTTGG (SEQ ID NO:5), TGGAGCTGTTGGTGGC (SEQ ID NO: 182), GGAGCTGTTGGTG (SEQ ID NO: 183), TGGAGCTGTTGGT (SEQ ID NO:184), and TGGAGCTGTaGGTGG (SEQ ID NO: 185); (3) a third probe comprising a sequence selected from the group consisting of TTGGAGCTAGTGGCGTA (SEQ ID NO:186), GCTAGTGGCGTAGGC (SEQ ID NO:187), AGCTAGTGGCGT (SEQ ID NO: 188), GTTGGAGCTAGTGG (SEQ ID NO: 189), and GGAGCTAGTGG (SEQ ID NO:190); and (4) a fourth probe comprising a sequence selected from the group consisting of GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), and GGAGCTGGTGACGT (SEQ ID NO:195); and wherein each of the four probes is coupled to a microcarrier with a different identifier. In some embodiments, the four probes comprise: (1) a first probe comprising a sequence selected from the group consisting of TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), and TTTTTTTTTTTTTGCTGATGGCGTA (SEQ ID NO:77); (2) a second probe comprising a sequence selected from the group consisting of TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), and TTTTTTTTTATGGAGCTGTAGGTGG (SEQ ID NO:81); (3) a third probe comprising a sequence selected from the group consisting of TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), and TTTTTTTTTTTTAAGGAGCTAGTGG (SEQ ID NO:86); and (4) a fourth probe comprising a sequence selected from the group consisting of TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), and TTTTTTTTTAAGGAGCTGGTGACGT (SEQ ID NO:91); and wherein each of the four probes is coupled to a microcarrier with a different identifier. In some embodiments, step (b) comprises amplifying the isolated DNA by PCR using a primer pair comprising the sequences GTACTGGTGGAGTATTTGATAGTG (SEQ ID NO:1) and ATCGTCAAGGCACTCTTGCCTAC (SEQ ID NO:2). In some embodiments, step (b) comprises amplifying the isolated DNA by PCR in the presence of a blocking nucleic acid comprising the sequence of TA*C*G*CC*A*CC*A*G*CT(invdT)*ₙ*, wherein n is 1, 2, or 3 (SEQ ID NO:3), TT*GG*A*G*CT*GGTGGC*GTA(invdT)*ₙ*, wherein *n* is 1, 2, or 3 (SEQ ID NO: 142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n* is 1, 2, or 3 (SEQ ID NO: 144), or TT*GG*A*G*CT*GG*T*GG*C*G*T(invdT)*ₙ*, wherein *n* is 1, 2, or 3 (SEQ ID NO: 145), with italicized nucleic acids representing locked nucleic acids.

In some embodiments, the one or more DNA mutations in the *BRAF* gene comprise two or more *BRAF* mutations encoding a V600E mutated BRAF protein. In some embodiments, the probes specific for one or more DNA mutations in the *BRAF* gene comprise two probes comprising the sequences TCTAGCTACAGAGAAAT (SEQ ID NO:11) and GTCTAGCTACAGAAAAAT (SEQ ID NO:12), and wherein each of the two probes is coupled to a microcarrier with a different identifier. In some embodiments, each of the two probes further comprises eight nucleotides at the 5' end, wherein the eight nucleotides at the 5' end are adenine or thymine nucleotides, and wherein each of the two probes comprises at least 24 total nucleotides. In some embodiments, the probes comprise: (1) a first probe comprising a sequence selected from the group consisting of TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), and TCTAGCTACAGAG (SEQ ID NO:200); and (2) a second probe comprising a sequence selected from the group consisting of GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204); and wherein each of the two probes is coupled to a microcarrier with a different identifier. In some embodiments, the probes comprise: (1) a first probe comprising a sequence selected from the group consisting of TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), and TTTTTTTTTTAATTTCTAGCTACAGAG (SEQ ID NO:96); and (2) a second probe comprising a sequence selected from the group consisting of TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and TTTTTTTTATTCTAGCTACAGAAAAATC (SEQ ID NO:100); and wherein each of the two probes is coupled to a microcarrier with a different identifier. In some embodiments, step (b) comprises amplifying the isolated DNA by PCR using a primer pair comprising the sequences GGACCCACTCCATCGAGATTT (SEQ ID NO:8) and CAGATATATTTCTTCATGAAGACCTCACAGTAA (SEQ ID NO:9). In some embodiments, step (b) comprises amplifying the isolated DNA by PCR in the presence of a blocking nucleic acid comprising the sequence of G*AGAT*T*TCAC*T*GTAGC*(invdT)*ₙ,* wherein *n* is 1, 2, or 3 (SEQ ID NO:10), G*AGAT*T*TCAC*T*GTAGC*(invdT)*ₙ,* wherein *n* is 1, 2, or 3 (SEQ ID NO:146), G*AGAT*T*TCAC*T*GTAGC*(invdT)*ₙ,* wherein *n* is 1, 2, or 3 (SEQ ID NO: 147), G*AGAT*T*TCAC*T*GTAGC*(invdT)*ₙ,* wherein *n* is 1, 2, or 3 (SEQ ID NO: 148), or G*AGAT*T*TCAC*T*GTAGC*(invdT)*ₙ,* wherein *n* is 1, 2, or 3 (SEQ ID NO: 149), with italicized nucleic acids representing locked nucleic acids.

In some embodiments, the one or more DNA mutations in the *CTNNB1* gene comprise *CTNNB1* mutations encoding T41A, T41I, S45F, and S45P mutated CTNNB1 proteins. In some embodiments, the probes specific for one or more DNA mutations in the *CTNNB1* gene comprise four probes comprising the sequences AGGAGCTGTGGCAG (SEQ ID NO:16), GGAGCTGTGATA (SEQ ID NO: 17), TTTACCACTCAGAAAAG (SEQ ID NO:21), and TACCACTCAGAGGAG (SEQ ID NO:22), and wherein each of the four probes is coupled to a microcarrier with a different identifier. In some embodiments, each of the four probes further comprises eight nucleotides at the 5' end, wherein the eight nucleotides at the 5' end are adenine or thymine nucleotides, and wherein each of the four probes comprises at least 24 total nucleotides. In some embodiments, the probes comprise: (1) a first probe comprising a sequence selected from the group consisting of AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), and AAGGAGCTGTGGCAG (SEQ ID NO:209); (2) a second probe comprising a sequence selected from the group consisting of GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), and GGAGCTGTGAT (SEQ ID NO:214); (3) a third probe comprising a sequence selected from the group consisting of ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), and CAGAAAAGGAGCTGTG (SEQ ID NO:219); and (4) a fourth probe comprising a sequence selected from the group consisting of ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), and TTACCAATCAGAGGAGG (SEQ ID NO:224); and wherein each of the four probes is coupled to a microcarrier with a different identifier. In some embodiments, the probes comprise: (1) a first probe comprising a sequence selected from the group consisting of TTTTTTTTTTTAGGAGCTGTGGCAG (SEQ ID NO:53), TTTTTTTTTTTAGGAGCTGTGGCAGTG (SEQ ID NO:101), TTTTTTTTTTTAGCTGTGGCAGTGGC (SEQ ID NO:102), TTTTTTTTTTTGCTGTGGCAGTGGCA (SEQ ID NO:103), and TTTTTTTTTTAAGGAGCTGTGGCAG (SEQ ID NO:104); (2) a second probe comprising a sequence selected from the group consisting of TTTTTTTTTTTTTGGAGCTGTGATA (SEQ ID NO:54), TTTTTTTTTGGAGCTGTGATAGTGG (SEQ ID NO:105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), and TTTTTTTTTTTTTTGGAGCTGTGAT (SEQ ID NO:109); (3) a third probe comprising a sequence selected from the group consisting of TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), and TTTTTTTTTCAGAAAAGGAGCTGTG (SEQ ID NO:114); and (4) a fourth probe comprising a sequence selected from the group consisting of TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and TTTTTTTTTATTACCAATCAGAGGAGG (SEQ ID NO:119), and wherein each of the four probes is coupled to a microcarrier with a different identifier. In some embodiments, step (b) comprises amplifying the isolated DNA by PCR using a first primer pair comprising the sequences GGAATCCATTCTGGTGCCACT (SEQ ID NO:13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO:19). In some embodiments, step (b) comprises amplifying the isolated DNA by PCR in the presence of a first blocking nucleic acid comprising the sequence of GC*CACTACCACAG*CT(invdT)*ₙ,* wherein *n* is 1, 2, or 3 (SEQ ID NO:15), T*G*C*CA*CT*ACCA*C*AG*(invdT)*ₙ,* wherein *n* is 1, 2, or 3 (SEQ ID NO:150), C*AC*T*ACCA*C*AGC*T*C*C(invdT)*ₙ,* wherein *n* is 1, 2, or 3 (SEQ ID NO:151), GC*CACTACCACAG*CT(invdT)*ₙ,* wherein *n* is 1, 2, or 3 (SEQ ID NO:152), or GC*CACTACCACAG*CT(invdT)*ₙ,* wherein *n* is 1, 2, or 3 (SEQ ID NO: 153), and a second blocking nucleic acid comprising the sequence of GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein *n* is 1, 2, or 3 (SEQ ID NO:174), G*C*T*CC*T*TC*TC*TGA*GT(invdT)n, wherein n is 1, 2, or 3 (SEQ ID NO:175), T*CC*TT*CT*CT*GAG*T*G*G(invdT)n, wherein n is 1, 2, or 3 (SEQ ID NO: 176), or G*C*T*CC*TT*CT*C*TGAG*T(invdT)n, wherein n is 1, 2, or 3 (SEQ ID NO: 177), with italicized nucleic acids representing locked nucleic acids.

In some embodiments, the one or more DNA mutations in the *APC* gene comprise *APC* mutations encoding Q1367*, R1450*, E1309 frameshift, S1465 frameshift, and T1556 frameshift mutated APC proteins. In some embodiments, the probes specific for one or more DNA mutations in the *APC* gene comprise five probes comprising the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), and AGAGGCAGAAAAAAACT (SEQ ID NO:42), and wherein each of the five probes is coupled to a microcarrier with a different identifier. In some embodiments, each of the five probes further comprises eight nucleotides at the 5' end, wherein the eight nucleotides at the 5' end are adenine or thymine nucleotides, and wherein each of the five probes comprises at least 24 total nucleotides. In some embodiments, the probes comprise: (1) a first probe comprising a sequence selected from the group consisting of AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (2) a second probe comprising a sequence selected from the group consisting of TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (3) a third probe comprising a sequence selected from the group consisting of AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (4) a fourth probe comprising a sequence selected from the group consisting of GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (5) a fifth probe comprising a sequence selected from the group consisting of GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246); and wherein each of the five probes is coupled to a microcarrier with a different identifier. In some embodiments, the probes comprise: (1) a first probe comprising a sequence selected from the group consisting of TTTTTTTTTTTTTGAAATAAAAGATTGG (SEQ ID NO:58), TTTTTTTTTTAAATAGCAGAAATAAAAG (SEQ ID NO: 120), TTTTTTTTTTTGAAATAAAAGATTGGAA (SEQ ID NO:121), TTTTTTTTTTTTTAGAAATAAAAGATTG (SEQ ID NO: 122), TTTTTTTTTTTTTGAAATAAATGAATGG (SEQ ID NO:123), and TTTTTTTTTTTTTCAGAAATAAAAGATT (SEQ ID NO: 124); (2) a second probe comprising a sequence selected from the group consisting of TTTTTTTTTTTTTGGGTGTCTAAG (SEQ ID NO:59), TTTTTTTTTATTTGGGTGTCTAAG (SEQ ID NO:125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO:128),and TTTTTTTTTGGTGTCTAAGCACCA (SEQ ID NO:129); (3) a third probe comprising a sequence selected from the group consisting of TTTTTTTTTACAAACCAAGTGAGAA (SEQ ID NO:60), TTTTTTTTAAGTGAGAAGTACCTAA (SEQ ID NO: 130), TTTTTTTTTTTTCAAACCAAGTGAG (SEQ ID NO:131), TTTTTTTTTTACCAAGTGAGAAGTA (SEQ ID NO: 132), and TTTTTTTTAGCTCAAACCAAGTGAG (SEQ ID NO: 133); (4) a fourth probe comprising a sequence selected from the group consisting of TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO: 136), and TTTTTTTTTCCTACTGCTGAAAAGAGA (SEQ ID NO: 137); and (5) a fifth probe comprising a sequence selected from the group consisting of TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO: 139), TTTTTTTAGAAAGAGGCAGAAAAAAACT (SEQ ID NO: 140), and TTTTTTTTTTTGAGGCAGAAAAAAACTA (SEQ ID NO:141); and wherein each of the five probes is coupled to a microcarrier with a different identifier. In some embodiments, step (b) comprises amplifying the isolated DNA by PCR using a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40). In some embodiments, step (b) comprises amplifying the isolated DNA by PCR in the presence of a first blocking nucleic acid comprising the sequence of *CCA*C*TC*TCTCT*C*T*TT*T*CAGC*(invdT)*ₙ,* wherein *n* is 1, 2, or 3 (SEQ ID NO:25), TA*GG*T*CC*ACTCTCTCTCT*TT*T*CAGC*A(invdT)n, wherein n is 1, 2, or 3 (SEQ ID NO:166), *TAGGTCCACTCTCTCTCTTTTCAGCA* (invdT)n, wherein n is 1, 2, or 3 (SEQ ID NO:167), *CCACTCTCTCTCTTTTCAGC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO: 169), a second blocking nucleic acid comprising the sequence of C*TTTTCTTTTAT*TT*C*T*GC*(invdT)*ₙ,* wherein *n is* 1, 2, or 3 (SEQ ID NO:29), *CTTTTCTTTTATTTCTGC(invdT)n,* wherein n is 1, 2, or 3 (SEQ ID NO:154), *CTTTTCTTTTATTTCTGC(invdT)n,* wherein n is 1, 2, or 3 (SEQ ID NO:155), C*TTT*TCT*TTT*A*T*T*TC*T*GC*(invdT)n, wherein n is 1, 2, or 3 (SEQ ID NO:156), or *CTTTTCTTTTATTTCTGC(invdT)n,* wherein n is 1, 2, or 3 (SEQ ID NO:157), a third blocking nucleic acid comprising the sequence of GTG*CTCA*G*ACAC*C(invdT)*ₙ,* wherein *n is* 1, 2, or 3 (SEQ ID NO:33), G*TGC*TC*A*G*A*C*ACC*(invdT)n, wherein n is 1, 2, or 3 (SEQ ID NO: 158), AGTGGTG*CTCAGAC*ACCCA(invdT)n, wherein n is 1, 2, or 3 (SEQ ID NO:159), *AGTGGTGCTCAGACACCCA(invdT)n,* wherein n is 1, 2, or 3 (SEQ ID NO:160), or A*GTG*GTGC*TCA*G*ACAC*C*C*A(invdT)n, wherein n is 1, 2, or 3 (SEQ ID NO:161), a fourth blocking nucleic acid comprising the sequence of C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO:162), *CTTCTCGCTTGGTT(invdT)n,* wherein n is 1, 2, or 3 (SEQ ID NO:163), GT*ACT*T*CTCG*CT*TGG*T(invdT)n, wherein n is 1, 2, or 3 (SEQ ID NO: 164), or GT*ACTTCTCGC*TT*GG*T(invdT)n, wherein n is 1, 2, or 3 (SEQ ID NO: 165), and a fifth blocking nucleic acid comprising the sequence of *C*A*ATAG*T*TTTT*T*CTGC*C(invdT)*ₙ,* wherein *n is* 1, 2, or 3 (SEQ ID NO:41), *GAATCAATAGTTTTTTCTGCCTC* (invdT)n, wherein n is 1, 2, or 3 (SEQ ID NO: 170), *TCAGAATCAATAGTTTTTTCTG* (invdT)n, wherein n is 1, 2, or 3 (SEQ ID NO: 171), *GAATCAATAGATTTTACTGCCTC* (invdT)n, wherein n is 1, 2, or 3 (SEQ ID NO: 172), or A*A*T*CAATAG*TTTTTTC*TGCCT*C (invdT)n, wherein n is 1, 2, or 3 (SEQ ID NO: 173), with italicized nucleic acids representing locked nucleic acids.

In some embodiments of any of the above embodiments, *n* is 1, 2, 3, 4, 5, 6, 7, 8, 9, or 10. In some embodiments, the method further comprises: amplifying a positive control DNA sequence using a primer pair specific for the positive control DNA sequence; hybridizing the amplified positive control gene sequence with a probe specific for the positive control gene sequence, wherein the probe specific for the positive control gene sequence is coupled to a microcarrier with an identifier corresponding to a positive control; detecting presence or absence of hybridization of the amplified positive control DNA sequence with the probe specific for the positive control gene sequence; and detecting the identifier corresponding to the positive control. In some embodiments, the positive control DNA sequence comprises a sequence of a human leukocyte antigen gene. In some embodiments, the primer pair specific for the positive control DNA sequence comprises the sequences TGAGTGTTACTTCTTCCCACACTC (SEQ ID NO:43) and ATTGCTTTTGCGCAATCCCT (SEQ ID NO:44). In some embodiments, the probe specific for the positive control gene sequence comprises the sequence TTTTTTTTTTTTGGAGACGGTCTG (SEQ ID NO:45). In some embodiments, the primer pair specific for the positive control DNA sequence comprises the sequences AATCCCATCACCATCTTCCA (SEQ ID NO:71) and TGGACTCCACGACGTACTCA (SEQ ID NO:72). In some embodiments, the probe specific for the positive control gene sequence comprises the sequence CTGTCTTCCACTCACTCC (SEQ ID NO:73).

In some embodiments, the method further comprises: detecting absence of hybridization of the amplified DNA with a microcarrier having an identifier corresponding to a negative control, wherein the microcarrier with the identifier corresponding to the negative control comprises a probe that does not hybridize with the amplified DNA; and detecting the identifier corresponding to the negative control. In some embodiments, the microcarrier with the identifier corresponding to the negative control comprises a probe comprising the sequence AATATAATATATTAT (SEQ ID NO:46). In some embodiments, the identifiers of the micocarriers comprise digital barcodes. In some embodiments, each of the microcarriers comprises: (i) a first photopolymer layer; (ii) a second photopolymer layer; and (iii) an intermediate layer between the first layer and the second layer, the intermediate layer having an encoded pattern representing the identifier defined thereon, wherein the intermediate layer is partially substantially transmissive and partially substantially opaque to light, representing a code corresponding to the microcarrier, wherein the outermost surface of the microcarrier comprises a photoresist photopolymer, and said photoresist photopolymer is functionalized with the probe specific for the DNA mutation, and wherein said microcarrier has about the same density as water. In some embodiments, the identifiers of the micocarriers comprise analog codes. In some embodiments, each of the microcarriers comprises: (i) a substantially transparent polymer layer having a first surface and a second surface, the first and the second surfaces being parallel to each other; (ii) a substantially non-transparent polymer layer, wherein the substantially non-transparent polymer layer is affixed to the first surface of the substantially transparent polymer layer and encloses a center portion of the substantially transparent polymer layer, and wherein the substantially non-transparent polymer layer comprises a two-dimensional shape representing an analog code, wherein the analog code represents the identifier; and (iii) the probe specific for the DNA mutation, wherein the probe is coupled to at least one of the first surface and the second surface of the substantially transparent polymer layer in at least the center portion of the substantially transparent polymer layer. In some embodiments, each of the microcarriers further comprises: (iv) a second substantially transparent polymer layer aligned with the first substantially transparent polymer layer, the second substantially transparent polymer layer having a center portion that is aligned with the center portion of the first substantially transparent polymer layer, wherein the second substantially transparent polymer layer is affixed to the second surface of the first substantially transparent polymer layer and does not extend beyond the two-dimensional shape of the first substantially transparent polymer layer; and (v) a magnetic, substantially non-transparent layer that encloses the center portion of the first substantially transparent polymer layer between the substantially non-transparent polymer layer and the center portion of the substantially transparent polymer layer, wherein the magnetic, substantially non-transparent layer is affixed between the first and the second substantially transparent polymer layers. In some embodiments, each of the microcarriers further comprises an orientation indicator for orienting the analog code of the substantially non-transparent polymer layer. In some embodiments, the two-dimensional shape of the substantially non-transparent polymer layer comprises a gear shape comprising a plurality of gear teeth, and wherein the analog code is represented by one or more aspects selected from the group consisting of the height of one or more gear teeth of the plurality, the width of one or more gear teeth of the plurality, the number of gear teeth in the plurality, and the arrangement of one or more gear teeth within the plurality. In some embodiments, each of the microcarriers further comprises: (vi) one or more columns projecting from the first surface of the first substantially transparent polymer layer, wherein the one or more columns are not within the center portion of the first substantially transparent polymer layer; and/or (vii) one or more columns projecting from the second surface of the first substantially transparent polymer layer or a surface of the second substantially transparent polymer layer that is not affixed to the first substantially transparent polymer layer, wherein the one or more columns are not within the center portions of the first or the second substantially transparent polymer layer. In some embodiments, the substantially transparent polymer of the first or the second substantially transparent polymer layer comprises an epoxy-based polymer. In some embodiments, the epoxy-based polymer is SU-8. In some embodiments, the sample is a stool sample. In some embodiments, multiple stool samples from a patient are obtained. In some embodiments, multiple stool samples from a single stool specimen are obtained. In some embodiments, the methods of the present disclosure comprise detecting the presence or absence of hybridization of amplified DNA with a total of between about 1 and about 1000 microcarriers per probe per well of an assay plate. In some embodiments, the methods of the present disclosure are used to detect colon cancer, rectal cancer, colorectal cancer, colon adenoma, rectal adenoma, or colorectal adenoma, *e.g*., in a patient from whom the sample is collected.

In some embodiments, each of the at least four blocking nucleic acids in the kit of the invention comprises: a single-stranded oligonucleotide that hybridizes with the corresponding wild-type DNA locus; and a 3' terminal moiety that blocks extension from the single-stranded oligonucleotide. In some embodiments, the 3' terminal moiety comprises one or more inverted deoxythymidines. In some embodiments, each of said at least four blocking nucleic acids comprises one or more modified nucleotides selected from the group consisting of locked nucleic acids (LNAs), peptide nucleic acids (PNAs), hexose nucleic acids (HNAs), threose nucleic acids (TNAs), glycol nucleic acids (GNAs), and cyclohexenyl nucleic acids (CeNAs). In some embodiments, the DNA mutation in the *KRAS* gene comprises one or more DNA mutations encoding a G12D, G12V, G12S, or G13D mutated KRAS protein. In some embodiments, at least one of the at least four blocking nucleic acids comprises the sequence TA*C*G*CC*A*CC*A*GC*T(invdT)*ₙ*, wherein n is 1, 2, or 3 (SEQ ID NO:3), TT*GG*A*G*CT*GGTGGC*GTA(invdT)*ₙ*, wherein *n* is 1, 2, or 3 (SEQ ID NO: 142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n* is 1, 2, or 3 (SEQ ID NO: 144), or TT*GG*A*G*CT*GG*T*GG*C*G*T(invdT)*ₙ*, wherein *n* is 1, 2, or 3 (SEQ ID NO: 145), with italicized nucleic acids representing locked nucleic acids. In some embodiments, the DNA mutation in the *BRAF* gene comprises one or more DNA mutations encoding a V600E mutated BRAF protein. In some embodiments, at least one of the at least four blocking nucleic acids comprises the sequence G*AGAT*T*TCAC*T*GTAGC*(invdT)*ₙ,* wherein *n* is 1, 2, or 3 (SEQ ID NO:10), G*AGAT*T*TCAC*T*GTAGC*(invdT)*ₙ,* wherein *n* is 1, 2, or 3 (SEQ ID NO:146), G*AGAT*T*TCAC*T*GTAGC*(invdT)*ₙ,* wherein *n* is 1, 2, or 3 (SEQ ID NO: 147), G*AGAT*T*TCAC*T*GTAGC*(invdT)*ₙ,* wherein *n* is 1, 2, or 3 (SEQ ID NO:148), or G*AGAT*T*TCAC*T*GTAGC*(invdT)*ₙ,* wherein *n* is 1, 2, or 3 (SEQ ID NO:149), with italicized nucleic acids representing locked nucleic acids. In some embodiments, the DNA mutation in the *CTNNB1* gene comprises at least a first *CTNNB1* mutation encoding a T41A or T41I mutated CTNNB1 protein. In some embodiments, at least one of the at least four blocking nucleic acids comprises the sequence GC*CACTACCACAG*CT(invdT)*ₙ,* wherein *n* is 1, 2, or 3 (SEQ ID NO:15), T*G*C*CA*CT*ACCA*C*AG*(invdT)*ₙ,* wherein *n is* 1, 2, or 3 (SEQ ID NO:150), C*AC*T*ACCA*C*AGC*T*C*C(invdT)*ₙ,* wherein *n is* 1, 2, or 3 (SEQ ID NO:151), G*CCACTACCACAG*CT(invdT)*ₙ,* wherein *n is* 1, 2, or 3 (SEQ ID NO:152), or GC*CACTACCACAG*CT(invdT)*ₙ,* wherein *n is* 1, 2, or 3 (SEQ ID NO: 153), with italicized nucleic acids representing locked nucleic acids. In some embodiments, the DNA mutation in the *CTNNB1* gene comprises at least a first *CTNNB1* mutation encoding an S45F or S45P mutated CTNNB1 protein. In some embodiments, at least one of the at least four blocking nucleic acids comprises the sequence GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein *n* is 1, 2, or 3 (SEQ ID NO:174), G*C*T*CC*T*TC*TC*TGA*GT(invdT)n, wherein n is 1, 2, or 3 (SEQ ID NO:175), T*CC*TT*CT*CT*GAG*T*G*G(invdT)n, wherein n is 1, 2, or 3 (SEQ ID NO:176), or G*C*T*CC*TT*CT*C*TGAG*T(invdT)n, wherein n is 1, 2, or 3 (SEQ ID NO:177), with italicized nucleic acids representing locked nucleic acids. In some embodiments, the DNA mutation in the *APC* gene comprise at least a first *APC* mutation encoding a Q1367* mutated APC protein. In some embodiments, at least one of the at least four blocking nucleic acids comprises the sequence GTG*CTCA*G*ACAC*C(invdT)*ₙ,* wherein *n is* 1, 2, or 3 (SEQ ID NO:33), G*TGC*TC*A*G*A*C*ACC*(invdT)n, wherein n is 1, 2, or 3 (SEQ ID NO:158), AGTGGTG*CTCAGAC*ACCCA(invdT)n, wherein n is 1, 2, or 3 (SEQ ID NO:159), *AGTGGTGCTCAGACACCCA(invdT)n,* wherein n is 1, 2, or 3 (SEQ ID NO:160), or A*G*T*G*GTGC*TCA*G*AC*A*C*C*C*A(invdT)n, wherein n is 1, 2, or 3 (SEQ ID NO:161), with italicized nucleic acids representing locked nucleic acids. In some embodiments, the DNA mutation in the *APC* gene comprises at least a first *APC* mutation encoding an R1450* mutated APC protein. In some embodiments, at least one of the at least four blocking nucleic acids comprises the sequence C*TTC*T*CGCT*T*GGT*T(invdT)*ₙ,* wherein *n* is 1, 2, or 3 (SEQ ID NO:37), G*TAC*T*T*C*TCG*CT*TGG*T(invdT)n, wherein n is 1, 2, or 3 (SEQ ID NO:162), *CTTCTCGCTTGGTT(invdT)n,* wherein n is 1, 2, or 3 (SEQ ID NO:163), GT*ACT*T*CTCG*CT*TGG*T(invdT)n, wherein n is 1, 2, or 3 (SEQ ID NO: 164), or GT*A*C*TTCTCGC*TT*GG*T(invdT)n, wherein n is 1, 2, or 3 (SEQ ID NO: 165), with italicized nucleic acids representing locked nucleic acids. In some embodiments, the DNA mutation in the *APC* gene comprises at least a first *APC* mutation encoding an E1309 frameshift mutated APC protein. In some embodiments, at least one of the at least four blocking nucleic acids comprises the sequence C*TTTTCTTTTAT*TT*C*T*GC*(invdT)*ₙ,* wherein *n is* 1, 2, or 3 (SEQ ID NO:29), *CTTTTCTTTTATTTCTGC(invdT)n,* wherein n is 1, 2, or 3 (SEQ ID NO: 154), *CTTTTCTTTTATTTCTGC(invdT)n,* wherein n is 1, 2, or 3 (SEQ ID NO:155), C*TTT*TCT*TTT*A*T*T*TC*T*GC*(invdT)n, wherein n is 1, 2, or 3 (SEQ ID NO: 156), or *CTTTTCTTTTATTTCTGC(invdT)n,* wherein n is 1, 2, or 3 (SEQ ID NO:157), with italicized nucleic acids representing locked nucleic acids. In some embodiments, the DNA mutation in the *APC* gene comprises at least a first *APC* mutation encoding an S1465 frameshift mutated APC protein. In some embodiments, at least one of the at least four blocking nucleic acids comprises the sequence *CCA*C*TC*TCTCT*C*T*TT*T*CAGC*(invdT)*ₙ,* wherein *n is* 1, 2, or 3 (SEQ ID NO:25), TA*GG*T*CC*ACTCTCTCTCT*TT*T*C*A*GC*A(invdT)n, wherein n is 1, 2, or 3 (SEQ ID NO:166), *TAGGTCCACTCTCTCTCTTTTCAGCA* (invdT)n, wherein n is 1, 2, or 3 (SEQ ID NO:167), C*CA*C*T*C*T*C*T*C*TC*TTTT*C*A*GC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO: 169), with italicized nucleic acids representing locked nucleic acids. In some embodiments, the DNA mutation in the *APC* gene comprises at least a first *APC* mutation encoding a T1556 frameshift mutated APC protein. In some embodiments, at least one of the at least four blocking nucleic acids comprises the sequence *C*A*ATAG*T*TTTT*T*CTGC*C(invdT)*ₙ,* wherein *n* is 1, 2, or 3 (SEQ ID NO:41), *G*A*A*T*CAATAG*TTTTTT*CTGCCT*C (invdT)n, wherein n is 1, 2, or 3 (SEQ ID NO:170), *TCAGAATCAATAGTTTTTTCTG* (invdT)n, wherein n is 1, 2, or 3 (SEQ ID NO:171), *G*A*A*T*CAATAG*ATTTTA*CTGCCT*C (invdT)n, wherein n is 1, 2, or 3 (SEQ ID NO: 172), or *AATCAATAGTTTTTTCTGCCTC* (invdT)n, wherein n is 1, 2, or 3 (SEQ ID NO: 173), with italicized nucleic acids representing locked nucleic acids. In some embodiments, the kit further comprises at least four primer pairs, wherein the kit comprises a primer pair specific for the locus of one or more DNA mutations in each of the *KRAS, BRAF, CTNNB1,* and *APC* genes. In some embodiments, each of said at least four primer pairs comprises a primer coupled to a detection reagent. In some embodiments, the detection reagent comprises a fluorescent detection reagent. In some embodiments, the detection reagent comprises biotin, and wherein the kit further comprises streptavidin conjugated to a signal-emitting entity. In some embodiments, the signal-emitting entity comprises phycoerythrin (PE).

In some embodiments, the DNA mutation in the *KRAS* gene comprises *KRAS* mutations encoding G12D, G12V, G12S, and G13D mutated KRAS proteins. In some embodiments, the kit comprises four probes comprising the sequences GGAGCTGATGG (SEQ ID NO:4), GGAGCTGTTGG (SEQ ID NO:5), TGGAGCTAGTGG (SEQ ID NO:6), and TGGAGCTGGTGACGT (SEQ ID NO:7), and wherein each of the four probes is coupled to a microcarrier with a different identifier. In some embodiments, the kit comprises: (1) a first probe comprising a sequence selected from the group consisting of GGAGCTGATGG (SEQ ID NO:4), AGCTGATGGCGTA (SEQ ID NO: 178), TGGAGCTGATGGCG (SEQ ID NO:179), TGGAGCTGATGG (SEQ ID NO:180), and GCTGATGGCGTA (SEQ ID NO:181); (2) a second probe comprising a sequence selected from the group consisting of GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO:184), and TGGAGCTGTaGGTGG (SEQ ID NO: 185); (3) a third probe comprising a sequence selected from the group consisting of TTGGAGCTAGTGGCGTA (SEQ ID NO:186), GCTAGTGGCGTAGGC (SEQ ID NO: 187), AGCTAGTGGCGT (SEQ ID NO: 188), GTTGGAGCTAGTGG (SEQ ID NO: 189), and GGAGCTAGTGG (SEQ ID NO: 190); and (4) a fourth probe comprising a sequence selected from the group consisting of GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), and GGAGCTGGTGACGT (SEQ ID NO:195), and wherein each of the four probes is coupled to a microcarrier with a different identifier. In some embodiments, each of the four probes further comprises eight nucleotides at the 5' end, wherein the eight nucleotides at the 5' end are adenine or thymine nucleotides, and wherein each of the four probes comprises at least 24 total nucleotides. In some embodiments, the kit comprises: (1) a first probe comprising a sequence selected from the group consisting of TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), and TTTTTTTTTTTTTGCTGATGGCGTA (SEQ ID NO:77); (2) a second probe comprising a sequence selected from the group consisting of TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), and TTTTTTTTTATGGAGCTGTAGGTGG (SEQ ID NO:81); (3) a third probe comprising a sequence selected from the group consisting of TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), and TTTTTTTTTTTTAAGGAGCTAGTGG (SEQ ID NO:86); and (4) a fourth probe comprising a sequence selected from the group consisting of TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), and TTTTTTTTTAAGGAGCTGGTGACGT (SEQ ID NO:91), and wherein each of the four probes is coupled to a microcarrier with a different identifier. In some embodiments, the kit further comprises a primer pair comprising the sequences GTACTGGTGGAGTATTTGATAGTG (SEQ ID NO:1) and ATCGTCAAGGCACTCTTGCCTAC (SEQ ID NO:2). In some embodiments, the kit further comprises a blocking nucleic acid comprising the sequence of TA*CGCC*A*CC*A*GC*T(invdT)*ₙ*, wherein *n* is 1, 2, or 3 (SEQ ID NO:3), TT*GG*A*G*CT*GGTGGC*GTA(invdT)*ₙ*, wherein *n is* 1, 2, or 3 (SEQ ID NO:142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n* is 1, 2, or 3 (SEQ ID NO:144), or TT*GG*A*G*CT*GG*T*GG*C*G*T(invdT)*ₙ*, wherein *n* is 1, 2, or 3 (SEQ ID NO:145), with italicized nucleic acids representing locked nucleic acids.

In some embodiments, the DNA mutations in the *BRAF* gene comprises two or more *BRAF* mutations encoding a V600E mutated BRAF protein. In some embodiments, the kit comprises two probes comprising the sequences TCTAGCTACAGAGAAAT (SEQ ID NO:11) and GTCTAGCTACAGAAAAAT (SEQ ID NO:12), and wherein each of the two probes is coupled to a microcarrier with a different identifier. In some embodiments, the kit comprises: (1) a first probe comprising a sequence selected from the group consisting of TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), and TCTAGCTACAGAG (SEQ ID NO:200); and (2) a second probe comprising a sequence selected from the group consisting of GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204); and wherein each of the two probes is coupled to a microcarrier with a different identifier. In some embodiments, each of the two probes further comprises eight nucleotides at the 5' end, wherein the eight nucleotides at the 5' end are adenine or thymine nucleotides, and wherein each of the two probes comprises at least 24 total nucleotides. In some embodiments, the kit comprises: (1) a first probe comprising a sequence selected from the group consisting of TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), and TTTTTTTTTTAATTTCTAGCTACAGAG (SEQ ID NO:96); and (2) a second probe comprising a sequence selected from the group consisting of TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and TTTTTTTTATTCTAGCTACAGAAAAATC (SEQ ID NO:100); and wherein each of the two probes is coupled to a microcarrier with a different identifier. In some embodiments, the kit further comprises a primer pair comprising the sequences GGACCCACTCCATCGAGATTT (SEQ ID NO:8) and CAGATATATTTCTTCATGAAGACCTCACAGTAA (SEQ ID NO:9). In some embodiments, the kit further comprises a blocking nucleic acid comprising the sequence of G*AGAT*T*TCAC*T*GTAGC*(invdT)*ₙ,* wherein *n* is 1, 2, or 3 (SEQ ID NO: 10), G*AGAT*T*TCAC*T*GTAGC*(invdT)*ₙ,* wherein *n* is 1, 2, or 3 (SEQ ID NO: 146), G*AGAT*T*TCAC*T*GTAGC*(invdT)*ₙ,* wherein *n* is 1, 2, or 3 (SEQ ID NO: 147), G*AGAT*T*TCAC*T*GTAGC*(invdT)*ₙ,* wherein *n* is 1, 2, or 3 (SEQ ID NO: 148), or G*AGAT*T*TCAC*T*GTAGC*(invdT)*ₙ,* wherein *n* is 1, 2, or 3 (SEQ ID NO: 149), with italicized nucleic acids representing locked nucleic acids.

In some embodiments, the DNA mutation in the *CTNNB1* gene comprises *CTNNB1* mutations encoding T41A, T41I, S45F, and S45P mutated CTNNB1 proteins. In some embodiments, the kit comprises four probes comprising the sequences AGGAGCTGTGGCAG (SEQ ID NO: 16), GGAGCTGTGATA (SEQ ID NO: 17), TTTACCACTCAGAAAAG (SEQ ID NO:21), and TACCACTCAGAGGAG (SEQ ID NO:22), and wherein each of the four probes is coupled to a microcarrier with a different identifier. In some embodiments, each of the four probes further comprises eight nucleotides at the 5' end, wherein the eight nucleotides at the 5' end are adenine or thymine nucleotides, and wherein each of the four probes comprises at least 24 total nucleotides. In some embodiments, the kit comprises: (1) a first probe comprising a sequence selected from the group consisting of AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), and AAGGAGCTGTGGCAG (SEQ ID NO:209); (2) a second probe comprising a sequence selected from the group consisting of GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), and GGAGCTGTGAT (SEQ ID NO:214); (3) a third probe comprising a sequence selected from the group consisting of ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), and CAGAAAAGGAGCTGTG (SEQ ID NO:219); and (4) a fourth probe comprising a sequence selected from the group consisting of ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), and TTACCAATCAGAGGAGG (SEQ ID NO:224); and wherein each of the four probes is coupled to a microcarrier with a different identifier. In some embodiments, each of the four probes further comprises eight nucleotides at the 5' end, wherein the eight nucleotides at the 5' end are adenine or thymine nucleotides, and wherein each of the four probes comprises at least 24 total nucleotides. In some embodiments, the kit comprises: (1) a first probe comprising a sequence selected from the group consisting of TTTTTTTTTTTAGGAGCTGTGGCAG (SEQ ID NO:53), TTTTTTTTTTTAGGAGCTGTGGCAGTG (SEQ ID NO:101), TTTTTTTTTTTAGCTGTGGCAGTGGC (SEQ ID NO:102), TTTTTTTTTTTGCTGTGGCAGTGGCA (SEQ ID NO:103), and TTTTTTTTTTAAGGAGCTGTGGCAG (SEQ ID NO:104); (2) a second probe comprising a sequence selected from the group consisting of TTTTTTTTTTTTTGGAGCTGTGATA (SEQ ID NO:54), TTTTTTTTTGGAGCTGTGATAGTGG (SEQ ID NO:105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), and TTTTTTTTTTTTTTGGAGCTGTGAT (SEQ ID NO:109); (3) a third probe comprising a sequence selected from the group consisting of TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), and TTTTTTTTTCAGAAAAGGAGCTGTG (SEQ ID NO:114); and (4) a fourth probe comprising a sequence selected from the group consisting of TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and TTTTTTTTTATTACCAATCAGAGGAGG (SEQ ID NO:119), and wherein each of the four probes is coupled to a microcarrier with a different identifier. In some embodiments, the kit further comprises a first primer pair comprising the sequences GGAATCCATTCTGGTGCCACT (SEQ ID NO: 13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO:19). In some embodiments, the kit further comprises a first blocking nucleic acid comprising the sequence of GC*CACTACCACAG*CT(invdT)*ₙ,* wherein *n* is 1, 2, or 3 (SEQ ID NO:15), T*G*C*CA*CT*ACCA*C*AG*(invdT)*ₙ,* wherein *n* is 1, 2, or 3 (SEQ ID NO:150), C*AC*T*ACCA*C*AGC*T*C*C(invdT)*ₙ,* wherein *n* is 1, 2, or 3 (SEQ ID NO:151), GC*CACTACCACAG*CT(invdT)*ₙ,* wherein *n* is 1, 2, or 3 (SEQ ID NO:152), or GC*CACTACCACAG*CT(invdT)*ₙ,* wherein *n* is 1, 2, or 3 (SEQ ID NO: 153), and a second blocking nucleic acid comprising the sequence of GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein *n* is 1, 2, or 3 (SEQ ID NO:174), G*C*T*CC*T*TC*TC*TGA*GT(invdT)n, wherein n is 1, 2, or 3 (SEQ ID NO:175), T*CC*TT*CT*CT*GAG*T*G*G(invdT)n, wherein n is 1, 2, or 3 (SEQ ID NO:176), or G*C*T*CC*TT*CT*C*TGAG*T(invdT)n, wherein n is 1, 2, or 3 (SEQ ID NO:177), with italicized nucleic acids representing locked nucleic acids.

In some embodiments, the DNA mutation in the *APC* gene comprises *APC* mutations encoding Q1367*, R1450*, E1309 frameshift, S1465 frameshift, and T1556 frameshift mutated APC proteins. In some embodiments, the kit comprises five probes comprising the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), and AGAGGCAGAAAAAAACT (SEQ ID NO:42), and wherein each of the five probes is coupled to a microcarrier with a different identifier. In some embodiments, the kit comprises: (1) a first probe comprising a sequence selected from AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (2) a second probe comprising a sequence selected from TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (3) a third probe comprising a sequence selected from the group consisting of AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (4) a fourth probe comprising a sequence selected from GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (5) a fifth probe comprising a sequence selected from GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246); and wherein each of the five probes is coupled to a microcarrier with a different identifier. In some embodiments, each of the five probes further comprises eight nucleotides at the 5' end, wherein the eight nucleotides at the 5' end are adenine or thymine nucleotides, and wherein each of the five probes comprises at least 24 total nucleotides. In some embodiments, the kit comprises: (1) a first probe comprising a sequence selected from the group consisting of TTTTTTTTTTTTTGAAATAAAAGATTGG (SEQ ID NO:58), TTTTTTTTTTAAATAGCAGAAATAAAAG (SEQ ID NO: 120), TTTTTTTTTTTGAAATAAAAGATTGGAA (SEQ ID NO:121), TTTTTTTTTTTTTAGAAATAAAAGATTG (SEQ ID NO: 122), TTTTTTTTTTTTTGAAATAAATGAATGG (SEQ ID NO:123), and TTTTTTTTTTTTTCAGAAATAAAAGATT (SEQ ID NO: 124); (2) a second probe comprising a sequence selected from the group consisting of TTTTTTTTTTTTTGGGTGTCTAAG (SEQ ID NO:59), TTTTTTTTTATTTGGGTGTCTAAG (SEQ ID NO: 125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128),and TTTTTTTTTGGTGTCTAAGCACCA (SEQ ID NO:129); (3) a third probe comprising a sequence selected from the group consisting of TTTTTTTTTACAAACCAAGTGAGAA (SEQ ID NO:60), TTTTTTTTAAGTGAGAAGTACCTAA (SEQ ID NO: 130), TTTTTTTTTTTTCAAACCAAGTGAG (SEQ ID NO:131), TTTTTTTTTTACCAAGTGAGAAGTA (SEQ ID NO: 132), and TTTTTTTTAGCTCAAACCAAGTGAG (SEQ ID NO:133); (4) a fourth probe comprising a sequence selected from the group consisting of TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO: 136), and TTTTTTTTTCCTACTGCTGAAAAGAGA (SEQ ID NO: 137); and (5) a fifth probe comprising a sequence selected from the group consisting of TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO: 139), TTTTTTTAGAAAGAGGCAGAAAAAAACT (SEQ ID NO: 140), and TTTTTTTTTTTGAGGCAGAAAAAAACTA (SEQ ID NO:141); and wherein each of the five probes is coupled to a microcarrier with a different identifier. In some embodiments, the kit further comprises a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40). In some embodiments, the kit further comprises a first blocking nucleic acid comprising the sequence of *CCA*C*TC*TCTCT*C*T*TT*T*CAGC*(invdT)*ₙ,* wherein *n* is 1, 2, or 3 (SEQ ID NO:25), TA*GG*T*CC*ACTCTCTCTCT*TT*T*C*A*GC*A(invdT)n, wherein n is 1, 2, or 3 (SEQ ID NO: 166), *TAGGTCCACTCTCTCTCTTTTCAGCA* (invdT)n, wherein n is 1, 2, or 3 (SEQ ID NO: 167), *CCACTCTCTCTCTTTTCAGC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO:169), a second blocking nucleic acid comprising the sequence of C*TTTTCTTTTAT*TT*C*T*GC*(invdT)*ₙ,* wherein *n is* 1, 2, or 3 (SEQ ID NO:29), C*TTTTCTTTTAT*TT*C*T*GC*(invdT)*ₙ,* wherein n is 1, 2, or 3 (SEQ ID NO:154), C*TTTTCTTTTAT*TT*C*T*GC*(invdT)*ₙ,* wherein n is 1, 2, or 3 (SEQ ID NO:155), C*TTTTCTTTTAT*TT*C*T*GC*(invdT)*ₙ,* wherein n is 1, 2, or 3 (SEQ ID NO:156), or *CTTTTCTTTTATTTCTGC(invdT)n,* wherein n is 1, 2, or 3 (SEQ ID NO:157), a third blocking nucleic acid comprising the sequence of GTG*CTCA*G*ACAC*C(invdT)*ₙ,* wherein *n* is 1, 2, or 3 (SEQ ID NO:33), G*TGC*TC*A*G*A*C*ACC*(invdT)n, wherein n is 1, 2, or 3 (SEQ ID NO:158), AGTGGTG*CTCAGAC*ACCCA(invdT)n, wherein n is 1, 2, or 3 (SEQ ID NO:159), A*GTG*GT*G*C*TC*AG*A*C*ACCC*A(invdT)n, wherein n is 1, 2, or 3 (SEQ ID NO:160), or AGTGGTGCTCAGACACCCA(invdT)n, wherein n is 1, 2, or 3 (SEQ ID NO:161), a fourth blocking nucleic acid comprising the sequence of C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO:162), *CTTCTCGCTTGGTT(invdT)n,* wherein n is 1, 2, or 3 (SEQ ID NO:163), *GTACTTCTCGCTTGGT(invdT)n,* wherein n is 1, 2, or 3 (SEQ ID NO:164), or GTACTTCTCGCTTGGT(invdT)n, wherein n is 1, 2, or 3 (SEQ ID NO:165), and a fifth blocking nucleic acid comprising the sequence of *C*A*ATAG*T*TTTT*T*CTGC*C(invdT)*ₙ,* wherein *n* is 1, 2, or 3 (SEQ ID NO:41), *G*A*A*T*CAATAG*TTTTTT*CTGCCT*C (invdT)n, wherein n is 1, 2, or 3 (SEQ ID NO:170), *TCAGAATCAATAGTTTTTTCTG* (invdT)n, wherein n is 1, 2, or 3 (SEQ ID NO:171), *GAATCAATAGATTTTACTGCCTC* (invdT)n, wherein n is 1, 2, or 3 (SEQ ID NO:172), or A*A*T*CAATAG*TTTTTTC*TGCCT*C (invdT)n, wherein n is 1, 2, or 3 (SEQ ID NO:173), with italicized nucleic acids representing locked nucleic acids.

In some embodiments of any of the above embodiments, *n* is 1, 2, 3, 4, 5, 6, 7, 8, 9, or 10. In some embodiments, the kit further comprises a microcarrier with an identifier corresponding to a positive control and to which a probe specific for a positive control gene sequence is coupled; and a primer pair specific for the positive control DNA sequence. In some embodiments, the positive control DNA sequence comprises a sequence of a human leukocyte antigen gene. In some embodiments, the primer pair specific for the positive control DNA sequence comprises the sequences TGAGTGTTACTTCTTCCCACACTC (SEQ ID NO:43) and ATTGCTTTTGCGCAATCCCT (SEQ ID NO:44). In some embodiments, the probe specific for the positive control gene sequence comprises the sequence TTTTTTTTTTTTGGAGACGGTCTG (SEQ ID NO:45). In some embodiments, the primer pair specific for the positive control DNA sequence comprises the sequences AATCCCATCACCATCTTCCA (SEQ ID NO:71) and TGGACTCCACGACGTACTCA (SEQ ID NO:72). In some embodiments, the probe specific for the positive control gene sequence comprises the sequence CTGTCTTCCACTCACTCC (SEQ ID NO:73). In some embodiments, the kit further comprises a microcarrier with an identifier corresponding to a negative control and to which is coupled a probe that does not hybridize with the amplified DNA. In some embodiments, the microcarrier with the identifier corresponding to the negative control comprises a probe comprising the sequence AATATAATATATTAT (SEQ ID NO:46).

In some embodiments, the identifiers of the micocarriers comprise digital barcodes. In some embodiments, each of the microcarriers comprises: (i) a first photopolymer layer; (ii) a second photopolymer layer; and (iii) an intermediate layer between the first layer and the second layer, the intermediate layer having an encoded pattern representing the identifier defined thereon, wherein the intermediate layer is partially substantially transmissive and partially substantially opaque to light, representing a code corresponding to the microcarrier, wherein the outermost surface of the microcarrier comprises a photoresist photopolymer, and said photoresist photopolymer is functionalized with the probe specific for the DNA mutation, and wherein said microcarrier has about the same density as water. In some embodiments, the identifiers of the micocarriers comprise analog codes. In some embodiments, each of the microcarriers comprises: (i) a substantially transparent polymer layer having a first surface and a second surface, the first and the second surfaces being parallel to each other; (ii) a substantially non-transparent polymer layer, wherein the substantially non-transparent polymer layer is affixed to the first surface of the substantially transparent polymer layer and encloses a center portion of the substantially transparent polymer layer, and wherein the substantially non-transparent polymer layer comprises a two-dimensional shape representing an analog code, wherein the analog code represents the identifier; and (iii) the probe specific for the DNA mutation, wherein the probe is coupled to at least one of the first surface and the second surface of the substantially transparent polymer layer in at least the center portion of the substantially transparent polymer layer. In some embodiments, each of the microcarriers further comprises: (iv) a second substantially transparent polymer layer aligned with the first substantially transparent polymer layer, the second substantially transparent polymer layer having a center portion that is aligned with the center portion of the first substantially transparent polymer layer, wherein the second substantially transparent polymer layer is affixed to the second surface of the first substantially transparent polymer layer and does not extend beyond the two-dimensional shape of the first substantially transparent polymer layer; and (v) a magnetic, substantially non-transparent layer that encloses the center portion of the first substantially transparent polymer layer between the substantially non-transparent polymer layer and the center portion of the substantially transparent polymer layer, wherein the magnetic, substantially non-transparent layer is affixed between the first and the second substantially transparent polymer layers. In some embodiments, each of the microcarriers further comprises an orientation indicator for orienting the analog code of the substantially non-transparent polymer layer. In some embodiments, the two-dimensional shape of the substantially non-transparent polymer layer comprises a gear shape comprising a plurality of gear teeth, and wherein the analog code is represented by one or more aspects selected from the group consisting of the height of one or more gear teeth of the plurality, the width of one or more gear teeth of the plurality, the number of gear teeth in the plurality, and the arrangement of one or more gear teeth within the plurality. In some embodiments, each of the microcarriers further comprises: (vi) one or more columns projecting from the first surface of the first substantially transparent polymer layer, wherein the one or more columns are not within the center portion of the first substantially transparent polymer layer; and/or(vii) one or more columns projecting from the second surface of the first substantially transparent polymer layer or a surface of the second substantially transparent polymer layer that is not affixed to the first substantially transparent polymer layer, wherein the one or more columns are not within the center portions of the first or the second substantially transparent polymer layer. In some embodiments, the substantially transparent polymer of the first or the second substantially transparent polymer layer comprises an epoxy-based polymer. In some embodiments, the epoxy-based polymer is SU-8. In some embodiments, the kit further comprises instructions for using the kit to detect colon cancer, rectal cancer, colorectal cancer, colon adenoma, rectal adenoma, or colorectal adenoma.

In another aspect, provided herein are kits comprising (a) a plurality of probes, the plurality of probes comprising a first probe comprising the sequence TTTTTTTTTTATGGAGCTGATGGCG (SEQ ID NO:75), a second probe comprising the sequence TTTTTTTTTTAAGGAGCTGTTGGTG (SEQ ID NO:79), a third probe comprising the sequence TTTTTTTTTTGGAGCTAGTGGCGTA (SEQ ID NO:82), a fourth probe comprising the sequence TTTTTTTTAAAGGTGACGTAGGCAA (SEQ ID NO:87), a fifth probe comprising the sequence TTTTTTTTTATACAGAGAAATCTCGAT (SEQ ID NO:92), a sixth probe comprising the sequence TTTTTTTTATTCTAGCTACAGAAAAATC (SEQ ID NO:100), a seventh probe comprising the sequence TTTTTTTTTTTAGGAGCTGTGGCAG (SEQ ID NO:53), an eighth probe comprising the sequence TTTTTTTTTGAGCTGTGATAGTGGC (SEQ ID NO:106), a ninth probe comprising the sequence TTTTTTAATACCACTCAGAAAAGGA (SEQ ID NO:111), a tenth probe comprising the sequence TTTTTTTTTTTATTACCACTCAGAGGA (SEQ ID NO:116), an eleventh probe comprising the sequence TTTTTTTTTTTTTAGAAATAAAAGATTG (SEQ ID NO:122), a twelfth probe comprising the sequence TTTTTTTTTATTTGGGTGTCTAAG (SEQ ID NO:125), a thirteenth probe comprising the sequence TTTTTTTTTTACCAAGTGAGAAGTA (SEQ ID NO:132), a fourteenth probe comprising the sequence TTTTTTTTACTGCTGAAAAGAGAGAGT (SEQ ID NO:57), and a fifteenth probe comprising the sequence TTTTTTTTTTTGAGGCAGAAAAAAACTA (SEQ ID NO:141); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO:13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and a plurality of blocking nucleic acids, the plurality of blocking nucleic acids comprising a first blocking nucleic acid comprising the sequence TA*C*G*CC*A*CC*A*G*CT(invdT)*ₙ*, wherein *n* is 1, 2, or 3 (SEQ ID NO:3), a second blocking nucleic acid comprising the sequence G*AGAT*T*TCAC*T*GTAGC*(invdT)*ₙ,* wherein *n* is 1, 2, or 3 (SEQ ID NO:10), a third blocking nucleic acid comprising the sequence T*G*C*CA*CT*ACCA*C*AG*(invdT)*ₙ,* wherein *n* is 1, 2, or 3 (SEQ ID NO:150), a fourth blocking nucleic acid comprising the sequence T*CC*TT*CT*CT*GAG*T*G*G(invdT)n, wherein n is 1, 2, or 3 (SEQ ID NO:176), a fifth blocking nucleic acid comprising the sequence A*G*T*G*GTGC*TCA*G*AC*A*C*C*C*A(invdT)n, wherein n is 1, 2, or 3 (SEQ ID NO:161), a sixth blocking nucleic acid comprising the sequence *CTTCTCGCTTGGTT(invdT)n,* wherein n is 1, 2, or 3 (SEQ ID NO:163), a seventh blocking nucleic acid comprising the sequence *CTTTTCTTTTATTTCTGC(invdT)n,* wherein n is 1, 2, or 3 (SEQ ID NO:157), an eighth blocking nucleic acid comprising the sequence *CCA*C*TC*TCTCT*C*T*TT*T*CAGC*(invdT)*ₙ,* wherein *n* is 1, 2, or 3 (SEQ ID NO:25), and a ninth blocking nucleic acid comprising the sequence *TCAGAATCAATAGTTTTTTCTG* (invdT)n, wherein n is 1, 2, or 3 (SEQ ID NO:171), with italicized nucleic acids representing locked nucleic acids.

In another aspect, provided herein are kits comprising (a) a plurality of probes, the plurality of probes comprising a first probe comprising the sequence TTTTTTTTTTATGGAGCTGATGGCG (SEQ ID NO:75), a second probe comprising the sequence TTTTTTTTTATGGAGCTGTAGGTGG (SEQ ID NO:81), a third probe comprising the sequence TTTTTTTTTTTATGGAGCTAGTGG (SEQ ID NO:49), a fourth probe comprising the sequence TTTTTTTTTATGGAGCTGGTGACGT (SEQ ID NO:50), a fifth probe comprising the sequence TTTTTTTTTATACAGAGAAATCTCGAT (SEQ ID NO:92), a sixth probe comprising the sequence TTTTTTTATGTCTAGCTACAGAAAAAT (SEQ ID NO:52), a seventh probe comprising the sequence TTTTTTTTTTTAGGAGCTGTGGCAGTG (SEQ ID NO:101), an eighth probe comprising the sequence TTTTTTTTTTTTTGGAGCTGTGATA (SEQ ID NO:54), a ninth probe comprising the sequence TTTTTTAATACCACTCAGAAAAGGA (SEQ ID NO:111), a tenth probe comprising the sequence TTTTTTTTTATTACCAATCAGAGGAGG (SEQ ID NO:119), an eleventh probe comprising the sequence TTTTTTTTTTTTTAGAAATAAAAGATTG (SEQ ID NO:122), a twelfth probe comprising the sequence TTTTTTTTTTTTTGGGTGTCTAAG (SEQ ID NO:59), a thirteenth probe comprising the sequence TTTTTTTTTTACCAAGTGAGAAGTA (SEQ ID NO:132), a fourteenth probe comprising the sequence TTTTTTTTTACCTACTGCTGAAAAG (SEQ ID NO:135), and a fifteenth probe comprising the sequence TTTTTTTTTTTGAGGCAGAAAAAAACTA (SEQ ID NO:141); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO:13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and (c) a plurality of blocking nucleic acids, the plurality of blocking nucleic acids comprising a first blocking nucleic acid comprising the sequence TT*GG*A*G*CT*GGTGGC*GTA(invdT)*ₙ*, wherein *n* is 1, 2, or 3 (SEQ ID NO:142), a second blocking nucleic acid comprising the sequence G*AGAT*T*TCAC*T*GTAGC*(invdT)*ₙ,* wherein *n* is 1, 2, or 3 (SEQ ID NO:148), a third blocking nucleic acid comprising the sequence GC*CACTACCACAG*CT(invdT)*ₙ,* wherein *n* is 1, 2, or 3 (SEQ ID NO: 15), a fourth blocking nucleic acid comprising the sequence T*CC*T*TCTC*T*GAG*T*G*G(invdT)*ₙ,* wherein *n* is 1, 2, or 3 (SEQ ID NO: 174), a fifth blocking nucleic acid comprising the sequence A*G*T*G*GTG*CTCA*G*AC*A*C*C*C*A(invdT)n, wherein n is 1, 2, or 3 (SEQ ID NO: 161), a sixth blocking nucleic acid comprising the sequence *CTTCTCGCTTGGTT(invdT)n,* wherein n is 1, 2, or 3 (SEQ ID NO:163), a seventh blocking nucleic acid comprising the sequence C*TTTTCTTTTAT*TT*C*T*GC*(invdT)*ₙ,* wherein *n is* 1, 2, or 3 (SEQ ID NO:29), an eighth blocking nucleic acid comprising the sequence *CCACTCTCTCTCTTTTCAGC* (invdT)n, wherein n is 1, 2, or 3 (SEQ ID NO:168), and a ninth blocking nucleic acid comprising the sequence *C*A*ATAG*T*TTTT*T*CTGC*C(invdT)*ₙ,* wherein n is 1, 2, or 3 (SEQ ID NO:41), with italicized nucleic acids representing locked nucleic acids.

In another aspect, provided herein are kits comprising (a) a plurality of probes, the plurality of probes comprising a first probe comprising the sequence TTTTTTTTTTTTAAGGAGCTGATGG (SEQ ID NO:47), a second probe comprising the sequence TTTTTTTTTTTTAAGGAGCTGTTGG (SEQ ID NO:48), a third probe comprising the sequence TTTTTTTTTTTTAAGGAGCTAGTGG (SEQ ID NO:86), a fourth probe comprising the sequence TTTTTTTTTAAGGAGCTGGTGACGT (SEQ ID NO:91), a fifth probe comprising the sequence TTTTTTTAATTACTAGCTACAGAGAAA (SEQ ID NO:95), a sixth probe comprising the sequence TTTTTTTATTTCTAGCTACAGAAAAAT (SEQ ID NO:99), a seventh probe comprising the sequence TTTTTTTTTTAAGGAGCTGTGGCAG (SEQ ID NO:104), an eighth probe comprising the sequence TTTTTTTTTTTTTTGGAGCTGTGAT (SEQ ID NO:109), a ninth probe comprising the sequence TTTTTTTTATTACCACTCAGAAAAG (SEQ ID NO: 113), a tenth probe comprising the sequence TTTTTTTTTATTACCAATCAGAGGAGG (SEQ ID NO:119), an eleventh probe comprising the sequence TTTTTTTTTTTTTAGAAATAAAAGATTG (SEQ ID NO:122), a twelfth probe comprising the sequence TTTTTTTTTATTTGGGTGTCTAAG (SEQ ID NO:125), a thirteenth probe comprising the sequence TTTTTTTTTACAAACCAAGTGAGAA (SEQ ID NO:60), a fourteenth probe comprising the sequence TTTTTTTTACTGCTGAAAAGAGAGAGT (SEQ ID NO:57), and a fifteenth probe comprising the sequence TTTTTTTTTTAGAGGCAGAAAAAAACT (SEQ ID NO:61); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO:13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and (c) a plurality of blocking nucleic acids, the plurality of blocking nucleic acids comprising a first blocking nucleic acid comprising the sequence a plurality of blocking nucleic acids, the plurality of blocking nucleic acids comprising a first blocking nucleic acid comprising the sequence TA*C*G*CC*A*CC*A*G*CT(invdT)*ₙ*, wherein n is 1, 2, or 3 (SEQ ID NO:3), a second blocking nucleic acid comprising the sequence G*AGAT*T*TCAC*T*GTAGC*(invdT)*ₙ,* wherein *n* is 1, 2, or 3 (SEQ ID NO: 10), a third blocking nucleic acid comprising the sequence GC*CACTACCACAG*CT(invdT)*ₙ,* wherein *n* is 1, 2, or 3 (SEQ ID NO: 15), a fourth blocking nucleic acid comprising the sequence GC*TCCTTCTCTG*AGT(invdT)*ₙ,* wherein *n* is 1, 2, or 3 (SEQ ID NO:20), a fifth blocking nucleic acid comprising the sequence GTG*CTCA*G*ACAC*C(invdT)*ₙ,* wherein *n* is 1, 2, or 3 (SEQ ID NO:33), a sixth blocking nucleic acid comprising the sequence C*TTC*T*CGCT*T*GGT*T(invdT)*ₙ,* wherein *n* is 1, 2, or 3 (SEQ ID NO:37), a seventh blocking nucleic acid comprising the sequence C*TTTTCTTTTAT*TT*C*T*GC*(invdT)*ₙ,* wherein *n* is 1, 2, or 3 (SEQ ID NO:29), an eighth blocking nucleic acid comprising the sequence *CCA*C*TC*TCTCT*C*T*TT*T*CAGC*(invdT)*ₙ,* wherein *n* is 1, 2, or 3 (SEQ ID NO:25), and a ninth blocking nucleic acid comprising the sequence *C*A*ATAG*T*TTTT*T*CTGC*C(invdT)*ₙ,* wherein *n* is 1, 2, or 3 (SEQ ID NO:41), with italicized nucleic acids representing locked nucleic acids. In some embodiments of any of the above embodiments, each probe of the plurality is coupled to a microcarrier that has a unique identifier corresponding to the probe coupled thereto.

In another aspect, provided herein are kits comprising (1) a primer pair for amplifying the locus of a *KRAS* mutation (*e.g*., encoding or resulting in a G12D, G12V, G12S, or G13D mutated KRAS protein); (2) a blocking nucleic acid comprising the sequence TA*C*G*CC*A*CC*A*G*CT(invdT)*ₙ*, wherein *n* is 1, 2, or 3 (SEQ ID NO:3), TT*GG*A*G*CT*GGTGGC*GTA(invdT)*ₙ*, wherein *n* is 1, 2, or 3 (SEQ ID NO: 142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n* is 1, 2, or 3 (SEQ ID NO: 144), or TT*GG*A*G*CT*GG*T*GG*C*G*T(invdT)*ₙ*, wherein *n* is 1, 2, or 3 (SEQ ID NO: 145), with italicized nucleic acids representing locked nucleic acids; (3a) a first probe comprising a sequence selected from the group consisting of GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO:180), and GCTGATGGCGTA (SEQ ID NO:181); (3b) a second probe comprising a sequence selected from the group consisting of GGAGCTGTTGG (SEQ ID NO:5), TGGAGCTGTTGGTGGC (SEQ ID NO: 182), GGAGCTGTTGGTG (SEQ ID NO:183), TGGAGCTGTTGGT (SEQ ID NO: 184), and TGGAGCTGTAGGTGG (SEQ ID NO: 185) (3c) a third probe comprising a sequence selected from the group consisting of TTGGAGCTAGTGGCGTA (SEQ ID NO:186), GCTAGTGGCGTAGGC (SEQ ID NO:187), AGCTAGTGGCGT (SEQ ID NO: 188), GTTGGAGCTAGTGG (SEQ ID NO: 189), and GGAGCTAGTGG (SEQ ID NO: 190); (3d) a fourth probe comprising a sequence selected from the group consisting of GGTGACGTAGGCAA (SEQ ID NO:191), TGACGTAGGCAAGAG (SEQ ID NO: 192), GCTGGTGACGTAGG (SEQ ID NO:193), AGCTGGTGACGTAG (SEQ ID NO: 194), and GGAGCTGGTGACGT (SEQ ID NO:195); and wherein each of the four probes is coupled to a microcarrier with a different identifier; (4) a primer pair for amplifying the locus of a *BRAF* mutation (*e.g.,* encoding or resulting in a V600E mutated BRAF protein); (5) a blocking nucleic acid comprising the sequence G*AGAT*T*TCAC*T*GTAGC*(invdT)*ₙ,* wherein *n* is 1, 2, or 3 (SEQ ID NO: 10), G*AGAT*T*TCAC*T*GTAGC*(invdT)*ₙ,* wherein *n* is 1, 2, or 3 (SEQ ID NO: 146), G*AGAT*T*TCAC*T*GTAGC*(invdT)*ₙ,* wherein *n* is 1, 2, or 3 (SEQ ID NO: 147), G*AGAT*T*TCAC*T*GTAGC*(invdT)*ₙ,* wherein *n* is 1, 2, or 3 (SEQ ID NO:148), or G*AGAT*T*TCAC*T*GTAGC*(invdT)*ₙ,* wherein *n* is 1, 2, or 3 (SEQ ID NO: 149), with italicized nucleic acids representing locked nucleic acids; (6a) a first probe comprising a sequence selected from the group consisting of TACAGAGAAATCTCGAT (SEQ ID NO: 196), TACAGAGAAATCTC (SEQ ID NO: 197), CTAGCTACAGAGAAAT (SEQ ID NO:198), CTAGCTACAGAGAAA (SEQ ID NO: 199), and TCTAGCTACAGAG (SEQ ID NO:200); (6b) a second probe comprising a sequence selected from the group consisting of GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204); (7) a primer pair for amplifying the locus of a *CTNNB1* mutation (*e.g.,* encoding or resulting in a T41A, T41I, S45F, and S45P mutated CTNNB 1 protein); (8a) a first blocking nucleic acid comprising the sequence of GC*CACTACCACAG*CT(invdT)*ₙ,* wherein *n is* 1, 2, or 3 (SEQ ID NO:15), T*G*C*CA*CT*ACCA*C*AG*(invdT)*ₙ,* wherein *n* is 1, 2, or 3 (SEQ ID NO:150), C*AC*T*ACCA*C*AGC*T*C*C(invdT)*ₙ,* wherein *n* is 1, 2, or 3 (SEQ ID NO:151), GC*CACTACCACAG*CT(invdT)*ₙ,* wherein *n* is 1, 2, or 3 (SEQ ID NO: 152), or GC*CACTACCACAG*CT(invdT)*ₙ,* wherein *n* is 1, 2, or 3 (SEQ ID NO: 153), with italicized nucleic acids representing locked nucleic acids; (8b) a second blocking nucleic acid comprising the sequence of GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein *n* is 1, 2, or 3 (SEQ ID NO: 174), G*C*T*CC*T*TC*TC*TGA*GT(invdT)n, wherein *n* is 1, 2, or 3 (SEQ ID NO: 175), T*CC*TT*CT*CT*GAG*T*G*G(invdT)n, wherein n is 1, 2, or 3 (SEQ ID NO: 176), or G*C*T*CC*TT*CT*C*TGAG*T(invdT)n, wherein n is 1, 2, or 3 (SEQ ID NO: 177), with italicized nucleic acids representing locked nucleic acids; (9a) a first probe comprising a sequence selected from the group consisting of AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (9b) a second probe comprising a sequence selected from the group consisting of TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (9c) a third probe comprising a sequence selected from the group consisting of AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (9d) a fourth probe comprising a sequence selected from the group consisting of GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); (9e) a fifth probe comprising a sequence selected from the group consisting of GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246); and wherein each of the five probes is coupled to a microcarrier with a different identifier; (10) a primer pair for amplifying the locus of an *APC* mutation (*e.g.,* encoding or resulting in the mutated APC proteins described *infra*); (11a) a first blocking nucleic acid comprising the sequence of *CCA*C*TC*TCTCT*C*T*TT*T*CAGC*(invdT)*ₙ,* wherein *n* is 1, 2, or 3 (SEQ ID NO:25), TAGGTCCACTCTCTCTCTTTTCAGCA(invdT)n, wherein n is 1, 2, or 3 (SEQ ID NO: 166), T*AGG*T*CCAC*T*CTCT*C*T*CTT*T*TC*AG*CA (invdT)n, wherein n is 1, 2, or 3 (SEQ ID NO: 167), *CCACTCTCTCTCTTTTCAGC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO:169), with italicized nucleic acids representing locked nucleic acids; (11b) a second blocking nucleic acid comprising the sequence of C*TTTTCTTTTAT*TT*C*T*GC*(invdT)*ₙ,* wherein *n* is 1, 2, or 3 (SEQ ID NO:29), *CTTTTCTTTTATTTCTGC(invdT)n,* wherein n is 1, 2, or 3 (SEQ ID NO:154), *CTTTTCTTTTATTTCTGC(invdT)n,* wherein n is 1, 2, or 3 (SEQ ID NO:155), C*TTT*TCT*TTT*A*T*T*TC*T*GC*(invdT)n, wherein n is 1, 2, or 3 (SEQ ID NO: 156), or *CTTTTCTTTTATTTCTGC(invdT)n,* wherein n is 1, 2, or 3 (SEQ ID NO:157), with italicized nucleic acids representing locked nucleic acids; (11c) a third blocking nucleic acid comprising the sequence of GTG*CTCA*G*ACAC*C(invdT)*ₙ,* wherein *n* is 1, 2, or 3 (SEQ ID NO:33), G*TGC*TC*A*G*A*C*ACC*(invdT)n, wherein n is 1, 2, or 3 (SEQ ID NO:158), AGTGGTG*CTCAGAC*ACCCA(invdT)n, wherein n is 1, 2, or 3 (SEQ ID NO:159), *AGTGGTGCTCAGACACCCA(invdT)n,* wherein n is 1, 2, or 3 (SEQ ID NO:160), or A*G*T*G*GTGC*TCA*G*ACAC*C*C*A(invdT)n, wherein n is 1, 2, or 3 (SEQ ID NO:161), with italicized nucleic acids representing locked nucleic acids; (11d) a fourth blocking nucleic acid comprising the sequence of C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO: 162), *CTTCTCGCTTGGTT(invdT)n,* wherein n is 1, 2, or 3 (SEQ ID NO:163), GT*ACT*T*CTCG*CT*TGG*T(invdT)n, wherein n is 1, 2, or 3 (SEQ ID NO: 164), or GT*A*C*TTCTCGC*TT*GG*T(invdT)n, wherein n is 1, 2, or 3 (SEQ ID NO: 165), with italicized nucleic acids representing locked nucleic acids; (11e) a fifth blocking nucleic acid comprising the sequence of *C*A*ATAG*T*TTTT*T*CTGC*C(invdT)*ₙ,* wherein *n* is 1, 2, or 3 (SEQ ID NO:41), *GAATCAATAGTTTTTTCTGCCTC* (invdT)n, wherein n is 1, 2, or 3 (SEQ ID NO: 170), *TCAGAATCAATAGTTTTTTCTG* (invdT)n, wherein n is 1, 2, or 3 (SEQ ID NO: 171), *GAATCAATAGATTTTACTGCCTC* (invdT)n, wherein n is 1, 2, or 3 (SEQ ID NO: 172), or *AATCAATAGTTTTTTCTGCCTC* (invdT)n, wherein n is 1, 2, or 3 (SEQ ID NO: 173), with italicized nucleic acids representing locked nucleic acids; (12a) a first probe comprising a sequence selected from AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (12b) a second probe comprising a sequence selected from TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (12c) a third probe comprising a sequence selected from AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (12d) a fourth probe comprising a sequence selected from GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (12e) a fifth probe comprising a sequence selected from GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246). In some embodiments, each of the probes is coupled to a microcarrier with a different identifier. In some embodiments, the primer pair for amplifying the locus of a *KRAS* mutation comprises the sequences GTACTGGTGGAGTATTTGATAGTG (SEQ ID NO:1) and ATCGTCAAGGCACTCTTGCCTAC (SEQ ID NO:2). In some embodiments, the primer pair for amplifying the locus of a *BRAF* mutation comprises the sequences GGACCCACTCCATCGAGATTT (SEQ ID NO:8) and CAGATATATTTCTTCATGAAGACCTCACAGTAA (SEQ ID NO:9). In some embodiments, the primer pair for amplifying the locus of a *CTNNB1* mutation comprises: a first primer pair comprising the sequences GGAATCCATTCTGGTGCCACT (SEQ ID NO:13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO:19). In some embodiments, the primer pair for amplifying the locus of an *APC* mutation comprises: a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40).

It is to be understood that one, some, or all of the properties of the various embodiments described herein may be combined to form other embodiments of the present invention. These and other aspects of the invention will become apparent to one of skill in the art.

### BRIEF DESCRIPTION OF THE DRAWINGS

**FIGS. 1A & 1B** show two views of an exemplary microcarrier.
**FIGS. 1C & 1D** show an exemplary assay for DNA detection using an exemplary microcarrier.
**FIGS. 2A & 2B** show two views of an exemplary microcarrier.
**FIG. 3** shows an exemplary analog encoding scheme that includes multiple shape variation points for generating unique analog codes.
**FIG. 4A** shows three examples of microcarriers, each having a unique analog code.
**FIG. 4B** shows examples of microcarriers with a unique analog code, in accordance with some embodiments.
**FIGS. 5A & 5B** show two views of an exemplary microcarrier.
**FIGS. 6A & 6B** show two views of an exemplary microcarrier.
**FIG. 6C** shows the dimensions of an exemplary analog code. Dimensions are based on µm units.
**FIG. 7** shows an exemplary microcarrier.
**FIG. 8A** shows an exemplary microcarrier that includes an asymmetric start position as an orientation indicator.
**FIG. 8B** shows an exemplary analog encoding scheme that includes multiple shape variation points for generating unique analog codes.
**FIGS. 9A-9C** show two views of an exemplary microcarrier (**FIG. 9A** and **FIG. 9B**), along with a depiction of an optional feature (**FIG. 9C**).
**FIG. 10** shows a method for producing an exemplary microcarrier.
**FIGS. 11A** **&** **11B** show a method for producing an exemplary microcarrier.
**FIGS. 12A-12E** show a method for producing an exemplary microcarrier.
**FIGS. 13A-13C** show a method for producing an exemplary microcarrier.
**FIG. 14** shows a flowchart illustrating an exemplary method for detecting the presence of DNA mutation(s), in accordance with some embodiments.
**FIGS. 15** **&** **16** illustrate an exemplary scheme for preferentially amplifying and detecting mutant (**FIG. 16**) over wild-type (**FIG. 15**) loci corresponding to a DNA mutation of interest, in accordance with some embodiments. Solid horizontal lines indicate amplified DNA sequences, dashed horizontal lines indicate primer/probe/blocking nucleic acid (NA) sequences, and vertical lines indicate Watson-Crick base pairing.
**FIG. 17** shows a flowchart illustrating an exemplary protocol for hybridizing amplified DNA, detecting the presence or absence of hybridization of amplified DNA with probe(s) coupled to microcarriers, and detecting analog identifiers of microcarriers, in accordance with some embodiments.
**FIGS. 18A** **&** **18B** show the results of detecting the presence of DNA mutations in the *KRAS, BRAF, CTNNB1,* and *APC* genes, in accordance with some embodiments. Numbers report the fluorescence signal (in arbitrary units, AU) observed with each pairwise combination of isolated DNA (bearing a mutation or a wild-type sequence, as indicated in columns) and probe (as indicated in rows).

### DETAILED DESCRIPTION

The invention provides a method for detecting the presence of DNA mutations in the *KRAS, BRAF, CTNNB1,* and *APC* genes, the method comprising: (a) isolating DNA from a sample; (b) amplifying the isolated DNA by polymerase chain reaction (PCR) using primer pairs specific for the loci of one or more DNA mutations in each of the *KRAS, BRAF, CTNNB1*, and *APC* genes, wherein the isolated DNA is amplified in the presence of at least four blocking nucleic acids, wherein each of said at least four blocking nucleic acids hybridizes with a wild-type DNA locus corresponding with one of the DNA mutations in the KRAS, BRAF, CTNNB1, or APC genes and prevents amplification of the wild-type DNA locus, wherein optionally the *KRAS, BRAF, CTNNB1,* and *APC* genes are human genes; (c) hybridizing the amplified DNA with at least four probes, said at least four probes comprising one or more probes specific for a DNA mutation in each of the *KRAS, BRAF, CTNNB1,* and *APC* genes, wherein each of said at least four probes is coupled to a microcarrier, and wherein each of the microcarriers comprises an identifier corresponding to the probe coupled thereto; (d) detecting presence or absence of hybridization of the amplified DNA with said at least four probes, wherein hybridization between the amplified DNA and one of the probes indicates the presence of the DNA mutation corresponding to the probe; (e) detecting the identifiers of the microcarriers; and (f) correlating the detected identifiers of the microcarriers with the detected presence or absence of hybridization of the amplified DNA to the corresponding probes of the microcarriers.
The invention also provides a kit comprising (a) at least four microcarriers, wherein each of said at least four microcarriers comprises: (i) a probe coupled to the microcarrier, wherein the probe is specific for a DNA mutation in the *KRAS, BRAF, CTNNB1*, or *APC* gene, wherein optionally the KRAS, BRAF, CTNNB1, and APC genes are human genes; and (ii) an identifier corresponding to the probe coupled thereto; wherein the kit comprises at least one microcarrier comprising a probe specific for a DNA mutation in the *KRAS* gene, at least one microcarrier comprising a probe specific for a DNA mutation in the *BRAF* gene, at least one microcarrier comprising a probe specific for a DNA mutation in the *CTNNB1* gene, and at least one microcarrier comprising a probe specific for a DNA mutation in the *APC* gene, and (b) at least four blocking nucleic acids, wherein each of said at least four blocking nucleic acids hybridizes with a wild-type DNA locus corresponding with one of the DNA mutations in the KRAS, BRAF, CTNNB1, or APC genes.

In another aspect, provided herein are kits comprising (a) a plurality of probes, the plurality of probes comprising a first probe comprising the sequence TTTTTTTTTTATGGAGCTGATGGCG (SEQ ID NO:75), a second probe comprising the sequence TTTTTTTTTTAAGGAGCTGTTGGTG (SEQ ID NO:79), a third probe comprising the sequence TTTTTTTTTTGGAGCTAGTGGCGTA (SEQ ID NO:82), a fourth probe comprising the sequence TTTTTTTTAAAGGTGACGTAGGCAA (SEQ ID NO:87), a fifth probe comprising the sequence TTTTTTTTTATACAGAGAAATCTCGAT (SEQ ID NO:92), a sixth probe comprising the sequence TTTTTTTTATTCTAGCTACAGAAAAATC (SEQ ID NO:100), a seventh probe comprising the sequence TTTTTTTTTTTAGGAGCTGTGGCAG (SEQ ID NO:53), an eighth probe comprising the sequence TTTTTTTTTGAGCTGTGATAGTGGC (SEQ ID NO:106), a ninth probe comprising the sequence TTTTTTAATACCACTCAGAAAAGGA (SEQ ID NO:111), a tenth probe comprising the sequence TTTTTTTTTTTATTACCACTCAGAGGA (SEQ ID NO:116), an eleventh probe comprising the sequence TTTTTTTTTTTTTAGAAATAAAAGATTG (SEQ ID NO:122), a twelfth probe comprising the sequence TTTTTTTTTATTTGGGTGTCTAAG (SEQ ID NO:125), a thirteenth probe comprising the sequence TTTTTTTTTTACCAAGTGAGAAGTA (SEQ ID NO:132), a fourteenth probe comprising the sequence TTTTTTTTACTGCTGAAAAGAGAGAGT (SEQ ID NO:57), and a fifteenth probe comprising the sequence TTTTTTTTTTTGAGGCAGAAAAAAACTA (SEQ ID NO:141); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO: 13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and a plurality of blocking nucleic acids, the plurality of blocking nucleic acids comprising a first blocking nucleic acid comprising the sequence TA*C*G*CC*A*CC*A*G*CT(invdT)*ₙ*, wherein *n* is 1, 2, or 3 (SEQ ID NO:3), a second blocking nucleic acid comprising the sequence G*AGAT*T*TCAC*T*GTAGC*(invdT)*ₙ,* wherein *n* is 1, 2, or 3 (SEQ ID NO: 10), a third blocking nucleic acid comprising the sequence T*G*C*CA*CTA*CCA*C*AG*invdTinvdTinvdT (SEQ ID NO:150), a fourth blocking nucleic acid comprising the sequence T*CC*TT*CT*CT*GAG*T*G*GinvdTinvdTinvdT (SEQ ID NO: 176), a fifth blocking nucleic acid comprising the sequence A*G*T*G*GTGC*TCA*G*ACAC*C*C*AinvdTinvdTinvdT (SEQ ID NO: 161), a sixth blocking nucleic acid comprising the sequence C*TTC*T*CGCT*T*GGT*TinvdTinvdTinvdT (SEQ ID NO: 163), a seventh blocking nucleic acid comprising the sequence C*T*TT*TCTTTTATTTC*TG*C*invdTinvdTinvdT (SEQ ID NO:157), an eighth blocking nucleic acid comprising the sequence *CCA*C*TC*TCTCT*C*T*TT*T*CAGC*invdTinvdTinvdT (SEQ ID NO:25), and a ninth blocking nucleic acid comprising the sequence *TCAGAATCAATAGTTTTTTCTG* invdTinvdTinvdT (SEQ ID NO: 171), with italicized nucleic acids representing locked nucleic acids. In another aspect, provided herein are kits comprising (a) a plurality of probes, the plurality of probes comprising a first probe comprising the sequence TTTTTTTTTTATGGAGCTGATGGCG (SEQ ID NO:75), a second probe comprising the sequence TTTTTTTTTATGGAGCTGTAGGTGG (SEQ ID NO:81), a third probe comprising the sequence TTTTTTTTTTTATGGAGCTAGTGG (SEQ ID NO:49), a fourth probe comprising the sequence TTTTTTTTTATGGAGCTGGTGACGT (SEQ ID NO:50), a fifth probe comprising the sequence TTTTTTTTTATACAGAGAAATCTCGAT (SEQ ID NO:92), a sixth probe comprising the sequence TTTTTTTATGTCTAGCTACAGAAAAAT (SEQ ID NO:52), a seventh probe comprising the sequence TTTTTTTTTTTAGGAGCTGTGGCAGTG (SEQ ID NO:101), an eighth probe comprising the sequence TTTTTTTTTTTTTGGAGCTGTGATA (SEQ ID NO:54), a ninth probe comprising the sequence TTTTTTAATACCACTCAGAAAAGGA (SEQ ID NO:111), a tenth probe comprising the sequence TTTTTTTTTATTACCAATCAGAGGAGG (SEQ ID NO:119), an eleventh probe comprising the sequence TTTTTTTTTTTTTAGAAATAAAAGATTG (SEQ ID NO:122), a twelfth probe comprising the sequence TTTTTTTTTTTTTGGGTGTCTAAG (SEQ ID NO:59), a thirteenth probe comprising the sequence TTTTTTTTTTACCAAGTGAGAAGTA (SEQ ID NO:132), a fourteenth probe comprising the sequence TTTTTTTTTACCTACTGCTGAAAAG (SEQ ID NO:135), and a fifteenth probe comprising the sequence TTTTTTTTTTTGAGGCAGAAAAAAACTA (SEQ ID NO:141); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO:13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and (c) a plurality of blocking nucleic acids, the plurality of blocking nucleic acids comprising a first blocking nucleic acid comprising the sequence TT*GG*A*GC*T*GGTGGC*GTAinvdTinvdTinvdT (SEQ ID NO:142), a second blocking nucleic acid comprising the sequence *GAGAT*T*TCACT*G*TAGC*invdTinvdTinvdT (SEQ ID NO:148), a third blocking nucleic acid comprising the sequence GC*CACTACCACAG*CT(invdT)*ₙ,* wherein *n* is 1, 2, or 3 (SEQ ID NO:15), a fourth blocking nucleic acid comprising the sequence T*CC*T*TCTC*T*G*A*G*T*G*GinvdTinvdTinvdT (SEQ ID NO:174), a fifth blocking nucleic acid comprising the sequence A*G*T*G*GTGC*TCA*G*ACAC*C*C*AinvdTinvdTinvdT (SEQ ID NO:161), a sixth blocking nucleic acid comprising the sequence C*TTC*T*CGCT*T*GGT*TinvdTinvdTinvdT (SEQ ID NO:163), a seventh blocking nucleic acid comprising the sequence C*TTTTCTTTTAT*TT*C*T*GC*invdTinvdTinvdT (SEQ ID NO:29), an eighth blocking nucleic acid comprising the sequence *CCACTCTCTCTCTTTTCAGC* invdTinvdTinvdT (SEQ ID NO:168), and a ninth blocking nucleic acid comprising the sequence *C*A*ATAG*T*TTTT*T*CTGC*CinvdTinvdTinvdT (SEQ ID NO:41), with italicized nucleic acids representing locked nucleic acids. In another aspect, provided herein are kits comprising (a) a plurality of probes, the plurality of probes comprising a first probe comprising the sequence TTTTTTTTTTTTAAGGAGCTGATGG (SEQ ID NO:47), a second probe comprising the sequence TTTTTTTTTTTTAAGGAGCTGTTGG (SEQ ID NO:48), a third probe comprising the sequence TTTTTTTTTTTTAAGGAGCTAGTGG (SEQ ID NO:86), a fourth probe comprising the sequence TTTTTTTTTAAGGAGCTGGTGACGT (SEQ ID NO:91), a fifth probe comprising the sequence TTTTTTTAATTACTAGCTACAGAGAAA (SEQ ID NO:95), a sixth probe comprising the sequence TTTTTTTATTTCTAGCTACAGAAAAAT (SEQ ID NO:99), a seventh probe comprising the sequence TTTTTTTTTTAAGGAGCTGTGGCAG (SEQ ID NO:104), an eighth probe comprising the sequence TTTTTTTTTTTTTTGGAGCTGTGAT (SEQ ID NO:109), a ninth probe comprising the sequence TTTTTTTTATTACCACTCAGAAAAG (SEQ ID NO: 113), a tenth probe comprising the sequence TTTTTTTTTATTACCAATCAGAGGAGG (SEQ ID NO:119), an eleventh probe comprising the sequence TTTTTTTTTTTTTAGAAATAAAAGATTG (SEQ ID NO:122), a twelfth probe comprising the sequence TTTTTTTTTATTTGGGTGTCTAAG (SEQ ID NO:125), a thirteenth probe comprising the sequence TTTTTTTTTACAAACCAAGTGAGAA (SEQ ID NO:60), a fourteenth probe comprising the sequence TTTTTTTTACTGCTGAAAAGAGAGAGT (SEQ ID NO:57), and a fifteenth probe comprising the sequence TTTTTTTTTTAGAGGCAGAAAAAAACT (SEQ ID NO:61); (b) a plurality of primer pairs, the plurality of primer pairs comprising a first primer pair comprising the sequences GTACTGGTGGAGTATTTGATAGTG (SEQ ID NO:1) and ATCGTCAAGGCACTCTTGCCTAC (SEQ ID NO:2), a second primer pair comprising the sequences GGACCCACTCCATCGAGATTT (SEQ ID NO:8) and CAGATATATTTCTTCATGAAGACCTCACAGTAA (SEQ ID NO:9), a third primer pair comprising the sequences GGAATCCATTCTGGTGCCACT (SEQ ID NO: 13) and AGAAAATCCCTGTTCCCACTCATA (SEQ ID NO:14), a fourth primer pair comprising the sequences GGTGCCACTACCACAGCTCCT (SEQ ID NO:18) and TCTCAAAACTGCATTCTGACTTTCA (SEQ ID NO:19), a fifth primer pair comprising the sequences TAAAAATAAAGCACCTACTGCTGAAA (SEQ ID NO:23) and AGCTTGCTTAGGTCCACTCTCTCT (SEQ ID NO:24), a sixth primer pair comprising the sequences TAGGATGTAATCAGACGACACAGGA (SEQ ID NO:27) and CAGCTGACCTAGTTCCAATCTTTTA (SEQ ID NO:28), a seventh primer pair comprising the sequences TCTCCCTCCAAAAGTGGTGCT (SEQ ID NO:31) and TGGCAATCGAACGACTCTCAA (SEQ ID NO:32), an eighth primer pair comprising the sequences GCAGAAGTAAAACACCTCCACCA (SEQ ID NO:35) and GGTGCTTTATTTTTAGGTACTTC (SEQ ID NO:36), and a ninth primer pair comprising the sequences CAGGAAAATGACAATGGGAATG (SEQ ID NO:39) and ATCTAATAGGTCCTTTTCAGAATCAATAG (SEQ ID NO:40), respectively; and (c) a plurality of blocking nucleic acids, the plurality of blocking nucleic acids comprising a first blocking nucleic acid comprising the sequence TA*C*G*CC*A*CC*A*G*CT(invdT)*ₙ*, wherein *n* is 1, 2, or 3 (SEQ ID NO:3), a second blocking nucleic acid comprising the sequence G*AGAT*T*TCAC*T*GTAGC*(invdT)*ₙ,* wherein *n* is 1, 2, or 3 (SEQ ID NO: 10), a third blocking nucleic acid comprising the sequence GC*CACTACCACAG*CT(invdT)*ₙ,* wherein *n* is 1, 2, or 3 (SEQ ID NO: 15), a fourth blocking nucleic acid comprising the sequence GC*TCCTTCTCTG*AGTinvdTinvdTinvdT (SEQ ID NO:20), a fifth blocking nucleic acid comprising the sequence GTG*CTCA*G*ACAC*CinvdTinvdTinvdT (SEQ ID NO:33), a sixth blocking nucleic acid comprising the sequence C*TTC*T*CGCT*T*GGT*TinvdTinvdTinvdT (SEQ ID NO:37), a seventh blocking nucleic acid comprising the sequence *CTTTTCTTTTATTTCTGCinvdTinvdTinvdT* (SEQ ID NO:29), an eighth blocking nucleic acid comprising the sequence *CCACTC*TCTCT*C*T*TT*T*CAGC*invdTinvdTinvdT (SEQ ID NO:25), and a ninth blocking nucleic acid comprising the sequence *C*A*ATAG*T*TTTT*T*CTGC*CinvdTinvdTinvdT (SEQ ID NO:41), with italicized nucleic acids representing locked nucleic acids. In some embodiments, each probe of the plurality is coupled to a microcarrier that has a unique identifier corresponding to the probe coupled thereto.

### I. General Techniques

The practice of the techniques described herein will employ, unless otherwise indicated, conventional techniques in polymer technology, microfabrication, micro-electromechanical systems (MEMS) fabrication, photolithography, microfluidics, organic chemistry, biochemistry, oligonucleotide synthesis and modification, bioconjugate chemistry, nucleic acid hybridization, molecular biology, microbiology, genetics, recombinant DNA, and related fields as are within the skill of the art. The techniques are described in the references cited herein and are fully explained in the literature.

For molecular biology and recombinant DNA techniques, see, for example, (Maniatis, T. et al. (1982), Molecular Cloning: A Laboratory Manual, Cold Spring Harbor; Ausubel, F. M. (1987), Current Protocols in Molecular Biology, Greene Pub. Associates and Wiley-Interscience; Ausubel, F. M. (1989), Short Protocols in Molecular Biology: A Compendium of Methods from Current Protocols in Molecular Biology, Greene Pub. Associates and Wiley-Interscience; Sambrook, J. et al. (1989), Molecular Cloning: A Laboratory Manual, Cold Spring Harbor; Innis, M. A. (1990), PCR Protocols: A Guide to Methods and Applications, Academic Press; Ausubel, F. M. (1992), Short Protocols in Molecular Biology: A Compendium of Methods from Current Protocols in Molecular Biology, Greene Pub. Associates; Ausubel, F. M. (1995), Short Protocols in Molecular Biology: A Compendium of Methods from Current Protocols in Molecular Biology, Greene Pub. Associates; Innis, M. A. et al. (1995), PCR Strategies, Academic Press*;* Ausubel, F. M. (1999), Short Protocols in Molecular Biology: A Compendium of Methods from Current Protocols in Molecular Biology, Wiley, and annual updates.

For DNA synthesis techniques and nucleic acids chemistry, see for example, Gait, M. J. (1990), Oligonucleotide Synthesis: A Practical Approach, IRL Press; Eckstein, F. (1991), Oligonucleotides and Analogues: A Practical Approach, IRL Press; Adams, R. L. et al. (1992), The Biochemistry of the Nucleic Acids, Chapman & Hall; Shabarova, Z. et al. (1994), Advanced Organic Chemistry of Nucleic Acids, Weinheim; Blackburn, G. M. et al. (1996), Nucleic Acids in Chemistry and Biology, Oxford University Press; Hermanson, G. T. (1996), Bioconjugate Techniques, Academic Press).

For microfabrication, see for example, (Campbell, S. A. (1996), The Science and Engineering of Microelectronic Fabrication, Oxford University Press; Zaut, P. V. (1996), Microarray Fabrication: a Practical Guide to Semiconductor Processing, Semiconductor Services; Madou, M. J. (1997), Fundamentals of Microfabrication, CRC Press; Rai-Choudhury, P. (1997). Handbook of Microlithography, Micromachining, & Microfabrication: Microlithography).

### II. Definitions

Before describing the invention in detail, it is to be understood that this invention is not limited to particular compositions or biological systems, which can, of course, vary. It is also to be understood that the terminology used herein is for the purpose of describing particular embodiments only, and is not intended to be limiting.

The term "microcarrier" as used herein may refer to a physical substrate onto which a capture agent or probe may be coupled. A microcarrier of the present disclosure may take any suitable geometric form or shape. In some embodiments, the microcarrier may be disc-shaped. Typically the form or shape of a microcarrier will include at least one dimension on the order of 10⁻⁴ to 10⁻⁷ m (hence the prefix "micro").

The term "polymer" as used herein may refer to any macromolecular structure comprising repeated monomers. A polymer may be natural (*e.g*., found in nature) or synthetic (*e.g*., man-made, such as a polymer composed of non-natural monomer(s) and/or polymerized in a configuration or combination not found in nature).

The terms "substantially transparent" and "substantially non-transparent" as used herein may refer to the ability of light (*e.g.,* of a particular wavelength, such as infrared, visible, UV, and so forth) to pass through a substrate, such as a polymer layer. A substantially transparent polymer may refer to one that is transparent, translucent, and/or pervious to light, whereas a substantially non-transparent polymer may refer to one that reflects and/or absorbs light. It is to be appreciated that whether a material is substantially transparent or substantially non-transparent may depend upon the wavelength and/or intensity of light illuminating the material, as well as the means detecting the light traveling through the material (or a decrease or absence thereof). In some embodiments, a substantially non-transparent material causes a perceptible decrease in transmitted light as compared to the surrounding material or image field, *e.g.,* as imaged by light microscopy (*e.g.,* bright field, dark field, phase contrast, differential interference contrast (DIC), Nomarski interference contrast (NIC), Nomarski, Hoffman modulation contrast (HMC), or fluorescence microscopy). In some embodiments, a substantially transparent material allows a perceptible amount of transmitted light to pass through the material, *e.g.,* as imaged by light microscopy (*e.g.,* bright field, dark field, phase contrast, differential interference contrast (DIC), Nomarski interference contrast (NIC), Nomarski, Hoffman modulation contrast (HMC), or fluorescence microscopy).

The term "analog code" as used herein may refer to any code in which the encoded information is represented in a non-quantized and/or non-discrete manner, *e.g*., as opposed to a digital code. For example, a digital code is sampled at discrete positions for a limited set of values (*e.g.,* 0/1 type values), whereas an analog code may be sampled at a greater range of positions (or as a continuous whole) and/or may contain a wider set of values (*e.g*., shapes). In some embodiments, an analog code may be read or decoded using one or more analog shape recognition techniques.

As used herein, "sample" refers to a composition containing a material, such as a molecule, to be detected. In one embodiment, the sample is a "biological sample" (*i.e.,* any material obtained from a living source (*e.g*. human, animal, plant, bacteria, fungi, protist, virus)). The biological sample can be in any form, including solid materials (*e.g*. stool, tissue, cell pellets and biopsies) and biological fluids (*e.g.* urine, blood, stool, saliva, lymph, tears, sweat, prostatic fluid, seminal fluid, semen, bile, mucus, amniotic fluid and mouth wash (containing buccal cells)). Solid materials typically are mixed with a fluid. Sample can also refer to an environmental sample such as water, air, soil, or any other environmental source.

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

### III. Methods of Detecting DNA Mutations

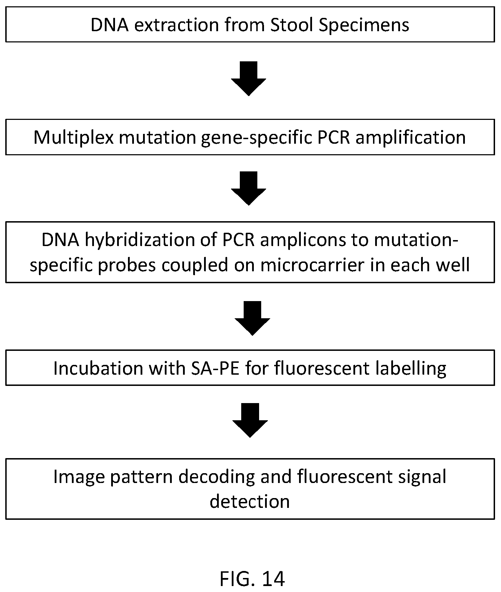

Certain aspects of the present disclosure relate to methods for detecting the presence of DNA mutations (*e.g.,* one or more mutations in the *KRAS, BRAF, CTNNB1,* and/or *APC* genes) by using microcarriers, *e.g.,* an encoded microcarrier described herein, or any of the microcarriers described in International Publication No. WO2016/198954. The methods of the present disclosure employ encoded microcarrier(s) with some or all of the microcarrier features and aspects described herein, *e.g.,* in sections IV, V, and VI. Advantageously, these encoded microcarriers allow for detection of DNA mutations in improved multiplex assays with a large number of potential unique microcarriers and reduced recognition error, as compared to traditional multiplex assays. A flowchart described an exemplary method for detection of DNA mutations is provided in **FIGS. 14-16****.** The detection methods used herein may be performed in any suitable assay vessel known in the art, for example a microplate, petri dish, or any number of other well-known assay vessels.

In some embodiments, the methods of the present disclosure include isolating DNA from a sample. Standard molecular techniques known in the art allow for the isolation of DNA from a variety of different types of samples. DNA isolation kits suitable for a variety of samples are commercially available. For example, the QIAamp^{®} DNA Stool Mini Kit (QIAGEN; Hilden, Germany) can be used to isolate DNA from a stool sample (*e.g*., using an elution volume of 50µL per 2g of stool sample) according to manufacturer's instructions.

In some embodiments, the methods of the present disclosure use DNA from a sample at a concentration of between about 0.5 and 2.5ng/µL. In some embodiments, the methods of the present disclosure use DNA from a sample at a concentration of at least about 0.5ng/µL. For example, 20 µL of a 0.5 ng/µL DNA Stock (total of 10 ng DNA) or a 2.5 ng/µL DNA stock (total of 50ng DNA) may be used (*e.g.,* for PCR amplification).

In some embodiments, the methods of the present disclosure are used to detect, monitor, screen for, or monitor treatment response of colon cancer, rectal cancer, colorectal cancer, colon adenoma, rectal adenoma, or colorectal adenoma, *e.g*., in a patient from whom the sample is collected. As used herein, colon, rectal, and colorectal cancer can refer to various types of cancers, including without limitation adenocarcinomas, lymphomas, stromal tumors, leiomyosarcomas, carcinoid tumors, and melanomas. Colorectal cancers are thought to progress from benign neoplasias like adenomas (*e.g*., a polyp) to malignancies such as carcinomas and metastatic disease as DNA mutations accumulate, but even some early adenomas have DNA mutations such as the *KRAS* activating mutations described herein. *See* Fearon, E.F. and Vogelstein, B. (1990) Cell 61:759-67. Detecting DNA mutations from stool samples is particularly of interest in the early detection of colorectal cancer. *See, e.g.,* U.S. Patent No. 7,833,757; Imperiale, T.F. et al. (2014) N. Engl. J. Med. 370:1287-97; and the COLOGUARD^{®} kit (Exact Sciences Corp.). Existing diagnostic techniques such as sigmoidoscopy and colonoscopy are highly invasive (and therefore can suffer from low patient acceptance) and can miss tumors that are very small and/or in inaccessible locations. In contrast, the methods described herein are thought to be highly accurate, robust, and non-invasive.

The methods of the present disclosure can be used to detect analytes (*e.g*., DNA mutations) in any suitable solution. In some embodiments, the solution comprises a biological sample. In some embodiments, the solution comprises DNA isolated from a biological sample and, optionally, a buffer. Suitable buffers for DNA isolation are well-known in the art. Examples of biological samples include without limitation stool, blood, urine, sputum, bile, cerebrospinal fluid, interstitial fluid of skin or adipose tissue, saliva, tears, bronchial-alveolar lavage, oropharyngeal secretions, intestinal fluids, cervico-vaginal or uterine secretions, and seminal fluid. In some embodiments, the sample is a stool sample. In some embodiments, multiple samples are obtained from a single specimen (*e.g*., representing different regions of the specimen, such as described in Example 2) and analyzed individually. In other embodiments, a single, large stool specimen is analyzed (*e.g.,* an entire stool or stool sample is analyzed instead of sampling discrete, smaller samples). In some embodiments, the biological sample may be from a human. In other embodiments, the solution comprises a sample that is not a biological sample, such as an environmental sample, a sample prepared in a laboratory (*e.g.,* a sample containing one or more analytes that have been prepared, isolated, purified, and/or synthesized), a fixed sample (*e.g.,* a formalin-fixed, paraffin-embedded or FFPE sample), and so forth.

In some embodiments, the methods of the present disclosure include amplifying DNA (*e.g.,* DNA isolated from a sample as described *supra*) by polymerase chain reaction (PCR). PCR techniques are well-known in the art. Briefly, a thermostable DNA polymerase is used to amplify copies of a DNA sequence of interest using template DNA strands (*e.g*., isolated from a sample and denatured) and a pair of oligonucleotide primers that are complementary to the 3' ends of the sense and anti-sense strands (respectively) of the DNA template. The DNA polymerase is mixed in a reaction with both primers, all four deoxynucleotides (dNTPs), a buffer, magnesium ions (*e.g.,* MgCl₂), and potassium ions (*e.g.,* KCl), and optionally other ingredients. The reaction mixture is then subjected to multiple cycles (*e.g.,* 20-40) of temperature changes that allow for denaturation of the DNA template, annealing of the primers to the denatured, single-stranded template, and primer extension by the DNA polymerase. Various DNA polymerases with different properties of interest (*e.g*., ability to amplify long or repetitive templates, high fidelity, hot start, etc.) have been characterized for use in PCR and are commercially available.

In some embodiments, the methods of the present disclosure include amplifying (*e.g*., by PCR) from isolated DNA the loci of one or more DNA mutations in one or more specific genes of interest. As herein, a "locus" of a DNA mutation comprises the mutation itself and sufficient adjacent sequence on one or both sides of the mutation for PCR amplification of, and/or probe hybridization to, the mutated DNA sequence. As is known in the art, the minimum sequence length sufficient for PCR amplification can be influenced by several factors, including without limitation the polymerase, the melting temperature of the primers, the propensity of the primers to form primer dimers, the ratio of the template to primers, etc. In some embodiments, the locus of a DNA mutation comprises at least about 100 base pairs of adjacent sequence (*i.e.,* including adjacent sequence both 5' and 3' to the DNA mutation). In some embodiments, the locus of a DNA mutation comprises less than or equal to about 200 base pairs of adjacent sequence (*i.e.,* including adjacent sequence both 5' and 3' to the DNA mutation). As described above, the locus of a DNA mutation can be amplified using a pair of primers specific to the locus, using the locus as the DNA template. While mutations are described herein as DNA mutations, it will be appreciated that similar techniques could be applied to detecting RNA mutations, *e.g*., using mRNA or cDNA from a sample.

In some embodiments, multiple PCR reactions can be used, *e.g.,* to detect various DNA mutations. In some embodiments, each PCR reaction can include multiple primer pairs, each specific for a DNA mutation of interest. For example, in some embodiments, as shown in Examples 1 and 2 below, the methods of the present disclosure can include two PCR reactions: a first PCR reaction with the reagents for detecting the following mutations: *KRAS* G12D, *KRAS* G12V, *KRAS* G12S, *KRAS* G13D, *BRAF* V600E1, *BRAF* V600E2, *CTNNB1* T41A, *CTNNB1* T41I, *APC* E1309, *APC* Q1367, *APC* R1450, and *APC* T1556; and a second PCR reaction with the reagents for detecting the following mutations: *CTNNB1* S45F, *CTNNB1* S45P, and *APC* S1465. In some embodiments, each PCR reaction includes: 10µL of PCR reaction mix, 10µL of primer mix, and 20 µL DNA (*e.g.,* 20 µL of a 0.5 ng/µL DNA Stock, for a total of 10 ng DNA). In some embodiments, multiple PCR reactions are performed using the same temperature cycling conditions.

### Mutations

In some embodiments, the methods of the present disclosure include amplifying the loci of one or more mutations in a *KRAS* gene. As used herein in reference to the *KRAS, BRAF, CTNNB1,* and *APC* genes, amplifying the locus of a DNA mutation encompasses amplifying either the mutant locus or the corresponding wild-type locus. It will be appreciated that in most instances, while four or more mutations are screened in a multiplex assay, any individual sample will typically include at most one of the mutations being screened. *KRAS* encodes the KRAS proto-oncogene, a small GTPase frequently mutated in human cancers, also known as the Kirsten rag sarcoma viral oncogene homolog, PR310 c-K-ras oncogene, c-Ki-ras, c-Kirsten-ras, K-Ras2, K-ras p21, GTPase KRas, cellular c-Ki-ras2 proto-oncogene, cellular transforming proto-oncogene, oncogene KRAS2, transforming protein p21, and v-Ki-ras2 Kirsten rat sarcoma 2 viral oncogene homolog. In some embodiments, the *KRAS* gene is a human *KRAS* gene. In some embodiments, a human *KRAS* gene refers to the gene described by NCBI Entrez Gene ID No. 3845, including mutants and variants thereof. In other embodiments, the *KRAS* gene is from one of the following organisms: mouse (*see, e.g.,* NCBI Entrez Gene ID No. 16653), rat (*see, e.g.,* NCBI Entrez Gene ID No. 24525), cynomolgus monkey (*see, e.g.,* NCBI Entrez Gene ID No. 102131483), fish (*see, e.g.,* NCBI Entrez Gene ID No. 445289), dog (*see, e.g.,* NCBI Entrez Gene ID No. 403871), cattle (*see, e.g.,* NCBI Entrez Gene ID No. 541140), horse (*see, e.g.,* NCBI Entrez Gene ID No. 100064473), chicken (*see, e.g.,* NCBI Entrez Gene ID No. 418207), chimpanzee (*see, e.g.,* NCBI Entrez Gene ID No. 473387), rhesus monkey (*see, e.g.,* NCBI Entrez Gene ID No. 707977), or cat (*see, e.g.,* NCBI Entrez Gene ID No. 751104).

A variety of *KRAS* mutations associated with cancer are known and may be suitably detected by the methods described herein; *see, e.g.,* Prior, I.A. et al. (2012) Cancer Res. 72:2457-67. For example, one study found that 30% of colorectal tumors in their cohort had *KRAS* mutations, predominantly at amino acid 12 (Smith, G. et al. (2002) Proc. Natl. Acad. Sci. 99:9433-8). In some embodiments described herein, a *KRAS* mutation is named based on the resulting amino acid substitution/deletion/frameshift according to a human KRAS protein, *e.g.,* as set forth in MTEYKLVVVGAGGVGKSALTIQLIQNHFVDEYDPTIEDSYRKQVVIDGETCLLDILDT AGQEEYSAMRDQYMRTGEGFLCVFAINNTKSFEDIHHYREQIKRVKDSEDVPMVLVG NKCDLPSRTVDTKQAQDLARSYGIPFIETSAKTRQGVDDAFYTLVREIRKHKEKMSKD GKKKKKKSKTKCVIM (SEQ ID NO:62). An exemplary human *KRAS* cDNA sequence is set forth in TGTGCTCGGAGCTCGATTTTCCTAGGCGGCGGCCGCGGCGGCGGAGGCAGCAGCGGCGGCGGCAGTGGCGGCGGCG AAGGTGGCGGCGGCTCGGCCAGTACTCCCGGCCCCCGCCATTTCGGACTGGGAGCGAGCGCGGCGCAGGCACTGAA GGCGGCGGCGGGGCCAGAGGCTCAGCGGCTCCCAGGCCTGCTGAAAATGACTGAATATAAACTTGTGGTAGTTGGA GCTGGTGGCGTAGGCAAGAGTGCCTTGACGATACAGCTAATTCAGAATCATTTTGTGGACGAATATGATCCAACAA TAGAGGATTCCTACAGGAAGCAAGTAGTAATTGATGGAGAAACCTGTCTCTTGGATATTCTCGACACAGCAGGTCA AGAGGAGTACAGTGCAATGAGGGACCAGTACATGAGGACTGGGGAGGGCTTTCTTTGTGTATTTGCCATAAATAAT ACTAAATCATTTGAAGATATTCACCATTATAGAGAACAAATTAAAAGAGTTAAGGACTCTGAAGATGTACCTATGG TCCTAGTAGGAAATAAATGTGATTTGCCTTCTAGAACAGTAGACACAAAACAGGCTCAGGACTTAGCAAGAAGTTA TGGAATTCCTTTTATTGAAACATCAGCAAAGACAAGACAGAGAGTGGAGGATGCTTTTTATACATTGGTGAGAGAG ATCCGACAATACAGATTGAAAAAAATCAGCAAAGAAGAAAAGACTCCTGGCTGTGTGAAAATTAAAAAATGCATTA TAATGTAATCTGGGTGTTGATGATGCCTTCTATACATTAGTTCGAGAAATTCGAAAACATAAAGAAAAGATGAGCA AAGATGGTAAAAAGAAGAAAAAG (SEQ ID NO:66). In some embodiments, a DNA mutation results in the mutation of G12 or G13 according to SEQ ID NO:62 or SEQ ID NO:66. For example, in some embodiments, a DNA mutation in a *KRAS* gene encodes or results in a G12D, G12V, G12S, or G13D mutated KRAS protein (numbering according to SEQ ID NO:62). These DNA mutations are also described by their nucleotide positions (rather than mutated polypeptide codons) in Table A *infra.* For example, in some embodiments, a DNA mutation in a *KRAS* gene results in a c.35G>A, c.35G>T, c.34G>A, or c.38G>A mutation in the corresponding cDNA sequence of SEQ ID NO:66.

Since, as described *supra,* the sequences of a variety of *KRAS* genes and corresponding mutations are known, one of ordinary skill in the art may suitably select a primer pair to amplify the locus of one or more of these DNA mutation(s). Various factors influencing primer design are known, and tools for identifying primer pairs suitable for amplifying a given DNA template sequence are available (*see, e.g.,* www.ncbi.nlm.nih.gov/tools/primer-blast/). In certain embodiments, a primer pair for amplifying the locus of a *KRAS* mutation (*e.g*., encoding or resulting in a G12D, G12V, G12S, or G13D mutated KRAS protein) comprises the sequences GTACTGGTGGAGTATTTGATAGTG (SEQ ID NO:1) and ATCGTCAAGGCACTCTTGCCTAC (SEQ ID NO:2), respectively.

In some embodiments, the methods of the present disclosure include amplifying the loci of one or more mutations in a *BRAF* gene. *BRAF* encodes the BRAF proto-oncogene, a serine/threonine kinase frequently mutated in human cancers, also known as B-Raf, BRAF1, B-RAF1, RAFB1, NS7, 94kDa B-raf protein, p94, murine sarcoma viral (v-raf) oncogene homolog B1, v-raf murine sarcoma viral oncogene homolog B, and v-raf murine sarcoma viral oncogene homolog B1. In some embodiments, the *BRAF* gene is a human *BRAF* gene. In some embodiments, a human *BRAF* gene refers to the gene described by NCBI Entrez Gene ID No. 673, including mutants and variants thereof. In other embodiments, the *BRAF* gene is from one of the following organisms: mouse (*see, e.g.,* NCBI Entrez Gene ID No. 109880), rat (*see, e.g.,* NCBI Entrez Gene ID No. 114486), cynomolgus monkey (*see, e.g.,* NCBI Entrez Gene ID No. 101866436), fish *(*s*ee, e.g.,* NCBI Entrez Gene ID No. 403065), dog (*see, e.g.,* NCBI Entrez Gene ID No. 475526), cattle (*see, e.g.,* NCBI Entrez Gene ID No. 536051), horse (*see, e.g.,* NCBI Entrez Gene ID No. 100065760), chicken (*see, e.g.,* NCBI Entrez Gene ID No. 396239), chimpanzee (*see, e.g.,* NCBI Entrez Gene ID No. 463781), rhesus monkey (*see, e.g.,* NCBI Entrez Gene ID No. 693554), or cat (*see, e.g.,* NCBI Entrez Gene ID No. 101092346).

A variety of *BRAF* mutations associated with cancer are known and may be suitably detected by the methods described herein; *see, e.g.,* Davies, H. et al. (2002) Nature 417:949-54; and Garnett, M.J. and Marais, R. (2004) Cancer Cell 6:313-9. For example, one review has described that *BRAF* mutations have been found in 9-14% of patients with colon and rectal cancers, are associated with poor overall survival, and are found in over 50% of microsatellite instability (MSI) high tumors (Clarke, C.N. and Kopetz, E.S. (2015) J. Gastrointest. Oncol. 6:660-7). In some embodiments described herein, a *BRAF* mutation is named based on the resulting amino acid substitution/deletion/frameshift according to a human BRAF protein, *e.g.,* as set forth in MAALSGGGGGGAEPGQALFNGDMEPEAGAGAGAAASSAADPAIPEEVWNIKQMIKL TQEHIEALLDKFGGEHNPPSIYLEAYEEYTSKLDALQQREQQLLESLGNGTDFSVSSSA SMDTVTSSSSSSLSVLPSSLSVFQNPTDVARSNPKSPQKPIVRVFLPNKQRTVVPARCG VTVRDSLKKALMMRGLIPECCAVYRIQDGEKKPIGWDTDISWLTGEELHVEVLENVPL TTHNFVRKTFFTLAFCDFCRKLLFQGFRCQTCGYKFHQRCSTEVPLMCVNYDQLDLLF VSKFFEHHPIPQEEASLAETALTSGSSPSAPASDSIGPQILTSPSPSKSIPIPQPFRPADEDH RNQFGQRDRSSSAPNVHINTIEPVNIDDLIRDQGFRGDGGSTTGLSATPPASLPGSLTNV KALQKSPGPQRERKSSSSSEDRNRMKTLGRRDSSDDWEIPDGQITVGQRIGSGSFGTVY KGKWHGDVAVKMLNVTAPTPQQLQAFKNEVGVLRKTRHVNILLFMGYSTKPQLAIV TQWCEGSSLYHHLHIIETKFEMIKLIDIARQTAQGMDYLHAKSIIHRDLKSNNIFLHEDL TVKIGDFGLATVKSRW SGSHQFEQLSGSILWMAPEVIRMQDKNPYSFQSDVYAFGIVL YELMTGQLPYSNINNRDQIIFMVGRGYLSPDLSKVRSNCPKAMKRLMAECLKKKRDE RPLFPQILASIELLARSLPKIHRSASEPSLNRAGFQTEDFSLYACASPKTPIQAGGYGAFP VH (SEQ ID NO:63). An exemplary human *BRAF* cDNA sequence is set forth in GTTATAAGATGGCGGCGCTGAGCGGTGGCGGTGGTGGCGGCGCGGAGCCGGGCCAGGCTCTGTTCAACGGGGACAT GGAGCCCGAGGCCGGCGCCGGCGCCGGCGCCGCGGCCTCTTCGGCTGCGGACCCTGCCATTCCGGAGGAGGTGTGG AATATCAAACAAATGATTAAGTTGACACAGGAACATATAGAGGCCCTATTGGACAAATTTGGTGGGGAGCATAATC CACCATCAATATATCTGGAGGCCTATGAAGAATACACCAGCAAGCTAGATGCACTCCAACAAAGAGAACAACAGTT ATTGGAATCTCTGGGGAACGGAACTGATTTTTCTGTTTCTAGCTCTGCATCAATGGATACCGTTACATCTTCTTCC TCTTCTAGCCTTTCAGTGCTACCTTCATCTCTTTCAGTTTTTCAAAATCCCACAGATGTGGCACGGAGCAACCCCA AGTCACCACAAAAACCTATCGTTAGAGTCTTCCTGCCCAACAAACAGAGGACAGTGGTACCTGCAAGGTGTGGAGT TACAGTCCGAGACAGTCTAAAGAAAGCACTGATGATGAGAGGTCTAATCCCAGAGTGCTGTGCTGTTTACAGAATT CAGGATGGAGAGAAGAAACCAATTGGTTGGGACACTGATATTTCCTGGCTTACTGGAGAAGAATTGCATGTGGAAG TGTTGGAGAATGTTCCACTTACAACACACAACTTTGTACGAAAAACGTTTTTCACCTTAGCATTTTGTGACTTTTG TCGAAAGCTGCTTTTCCAGGGTTTCCGCTGTCAAACATGTGGTTATAAATTTCACCAGCGTTGTAGTACAGAAGTT CCACTGATGTGTGTTAATTATGACCAACTTGATTTGCTGTTTGTCTCCAAGTTCTTTGAACACCACCCAATACCAC AGGAAGAGGCGTCCTTAGCAGAGACTGCCCTAACATCTGGATCATCCCCTTCCGCACCCGCCTCGGACTCTATTGG GCCCCAAATTCTCACCAGTCCGTCTCCTTCAAAATCCATTCCAATTCCACAGCCCTTCCGACCAGCAGATGAAGAT CATCGAAATCAATTTGGGCAACGAGACCGATCCTCATCAGCTCCCAATGTGCATATAAACACAATAGAACCTGTCA ATATTGATGACTTGATTAGAGACCAAGGATTTCGTGGTGATGGAGCCCCTTTGAACCAGCTGATGCGCTGTCTTCG GAAATACCAATCCCGGACTCCCAGTCCCCTCCTACATTCTGTCCCCAGTGAAATAGTGTTTGATTTTGAGCCTGGC CCAGTGTTCAGAGGATCAACCACAGGTTTGTCTGCTACCCCCCCTGCCTCATTACCTGGCTCACTAACTAACGTGA AAGCCTTACAGAAATCTCCAGGACCTCAGCGAGAAAGGAAGTCATCTTCATCCTCAGAAGACAGGAATCGAATGAA AACACTTGGTAGACGGGACTCGAGTGATGATTGGGAGATTCCTGATGGGCAGATTACAGTGGGACAAAGAATTGGA TCTGGATCATTTGGAACAGTCTACAAGGGAAAGTGGCATGGTGATGTGGCAGTGAAAATGTTGAATGTGACAGCAC CTACACCTCAGCAGTTACAAGCCTTCAAAAATGAAGTAGGAGTACTCAGGAAAACACGACATGTGAATATCCTACT CTTCATGGGCTATTCCACAAAGCCACAACTGGCTATTGTTACCCAGTGGTGTGAGGGCTCCAGCTTGTATCACCAT CTCCATATCATTGAGACCAAATTTGAGATGATCAAACTTATAGATATTGCACGACAGACTGCACAGGGCATGGATT ACTTACACGCCAAGTCAATCATCCACAGAGACCTCAAGAGTAATAATATATTTCTTCATGAAGACCTCACAGTAAA AATAGGTGATTTTGGTCTAGCTACAGTGAAATCTCGATGGAGTGGGTCCCATCAGTTTGAACAGTTGTCTGGATCC ATTTTGTGGATGGCACCAGAAGTCATCAGAATGCAAGATAAAAATCCATACAGCTTTCAGTCAGATGTATATGCAT TTGGAATTGTTCTGTATGAATTGATGACTGGACAGTTACCTTATTCAAACATCAACAACAGGGACCAGATAATTTT TATGGTGGGACGAGGATACCTGTCTCCAGATCTCAGTAAGGTACGGAGTAACTGTCCAAAAGCCATGAAGAGATTA ATGGCAGAGTGCCTCAAAAAGAAAAGAGATGAGAGACCACTCTTTCCCCAAATTCTCGCCTCTATTGAGCTGCTGG CCCGCTCATTGCCAAAAATTCACCGCAGTGCATCAGAACCCTCCTTGAATCGGGCTGGTTTCCAAACAGAGGATTT TAGTCTATATGCTTGTGCTTCTCCAAAAACACCCATCCAGGCAGGGGGATATGGAGAATTTGCAGCCTTCAAGTAG CCACCATCATGGCAGCATCTGCTCTTATTTCTTAAGTCTTGTGTTCGTACAATTTGTTAACATCAAAACACAGTTC TGTTCCTCAAATCTTTTTTTAAAGATACAAAATTTCCAATGCATAAGCTGATGTGGAACAGAATGGAATTTCCCAT CCAACAAAAGAGGAAAGAATGTTTTAGGAACCAGAATTCTCTGCTGCCAGTGTTTCTTCAACAAAAATACCACGAG CATACAAGTCTGCCCAGTCCCAGGAAGAAAGAGGAGAGACCCTGAATTCTGACCTTTTGATGGTCAGGCATGATGG AAAGAAACTGCTGCTACAGCTTGGGAGATTTGCTATGGAAAGTCTGCCAGTCAACTTTGCCCTTCTAACCACCAGA TCAATTTGTGGCTGATCATCTGATGGGGCAGTTTCAATCACCAAGCATCGTTCTCTTTCCTGTTCTGGAATTTTGT TTTGGAGCTCTTTCCCCTAGTGACCACCAGTTAGTTTCTGAGGGATGGAACAAAAATGCAGCTTGCCCTTTCTATG TGGTGCGTGTTCAGGCCTTGACAGATTTTATCAAAAGGAAACTATTTTATTTAAATGGAGGCTGAGTGGTGAGTAG ATGTGTCTTGGTATGGAGGAAAAGGGCATGCTGCATCTTCTTCCTGACCTCCGGGGTCTCTGGCCTTTTGTTTCCT TGCTCACTGAGGGGTCTGTCTAACCAAGCAGGCTAGATAGTGCTGGCACACATTGCCTTCTTTCTCATTGGGTCCA GCAATGAAGATAAGTGTTTGGGTTTTTTTTTTTTCCTCCACAATGTAGCAAATTCTCAGGAAATACAGTTTATATC TTCCTCCTATGCTCTTCCAGTCACCAACTACTTATGCGGCTACTTTGTCCAGGGCACAAAATGCCGTGGCAGTATC TAACTAAACCCCCACAAAACTGCTTAATAACAGTTTTGAATGTGAGAAATTTAGATAATTTAAATATAAGGTACAG GTTTTAATTTCTGAGTTTCTTCTTTTCTATTTTTATTAAAAAGAAAATAATTTTCAGATTTAATTGAATTGGAAAA AAACAATACTTCCCACCAGAATTATATATCCTGAAAATTGTATTTTTGTTATATAAACAACTTTTAAGAAAGATCA TTATCCTTTTCTCTACCTAAATATGAGGAGTCTTAGCATAATGACAAATATTTATAATTTTTCAATTAATGGTACT TGCTGGATCCACACTAACATCTTTGCTAATAATCTCATTGTTTCTTCCAACTGATTCCTAACACTATATCCCACAT CTTCTTTCTAGTCTTTTATCTAGAATATGCAACCTAAAATAAAAATGGTGGCGTCTCCATTCATTCTCCTTCTTCC TTTTTTCCCAAGCCTGGTCTTCAAAAGGTTGGGCAATTTGGCAGCTGAATTCCCAGACAGAGAATAGAGCAATTTT AGGGATATTAGGACTGAGGGAGGGTGTGGGAAAGCTGTCATCAGTTGTTTTTATAGAAAGAACTGGCATTCATTAA GAACCTAAATCTTATCTTTGCACAAATGGAAAATATAACCTAGTTATAGCTTCCTTTGGCCTTTATTAAAGGGTAA TATCAATCACAGTCATAGCAAAGAAAGCGGATGTATTAATGGCAAATTAATGGAAAACCTCCCTTATCAGGAATCT AGACTCAGAATTTAGGAACACAAATCAAATCAGACCAACCAAGCTATAGCCAAGGACTTGAAAGAAATTAAACAAG ACCCAGAATAAATCAAGGAATTAGAAATTGTTATTTAAAAATTTCAGATTGTAACTCCAGGCCCTGCTGTCTATAT TGCAGCCACTAAAAGCTCACTACCATTAGATTTTTGCTAACATACATGTATTCAGAAGAAAGCCTATTGAAATTTT CATTGTCTTGTAAAAGGTTGTCCTAGTAAAATGGAAAAGATCCTTAAGTTATTAATCAGTTTGAAAAGCAAATTTG TTTTTAAGTTTTACATCAGCAGGGCAGTGTCTTACAAAATTCAGAAATTGCAAAGGTGGAAATAATTCACGCTGAT TTGAAGAACATCTTCTGTGCAATAATACTGCCTCTCTTGAAAAGCATTGGCTGTTTTTTCTTTTTAAATATATCTC TAGATGCTTTTAAATGTGGCTGTGTTCCCTTTACCAAGATTGGCTTCAAGTTTCCGCAGGTAGAGAGACCTGGGCT TGAACAAGAGGATGTGTTTCATGTCCTGCTGAGGAGGTAGAACATGTGCAGCCTGGGTCCGGGACTGCCTCCGTGG GGCAGGGGCAGGGGCGGTACCATTAGGGAGGAAGCTTAGCATTTCAGTTTCTTAAACAATATTCAGGGTGATACAC TTTTTCTTCCCTTGCATTTTAGAATAGGCTGGTATCTCATTTGAACGGGGGAGCAGACTTGATCTCAAATGAAGCT GTGCCCAGGAGCCAGGCTTAGCATATTGAGATTTTTATAGATACCTTAAAAAATAAAATATTTAAACCTCTCTTTT CTTCCTTTTTCTATGAAATAGGTTTTTTCTCTAGTTTACAAATGACATGAAAATAGGTTTTATTTGTGTTTTATCT GCTTTATTTTTTGATGCTTAGACAACAGTTAGACTTACTGAGCTCCTAAAAAAACGAGGAAGAAGTCCTTATTTGT GAAAAGCACTTTATGAGTAATTGTATAGACAGTATGTGGCTGCGTCACTGATCATCTTGTAAGGGTGTAACAGTCT TGTCTGTAAAGTGGCTGCAGTGCCTTCTGTAGTGTGTTTTATTTTTGGTAGGGAGAGGTGAAGCCTTCTGAAAAAT TTGAGAGCAACTACAGAGGATTGTTTGTAACTGTGTAGTATTCCTGATGGACTTTTTTCATCGTTAGAGTCAAGGA CCTAGACTTTTGCCACTGAAATAATATTGACCAAAAAAATAGTTTATAAAAGGGATTTGTGAATAGAAAATTCAGT GTGATCATTTGTTGTTAATGTGCACCTTAAAAGAAGATTCTGTCTAGCTGTCAAATTCTGGTTCCCGAATATCTCA CCCCTGATTGTATTTGAGATCTAGTAGGGCATACTGGGGCATTTTAGAAGATAAAATCCCATACAAATGATATATG CTATATTTATGTTGGTGTTGGAGAAGAAAGAGCAGTATATAAAGAAATAATTCAAGACTGCAGCACTGTCAACCTG AAACTTTGTAAATATTTCCTAGCTTCTGGTTTGGTGCGGTGACAGCACTTTCATCACAGGATGTTACCTTGTATTC ACCAGGCGGAGTGCGAGCTGCTGCACATCCTCCTCAGATCTCACCTGTCCCCACTGTACATCCACCCGCCAGCTGC TTGCAAACCTCATCTCTAGCTTTAGTTCGAAACCACATTGCAGGGTTCAGGTGACCTCTACAAAAAACTACCTCTT CAGAATGAGGTAATGAATAGTTATTTATTTTAAAATATGAAAAGTCAGGAGCTCTAGAACATGACGATGATTTAAG ATTTTAACTTTTTTGTGTACTTGTATTTGAGCACTCTCATTTTGTCCTAAAGGGCATTATACATTTAAGCAGTAAT ACTGTAAAAAAATGTGTTGCTCGGAATATCTGAATGTTGTTGAAAGTGGTGCCAGAACCGGTTTAGGGGTACGTTT CAGAATCTTAACCTTGAGTCAATTGCATGAAATTAAATAGCTGTGGTATCACTTCACTAACAGTGATGTAATTTTA ATTTTCAGTAGGCTTGGCATGACAGTACATCCTCATAATGAGTTTGCTGCAGCTTTGTCACATGCACAGGCATTCA TAGAAAGACCACCCAGCTAAGAGGGTAGAATGATTACTCTTTTTGCAAGATTCTCTTCTTTGTCCAAGTTGGCATT GTTAGTGCTAGGAATACCAGCACCTTGAGACGAGCAGATTCCAACCATTAGGCTATAAACACCATAGCCAGAGATG GAAGGTTTACTGTGAGTATGAACAGCAAATAGCTTACAGGTCATGAGTTGAAATGGTGTAGGTGAGGCTCTAGAAA AATACCTTGACAATTTGCCAAATGATCTTACTGTGCCTTCATGATGCAATAAAAAAGCTAACATTTTAGCAGAAAT CAGTGATTTGTGAAGAGAGCAGCCACTCTGGTTTAACTCAGCTGTGTTAATAATTTTTAGAGTGCAATTTAGACTG CATAGGTAAATGCACTAAAGAGTTTATAGCCAAAATCACATTTAACAATGAGAAAACACACAGGTAAATTTTCAGT GAACAAAATTATTTTTTTAAAGCACATAATCCCTAGTATAGTCAGATATATTTATCACATAGAGCAACTAGGTTGC AAATATAGTTCAGTGACATTTCTAGAGAAACTTTTTCTACTCCCATAGGCTCTTCAAAGCATGGAACTTTTATACA ACAGAAATGTTGACAGAAATTGCTGTAGTTTAGGGTTGAAGTACTGTATGATGGGCAGCAATCATGTATTAACTTA GAAGGGGAAATTGAAATATAGGACCGAATTTGGTTTTATCAGTTTCCAGAGTACTGCTGCCAACCTAGACACTGAT TTTTCAGAGTTTGAAATGTAAATTTCTTCCCGGGACTTGATTGCACATGAAGCTGGACTGCGTTAGTCATCCTGTC CCAAAGCGCTGTGGGGGCCAGGGTGGAGGTCTCAAGGCATCCTTTATGACCTGGCCATTGGATGTAAAAGAAAACA TATTCCATGCTGTGGTTCTTGTATCTTGTTTCATTCCTCACCATTGAAAGAGAAAGTCCATGTATTGTCTCCAGCA CATCCTTGAAATGTTATACTGGGATGGATTACTGATGCCCATCGGTAGTTGAGCCCCAGAAGAGGGTAGTAGCATC TCTGCCTCAGGTGATGATTTGTAGCTTGGCCAGAGGAGAGCGGAGTCACCAGTATATCTGTGGTCCATGTTGCTAG CTCTGGTAAAATTAAAAATACTGGTAAGATGTTTGTTTTATTAGTACACTAGACAGTAAGCTCTGTTTTGTTGTTT TCAAATAACCTATTTTCACTTTTGTTTGGGCAAAGACATTTAAATTGAAATTCAATTCTAATTTTTGTTAATTGTG GAAAGGGTAATTAACAGTTCCTATCAGGTATTTTTAATGTGGAAAAGGACAGAAACCCAACTCCTAAAATCTTAAA TTAAGGTAACAGTGCTTTAAAAAAAAAAAATGCATGGGGCAATTAGTCGGCAACTCAATGAGTGACTAAAGTACTT TTATTTAACATCCACAACTTCAACTGTTAAGTTTTATTAATTACTAAATCAGCTTTATTAAAATGTTGACATTTAT TTAGCTATTTTGAATAATTATAGTGACTTGACGAGTGTGTATGAGGACACAGCCAATGTAAGCCAGTGTATCCATT TTTTAGAGGTGCATTTTTTTTTAAAGAATTCTGTAGATAGAAGTGCTCTGAAAACAACTAAAATATGTTTATTCAT GGTAGTATCAAAAAATGTTTGTACAAACCATCTGCTTCTCCCGGCCAGCCGAGTTCATTCTCCAGCACCGTGACCG CTGGTTCTCATGTACAGCACATATGCGGGAGAGTTGGCAGAAAATTTGTGAAGAGATGCCGCAAAGGAAGGGTCTG TTGACGGGTGGGATTGGGGGTTTTGATGAAGTTGCTTAGTCCTGGTTTTGTTTTGAAAATTACTGCGTTGCATTTT TGTGTTAAGTTTTTGAACCCACGTGTGTTTTGGTGGAGTATGAGTTGGAAGTCACTGCAAACTAGCATAAACAACA AAGCTCACAGAGTAGGCACAGATGTAGAGAACAGAGACCAAAATGGGGTGAGGTGGCAGTAAATCTAGGATAGGGA AAAATTAATGTGAGGGTGGGAAATAAACTGTAATTACCTGAAATCAAATGTAAGAGTGCAATAAGTATGCTTTTTA TTCTAAGCTGTGAACGGTTTTTTTAAGAATCATTCCTTCCTAATACATTTGTGTATGTTCCATAGCTGATTAAAAC CAGCTATATCAACATATAATGCCTTTTTATTCATGTTAATGACCAACGTAAGTGGCTAGCCTTTATGTCTTATTTA TCTTCATGTTATGTTAGTTTACATACAGGGGTGTATGTCTCTGTGCTGTCCCCTTCTCCTGCCTTCATTTTAAAAT GCATCCATGGGTCCTCCGTGTTTCCTTTGGCCATGCCACATATATAGACTCAGTTTGGCCTTCATGATATCGCCTG ATTTTTGAGGACTGTATCACAGTGATATGTATTTGTGGTAATCTCATTTGTTGGTTGTACATCTGATCCTTTCCTC AACATGGCAATTGCTGCCTTTCCTAAGATAGGATCATACAACTGATCAGGGGATTGAATTTGATCATTCATCAACA TGTGTCTCTGAATTTTATTCAGTAGTTGTCATTGCTCTTTGGTTTAGACCAAGAAAAAGGAAATCCCCCCTTTTCA TGTATTCCTTGGTTTGAGGACATGACTCCTGTAAGGGAGAGGAAAGGGAGATGCTTCCTGTTTGAACTGCAGTGAA TTCACGGTTCCTGTTTCACCACTCCAAACCTTATGGCGACTCACACACACATTCCTCTTTTCTGTTACTGCCAAAG GTTCGGGTTTAGTACACTTCAGTTCCACTCAAGCATTGAAAAGGTTCTCGTGGAGTCTGGGGCGTGCCCAGTGAAA AGATGGGGACTTTTTAATTGTCCACAGACCTCTCTATACCTGCTTTGCAAAAATTACAATGGAGTAACTATTTTTA AAGCTTATTTTTCAATTCATAAAAAAGACATTTATTTTCAGTCAAATGGATGATGTCTCCCTCTTTTCCCCTATTC TCAATGTTTGCTTGAATCTTTTATTATTTTTTTTAATTCTCCCCCATACCCACTTCCTGATACTTTGGTTCTCTTT CCTGCTCAGGTCCCTTCATTTGTACTTTGGAGTTTTTCTCATGTAAATTTGTATAACAGAAAATATTGTTCAGTTT GGATAGAAAGCATGGAGAATAAAAAAAGATAGCTGAAATTCAGATTGAAGAAATTTATTTCTGTGTAAAGTTATTT AAAAACTGTATTATATAAAAGGCAAAAAAAGTTCTATGTACTTGATGTGAATATGCGAATACTGCTATAATAAAGA TTGACTGCATGGAGAAGTC (SEQ ID NO:67). In some embodiments, a DNA mutation results in the mutation of V600 according to SEQ ID NO:63 or SEQ ID NO:67. For example, in some embodiments, a DNA mutation in a *BRAF* gene encodes or results in a V600E mutated BRAF protein (numbering according to SEQ ID NO:63). These DNA mutations are also described by their nucleotide positions (rather than mutated polypeptide codons) in Table A *infra.* It is to be appreciated that some references to the V600E BRAF mutation refer to it as V599E due to an early, incorrect BRAF protein sequence that was missing a codon at approximately amino acid 31 (*see* Garnett, M.J. and Marais, R. (2004) Cancer Cell 6:313-9 for description). For example, in some embodiments, a DNA mutation in a *BRAF* gene results in a c.1799T> A or c.1799 _1800TG>AA mutation in the corresponding cDNA sequence of SEQ ID NO:67.

Since, as described *supra,* the sequences of a variety of *BRAF* genes and corresponding mutations are known, one of ordinary skill in the art may suitably select a primer pair to amplify the locus of one or more of these DNA mutation(s). Various factors influencing primer design are known, and tools for identifying primer pairs suitable for amplifying a given DNA template sequence are available (*see, e.g.,* www.ncbi.nlm.nih.gov/tools/primer-blast/). In some embodiments, a primer pair for amplifying the locus of a *BRAF* mutation (*e.g.,* encoding or resulting in a V600E mutated BRAF protein) comprises the sequences GGACCCACTCCATCGAGATTT (SEQ ID NO:8) and CAGATATATTTCTTCATGAAGACCTCACAGTAA (SEQ ID NO:9), respectively.

In some embodiments, the methods of the present disclosure include amplifying the loci of one or more mutations in a *CTNNB1* gene. *CTNNB1* encodes the CTNNB1 beta catenin 1 protein, a subunit of the cadherin protein complex that transduces Wnt signaling and is frequently mutated in human cancers, also known as CTNNB, MRD19, Armadillo, catenin (cadherin-associated protein) beta 1, and catenin (cadherin-associated protein) beta 1 88kDa. In some embodiments, the *CTNNB1* gene is a human *CTNNB1* gene. In some embodiments, a human *CTNNB1* gene refers to the gene described by NCBI Entrez Gene ID No. 1499, including mutants and variants thereof. In other embodiments, the *CTNNB1* gene is from one of the following organisms: mouse (*see, e.g.,* NCBI Entrez Gene ID No. 12387), rat (*see, e.g.,* NCBI Entrez Gene ID No. 84353), cynomolgus monkey (*see, e.g.,* NCBI Entrez Gene ID No. 102146984), fish (*see, e.g.,* NCBI Entrez Gene ID No. 30265), dog (*see, e.g.,* NCBI Entrez Gene ID No. 477032), cattle (*see, e.g.,* NCBI Entrez Gene ID No. 539003), horse (*see, e.g.,* NCBI Entrez Gene ID No. 100055241), chicken (*see, e.g.,* NCBI Entrez Gene ID No. 395964), chimpanzee (*see, e.g.,* NCBI Entrez Gene ID No. 450183), rhesus monkey (*see, e.g.,* NCBI Entrez Gene ID No. 574265), or cat (*see, e.g.,* NCBI Entrez Gene ID No. 101097342).

A variety of *CTNNB1* mutations associated with cancer are known and may be suitably detected by the methods described herein; *see, e.g.,* Morin, P.J. et al. (1997) Science 275:1787-90 and Polakis, P. (2000) Genes Dev. 14:1837-51. For example, *CTNNB1* mutations resulting in amino acid changes at positions T41 and S45 of the human CTNNB1 protein have been found in both colorectal adenomas and carcinomas, particularly in tumors that lack *APC* mutations (Sparks, A.B. et al. (1998) Cancer Res. 58:1130-4). In some embodiments described herein, a *CTNNB1* mutation is named based on the resulting amino acid substitution/deletion/frameshift according to a human CTNNB 1 protein, *e.g.,* as set forth in MATQADLMELDMAMEPDRKAAVSHWQQQSYLDSGIHSGATTTAPSLSGKGNPEEED VDTSQVLYEWEQGFSQSFTQEQVADIDGQYAMTRAQRVRAAMFPETLDEGMQIPSTQ FDAAHPTNVQRLAEPSQMLKHAVVNLINYQDDAELATRAIPELTKLLNDEDQVVVNK AAVMVHQLSKKEASRHAIMRSPQMVSAIVRTMQNTNDVETARCTAGTLHNLSHHRE GLLAIFKSGGIPALVKMLGSPVDSVLFYAITTLHNLLLHQEGAKMAVRLAGGLQKMV ALLNKTNVKFLAITTDCLQILAYGNQESKLIILASGGPQALVNIMRTYTYEKLLWTTSR VLKVLSVCSSNKPAIVEAGGMQALGLHLTDPSQRLVQNCLWTLRNLSDAATKQEGM EGLLGTLVQLLGSDDINVVTCAAGILSNLTCNNYKNKMMVCQVGGIEALVRTVLRAG DREDITEPAICALRHLTSRHQEAEMAQNAVRLHYGLPVVVKLLHPPSHWPLIKATVGL IRNLALCPANHAPLREQGAIPRLVQLLVRAHQDTQRRTSMGGTQQQFVEGVRMEEIVE GCTGALHILARDVHNRIVIRGLNTIPLFVQLLYSPIENIQRVAAGVLCELAQDKEAAEAI EAEGATAPLTELLHSRNEGVATYAAAVLFRMSEDKPQDYKKRLSVELTSSLFRTEPMA WNETADLGLDIGAQGEPLGYRQDDPSYRSFHSGGYGQDALGMDPMMEHEMGGHHP GADYPVDGLPDLGHAQDLMDGLPPGDSNQLAWFDTDL (SEQ ID NO:64). An exemplary human *CTNNB1* cDNA sequence is set forth in AAGTCCCATCAGTCCTGGGGATCGGACCAGTGGACTTTCTCTTAAGATTTCCTCTTTCATTCTTAAGAATAGAAGT GTTATTATTTTTTTTAATGCCCTGGCTATGTGAGTTTGAATCGAAGCAACTTTAAACCTTAGAGCAACTAAACTCT AAGTGCAGCGGGTGCGATGCGTCAGTAGGGTGAGCACATAAAAAATCCATGTCTTGCACCTGTATTTTAGCGTACT ATGCAGGGTATTTGAAGTATACCATACAACTGTTTTGAAAATCCAGCGTGGACAATGGCTACTCAAGCTGATTTGA TGGAGTTGGACATGGCCATGGAACCAGACAGAAAAGCGGCTGTTAGTCACTGGCAGCAACAGTCTTACCTGGACTC TGGAATCCATTCTGGTGCCACTACCACAGCTCCTTCTCTGAGTGGTAAAGGCAATCCTGAGGAAGAGGATGTGGAT ACCTCCCAAGTCCTGTATGAGTGGGAACAGGGATTTTCTCAGTCCTTCACTCAAGAACAAGTAGCTGATATTGATG GACAGTATGCAATGACTCGAGCTCAGAGGGTACGAGCTGCTATGTTCCCTGAGACATTAGATGAGGGCATGCAGAT CCCATCTACACAGTTTGATGCTGCTCATCCCACTAATGTCCAGCGTTTGGCTGAACCATCACAGATGCTGAAACAT GCAGTTGTAAACTTGATTAACTATCAAGATGATGCAGAACTTGCCACACGTGCAATCCCTGAACTGACAAAACTGC TAAATGACGAGGACCAGGTGGTGGTTAATAAGGCTGCAGTTATGGTCCATCAGCTTTCTAAAAAGGAAGCTTCCAG ACACGCTATCATGCGTTCTCCTCAGATGGTGTCTGCTATTGTACGTACCATGCAGAATACAAATGATGTAGAAACA GCTCGTTGTACCGCTGGGACCTTGCATAACCTTTCCCATCATCGTGAGGGCTTACTGGCCATCTTTAAGTCTGGAG GCATTCCTGCCCTGGTGAAAATGCTTGGTTCACCAGTGGATTCTGTGTTGTTTTATGCCATTACAACTCTCCACAA CCTTTTATTACATCAAGAAGGAGCTAAAATGGCAGTGCGTTTAGCTGGTGGGCTGCAGAAAATGGTTGCCTTGCTC AACAAAACAAATGTTAAATTCTTGGCTATTACGACAGACTGCCTTCAAATTTTAGCTTATGGCAACCAAGAAAGCA AGCTCATCATACTGGCTAGTGGTGGACCCCAAGCTTTAGTAAATATAATGAGGACCTATACTTACGAAAAACTACT GTGGACCACAAGCAGAGTGCTGAAGGTGCTATCTGTCTGCTCTAGTAATAAGCCGGCTATTGTAGAAGCTGGTGGA ATGCAAGCTTTAGGACTTCACCTGACAGATCCAAGTCAACGTCTTGTTCAGAACTGTCTTTGGACTCTCAGGAATC TTTCAGATGCTGCAACTAAACAGGAAGGGATGGAAGGTCTCCTTGGGACTCTTGTTCAGCTTCTGGGTTCAGATGA TATAAATGTGGTCACCTGTGCAGCTGGAATTCTTTCTAACCTCACTTGCAATAATTATAAGAACAAGATGATGGTC TGCCAAGTGGGTGGTATAGAGGCTCTTGTGCGTACTGTCCTTCGGGCTGGTGACAGGGAAGACATCACTGAGCCTG CCATCTGTGCTCTTCGTCATCTGACCAGCCGACACCAAGAAGCAGAGATGGCCCAGAATGCAGTTCGCCTTCACTA TGGACTACCAGTTGTGGTTAAGCTCTTACACCCACCATCCCACTGGCCTCTGATAAAGGCTACTGTTGGATTGATT CGAAATCTTGCCCTTTGTCCCGCAAATCATGCACCTTTGCGTGAGCAGGGTGCCATTCCACGACTAGTTCAGTTGC TTGTTCGTGCACATCAGGATACCCAGCGCCGTACGTCCATGGGTGGGACACAGCAGCAATTTGTGGAGGGGGTCCG CATGGAAGAAATAGTTGAAGGTTGTACCGGAGCCCTTCACATCCTAGCTCGGGATGTTCACAACCGAATTGTTATC AGAGGACTAAATACCATTCCATTGTTTGTGCAGCTGCTTTATTCTCCCATTGAAAACATCCAAAGAGTAGCTGCAG GGGTCCTCTGTGAACTTGCTCAGGACAAGGAAGCTGCAGAAGCTATTGAAGCTGAGGGAGCCACAGCTCCTCTGAC AGAGTTACTTCACTCTAGGAATGAAGGTGTGGCGACATATGCAGCTGCTGTTTTGTTCCGAATGTCTGAGGACAAG CCACAAGATTACAAGAAACGGCTTTCAGTTGAGCTGACCAGCTCTCTCTTCAGAACAGAGCCAATGGCTTGGAATG AGACTGCTGATCTTGGACTTGATATTGGTGCCCAGGGAGAACCCCTTGGATATCGCCAGGATGATCCTAGCTATCG TTCTTTTCACTCTGGTGGATATGGCCAGGATGCCTTGGGTATGGACCCCATGATGGAACATGAGATGGGTGGCCAC CACCCTGGTGCTGACTATCCAGTTGATGGGCTGCCAGATCTGGGGCATGCCCAGGACCTCATGGATGGGCTGCCTC CAGGTGACAGCAATCAGCTGGCCTGGTTTGATACTGACCTGTAAATCATCCTTTAGGAGTAACAATACAAATGGAT TTTGGGAGTGACTCAAGAAGTGAAGAATGCACAAGAATGGATCACAAGATGGAATTTATCAAACCCTAGCCTTGCT TGTTAAATTTTTTTTTTTTTTTTTTTAAGAATATCTGTAATGGTACTGACTTTGCTTGCTTTGAAGTAGCTCTTTT TTTTTTTTTTTTTTTTTTTTTGCAGTAACTGTTTTTTAAGTCTCTCGTAGTGTTAAGTTATAGTGAATACTGCTAC AGCAATTTCTAATTTTTAAGAATTGAGTAATGGTGTAGAACACTAATTCATAATCACTCTAATTAATTGTAATCTG AATAAAGTGTAACAATTGTGTAGCCTTTTTGTATAAAATAGACAAATAGAAAATGGTCCAATTAGTTTCCTTTTTA ATATGCTTAAAATAAGCAGGTGGATCTATTTCATGTTTTTGATCAAAAACTATTTGGGATATGTATGGGTAGGGTA AATCAGTAAGAGGTGTTATTTGGAACCTTGTTTTGGACAGTTTACCAGTTGCCTTTTATCCCAAAGTTGTTGTAAC CTGCTGTGATACGATGCTTCAAGAGAAAATGCGGTTATAAAAAATGGTTCAGAATTAAACTTTTAATTCATTCGA (SEQ ID NO:68). In some embodiments, a DNA mutation results in the mutation of T41 or S45 according to SEQ ID NO:64 or SEQ ID NO:68. For example, in some embodiments, a DNA mutation in a *CTNNB1* gene encodes or results in a T41A, T41I, S45F, or S45P mutated CTNNB 1 protein (numbering according to SEQ ID NO:64). These DNA mutations are also described by their nucleotide positions (rather than mutated polypeptide codons) in Table A *infra.* For example, in some embodiments, a DNA mutation in a *CTNNB1* gene results in a 121A>G, 122C>T, 133T>C, or 134C>T mutation in the corresponding cDNA sequence of SEQ ID NO:68.

Since, as described *supra,* the sequences of a variety of *CTNNB1* genes and corresponding mutations are known, one of ordinary skill in the art may suitably select a primer pair to amplify the locus of one or more of these DNA mutation(s). Various factors influencing primer design are known, and tools for identifying primer pairs suitable for amplifying a given DNA template sequence are available (*see, e.g.,* www.ncbi.nlm.nih.gov/tools/primer-blast/). In some embodiments, a primer pair for amplifying the locus of a *CTNNB1* mutation (*e.g.,* encoding or resulting in a T41A, T41I, S45F, and S45P mutated CTNNB 1 protein) comprises the sequences GGAATCCATTCTGGTGCCACT (SEQ ID NO: 13) and AGAAAATCCCTGTTCCCACTCATA (SEQ ID NO: 14), respectively (for the T41 locus); or GGTGCCACTACCACAGCTCCT (SEQ ID NO:18) and TCTCAAAACTGCATTCTGACTTTCA (SEQ ID NO:19), respectively (for the S45 locus). In some embodiments, two primer pairs are used for PCR: a first primer pair comprising the sequences GGAATCCATTCTGGTGCCACT (SEQ ID NO:13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO:19).

In some embodiments, the methods of the present disclosure include amplifying the loci of one or more mutations in an *APC* gene. *APC* encodes the APC tumor suppressor, a negative regulator of beta catenin and Wnt signaling that is frequently mutated in human cancers, also known as GS, DP2, DP3, BTPS2, DP2.5, PPP1R46, adenomatous polyposis coli protein, WNT signaling pathway negative regulator, adenomatous polyposis coli tumor suppressor, deleted in polyposis 2.5, protein phosphatase 1 regulatory subunit 46, and truncated adenomatous polyposis coli. In some embodiments, the *APC* gene is a human *APC* gene. In some embodiments, a human *APC* gene refers to the gene described by NCBI Entrez Gene ID No. 324, including mutants and variants thereof. In other embodiments, the *APC* gene is from one of the following organisms: mouse (*see, e.g.,* NCBI Entrez Gene ID No. 11789), rat (*see, e.g.,* NCBI Entrez Gene ID No. 24205), cynomolgus monkey (*see, e.g.,* NCBI Entrez Gene ID No. 102126553), fish (*see, e.g.,* NCBI Entrez Gene ID No. 386762), dog (*see, e.g.,* NCBI Entrez Gene ID No. 479139), cattle (*see, e.g.,* NCBI Entrez Gene ID No. 533233), horse (*see, e.g.,* NCBI Entrez Gene ID No. 100064431), chicken (*see, e.g.,* NCBI Entrez Gene ID No. 415607), chimpanzee (*see, e.g.,* NCBI Entrez Gene ID No. 461999), rhesus monkey (*see, e.g.,* NCBI Entrez Gene ID No. 693443), or cat (*see, e.g.,* NCBI Entrez Gene ID No. 101096138).

A variety of *APC* mutations associated with cancer are known and may be suitably detected by the methods described herein; *see, e.g.,* Morin, P.J. et al. (1997) Science 275:1787-90 and Polakis, P. (2000) Genes Dev. 14:1837-51. *APC* mutations often result in frameshifted or truncated APC mutant proteins, and are particularly frequent in a region known as the mutation cluster region (MCR). *See* Miyoshi, Y. et al. (1992) Hum. Mol. Genet. 1:229-33; and Segditsas, S. and Tomlinson, I. (2006) Oncogene 25:7531-7. One study found mutations in the MCR of the *APC gene* in 72% of colorectal cancer patients, and frameshift or nonsense mutations at the codons encoding human APC residues Q1367, R1450, E1309, and S1465 were frequently observed (Luchtenborg, M. et al. (2004) Carcinogenesis 25:1219-26). In some embodiments described herein, an *APC* mutation is named based on the resulting amino acid substitution/deletion/frameshift according to a human APC protein, *e.g.,* as set forth in MAAASYDQLLKQVEALKMENSNLRQELEDNSNHLTKLETEASNMKEVLKQLQGSIE DEAMASSGQIDLLERLKELNLDSSNFPGVKLRSKMSLRSYGSREGSVSSRSGECSPVPM GSFPRRGFVNGSRESTGYLEELEKERSLLLADLDKEEKEKDWYYAQLQNLTKRIDSLP LTENFSLQTDMTRRQLEYEARQIRVAMEEQLGTCQDMEKRAQRRIARIQQIEKDILRIR QLLQSQATEAERSSQNKHETGSHDAERQNEGQGVGEINMATSGNGQGSTTRMDHET ASVLSSSSTHSAPRRLTSHLGTKVEMVYSLLSMLGTHDKDDMSRTLLAMSSSQDSCIS MRQSGCLPLLIQLLHGNDKDSVLLGNSRGSKEARARASAALHNIIHSQPDDKRGRREI RVLHLLEQIRA YCETCWEWQEAHEPGMDQDKNPMP APVEHQICP A VCVLMKLSFDE EHRHAMNELGGLQAIAELLQVDCEMYGLTNDHYSITLRRYAGMALTNLTFGDVANK ATLCSMKGCMRALVAQLKSESEDLQQVIASVLRNLSWRADVNSKKTLREVGSVKAL MECALEVKKESTLKSVLSALWNLSAHCTENKADICAVDGALAFLVGTLTYRSQTNTL AIIESGGGILRNVSSLIATNEDHRQILRENNCLQTLLQHLKSHSLTIVSNACGTLWNLSA RNPKDQEALWDMGAVSMLKNLIHSKHKMIAMGSAAALRNLMANRPAKYKDANIMS PGSSLPSLHVRKQKALEAELDAQHLSETFDNIDNLSPKASHRSKQRHKQSLYGDYVFD TNRHDDNRSDNFNTGNMTVLSPYLNTTVLPSSSSSRGSLDSSRSEKDRSLERERGIGLG NYHPATENPGTSSKRGLQISTTAAQIAKVMEEVSAIHTSQEDRSSGSTTELHCVTDERN ALRRSSAAHTHSNTYNFTKSENSNRTCSMPYAKLEYKRSSNDSLNSVSSSDGYGKRGQ MKPSIESYSEDDESKFCSYGQYPADLAHKIHSANHMDDNDGELDTPINYSLKYSDEQL NSGRQSPSQNERWARPKHIIEDEIKQSEQRQSRNQSTTYPVYTESTDDKHLKFQPHFGQ QECVSPYRSRGANGSETNRVGSNHGINQNVSQSLCQEDDYEDDKPTNYSERYSEEEQ HEEEERPTNYSIKYNEEKRHVDQPIDYSLKYATDIPSSQKQSFSFSKSSSGQSSKTERMS SSSENTSTPSSNAKRQNQLHPSSAQSRSGQPQKAATCKVSSINQETIQTYCVEDTPICFS RCSSLSSLSSAEDEIGCNQTTQEADSANTLQIAEIKEKIGTRSAEDPVSEVPAVSQHPRT KSSRLQGSSLSSESARHKAVEFSSGAKSPSKSGAQTPKSPPEHYVQETPLMFSRCTSVSS LDSFESRSIASSVQSEPCSGMVSGIISPSDLPDSPGQTMPPSRSKTPPPPPQTAQTKREVP KNKAPTAEKRESGPKQAAVNAAVQRVQVLPDADTLLHFATESTPDGFSCSSSLSALSL DEPFIQKDVELRIMPPVQENDNGNETESEQPKESNENQEKEAEKTIDSEKDLLDDSDDD DIEILEECIISAMPTKSSRKAKKPAQTASKLPPPVARKPSQLPVYKLLPSQNRLQPQKHV SFTPGDDMPRVYCVEGTPINFSTATSLSDLTIESPPNELAAGEGVRGGAQSGEFEKRDTI PTEGRSTDEAQGGKTSSVTIPELDDNKAEEGDILAECINSAMPKGKSHKPFRVKKIMDQ VQQASASSSAPNKNQLDGKKKKPTSPVKPIPQNTEYRTRVRKNADSKNNLNAERVFS DNKDSKKQNLKNNSKVFNDKLPNNEDRVRGSFAFDSPHHYTPIEGTPYCFSRNDSLSS LDFDDDDVDLSREKAELRKAKENKESEAKVTSHTELTSNQQSANKTQAIAKQPINRGQ PKPILQKQSTFPQSSKDIPDRGAATDEKLQNFAIENTPVCFSHNSSLSSLSDIDQENNNK ENEPIKETEPPDSQGEPSKPQASGYAPKSFHVEDTPVCFSRNSSLSSLSIDSEDDLLQECI SSAMPKKKKPSRLKGDNEKHSPRNMGGILGEDLTLDLKDIQRPDSEHGLSPDSENFDW KAIQEGANSIVSSLHQAAAAACLSRQASSDSDSILSLKSGISLGSPFHLTPDQEEKPFTSN KGPRILKPGEKSTLETKKIESESKGIKGGKKVYKSLITGKVRSNSEISGQMKQPLQANM PSISRGRTMIHIPGVRNSSSSTSPVSKKGPPLKTPASKSPSEGQTATTSPRGAKPSVKSEL SPVARQTSQIGGSSKAPSRSGSRDSTPSRPAQQPLSRPIQSPGRNSISPGRNGISPPNKLSQ LPRTSSPSTASTKSSGSGKMSYTSPGRQMSQQNLTKQTGLSKNASSIPRSESASKGLNQ MNNGNGANKKVELSRMSSTKSSGSESDRSERPVLVRQSTFIKEAPSPTLRRKLEESASF ESLSPSSRPASPTRSQAQTPVLSPSLPDMSLSTHSSVQAGGWRKLPPNLSPTIEYNDGRP AKRHDIARSHSESPSRLPINRSGTWKREHSKHSSSLPRVSTWRRTGSSSSILSASSESSEK AKSEDEKHVNSISGTKQSKENQVSAKGTWRKIKENEFSPTNSTSQTVSSGATNGAESK TLIYQMAPAVSKTEDVWVRIEDCPINNPRSGRSPTGNTPPVIDSVSEKANPNIKDSKDN QAKQNVGNGSVPMRTVGLENRLNSFIQVDAPDQKGTEIKPGQNNPVPVSETNESSIVE RTPFSSSSSSKHSSPSGTVAARVTPFNYNPSPRKSSADSTSARPSQIPTPVNNNTKKRDS KTDSTESSGTQSPKRHSGSYLVTSV (SEQ ID NO:65). An exemplary human *APC* cDNA sequence is set forth in GTATTGGTGCAGCCCGCCAGGGTGTCACTGGAGACAGAATGGAGGTGCTGCCGGACTCGGAAATGGGGTCCAAGGG TAGCCAAGGATGGCTGCAGCTTCATATGATCAGTTGTTAAAGCAAGTTGAGGCACTGAAGATGGAGAACTCAAATC TTCGACAAGAGCTAGAAGATAATTCCAATCATCTTACAAAACTGGAAACTGAGGCATCTAATATGAAGGAAGTACT TAAACAACTACAAGGAAGTATTGAAGATGAAGCTATGGCTTCTTCTGGACAGATTGATTTATTAGAGCGTCTTAAA GAGCTTAACTTAGATAGCAGTAATTTCCCTGGAGTAAAACTGCGGTCAAAAATGTCCCTCCGTTCTTATGGAAGCC GGGAAGGATCTGTATCAAGCCGTTCTGGAGAGTGCAGTCCTGTTCCTATGGGTTCATTTCCAAGAAGAGGGTTTGT AAATGGAAGCAGAGAAAGTACTGGATATTTAGAAGAACTTGAGAAAGAGAGGTCATTGCTTCTTGCTGATCTTGAC AAAGAAGAAAAGGAAAAAGACTGGTATTACGCTCAACTTCAGAATCTCACTAAAAGAATAGATAGTCTTCCTTTAA CTGAAAATTTTTCCTTACAAACAGATATGACCAGAAGGCAATTGGAATATGAAGCAAGGCAAATCAGAGTTGCGAT GGAAGAACAACTAGGTACCTGCCAGGATATGGAAAAACGAGCACAGCGAAGAATAGCCAGAATTCAGCAAATCGAA AAGGACATACTTCGTATACGACAGCTTTTACAGTCCCAAGCAACAGAAGCAGAGAGGTCATCTCAGAACAAGCATG AAACCGGCTCACATGATGCTGAGCGGCAGAATGAAGGTCAAGGAGTGGGAGAAATCAACATGGCAACTTCTGGTAA TGGTCAGGGTTCAACTACACGAATGGACCATGAAACAGCCAGTGTTTTGAGTTCTAGTAGCACACACTCTGCACCT CGAAGGCTGACAAGTCATCTGGGAACCAAGGTGGAAATGGTGTATTCATTGTTGTCAATGCTTGGTACTCATGATA AGGATGATATGTCGCGAACTTTGCTAGCTATGTCTAGCTCCCAAGACAGCTGTATATCCATGCGACAGTCTGGATG TCTTCCTCTCCTCATCCAGCTTTTACATGGCAATGACAAAGACTCTGTATTGTTGGGAAATTCCCGGGGCAGTAAA GAGGCTCGGGCCAGGGCCAGTGCAGCACTCCACAACATCATTCACTCACAGCCTGATGACAAGAGAGGCAGGCGTG AAATCCGAGTCCTTCATCTTTTGGAACAGATACGCGCTTACTGTGAAACCTGTTGGGAGTGGCAGGAAGCTCATGA ACCAGGCATGGACCAGGACAAAAATCCAATGCCAGCTCCTGTTGAACATCAGATCTGTCCTGCTGTGTGTGTTCTA ATGAAACTTTCATTTGATGAAGAGCATAGACATGCAATGAATGAACTAGGGGGACTACAGGCCATTGCAGAATTAT TGCAAGTGGACTGTGAAATGTATGGGCTTACTAATGACCACTACAGTATTACACTAAGACGATATGCTGGAATGGC TTTGACAAACTTGACTTTTGGAGATGTAGCCAACAAGGCTACGCTATGCTCTATGAAAGGCTGCATGAGAGCACTT GTGGCCCAACTAAAATCTGAAAGTGAAGACTTACAGCAGGTTATTGCGAGTGTTTTGAGGAATTTGTCTTGGCGAG CAGATGTAAATAGTAAAAAGACGTTGCGAGAAGTTGGAAGTGTGAAAGCATTGATGGAATGTGCTTTAGAAGTTAA AAAGGAATCAACCCTCAAAAGCGTATTGAGTGCCTTATGGAATTTGTCAGCACATTGCACTGAGAATAAAGCTGAT ATATGTGCTGTAGATGGTGCACTTGCATTTTTGGTTGGCACTCTTACTTACCGGAGCCAGACAAACACTTTAGCCA TTATTGAAAGTGGAGGTGGGATATTACGGAATGTGTCCAGCTTGATAGCTACAAATGAGGACCACAGGCAAATCCT AAGAGAGAACAACTGTCTACAAACTTTATTACAACACTTAAAATCTCATAGTTTGACAATAGTCAGTAATGCATGT GGAACTTTGTGGAATCTCTCAGCAAGAAATCCTAAAGACCAGGAAGCATTATGGGACATGGGGGCAGTTAGCATGC TCAAGAACCTCATTCATTCAAAGCACAAAATGATTGCTATGGGAAGTGCTGCAGCTTTAAGGAATCTCATGGCAAA TAGGCCTGCGAAGTACAAGGATGCCAATATTATGTCTCCTGGCTCAAGCTTGCCATCTCTTCATGTTAGGAAACAA AAAGCCCTAGAAGCAGAATTAGATGCTCAGCACTTATCAGAAACTTTTGACAATATAGACAATTTAAGTCCCAAGG CATCTCATCGTAGTAAGCAGAGACACAAGCAAAGTCTCTATGGTGATTATGTTTTTGACACCAATCGACATGATGA TAATAGGTCAGACAATTTTAATACTGGCAACATGACTGTCCTTTCACCATATTTGAATACTACAGTGTTACCCAGC TCCTCTTCATCAAGAGGAAGCTTAGATAGTTCTCGTTCTGAAAAAGATAGAAGTTTGGAGAGAGAACGCGGAATTG GTCTAGGCAACTACCATCCAGCAACAGAAAATCCAGGAACTTCTTCAAAGCGAGGTTTGCAGATCTCCACCACTGC AGCCCAGATTGCCAAAGTCATGGAAGAAGTGTCAGCCATTCATACCTCTCAGGAAGACAGAAGTTCTGGGTCTACC ACTGAATTACATTGTGTGACAGATGAGAGAAATGCACTTAGAAGAAGCTCTGCTGCCCATACACATTCAAACACTT ACAATTTCACTAAGTCGGAAAATTCAAATAGGACATGTTCTATGCCTTATGCCAAATTAGAATACAAGAGATCTTC AAATGATAGTTTAAATAGTGTCAGTAGTAGTGATGGTTATGGTAAAAGAGGTCAAATGAAACCCTCGATTGAATCC TATTCTGAAGATGATGAAAGTAAGTTTTGCAGTTATGGTCAATACCCAGCCGACCTAGCCCATAAAATACATAGTG CAAATCATATGGATGATAATGATGGAGAACTAGATACACCAATAAATTATAGTCTTAAATATTCAGATGAGCAGTT GAACTCTGGAAGGCAAAGTCCTTCACAGAATGAAAGATGGGCAAGACCCAAACACATAATAGAAGATGAAATAAAA CAAAGTGAGCAAAGACAATCAAGGAATCAAAGTACAACTTATCCTGTTTATACTGAGAGCACTGATGATAAACACC TCAAGTTCCAACCACATTTTGGACAGCAGGAATGTGTTTCTCCATACAGGTCACGGGGAGCCAATGGTTCAGAAAC AAATCGAGTGGGTTCTAATCATGGAATTAATCAAAATGTAAGCCAGTCTTTGTGTCAAGAAGATGACTATGAAGAT GATAAGCCTACCAATTATAGTGAACGTTACTCTGAAGAAGAACAGCATGAAGAAGAAGAGAGACCAACAAATTATA GCATAAAATATAATGAAGAGAAACGTCATGTGGATCAGCCTATTGATTATAGTTTAAAATATGCCACAGATATTCC TTCATCACAGAAACAGTCATTTTCATTCTCAAAGAGTTCATCTGGACAAAGCAGTAAAACCGAACATATGTCTTCA AGCAGTGAGAATACGTCCACACCTTCATCTAATGCCAAGAGGCAGAATCAGCTCCATCCAAGTTCTGCACAGAGTA GAAGTGGTCAGCCTCAAAAGGCTGCCACTTGCAAAGTTTCTTCTATTAACCAAGAAACAATACAGACTTATTGTGT AGAAGATACTCCAATATGTTTTTCAAGATGTAGTTCATTATCATCTTTGTCATCAGCTGAAGATGAAATAGGATGT AATCAGACGACACAGGAAGCAGATTCTGCTAATACCCTGCAAATAGCAGAAATAAAAGAAAAGATTGGAACTAGGT CAGCTGAAGATCCTGTGAGCGAAGTTCCAGCAGTGTCACAGCACCCTAGAACCAAATCCAGCAGACTGCAGGGTTC TAGTTTATCTTCAGAATCAGCCAGGCACAAAGCTGTTGAATTTTCTTCAGGAGCGAAATCTCCCTCCAAAAGTGGT GCTCAGACACCCAAAAGTCCACCTGAACACTATGTTCAGGAGACCCCACTCATGTTTAGCAGATGTACTTCTGTCA GTTCACTTGATAGTTTTGAGAGTCGTTCGATTGCCAGCTCCGTTCAGAGTGAACCATGCAGTGGAATGGTAAGTGG CATTATAAGCCCCAGTGATCTTCCAGATAGCCCTGGACAAACCATGCCACCAAGCAGAAGTAAAACACCTCCACCA CCTCCTCAAACAGCTCAAACCAAGCGAGAAGTACCTAAAAATAAAGCACCTACTGCTGAAAAGAGAGAGAGTGGAC CTAAGCAAGCTGCAGTAAATGCTGCAGTTCAGAGGGTCCAGGTTCTTCCAGATGCTGATACTTTATTACATTTTGC CACGGAAAGTACTCCAGATGGATTTTCTTGTTCATCCAGCCTGAGTGCTCTGAGCCTCGATGAGCCATTTATACAG AAAGATGTGGAATTAAGAATAATGCCTCCAGTTCAGGAAAATGACAATGGGAATGAAACAGAATCAGAGCAGCCTA AAGAATCAAATGAAAACCAAGAGAAAGAGGCAGAAAAAACTATTGATTCTGAAAAGGACCTATTAGATGATTCAGA TGATGATGATATTGAAATACTAGAAGAATGTATTATTTCTGCCATGCCAACAAAGTCATCACGTAAAGCAAAAAAG CCAGCCCAGACTGCTTCAAAATTACCTCCACCTGTGGCAAGGAAACCAAGTCAGCTGCCTGTGTACAAACTTCTAC CATCACAAAACAGGTTGCAACCCCAAAAGCATGTTAGTTTTACACCGGGGGATGATATGCCACGGGTGTATTGTGT TGAAGGGACACCTATAAACTTTTCCACAGCTACATCTCTAAGTGATCTAACAATCGAATCCCCTCCAAATGAGTTA GCTGCTGGAGAAGGAGTTAGAGGAGGGGCACAGTCAGGTGAATTTGAAAAACGAGATACCATTCCTACAGAAGGCA GAAGTACAGATGAGGCTCAAGGAGGAAAAACCTCATCTGTAACCATACCTGAATTGGATGACAATAAAGCAGAGGA AGGTGATATTCTTGCAGAATGCATTAATTCTGCTATGCCCAAAGGGAAAAGTCACAAGCCTTTCCGTGTGAAAAAG ATAATGGACCAGGTCCAGCAAGCATCTGCGTCTTCTTCTGCACCCAACAAAAATCAGTTAGATGGTAAGAAAAAGA AACCAACTTCACCAGTAAAACCTATACCACAAAATACTGAATATAGGACACGTGTAAGAAAAAATGCAGACTCAAA AAATAATTTAAATGCTGAGAGAGTTTTCTCAGACAACAAAGATTCAAAGAAACAGAATTTGAAAAATAATTCCAAG GTCTTCAATGATAAGCTCCCAAATAATGAAGATAGAGTCAGAGGAAGTTTTGCTTTTGATTCACCTCATCATTACA CGCCTATTGAAGGAACTCCTTACTGTTTTTCACGAAATGATTCTTTGAGTTCTCTAGATTTTGATGATGATGATGT TGACCTTTCCAGGGAAAAGGCTGAATTAAGAAAGGCAAAAGAAAATAAGGAATCAGAGGCTAAAGTTACCAGCCAC ACAGAACTAACCTCCAACCAACAATCAGCTAATAAGACACAAGCTATTGCAAAGCAGCCAATAAATCGAGGTCAGC CTAAACCCATACTTCAGAAACAATCCACTTTTCCCCAGTCATCCAAAGACATACCAGACAGAGGGGCAGCAACTGA TGAAAAGTTACAGAATTTTGCTATTGAAAATACTCCGGTTTGCTTTTCTCATAATTCCTCTCTGAGTTCTCTCAGT GACATTGACCAAGAAAACAACAATAAAGAAAATGAACCTATCAAAGAGACTGAGCCCCCTGACTCACAGGGAGAAC CAAGTAAACCTCAAGCATCAGGCTATGCTCCTAAATCATTTCATGTTGAAGATACCCCAGTTTGTTTCTCAAGAAA CAGTTCTCTCAGTTCTCTTAGTATTGACTCTGAAGATGACCTGTTGCAGGAATGTATAAGCTCCGCAATGCCAAAA AAGAAAAAGCCTTCAAGACTCAAGGGTGATAATGAAAAACATAGTCCCAGAAATATGGGTGGCATATTAGGTGAAG ATCTGACACTTGATTTGAAAGATATACAGAGACCAGATTCAGAACATGGTCTATCCCCTGATTCAGAAAATTTTGA TTGGAAAGCTATTCAGGAAGGTGCAAATTCCATAGTAAGTAGTTTACATCAAGCTGCTGCTGCTGCATGTTTATCT AGACAAGCTTCGTCTGATTCAGATTCCATCCTTTCCCTGAAATCAGGAATCTCTCTGGGATCACCATTTCATCTTA CACCTGATCAAGAAGAAAAACCCTTTACAAGTAATAAAGGCCCACGAATTCTAAAACCAGGGGAGAAAAGTACATT GGAAACTAAAAAGATAGAATCTGAAAGTAAAGGAATCAAAGGAGGAAAAAAAGTTTATAAAAGTTTGATTACTGGA AAAGTTCGATCTAATTCAGAAATTTCAGGCCAAATGAAACAGCCCCTTCAAGCAAACATGCCTTCAATCTCTCGAG GCAGGACAATGATTCATATTCCAGGAGTTCGAAATAGCTCCTCAAGTACAAGTCCTGTTTCTAAAAAAGGCCCACC CCTTAAGACTCCAGCCTCCAAAAGCCCTAGTGAAGGTCAAACAGCCACCACTTCTCCTAGAGGAGCCAAGCCATCT GTGAAATCAGAATTAAGCCCTGTTGCCAGGCAGACATCCCAAATAGGTGGGTCAAGTAAAGCACCTTCTAGATCAG GATCTAGAGATTCGACCCCTTCAAGACCTGCCCAGCAACCATTAAGTAGACCTATACAGTCTCCTGGCCGAAACTC AATTTCCCCTGGTAGAAATGGAATAAGTCCTCCTAACAAATTATCTCAACTTCCAAGGACATCATCCCCTAGTACT GCTTCAACTAAGTCCTCAGGTTCTGGAAAAATGTCATATACATCTCCAGGTAGACAGATGAGCCAACAGAACCTTA CCAAACAAACAGGTTTATCCAAGAATGCCAGTAGTATTCCAAGAAGTGAGTCTGCCTCCAAAGGACTAAATCAGAT GAATAATGGTAATGGAGCCAATAAAAAGGTAGAACTTTCTAGAATGTCTTCAACTAAATCAAGTGGAAGTGAATCT GATAGATCAGAAAGACCTGTATTAGTACGCCAGTCAACTTTCATCAAAGAAGCTCCAAGCCCAACCTTAAGAAGAA AATTGGAGGAATCTGCTTCATTTGAATCTCTTTCTCCATCATCTAGACCAGCTTCTCCCACTAGGTCCCAGGCACA AACTCCAGTTTTAAGTCCTTCCCTTCCTGATATGTCTCTATCCACACATTCGTCTGTTCAGGCTGGTGGATGGCGA AAACTCCCACCTAATCTCAGTCCCACTATAGAGTATAATGATGGAAGACCAGCAAAGCGCCATGATATTGCACGGT CTCATTCTGAAAGTCCTTCTAGACTTCCAATCAATAGGTCAGGAACCTGGAAACGTGAGCACAGCAAACATTCATC ATCCCTTCCTCGAGTAAGCACTTGGAGAAGAACTGGAAGTTCATCTTCAATTCTTTCTGCTTCATCAGAATCCAGT GAAAAAGCAAAAAGTGAGGATGAAAAACATGTGAACTCTATTTCAGGAACCAAACAAAGTAAAGAAAACCAAGTAT CCGCAAAAGGAACATGGAGAAAAATAAAAGAAAATGAATTTTCTCCCACAAATAGTACTTCTCAGACCGTTTCCTC AGGTGCTACAAATGGTGCTGAATCAAAGACTCTAATTTATCAAATGGCACCTGCTGTTTCTAAAACAGAGGATGTT TGGGTGAGAATTGAGGACTGTCCCATTAACAATCCTAGATCTGGAAGATCTCCCACAGGTAATACTCCCCCGGTGA TTGACAGTGTTTCAGAAAAGGCAAATCCAAACATTAAAGATTCAAAAGATAATCAGGCAAAACAAAATGTGGGTAA TGGCAGTGTTCCCATGCGTACCGTGGGTTTGGAAAATCGCCTGAACTCCTTTATTCAGGTGGATGCCCCTGACCAA AAAGGAACTGAGATAAAACCAGGACAAAATAATCCTGTCCCTGTATCAGAGACTAATGAAAGTTCTATAGTGGAAC GTACCCCATTCAGTTCTAGCAGCTCAAGCAAACACAGTTCACCTAGTGGGACTGTTGCTGCCAGAGTGACTCCTTT TAATTACAACCCAAGCCCTAGGAAAAGCAGCGCAGATAGCACTTCAGCTCGGCCATCTCAGATCCCAACTCCAGTG AATAACAACACAAAGAAGCGAGATTCCAAAACTGACAGCACAGAATCCAGTGGAACCCAAAGTCCTAAGCGCCATT CTGGGTCTTACCTTGTGACATCTGTTTAAAAGAGAGGAAGAATGAAACTAAGAAAATTCTATGTTAATTACAACTG CTATATAGACATTTTGTTTCAAATGAAACTTTAAAAGACTGAAAAATTTTGTAAATAGGTTTGATTCTTGTTAGAG GGTTTTTGTTCTGGAAGCCATATTTGATAGTATACTTTGTCTTCACTGGTCTTATTTTGGGAGGCACTCTTGATGG TTAGGAAAAAAATAGTAAAGCCAAGTATGTTTGTACAGTATGTTTTACATGTATTTAAAGTAGCATCCCATCCCAA CTTCCTTTAATTATTGCTTGTCTTAAAATAATGAACACTACAGATAGAAAATATGATATATTGCTGTTATCAATCA TTTCTAGATTATAAACTGACTAAACTTACATCAGGGAAAAATTGGTATTTATGCAAAAAAAAATGTTTTTGTCCTT GTGAGTCCATCTAACATCATAATTAATCATGTGGCTGTGAAATTCACAGTAATATGGTTCCCGATGAACAAGTTTA CCCAGCCTGCTTTGCTTTACTGCATGAATGAAACTGATGGTTCAATTTCAGAAGTAATGATTAACAGTTATGTGGT CACATGATGTGCATAGAGATAGCTACAGTGTAATAATTTACACTATTTTGTGCTCCAAACAAAACAAAAATCTGTG TAACTGTAAAACATTGAATGAAACTATTTTACCTGAACTAGATTTTATCTGAAAGTAGGTAGAATTTTTGCTATGC TGTAATTTGTTGTATATTCTGGTATTTGAGGTGAGATGGCTGCTCTTTTATTAATGAGACATGAATTGTGTCTCAA CAGAAACTAAATGAACATTTCAGAATAAATTATTGCTGTATGTAAACTGTTACTGAAATTGGTATTTGTTTGAAGG GTCTTGTTTCACATTTGTATTAATAATTGTTTAAAATGCCTCTTTTAAAAGCTTATATAAATTTTTTTCTTCAGCT TCTATGCATTAAGAGTAAAATTCCTCTTACTGTAATAAAAACAATTGAAGAAGACTGTTGCCACTTAACCATTCCA TGCGTTGGCACTTATCTATTCCTGAAATTTCTTTTATGTGATTAGCTCATCTTGATTTTTAATATTTTTCCACTTA AACTTTTTTTTCTTACTCCACTGGAGCTCAGTAAAAGTAAATTCATGTAATAGCAATGCAAGCAGCCTAGCACAGA CTAAGCATTGAGCATAATAGGCCCACATAATTTCCTCTTTCTTAATATTATAGAATTCTGTACTTGAAATTGATTC TTAGACATTGCAGTCTCTTCGAGGCTTTACAGTGTAAACTGTCTTGCCCCTTCATCTTCTTGTTGCAACTGGGTCT GACATGAACACTTTTTATCACCCTGTATGTTAGGGCAAGATCTCAGCAGTGAAGTATAATCAGCACTTTGCCATGC TCAGAAAATTCAAATCACATGGAACTTTAGAGGTAGATTTAATACGATTAAGATATTCAGAAGTATATTTTAGAAT CCCTGCCTGTTAAGGAAACTTTATTTGTGGTAGGTACAGTTCTGGGGTACATGTTAAGTGTCCCCTTATACAGTGG AGGGAAGTCTTCCTTCCTGAAGGAAAATAAACTGACACTTATTAACTAAGATAATTTACTTAATATATCTTCCCTG ATTTGTTTTAAAAGATCAGAGGGTGACTGATGATACATGCATACATATTTGTTGAATAAATGAAAATTTATTTTTA GTGATAAGATTCATACACTCTGTATTTGGGGAGGGAAAACCTTTTTAAGCATGGTGGGGCACTCAGATAGGAGTGA ATACACCTACCTGGTGCCTTGAAAATCACATCAAGTAGTTAATTATCTACCCCTTACCTGTGTTTATAACTTCCAG GTAATGAGAATGATTTTTTTTAAAGCTAAAATGCCAGTAAATAAAAGTGCTATGACTTGAGCTAAGATATTTGACT CCAATGCCTGTACTGTGTCTACTGCACCACTTTGTAAACACTTCAATTTACTATCTTTGAAATGATTGACCTTTAA ATTTTTGCCAAATGTTATCTGAAATTGTCTATGAATACCATCTACTTCTGTTGTTTTCCCAGGCTTCCATAAACAA TGGAGATACATGCAAAAAAAAAAA (SEQ ID NO:69). In some embodiments, a DNA mutation results in a truncation of APC protein at exon 15. In some embodiments, a DNA mutation results in a truncation of APC protein between codons 1250 and 1560, or between codons 1286 and 1513. In some embodiments, a DNA mutation results in the mutation of Q1367, R1450, E1309, S1465, or T1556 according to SEQ ID NO:65 or SEQ ID NO:69. For example, in some embodiments, a DNA mutation in an *APC* gene encodes or results in a Q1367*, R1450*, E1309 frameshift, S1465 frameshift, or T1556 frameshift mutated APC protein (numbering according to SEQ ID NO:65). These DNA mutations are also described by their nucleotide positions (rather than mutated polypeptide codons) in Table A *infra.* For example, in some embodiments, a DNA mutation in an *APC* gene results in a 3927-3931delAAAGA, 4099C>T, 4348C>T, 4385-4386delAG, or 4666-4667insA mutation in the corresponding cDNA sequence of SEQ ID NO:69.

Since, as described *supra,* the sequences of a variety of *APC* genes and corresponding mutations are known, one of ordinary skill in the art may suitably select a primer pair to amplify the locus of one or more of these DNA mutation(s). Various factors influencing primer design are known, and tools for identifying primer pairs suitable for amplifying a given DNA template sequence are available (*see, e.g.,* www.ncbi.nlm.nih.gov/tools/primer-blast/). In some embodiments, a primer pair for amplifying the locus of an *APC* mutation (*e.g.,* encoding or resulting in the mutated APC proteins described *supra*) comprises the sequences TAAAAATAAAGCACCTACTGCTGAAA (SEQ ID NO:23) and AGCTTGCTTAGGTCCACTCTCTCT (SEQ ID NO:24), respectively (for the S1465 locus); TAGGATGTAATCAGACGACACAGGA (SEQ ID NO:27) and CAGCTGACCTAGTTCCAATCTTTTA (SEQ ID NO:28), respectively (for the E1309 locus); TCTCCCTCCAAAAGTGGTGCT (SEQ ID NO:31) and TGGCAATCGAACGACTCTCAA (SEQ ID NO:32), respectively (for the Q1367 locus); GCAGAAGTAAAACACCTCCACCA (SEQ ID NO:35) and GGTGCTTTATTTTTAGGTACTTC (SEQ ID NO:36), respectively (for the R1450 locus); or CAGGAAAATGACAATGGGAATG (SEQ ID NO:39) and ATCTAATAGGTCCTTTTCAGAATCAATAG (SEQ ID NO:40), respectively (for the T1556 locus). In some embodiments, five primer pairs are used for PCR: a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36); and a fifth primer pair comprising the sequences CAGGAAAATGACAATGGGAATG (SEQ ID NO:39) and ATCTAATAGGTCCTTTTCAGAATCAATAG (SEQ ID NO:40). In some embodiments, a primer pair for amplifying the locus of one or more *APC* mutations (*e.g.,* encoding or resulting in the mutated APC protein(s) described *supra*) comprises the sequences GCAGAAGTAAAACACCTCCACCA (SEQ ID NO:35) and AGCTTGCTTAGGTCCACTCTCTCT (SEQ ID NO:24).

In some embodiments, a primer pair of the present disclosure comprises one or more modified nucleotides, *e.g.,* locked nucleic acids (as described in greater detail *infra*)*.* For example, in some embodiments, a primer pair for amplifying the locus of an *APC* mutation (*e.g.,* encoding or resulting in the mutated APC proteins described *supra*) comprises the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids. In some embodiments, a primer pair for amplifying the locus of an *APC* mutation (*e.g.,* encoding or resulting in the mutated APC proteins described *supra*) comprises the sequences GCAGAAGTAAAACACCTCCACCA (SEQ ID NO:35) and GGTGCTTTATTTTTAGGTACTTC (SEQ ID NO:36), with underlined nucleic acids representing locked nucleic acids. In some embodiments, five primer pairs are used for PCR to amplify loci of an *APC* gene: a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40). In some embodiments, five primer pairs are used for PCR to amplify loci of an *APC* gene: a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with underlined nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40).

A multiplex assay of the present disclosure may include detecting two or more of the mutations described above in combination. For example, in some embodiments, the methods of the present disclosure include amplifying the loci of one or more mutations in a *KRAS* gene, one or more mutations in a *BRAF* gene, one or more mutations in a *CTNNB1* gene, and one or more mutations in an *APC* gene. In certain embodiments, the methods of the present disclosure include amplifying the locus of a mutation encoding a G12D, G12V, G12S, or G13D mutated KRAS protein; the locus of a mutation encoding a V600E mutated BRAF protein; the locus of a mutation encoding a T41A or T41I mutated CTNNB 1 protein and/or the locus of a mutation encoding an S45F or S45P mutated CTNNB1 protein; and one or more of: the locus of a mutation encoding a Q1367* mutated APC protein, the locus of a mutation encoding an R1450* mutated APC protein, the locus of a mutation encoding an E1309 frameshift mutated APC protein, the locus of a mutation encoding an S1465 frameshift mutated APC protein, and the locus of a mutation encoding a T1556 frameshift mutated APC protein. In certain embodiments, the methods of the present disclosure include amplifying the loci of mutations encoding G12D, G12V, G12S, and G13D mutated KRAS proteins; the loci of two or more different mutations encoding a V600E mutated BRAF protein; the loci of mutations encoding a T41A or T41I mutated CTNNB1 protein and/or the loci of mutations encoding an S45F or S45P mutated CTNNB1 protein; and the locus of a mutation encoding a Q1367* mutated APC protein, the locus of a mutation encoding an R1450* mutated APC protein, the locus of a mutation encoding an E1309 frameshift mutated APC protein, the locus of a mutation encoding an S1465 frameshift mutated APC protein, and the locus of a mutation encoding a T1556 frameshift mutated APC protein.

### Blocking nucleic acids

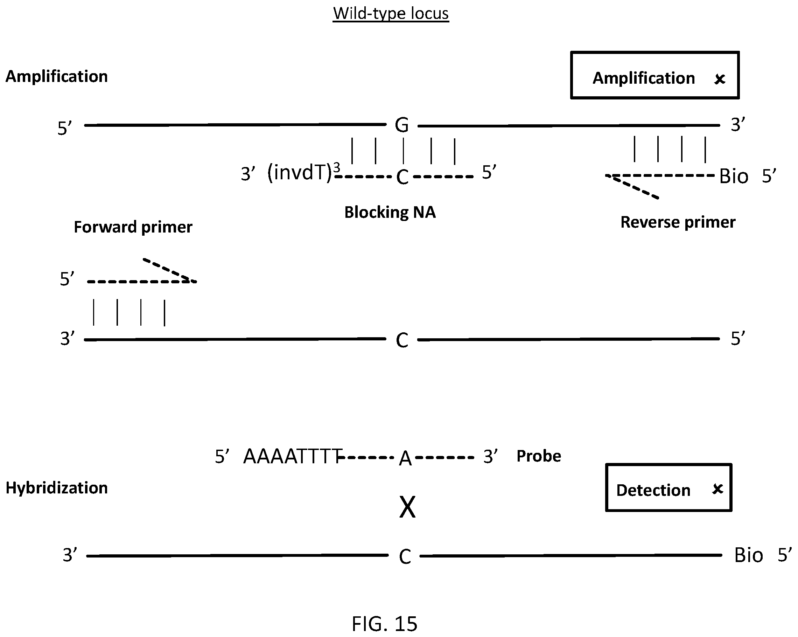

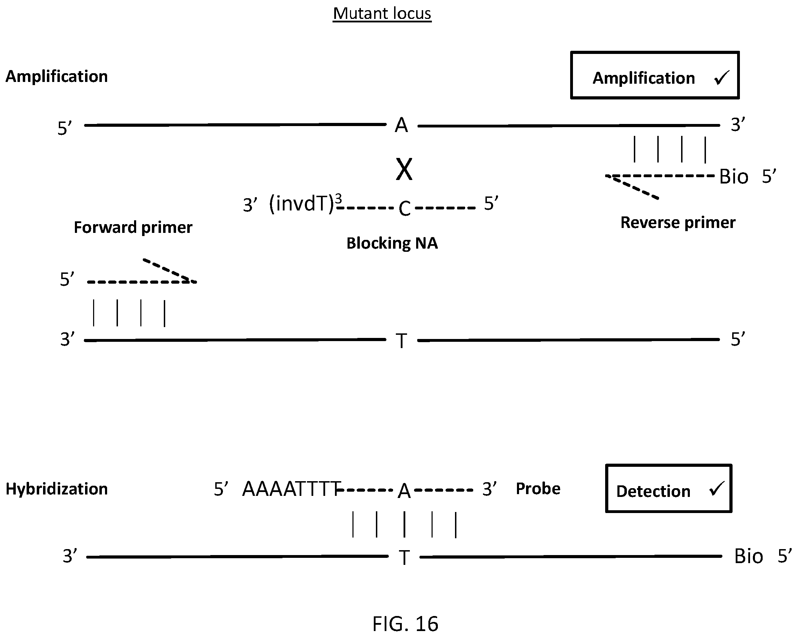

The methods of the present disclosure include amplifying isolated DNA by PCR in the presence of one or more blocking nucleic acid(s) (*e.g.,* a blocking nucleic acid corresponding to each DNA mutation of interest). Advantageously, the blocking nucleic acid prevents amplification of the wild-type DNA locus, thus increasing the sensitivity of detecting the DNA mutation (cf. **FIGS. 15** **&** **16**). The methods include amplifying isolated DNA by PCR in the presence of at least four blocking nucleic acids, each of which hybridizes with the wild-type DNA locus corresponding with a DNA mutation in the *KRAS, BRAF, CTNNB1,* or *APC* gene.

In some embodiments, a blocking nucleic acid of the present disclosure comprises: a single-stranded oligonucleotide that hybridizes with the corresponding wild-type DNA locus, and a 3' terminal moiety that blocks extension from the single-stranded oligonucleotide, thereby preventing amplification of the wild-type DNA locus. In some embodiments, the 3' terminal moiety comprises one or more inverted deoxythymidines (invdTs). In certain embodiments, the 3' terminal moiety comprises three consecutive inverted deoxythymidines.

In some embodiments, a blocking nucleic acid of the present disclosure comprises one or more modified nucleotides. Oligonucleotides comprising modified nucleotides in some or all sequence positions are contemplated and may have improved hybridization properties particularly advantageous for use as a blocking nucleic acid during PCR. For example, it is known that oligonucleotides partly or completely synthesized using locked nucleic acids (LNAs) possess greater thermal stability than corresponding oligonucleotides synthesized with only conventional nucleotides, thereby increasing the melting temperature of an oligo:LNA duplex and allowing for shorter sequences that retain stable hybridization during thermocycling. *See* Koshkin, A.A. et al. (1998) Tetrahedron 54:3607-30. A variety of modified nucleotides are known and include without limitation locked nucleic acids (LNAs), peptide nucleic acids (PNAs), hexose nucleic acids (HNAs), threose nucleic acids (TNAs), glycol nucleic acids (GNAs), and cyclohexenyl nucleic acids (CeNAs). For more detailed description of exemplary modified nucleotides, *see, e.g.,* Schmidt, M. (2010) BioEssays 32:322-31.

In some embodiments, a blocking nucleic acid of the present disclosure hybridizes with a wild-type *KRAS* locus corresponding with the locus of one or more DNA mutations encoding a G12D, G12V, G12S, or G13D mutated KRAS protein. In some embodiments, the blocking nucleic acid comprises the sequence TA*C*G*CC*A*CC*A*G*CT(invdT)*ₙ*, wherein *n* is 1, 2, or 3 (SEQ ID NO:3), TT*GG*A*G*CT*GGTGGC*GTA(invdT)*ₙ*, wherein *n* is 1, 2, or 3 (SEQ ID NO:142), GCT*GG*T*GG*C*G*TA*G*G*C*A(invdT)*ₙ*, wherein *n* is 1, 2, or 3 (SEQ ID NO:143), *GCTGGTGGCGTA*GGC(invdT)*ₙ,* wherein *n is* 1, 2, or 3 (SEQ ID NO:144), or TT*GG*A*G*CT*GG*T*GG*C*G*T(invdT)*ₙ*, wherein *n* is 1, 2, or 3 (SEQ ID NO:145), with italicized nucleic acids representing locked nucleic acids. In some embodiments, *n* is 1, 2, 3, 4, 5, 6, 7, 8, 9, or 10. For example, in certain embodiments, the blocking nucleic acid comprises the sequence TA*C*G*CC*A*CC*A*G*CTinvdTinvdTinvdT (SEQ ID NO:3), TT*GG*A*G*CT*GGTGGC*GTAinvdTinvdTinvdT (SEQ ID NO:142), GCT*GG*T*GG*C*G*TA*G*G*C*AinvdTinvdTinvdT (SEQ ID NO:143), *GCTGGTGGCGTA*GGCinvdTinvdTinvdT (SEQ ID NO:144), or TT*GG*A*G*CT*GG*T*GG*C*G*TinvdTinvdTinvdT (SEQ ID NO:145), with italicized nucleic acids representing locked nucleic acids. In certain embodiments, the blocking nucleic acid comprises the sequence TACGCCACCAGCTinvdTinvdTinvdT (SEQ ID NO:3), TTGGAGCTGGTGGCGTAinvdTinvdTinvdT (SEQ ID NO: 142), GCTGGTGGCGTAGGCAinvdTinvdTinvdT (SEQ ID NO: 143), GCTGGTGGCGTAGGCinvdTinvdTinvdT (SEQ ID NO: 144), or TTGGAGCTGGTGGCGTinvdTinvdTinvdT (SEQ ID NO: 145), with underlined nucleic acids representing locked nucleic acids. In some embodiments, the blocking nucleic acid comprises the sequence of SEQ ID NO:3 or 142-145 but optionally includes a different pattern or type of modified nucleotide(s). In some embodiments, the blocking nucleic acid comprises the sequence of SEQ ID NO:3 or 142-145 but includes a different 3' terminal moiety.

In some embodiments, a blocking nucleic acid of the present disclosure hybridizes with a wild-type *BRAF* locus corresponding with the locus of one or more DNA mutations encoding a V600E mutated BRAF protein. In some embodiments, the blocking nucleic acid comprises the sequence G*AGAT*T*TCAC*T*GTAGC*(invdT)*ₙ*, wherein *n* is 1, 2, or 3 (SEQ ID NO:10), G*AGAT*T*TCAC*T*GTAGC*(invdT)*ₙ*, wherein *n is* 1, 2, or 3 (SEQ ID NO: 146), G*AGAT*T*TCAC*T*GTAGC*(invdT)*ₙ*, wherein *n is* 1, 2, or 3 (SEQ ID NO: 147), G*AGAT*T*TCAC*T*GTAGC*(invdT)*ₙ*, wherein *n is* 1, 2, or 3 (SEQ ID NO:148), or G*AGAT*T*TCAC*T*GTAGC*(invdT)*ₙ*, wherein *n is* 1, 2, or 3 (SEQ ID NO: 149), with italicized nucleic acids representing locked nucleic acids. In some embodiments, *n* is 1, 2, 3, 4, 5, 6, 7, 8, 9, or 10. For example, in certain embodiments, the blocking nucleic acid comprises the sequence G*AGAT*T*TCAC*T*GTAGC*invdTinvdTinvdT (SEQ ID NO:10), GA*G*AT*TTCACTGT*AGCinvdTinvdTinvdT (SEQ ID NO: 146), G*AGA*TT*TC*AC*TGTAG*C invdTinvdTinvdT (SEQ ID NO: 147), *GAGAT*T*TCACT*G*TAGC*invdTinvdTinvdT (SEQ ID NO:148), or GA*GA*T*TT*C*ACT*G*T*A*GC*invdTinvdTinvdT (SEQ ID NO: 149), with italicized nucleic acids representing locked nucleic acids. In certain embodiments, the blocking nucleic acid comprises the sequence GAGATTTCACTGTAGCinvdTinvdTinvdT (SEQ ID NO: 10), GAGATTTCACTGTAGCinvdTinvdTinvdT (SEQ ID NO: 146), GAGATTTCACTGTAGC invdTinvdTinvdT (SEQ ID NO: 147), GAGATTTCACTGTAGCinvdTinvdTinvdT (SEQ ID NO:148), or GAGATTTCACTGTAGCinvdTinvdTinvdT (SEQ ID NO: 149), with underlined nucleic acids representing locked nucleic acids. In some embodiments, the blocking nucleic acid comprises the sequence of SEQ ID NO: 10 or 146-149 but optionally includes a different pattern or type of modified nucleotide(s). In some embodiments, the blocking nucleic acid comprises the sequence of SEQ ID NO: 10 or 146-149 but includes a different 3' terminal moiety.

In some embodiments, a blocking nucleic acid of the present disclosure hybridizes with a wild-type *CTNNB1* locus corresponding with the locus of one or more mutations encoding a T41A or T41I mutated CTNNB1 protein. In some embodiments, the blocking nucleic acid comprises the sequence GC*CACTACCACAG*CT(invdT)*ₙ*, wherein *n is* 1, 2, or 3 (SEQ ID NO: 15), T*G*C*CA*CT*ACCA*CA*G*(invdT)*ₙ*, wherein *n is* 1, 2, or 3 (SEQ ID NO: 150), C*AC*T*ACCA*C*AGC*T*C*C(invdT)*ₙ,* wherein *n is* 1, 2, or 3 (SEQ ID NO:151), G*CCACT*A*CCA*C*AG*C*T*(invdT)*ₙ,* wherein *n is* 1, 2, or 3 (SEQ ID NO: 152), or *GC*C*ACTA*CCA*CAG*CT(invdT)*ₙ,* wherein *n is* 1, 2, or 3 (SEQ ID NO: 153), with italicized nucleic acids representing locked nucleic acids. In some embodiments, *n* is 1, 2, 3, 4, 5, 6, 7, 8, 9, or 10. For example, in certain embodiments, the blocking nucleic acid comprises the sequence GC*CACTACCACAG*CTinvdTinvdTinvdT (SEQ ID NO:15), T*G*C*CA*CT*ACCA*C*AG*invdTinvdTinvdT (SEQ ID NO:150), C*AC*T*ACCA*C*AGC*T*C*CinvdTinvdTinvdT (SEQ ID NO:151), G*CCACT*A*CCA*C*AG*C*T*invdTinvdTinvdT (SEQ ID NO: 152), or *GC*C*ACTA*CCA*CAG*CTinvdTinvdTinvdT (SEQ ID NO: 153), with italicized nucleic acids representing locked nucleic acids. In certain embodiments, the blocking nucleic acid comprises the sequence GCCACTACCACAGCTinvdTinvdTinvdT (SEQ ID NO:15), TGCCACTACCACAGinvdTinvdTinvdT (SEQ ID NO:150), CACTACCACAGCTCCinvdTinvdTinvdT (SEQ ID NO:151), GCCACTACCACAGCTinvdTinvdTinvdT (SEQ ID NO:152), or GCCACTACCACAGCTinvdTinvdTinvdT (SEQ ID NO: 153), with underlined nucleic acids representing locked nucleic acids. In some embodiments, the blocking nucleic acid comprises the sequence of SEQ ID NO: 15 or 150-153 but optionally includes a different pattern or type of modified nucleotide(s). In some embodiments, the blocking nucleic acid comprises the sequence of SEQ ID NO: 15 or 150-153 but includes a different 3' terminal moiety.

In some embodiments, a blocking nucleic acid of the present disclosure hybridizes with a wild-type *CTNNB1* locus corresponding with the locus of one or more mutations encoding an S45F or S45P mutated CTNNB1 protein. In some embodiments, the blocking nucleic acid comprises the sequence GC*TCCTTCTCTG*AGT(invdT)*ₙ,* wherein *n is* 1, 2, or 3 (SEQ ID NO:20), T*CC*T*TCTC*T*G*A*G*T*G*G(invdT)*ₙ,* wherein *n is* 1, 2, or 3 (SEQ ID NO: 174), G*C*T*CC*T*TC*TC*TGA*GT(invdT)n, wherein n is 1, 2, or 3 (SEQ ID NO: 175), T*CC*TT*CT*CT*GAG*T*G*G(invdT)n, wherein n is 1, 2, or 3 (SEQ ID NO: 176), or G*C*T*CC*TT*CT*C*TGAG*T(invdT)n, wherein n is 1, 2, or 3 (SEQ ID NO: 177), with italicized nucleic acids representing locked nucleic acids. In some embodiments, *n* is 1, 2, 3, 4, 5, 6, 7, 8, 9, or 10. For example, in certain embodiments, the blocking nucleic acid comprises the sequence GC*TCCTTCTCTG*AGTinvdTinvdTinvdT (SEQ ID NO:20), T*CC*T*TCT*CT*G*A*G*T*G*GinvdTinvdTinvdT (SEQ ID NO: 174), G*C*T*CC*T*TC*TC*TGA*GTinvdTinvdTinvdT (SEQ ID NO: 175), T*CC*TT*CT*CT*GAG*T*G*GinvdTinvdTinvdT (SEQ ID NO: 176), or G*C*T*CC*TT*CT*C*TGAG*TinvdTinvdTinvdT (SEQ ID NO: 177), with italicized nucleic acids representing locked nucleic acids. In certain embodiments, the blocking nucleic acid comprises the sequence GCTCCTTCTCTGAGTinvdTinvdTinvdT (SEQ ID NO:20), TCCTTCTCTGAGTGGinvdTinvdTinvdT (SEQ ID NO: 174), GCTCCTTCTCTGAGTinvdTinvdTinvdT (SEQ ID NO: 175), TCCTTCTCTGAGTGG invdTinvdTinvdT (SEQ ID NO: 176), or GCTCCTTCTCTGAGT invdTinvdTinvdT (SEQ ID NO: 177), with underlined nucleic acids representing locked nucleic acids. In some embodiments, the blocking nucleic acid comprises the sequence of SEQ ID NO:20 or 174-177 but optionally includes a different pattern or type of modified nucleotide(s). In some embodiments, the blocking nucleic acid comprises the sequence of SEQ ID NO: 20 or 174-177 but includes a different 3' terminal moiety.

In some embodiments, a blocking nucleic acid of the present disclosure hybridizes with a wild-type *APC* locus corresponding with the locus of one or more mutations encoding a Q1367* mutated APC protein. In some embodiments, the blocking nucleic acid comprises the sequence GTG*CTCA*G*ACAC*C(invdT)*ₙ,* wherein *n is* 1, 2, or 3 (SEQ ID NO:33), G*TGC*TC*A*G*A*C*ACC*(invdT)n, wherein n is 1, 2, or 3 (SEQ ID NO:158), AGTGGTG*CTCAGAC*ACCCA(invdT)n, wherein n is 1, 2, or 3 (SEQ ID NO:159), A*GTG*GT*G*C*TC*AG*A*C*ACCC*A(invdT)n*,* wherein n is 1, 2, or 3 (SEQ ID NO:160), or A*G*T*G*GTGC*TCA*G*AC*A*C*C*CA*(invdT)n, wherein n is 1, 2, or 3 (SEQ ID NO:161), with italicized nucleic acids representing locked nucleic acids. In some embodiments, n is 1, 2, 3, 4, 5, 6, 7, 8, 9, or 10. For example, in certain embodiments, the blocking nucleic acid comprises the sequence GTG*CTCA*G*ACAC*CinvdTinvdTinvdT (SEQ ID NO:33), G*TGC*TC*A*G*A*C*ACC*invdTinvdTinvdT (SEQ ID NO: 158), AGTGGTG*CTCAGAC*ACCCAinvdTinvdTinvdT (SEQ ID NO:159), A*GTG*GT*G*C*TC*AG*A*CA*CCC*AinvdTinvdTinvdT (SEQ ID NO: 160), or A*G*T*G*GTGC*TCA*G*AC*A*C*C*C*AinvdTinvdTinvdT (SEQ ID NO: 161), with italicized nucleic acids representing locked nucleic acids. In certain embodiments, the blocking nucleic acid comprises the sequence GTGCTCAGACACCinvdTinvdTinvdT (SEQ ID NO:33), GTGCTCAGACACCinvdTinvdTinvdT (SEQ ID NO:158), AGTGGTGCTCAGACACCCAinvdTinvdTinvdT (SEQ ID NO: 159), AGTGGTGCTCAGACACCCAinvdTinvdTinvdT (SEQ ID NO: 160), or AGTGGTGCTCAGACACCCAinvdTinvdTinvdT (SEQ ID NO:161), with underlined nucleic acids representing locked nucleic acids. In some embodiments, the blocking nucleic acid comprises the sequence of SEQ ID NO:33 or 158-161 but optionally includes a different pattern or type of modified nucleotide(s). In some embodiments, the blocking nucleic acid comprises the sequence of SEQ ID NO: 33 or 158-161 but includes a different 3' terminal moiety.

In some embodiments, a blocking nucleic acid of the present disclosure hybridizes with a wild-type *APC* locus corresponding with the locus of one or more mutations encoding an R1450* mutated APC protein. In some embodiments, the blocking nucleic acid comprises the sequence C*TTC*T*CGCT*T*GGT*T(invdT)*ₙ,* wherein *n is* 1, 2, or 3 (SEQ ID NO:37), *GTACTTCTCGCTTGGT(invdT)n,* wherein n is 1, 2, or 3 (SEQ ID NO: 162), *CTTCTCGCTTGGTT(invdT)n,* wherein n is 1, 2, or 3 (SEQ ID NO: 163), GT*ACT*T*CTCG*CT*TGG*T(invdT)n, wherein n is 1, 2, or 3 (SEQ ID NO: 164), or GT*A*C*TTCTCGC*TT*GG*T(invdT)n, wherein n is 1, 2, or 3 (SEQ ID NO: 165), with italicized nucleic acids representing locked nucleic acids. In some embodiments, n is 1, 2, 3, 4, 5, 6, 7, 8, 9, or 10. For example, in certain embodiments, the blocking nucleic acid comprises the sequence C*TTC*T*CGCT*T*GGT*TinvdTinvdTinvdT (SEQ ID NO:37), G*TAC*T*T*C*TCG*CT*TGG*TinvdTinvdTinvdT (SEQ ID NO: 162), C*TTC*T*CGCT*T*GGT*TinvdTinvdTinvdT (SEQ ID NO: 163), GTA*CT*T*CTCG*CT*TGG*TinvdTinvdTinvdT (SEQ ID NO: 164), or GT*ACTTCTCGC*TT*GG*TinvdTinvdTinvdT (SEQ ID NO: 165), with italicized nucleic acids representing locked nucleic acids. In certain embodiments, the blocking nucleic acid comprises the sequence CTTCTCGCTTGGTTinvdTinvdTinvdT (SEQ ID NO:37), GTACTTCTCGCTTGGTinvdTinvdTinvdT (SEQ ID NO: 162), CTTCTCGCTTGGTTinvdTinvdTinvdT (SEQ ID NO: 163), GTACTTCTCGCTTGGTinvdTinvdTinvdT (SEQ ID NO: 164), or GTACTTCTCGCTTGGTinvdTinvdTinvdT (SEQ ID NO:165), with underlined nucleic acids representing locked nucleic acids. In some embodiments, the blocking nucleic acid comprises the sequence of SEQ ID NO:37 OR 162-165 but optionally includes a different pattern or type of modified nucleotide(s). In some embodiments, the blocking nucleic acid comprises the sequence of SEQ ID NO: 37 OR 162-165 but includes a different 3' terminal moiety.

In some embodiments, a blocking nucleic acid of the present disclosure hybridizes with a wild-type *APC* locus corresponding with the locus of one or more mutations encoding an E1309 frameshift mutated APC protein. In some embodiments, the blocking nucleic acid comprises the sequence *CTTTTCTTTTAT*TT*C*T*GC*(invdT)*ₙ,* wherein *n is* 1, 2, or 3 (SEQ ID NO:29), C*TTTTC*T*TTTA*T*I*T*C*TG*C*(invdT)n, wherein n is 1, 2, or 3 (SEQ ID NO:154), *CTTTTCTTTTATTTCTGC(invdT)n,* wherein n is 1, 2, or 3 (SEQ ID NO:155), *CTTTTCTTTTATTTCTGC(invdT)n,* wherein n is 1, 2, or 3 (SEQ ID NO:156), or *CTTTTCTTTTATTTCTGC(invdT)n,* wherein n is 1, 2, or 3 (SEQ ID NO:157), with italicized nucleic acids representing locked nucleic acids. In some embodiments, *n* is 1, 2, 3, 4, 5, 6, 7, 8, 9, or 10. For example, in certain embodiments, the blocking nucleic acid comprises the sequence C*TTTTCTTTTAT*TT*C*T*GC*invdTinvdTinvdT (SEQ ID NO:29), C*TTTTC*T*TTTA*T*T*T*C*TG*C*invdTinvdTinvdT (SEQ ID NO:154), C*T*TT*TC*T*T*T*TATTTCTGC*invdTinvdTinvdT (SEQ ID NO:155), C*TTT*TCT*TTT*A*T*T*TC*TG*C*invdTinvdTinvdT (SEQ ID NO:156), or C*T*TT*TCTTTTATTTC*TG*C*invdTinvdTinvdT (SEQ ID NO:157), with italicized nucleic acids representing locked nucleic acids. In certain embodiments, the blocking nucleic acid comprises the sequence CTTTTCTTTTATTTCTGCinvdTinvdTinvdT (SEQ ID NO:29), CTTTTCTTTTATTTCTGCinvdTinvdTinvdT (SEQ ID NO:154), CTTTTCTTTTATTTCTGCinvdTinvdTinvdT (SEQ ID NO:155), CTTTTCTTTTATTTCTGCinvdTinvdTinvdT (SEQ ID NO:156), or CTTTTCTTTTATTTCTGCinvdTinvdTinvdT (SEQ ID NO:157), with underlined nucleic acids representing locked nucleic acids. In some embodiments, the blocking nucleic acid comprises the sequence of SEQ ID NO:29or 154-157 but optionally includes a different pattern or type of modified nucleotide(s). In some embodiments, the blocking nucleic acid comprises the sequence of SEQ ID NO: 29or 154-157 but includes a different 3' terminal moiety.

In some embodiments, a blocking nucleic acid of the present disclosure hybridizes with a wild-type *APC* locus corresponding with the locus of one or more mutations encoding an S1465 frameshift mutated APC protein. In some embodiments, the blocking nucleic acid comprises the sequence *CCA*C*TC*TCTCT*C*T*TT*T*CAGC*(invdT)*ₙ,* wherein *n is* 1, 2, or 3 (SEQ ID NO:25), TA*GG*T*CC*ACTCTCTCTCT*TT*T*C*A*GC*A(invdT)n, wherein n is 1, 2, or 3 (SEQ ID NO:166), T*AGG*T*CCAC*T*CTCT*C*T*CTT*T*TC*AG*CA (invdT)n, wherein n is 1, 2, or 3 (SEQ ID NO:167), *CCACTCTCTCTCTTTTCAGC* (invdT)n, wherein n is 1, 2, or 3 (SEQ ID NO:168), or TA*G*GT*CC*AC*T*CT*C*TCT*C*T*T*TT*C*A*GC*A (invdT)n, wherein n is 1, 2, or 3 (SEQ ID NO:169), with italicized nucleic acids representing locked nucleic acids. In some embodiments, n is 1, 2, 3, 4, 5, 6, 7, 8, 9, or 10. For example, in certain embodiments, the blocking nucleic acid comprises the sequence *CCA*C*TC*TCTCT*C*T*TT*T*CAGC*invdTinvdTinvdT (SEQ ID NO:25), TA*GG*T*CC*ACTCTCTCTCT*TT*T*C*A*GC*AinvdTinvdTinvdT (SEQ ID NO:166), T*AGG*T*CCAC*T*CTCT*CTCTT*T*TC*AG*CA invdTinvdTinvdT (SEQ ID NO:167), C*CA*C*T*C*T*C*T*C*T*CTTTT*C*A*GC* invdTinvdTinvdT (SEQ ID NO:168), or TA*G*GT*CC*AC*T*CT*C*TCT*C*T*T*TT*C*A*GC*A invdTinvdTinvdT (SEQ ID NO:169), with italicized nucleic acids representing locked nucleic acids. In certain embodiments, the blocking nucleic acid comprises the sequence CCACTCTCTCTCTTTTCAGCinvdTinvdTinvdT (SEQ ID NO:25), TAGGTCCACTCTCTCTCTTTTCAGCAinvdTinvdTinvdT (SEQ ID NO:166), TAGGTCCACTCTCTCTCTTTTCAGCA invdTinvdTinvdT (SEQ ID NO:167), CCACTCTCTCTCTTTTCAGC invdTinvdTinvdT (SEQ ID NO:168), or TAGGTCCACTCTCTCTCTTTTCAGCA invdTinvdTinvdT (SEQ ID NO:169), with underlined nucleic acids representing locked nucleic acids. In some embodiments, the blocking nucleic acid comprises the sequence of SEQ ID NO:25 or 166-169 but optionally includes a different pattern or type of modified nucleotide(s). In some embodiments, the blocking nucleic acid comprises the sequence of SEQ ID NO: 25 or 166-169 but includes a different 3' terminal moiety.

In some embodiments, a blocking nucleic acid of the present disclosure hybridizes with a wild-type *APC* locus corresponding with the locus of one or more mutations encoding a T1556 frameshift mutated APC protein. In some embodiments, the blocking nucleic acid comprises the sequence *C*A*ATAG*T*TTTT*T*CTGC*C(invdT)*ₙ,* wherein *n is* 1, 2, or 3 (SEQ ID *NO:41), GAATCAATAGTTTTTTCTGCCTC* (invdT)n, wherein n is 1, 2, or 3 (SEQ ID NO:170), *TCAGAATCAATAGTTTTTTCTG* (invdT)n, wherein n is 1, 2, or 3 (SEQ ID NO:171), *GAATCAATAGATTTTACTGCCTC* (invdT)n, wherein n is 1, 2, or 3 (SEQ ID NO:172), or *AATCAATAGTTTTTTCTGCCTC* (invdT)n, wherein n is 1, 2, or 3 (SEQ ID NO:173), with italicized nucleic acids representing locked nucleic acids. In some embodiments, n is 1, 2, 3, 4, 5, 6, 7, 8, 9, or 10. For example, in certain embodiments, the blocking nucleic acid comprises the sequence *C*A*ATAGTTTT*T*CTGC*CinvdTinvdTinvdT (SEQ ID NO:41), *G*A*A*T*CAATAG*TTTTTT*CTGCCT*C invdTinvdTinvdT (SEQ ID NO:170), T*CAG*A*ATC*A*ATAG*TTTTT*TCTG* invdTinvdTinvdT (SEQ ID NO:171), *G*A*A*T*CAATAG*ATTTTA*CTGCCT*C invdTinvdTinvdT (SEQ ID NO:172), or A*A*T*CAATAG*TTTTTTC*TGCCT*C invdTinvdTinvdT (SEQ ID NO:173), with italicized nucleic acids representing locked nucleic acids. In certain embodiments, the blocking nucleic acid comprises the sequence CAATAGTTTTTTCTGCCinvdTinvdTinvdT (SEQ ID NO:41), GAATCAATAGTTTTTTCTGCCTC invdTinvdTinvdT (SEQ ID NO: 170), TCAGAATCAATAGTTTTTTCTG invdTinvdTinvdT (SEQ ID NO:171), GAATCAATAGATTTTACTGCCTC invdTinvdTinvdT (SEQ ID NO: 172), or AATCAATAGTTTTTTCTGCCTC invdTinvdTinvdT (SEQ ID NO: 173), with underlined nucleic acids representing locked nucleic acids. In some embodiments, the blocking nucleic acid comprises the sequence of SEQ ID NO:41 or 170-173 but optionally includes a different pattern or type of modified nucleotide(s). In some embodiments, the blocking nucleic acid comprises the sequence of SEQ ID NO: 41 or 170-173 but includes a different 3' terminal moiety.

### Hybridization

In some embodiments, the methods of the present disclosure include hybridizing amplified DNA with one or more probes specific for a DNA mutation of the present disclosure (*e.g.,* a DNA mutation in the *KRAS, BRAF, CTNNB1,* or *APC* gene as described *supra*)*.* In some embodiments, the methods include hybridizing amplified DNA with at least four probes, comprising one or more probes specific for a DNA mutation in each of the *KRAS, BRAF, CTNNB1, and APC* genes (*e.g.,* one or more probes representing a mutation in each gene). As used herein, a probe may refer to an oligonucleotide that is capable of hybridization with at least a portion of the locus of a DNA mutation of interest. For example, a probe may include a single-stranded oligonucleotide that is able to base pair with most or all of the base pairs of a single-stranded DNA template that includes a DNA mutation of interest. For specific detection of a DNA mutation, the probe is able to hybridize with a locus bearing the DNA mutation, but not with the corresponding wild-type locus (cf. **FIGS. 15** **&** **16**). Conditions suitable for hybridization of a probe with amplified DNA are known in the art (*e.g.,* as referenced in the materials cited herein) and exemplified *infra.*

In some embodiments, a probe of the present disclosure is coupled to an encoded microcarrier of the present disclosure, *e.g*., as described in section IV. Exemplary methods for coupling a polynucleotide probe to a microcarrier surface are known in the art and provided in section IV. For multiplex assays, each type of probe can be coupled to a microcarrier with a particular identifier corresponding to the probe type. Advantageously, this allows the user to correlate a signal detected from the probe with the probe's identity, enabling multiplex assays in which multiple probes are utilized. In some embodiments, a probe of the present disclosure comprises a 5' modification, *e.g*., a 5'amino modifier C6.

In some embodiments, a probe of the present disclosure comprises (1) a sequence that hybridizes with at least a portion of the locus of a DNA mutation of interest; and (2) one or more additional nucleotides. The one or more additional nucleotides may be used, *e.g.,* to couple the probe to the microcarrier surface and/or to provide spacing to reduce steric hindrance between the microcarrier surface and the amplified DNA during hybridization. In some embodiments, the one or more additional nucleotides are at the 5' end of the probe sequence. In other embodiments, the one or more additional nucleotides are at the 3' end of the probe sequence. In some embodiments, the one or more additional nucleotides are adenine or thymine nucleotides. Advantageously, this reduces the affinity of non-specific binding. In some embodiments, a probe of the present disclosure comprises 1 or more, 2 or more, 3 or more, 4 or more, 5 or more, 6 or more, 7 or more, or 8 or more adenine or thymine nucleotides at the 5' end. In some embodiments, a probe of the present disclosure comprises at least 20, at least 24, at least 25, or at least 30 total nucleotides.

Exemplary probe sequences are provided below. One of ordinary skill in the art can readily select any number of probe sequences suitable for hybridization with an amplified DNA sequence of interest (*e.g.,* a locus comprising a DNA mutation of interest) using well-known techniques.

In some embodiments, a probe specific for a DNA mutation in a *KRAS* gene comprises the sequence GGAGCTGATGG (SEQ ID NO:4), AGCTGATGGCGTA (SEQ ID NO:178), TGGAGCTGATGGCG (SEQ ID NO:179), TGGAGCTGATGG (SEQ ID NO:180), GCTGATGGCGTA (SEQ ID NO:181), GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO:184), TGGAGCTGTAGGTGG (SEQ ID NO:185), TGGAGCTAGTGG (SEQ ID NO:6), TTGGAGCTAGTGGCGTA (SEQ ID NO: 186), GCTAGTGGCGTAGGC (SEQ ID NO: 187), AGCTAGTGGCGT (SEQ ID NO: 188), GTTGGAGCTAGTGG (SEQ ID NO:189), GGAGCTAGTGG (SEQ ID NO: 190), or TGGAGCTGGTGACGT (SEQ ID NO:7). For example, a probe comprising the sequence GGAGCTGATGG (SEQ ID NO:4), AGCTGATGGCGTA (SEQ ID NO:178), TGGAGCTGATGGCG (SEQ ID NO: 179), TGGAGCTGATGG (SEQ ID NO: 180), or GCTGATGGCGTA (SEQ ID NO:181) can be used to detect a mutation encoding a G12D mutated KRAS protein; a probe comprising the sequence GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO: 184), or TGGAGCTGTaGGTGG (SEQ ID NO: 185) can be used to detect a mutation encoding a G12V mutated KRAS protein; a probe comprising the sequence TGGAGCTAGTGG (SEQ ID NO:6), TTGGAGCTAGTGGCGTA (SEQ ID NO:186), GCTAGTGGCGTAGGC (SEQ ID NO: 187), AGCTAGTGGCGT (SEQ ID NO: 188), GTTGGAGCTAGTGG (SEQ ID NO:189), or GGAGCTAGTGG (SEQ ID NO:190) can be used to detect a mutation encoding a G12S mutated KRAS protein; and/or a probe comprising the sequence TGGAGCTGGTGACGT (SEQ ID NO:7), GGTGACGTAGGCAA (SEQ ID NO:191), TGACGTAGGCAAGAG (SEQ ID NO: 192), GCTGGTGACGTAGG (SEQ ID NO:193), AGCTGGTGACGTAG (SEQ ID NO: 194), or GGAGCTGGTGACGT (SEQ ID NO: 195) can be used to detect a mutation encoding a G13D mutated KRAS protein. As described *supra,* in some embodiments, one or more probes of the present disclosure can comprise eight or more nucleotides (*e.g*., adenines or thymines) at its 5' end. In certain embodiments, a probe comprising the sequence TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), or TTTTTTTTTTTTTGCTGATGGCGTA (SEQ ID NO:77) can be used to detect a mutation encoding a G12D mutated KRAS protein. In certain embodiments, a probe comprising the sequence TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), or TTTTTTTTTATGGAGCTGTAGGTGG (SEQ ID NO:81) can be used to detect a mutation encoding a G12V mutated KRAS protein. In certain embodiments, a probe comprising the sequence TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), or TTTTTTTTTTTTAAGGAGCTAGTGG (SEQ ID NO:86) can be used to detect a mutation encoding a G12S mutated KRAS protein. In certain embodiments, a probe comprising the sequence TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), or TTTTTTTTTAAGGAGCTGGTGACGT (SEQ ID NO:91) can be used to detect a mutation encoding a G13D mutated KRAS protein (probes comprising these sequences exclusive of the 5' adenine and/or thymines are also contemplated).

In some embodiments, a probe specific for a DNA mutation in a *BRAF* gene comprises the sequence TCTAGCTACAGAGAAAT (SEQ ID NO:11) or GTCTAGCTACAGAAAAAT (SEQ ID NO:12). For example, a probe comprising the sequence TCTAGCTACAGAGAAAT (SEQ ID NO:11), TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), or TCTAGCTACAGAG (SEQ ID NO:200) can be used to detect a mutation encoding a V600E mutated BRAF protein (*e.g.,* a c.1799T>A DNA mutation), and/or a probe comprising the sequence GTCTAGCTACAGAAAAAT (SEQ ID NO:12) can be used to detect a mutation encoding a V600E mutated BRAF protein (*e.g.,* a c.1799 _1800TG>AA DNA mutation). As described *supra,* in some embodiments, one or more probes of the present disclosure can comprise eight or more nucleotides (*e.g*., adenines or thymines) at its 5' end and/or comprise at least 24 total nucleotides. In certain embodiments, a probe comprising the sequence TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), or TTTTTTTTTTAATTTCTAGCTACAGAG (SEQ ID NO:96) can be used to detect a mutation encoding a V600E mutated BRAF protein (*e.g.,* a c.1799T>A DNA mutation), and/or a probe comprising the sequence TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), or TTTTTTTTATTCTAGCTACAGAAAAATC (SEQ ID NO:100) can be used to detect a mutation encoding a V600E mutated BRAF protein (*e.g.,* a c.1799 _1800TG>AA DNA mutation) (probes comprising these sequences exclusive of the 5' adenine and/or thymines are also contemplated).

In some embodiments, a probe specific for a DNA mutation in a *CTNNB1* gene comprises the sequence AGGAGCTGTGGCAG (SEQ ID NO:16), GGAGCTGTGATA (SEQ ID NO:17), TTTACCACTCAGAAAAG (SEQ ID NO:21), TACCACTCAGAGGAG (SEQ ID NO:22), AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), AAGGAGCTGTGGCAG (SEQ ID NO:209), GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), GGAGCTGTGAT (SEQ ID NO:214), ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), CAGAAAAGGAGCTGTG (SEQ ID NO:219), ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), or TTACCAATCAGAGGAGG (SEQ ID NO:224). For example, a probe comprising the sequence AGGAGCTGTGGCAG (SEQ ID NO:16), AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), or AAGGAGCTGTGGCAG (SEQ ID NO:209) can be used to detect a mutation encoding a T41A mutated CTNNB1 protein; a probe comprising the sequence GGAGCTGTGATA (SEQ ID NO:17), GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), or GGAGCTGTGAT (SEQ ID NO:214) can be used to detect a mutation encoding a T41I mutated CTNNB1protein; a probe comprising the sequence TTTACCACTCAGAAAAG (SEQ ID NO:21), ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), or CAGAAAAGGAGCTGTG (SEQ ID NO:219 can be used to detect a mutation encoding an S45F mutated CTNNB1 protein; and/or a probe comprising the sequence TACCACTCAGAGGAG (SEQ ID NO:22), ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), or TTACCAATCAGAGGAGG (SEQ ID NO:224) can be used to detect a mutation encoding an S45P mutated CTNNB1 protein. As described *supra,* in some embodiments, one or more probes of the present disclosure can comprise eight or more nucleotides (*e.g*., adenines or thymines) at its 5' end and/or comprise at least 24 total nucleotides. In certain embodiments, a probe comprising the sequence TTTTTTTTTTTAGGAGCTGTGGCAG (SEQ ID NO:53), TTTTTTTTTTTAGGAGCTGTGGCAGTG (SEQ ID NO: 101), TTTTTTTTTTTAGCTGTGGCAGTGGC (SEQ ID NO: 102), TTTTTTTTTTTGCTGTGGCAGTGGCA (SEQ ID NO:103), or TTTTTTTTTTAAGGAGCTGTGGCAG (SEQ ID NO: 104) can be used to detect a mutation encoding a T41A mutated CTNNB1protein; a probe comprising the sequence TTTTTTTTTTTTTGGAGCTGTGATA (SEQ ID NO:54), TTTTTTTTTGGAGCTGTGATAGTGG (SEQ ID NO: 105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), or TTTTTTTTTTTTTTGGAGCTGTGAT (SEQ ID NO:109) can be used to detect a mutation encoding a T41I mutated CTNNB1 protein; a probe comprising the sequence TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), or TTTTTTTTTCAGAAAAGGAGCTGTG (SEQ ID NO:114) can be used to detect a mutation encoding an S45F mutated CTNNB1 protein; and/or a probe comprising the sequence TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), or TTTTTTTTTATTACCAATCAGAGGAGG (SEQ ID NO:119) can be used to detect a mutation encoding an S45P mutated CTNNB1 protein (probes comprising these sequences exclusive of the 5' adenine and/or thymines are also contemplated).

In some embodiments, a probe specific for a DNA mutation in an *APC* gene comprises the sequence ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), AGAGGCAGAAAAAAACT (SEQ ID NO:42), AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), CAGAAATAAAAGATT (SEQ ID NO:229), TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), GGTGTCTAAGCACCA (SEQ ID NO:234), AAGTGAGAAGTACCTAA (SEQ ID NO:235), TCAAACCAAGTGAG (SEQ ID NO:236), ACCAAGTGAGAAGTA (SEQ ID NO:237), AGCTCAAACCAAGTGAG (SEQ ID NO:238), GCACCTACTGCTGAA (SEQ ID NO:239), ACCTACTGCTGAAAAG (SEQ ID NO:240), TGCTGAAAAGAGAGAGT (SEQ ID NO:241), CCTACTGCTGAAAAGAGA (SEQ ID NO:242), GCAGAAAAAAACTATTG (SEQ ID NO:243), CAGAAAAAAACTATTGATT (SEQ ID NO:244), AGAAAGAGGCAGAAAAAAACT (SEQ ID NO:245), or GAGGCAGAAAAAAACTA (SEQ ID NO:246). For example, a probe comprising the sequence ACTGCTGAAAAGAGAGAGT (SEQ ID NO:26), GCACCTACTGCTGAA (SEQ ID NO:239), ACCTACTGCTGAAAAG (SEQ ID NO:240), TGCTGAAAAGAGAGAGT (SEQ ID NO:241), or CCTACTGCTGAAAAGAGA (SEQ ID NO:242) can be used to detect a mutation encoding an S1465 frameshift mutated APC protein; a probe comprising the sequence GAAATAAAAGATTGG (SEQ ID NO:30), AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), or CAGAAATAAAAGATT (SEQ ID NO:229) can be used to detect a mutation encoding an E1309 frameshift mutated APC protein; a probe comprising the sequence TTTTGGGTGTCTAAG (SEQ ID NO:34), TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), or GGTGTCTAAGCACCA (SEQ ID NO:234) can be used to detect a mutation encoding a Q1367* mutated APC protein; a probe comprising the sequence CAAACCAAGTGAGAA (SEQ ID NO:38), AAGTGAGAAGTACCTAA (SEQ ID NO:235), TCAAACCAAGTGAG (SEQ ID NO:236), ACCAAGTGAGAAGTA (SEQ ID NO:237), or AGCTCAAACCAAGTGAG (SEQ ID NO:238) can be used to detect a mutation encoding a R1450* mutated APC protein; and/or a probe comprising the sequence GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246) can be used to detect a mutation encoding a T1556 frameshift mutated APC protein. As described *supra,* in some embodiments, one or more probes of the present disclosure can comprise eight or more nucleotides (*e.g*., adenines or thymines) at its 5' end and/or comprise at least 24 total nucleotides. In certain embodiments, a probe comprising the sequence TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO:136), or TTTTTTTTTCCTACTGCTGAAAAGAGA (SEQ ID NO: 137) can be used to detect a mutation encoding an S1465 frameshift mutated APC protein; a probe comprising the sequence TTTTTTTTTTTTTGAAATAAAAGATTGG (SEQ ID NO:58), TTTTTTTTTTAAATAGCAGAAATAAAAG (SEQ ID NO: 120), TTTTTTTTTTTGAAATAAAAGATTGGAA (SEQ ID NO:121), TTTTTTTTTTTTTAGAAATAAAAGATTG (SEQ ID NO: 122), TTTTTTTTTTTTTGAAATAAATGAATGG (SEQ ID NO:123), or TTTTTTTTTTTTTCAGAAATAAAAGATT (SEQ ID NO:124) can be used to detect a mutation encoding an E1309 frameshift mutated APC protein; a probe comprising the sequence TTTTTTTTTTTTTGGGTGTCTAAG (SEQ ID NO:59), TTTTTTTTTATTTGGGTGTCTAAG (SEQ ID NO: 125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128), or TTTTTTTTTGGTGTCTAAGCACCA (SEQ ID NO: 129) can be used to detect a mutation encoding a Q1367* mutated APC protein; a probe comprising the sequence TTTTTTTTTACAAACCAAGTGAGAA (SEQ ID NO:60), TTTTTTTTAAGTGAGAAGTACCTAA (SEQ ID NO:130), TTTTTTTTTTTTCAAACCAAGTGAG (SEQ ID NO:131), TTTTTTTTTTACCAAGTGAGAAGTA (SEQ ID NO: 132), or TTTTTTTTAGCTCAAACCAAGTGAG (SEQ ID NO: 133) can be used to detect a mutation encoding a R1450* mutated APC protein; and/or a probe comprising the sequence TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO: 139), TTTTTTTAGAAAGAGGCAGAAAAAAACT (SEQ ID NO: 140), or TTTTTTTTTTTGAGGCAGAAAAAAACTA (SEQ ID NO: 141) can be used to detect a mutation encoding a T1556 frameshift mutated APC protein (probes comprising these sequences exclusive of the 5' adenine and/or thymines are also contemplated).

### Detection

In some embodiments, the methods of the present disclosure include detecting presence or absence of hybridization of amplified DNA with a probe of the present disclosure. Hybridization between the amplified DNA and one of the probes indicates the presence of the DNA mutation corresponding to the probe in the amplified DNA. Exemplary hybridization conditions and detection techniques are described and exemplified herein. In some embodiments, hybridization is performed using 5X SSPE buffer.

In some embodiments, the amplified DNA is labeled with a detection reagent, and hybridization is measured by signal of the detection reagent associated with the microcarrier, *e.g.,* after a washing step to reduce or eliminate non-specific binding. In some embodiments, a primer pair of the present disclosure comprises one or both primers coupled to the detection reagent. Thus, the amplified DNA is labeled with the detection reagent after PCR amplification using the labeled primer(s). In some embodiments, the detection reagent can be fluorescence-based including, but not limited to, phycoerythrin (PE), blue fluorescent protein, green fluorescent protein, yellow fluorescent protein, cyan fluorescent protein, and derivatives thereof. In other embodiments, the detection reagent can be radioisotope based, including, but not limited to, molecules labeled with ³²P, ³³P, ²²Na, ³⁶Cl, ²H, ³H, ³⁵S, and ¹²³I. In other embodiments, the detection reagent is light-based including, but not limited to, luciferase (*e.g*. chemiluminescence-based), horseradish peroxidase, alkaline phosphatase and derivatives thereof. In some embodiments, the amplified DNA can be labeled with the detection reagent prior to contact with the microcarrier composition. In some embodiments, the detection reagent emits a signal when in close proximity to the probe, *e.g.,* as with Förster resonance energy transfer (FRET). A variety of nucleic acid labeling techniques are known in the art; *see, e.g.,* Gibriel, A.A.Y. (2012) Briefings in Functional Genomics 11:311-8.

In some embodiments, the detection reagent can be a fluorescent detection reagent. In some embodiments, detecting the presence or absence of hybridization of amplified DNA with a probe is performed by fluorescence microscopy (*e.g*., a fluorescent microscope or plate reader). In some embodiments, the detection reagent can be colorimetric based. In some embodiments, the detection reagent can be luminescence based. In some embodiments, detecting the presence or absence of hybridization of amplified DNA with a probe is performed by luminescence microscopy (*e*.*g*., a luminescent microscope or plate reader).

In some embodiments, the detection reagent comprises a tag or other moiety that can be detected by the addition of a secondary reagent conjugated to a signal-emitting entity (*e.g.,* as described above). For example, in some embodiments, the detection reagent comprises biotin (*e.g.,* a primer such as the reverse or antisense primer may be labeled at the 5' end with biotin). Thus, in some embodiments, the detecting presence or absence of hybridization can include, after hybridization and optional washing, contacting microcarrier(s) with a secondary reagent conjugated to a signal-emitting entity and detecting a signal from the signal-emitting entity in association with the microcarrier(s) (*e.g*., after optional washing). For example, if the detection reagent comprises biotin, the microcarriers can be contacted with streptavidin conjugated to a signal-emitting entity such as phycoerythrin (PE), and signal from the signal-emitting entity can be detected.

Depending upon the particular detection reagent, a variety of techniques may be used to detect hybridization of the amplified DNA to a probe. For example, if the detection reagent comprises a fluorescent detection reagent, detecting the presence or absence of hybridization of the amplified DNA can comprise fluorescence imaging of the fluorescent detection reagent.

In some embodiments, detecting may include one or more washing steps, *e.g.,* to reduce contaminants, remove any substances non-specifically bound to the probe, DNA, and/or microcarrier surface, and so forth. In some embodiments, a magnetic separation step may be used to wash a microcarrier containing a magnetic layer or material of the present disclosure. In other embodiments, other separation steps known in the art may be used.

In some embodiments, the methods of the present disclosure comprise detecting the presence or absence of hybridization of amplified DNA with a total of between about 1 and about 1000 microcarriers per probe per assay. In some embodiments, the methods of the present disclosure comprise detecting the presence or absence of hybridization of amplified DNA with a total of between about 1 and about 1000 microcarriers per probe per well of an assay plate. In some embodiments, the methods of the present disclosure comprise detecting the presence or absence of hybridization of amplified DNA with at least about 50 microcarriers per probe per assay. In some embodiments, the methods of the present disclosure comprise detecting the presence or absence of hybridization of amplified DNA with at least about 50 microcarriers per probe per well of an assay plate. In some embodiments, a microcarrier of the present disclosure comprises the probe coupled thereto at a concentration of 1µM.

In some embodiments, the methods of the present disclosure comprise detecting the identifier of an encoded microcarrier. For example, in some embodiments, an image of the identifier of an encoded microcarrier can be obtained and decoded to identify the microcarrier and its corresponding probe. In some embodiments, the identifier detection step(s) may occur after the hybridization detection step(s). In other embodiments, the identifier detection step(s) may occur before the hybridization detection step(s). In still other embodiments, the identifier detection step(s) may occur simultaneously with the hybridization detection step(s). In some embodiments, after the hybridization detection step(s) and the identifier detection step(s), the methods of the present disclosure further comprise correlating the detected identifiers of the microcarriers with the detected presence or absence of hybridization of the amplified DNA to the corresponding probes of the microcarriers.

A variety of microcarrier coding schemes are described in section IV. In some embodiments, detecting the identifier of an encoded microcarrier comprises imaging a digital barcode of the microcarrier. In one embodiment, the coded microcarrier comprises a body having a series of alternating light transmissive and opaque sections, with relative widths bar code image (*e.g*., a series of narrow slits representing a "0" code and wide slits representing a "1" code, or vice versa). When the microcarrier is illuminated with a light beam, based on the either the "total intensity" of the transmission peak or the "bandwidth" of the transmission peak from the slit, the digital barcode either 0 or 1 can be determined by a line scan camera, a frame grabber, and a digital signal processor. In one embodiment, the bar code pattern with a series of narrow and wide bands provides an unambiguous signal and differentiation for 0's and 1's. The position of the slits on the pallet will determine which of the bits is the least significant (LSB) and most significant bit (MSB). The LSB will be placed closer to the edge of the pallet to distinguish it from the MSB at the other, longer end.

In some embodiments, detecting the identifier of an encoded microcarrier comprises imaging the identifier of the microcarrier, *e.g*., by bright field imaging of the identifier.

A variety of decoding techniques are contemplated. In some embodiments, an identifier is detected using analog shape recognition to identify the identifier (*e.g.,* an analog-encoded identifier). Conceptually, this decoding may involve, for example, imaging the analog code of each microcarrier (*e.g*., in a solution or sample), comparing each image against a library of analog codes, and matching each image to an image from the library, thus positively identifying the code. Optionally, as described herein, when using microcarriers that include an orientation indicator (*e.g*., an asymmetry), the decoding may further include a step of rotating each image to align with a particular orientation (based in part, *e.g.,* on the orientation indicator). For example, if the orientation indicator includes a gap, the image could be rotated until the gap reaches a predetermined position or orientation (*e.g*., a 0° position of the image).

Various shape recognition software, tools, and methods are known in the art. Examples of such APIs and tools include without limitation Microsoft^{®} Research FaceSDK, OpenBR, Face and Scene Recognition from ReKognition, Betaface API, and various ImageJ plugins. In some embodiments, the analog shape recognition may include without limitation image processing steps such as foreground extraction, shape detection, thresholding (*e.g*., automated or manual image thresholding), and the like.

It will be appreciated by one of skill in the art that the methods and microcarriers described herein may be adapted for various imaging devices, including without limitation a microscope, plate reader, and the like. In some embodiments, decoding an identifier (*e.g.,* an analog code) can include illuminating the microcarrier by passing light through a substantially transparent portion (*e.g*., substantially transparent polymer layer(s)) of the microcarrier and/or the surrounding solution). The light may then fail to pass through, or pass through with a lower intensity or other appreciable difference, the substantially non-transparent portions (*e.g*., substantially non-transparent polymer layer(s)) of the microcarrier to generate an analog-coded light pattern corresponding to the identifier. That is to say, the pattern of imaged light may correspond to the pattern of substantially transparent/substantially non-transparent areas of the microcarrier, thus producing an image of the analog code identifier. This imaging may include steps including without limitation capturing the image, thresholding the image, and any other image processing step desired to achieve more accurate, precise, or robust imaging of the identifier.

Any type of light microscopy may be used for the methods of the present disclosure, including without limitation one or more of: bright field, dark field, phase contrast, differential interference contrast (DIC), Nomarski interference contrast (NIC), Nomarski, Hoffman modulation contrast (HMC), or fluorescence microscopy. In certain embodiments, the identifiers may be decoded using bright field microscopy, and hybridization may be detected using fluorescence microscopy.

In some embodiments, decoding the identifiers may further include using analog shape recognition to match an analog-coded image with an analog code. In some embodiments, an image may be matched with an analog code (*e.g.,* an image file from a library of image files, with each image file corresponding to a unique two-dimensional shape/analog code) within a predetermined threshold, *e.g*., that tolerates a predetermined amount of deviation or mismatch between the image and the exemplar analog code image. Such a threshold may be empirically determined and may naturally be based on the particular type of two-dimensional shapes used for the analog codes and the extent of variation among the set of potential two-dimensional shapes.

In some embodiments, the methods of the present disclosure further comprise the use of microcarriers with an identifier corresponding to a positive or negative control. In some embodiments, the methods of the present disclosure comprise amplifying a positive control DNA sequence using a primer pair specific for the positive control DNA sequence. The positive control DNA sequence can be any sequence that is likely to be present in all samples of a given type, *e.g.,* a non-mutated or endogenous gene sequence from the organism from whence the sample is obtained. The positive control indicates that DNA (*e.g*., human DNA) is present in the sample at levels sufficient for detection. Like the mutated DNA sequences of interest, the positive control sequence is detected by amplifying a positive control DNA sequence using a primer pair specific for the positive control DNA sequence; hybridizing the amplified positive control gene sequence with a probe specific for the positive control gene sequence (the probe specific for the positive control gene sequence is coupled to a microcarrier with an identifier corresponding to a positive control); detecting presence or absence of hybridization of the amplified positive control DNA sequence with the probe specific for the positive control gene sequence; and detecting an analog code of the microcarrier with the identifier corresponding to the positive control.

In some embodiments, as exemplified *infra,* the positive control DNA sequence comprises a sequence of a human leukocyte antigen (HLA) gene. The sequences of a more than 150 HLA genes are known, including without limitation HLA-DRA (*see* NCBI Entrez Gene ID No. 3122), HLA-DRB1 (*see* NCBI Entrez Gene ID No. 3123), HLA-A (*see* NCBI Entrez Gene ID No. 3105), HLA-B (*see* NCBI Entrez Gene ID No. 3106), HLA-C (*see* NCBI Entrez Gene ID No. 3107), HLA-DQB1 (*see* NCBI Entrez Gene ID No. 3119), HLA-DPB1 (*see* NCBI Entrez Gene ID No. 3115), HLA-E *(*s*ee* NCBI Entrez Gene ID No. 3133), HLA-DQA2 (*see* NCBI Entrez Gene ID No. 3118), HLA-DPA1 (*see* NCBI Entrez Gene ID No. 3113), HLA-G (*see* NCBI Entrez Gene ID No. 3135), HLA-DRB5 (*see* NCBI Entrez Gene ID No. 3127), HLA-F (*see* NCBI Entrez Gene ID No. 3134), and HLA-DOA (*see* NCBI Entrez Gene ID No. 3111). For descriptions of other HLA genes, *see* Shiina, T. et al. (2009) J. Hum. Genet. 54:15-39.

In some embodiments, a primer pair specific for the positive control DNA sequence comprises the sequences TGAGTGTTACTTCTTCCCACACTC (SEQ ID NO:43) and ATTGCTTTTGCGCAATCCCT (SEQ ID NO:44). In some embodiments, the probe specific for the positive control gene sequence comprises the sequence TTTTTTTTTTTTGGAGACGGTCTG (SEQ ID NO:45). In some embodiments, a primer pair specific for the positive control DNA sequence comprises the sequences AATCCCATCACCATCTTCCA (SEQ ID NO:71) and TGGACTCCACGACGTACTCA (SEQ ID NO:72). In some embodiments, the probe specific for the positive control gene sequence comprises the sequence CTGTCTTCCACTCACTCC (SEQ ID NO:73).

In some embodiments, the methods of the present disclosure comprise detecting absence of hybridization of amplified DNA with a microcarrier having an identifier corresponding to a negative control. For example, the microcarrier with the identifier corresponding to the negative control can comprise a probe that does not hybridize with amplified DNA. The use of a microcarrier with the identifier corresponding to the negative control improves the reliability of detecting presence or absence of hybridization. In some embodiments, the microcarrier comprises a probe comprising the sequence AATATAATATATTAT (SEQ ID NO:46).

Exemplary and non-limiting descriptions of microcarriers, and optional aspects thereof, suitable for use in the methods of the present disclosure are provided *infra.*

### IV. Encoded Microcarriers

Provided herein are encoded microcarriers suitable for analyte detection, *e.g*., multiplex analyte detection. Multiple configurations for encoded microcarriers are contemplated, described, and exemplified herein. As used herein, an "encoded" microcarrier may refer to a microcarrier with an identifier that corresponds to the identity of a probe coupled thereto. This enables the data of an assay using the microcarrier to be associated with the identity of the probe, allowing for the use of multiple microcarriers in a single multiplex assay, since the results of any individual microcarrier can be correlated with the identity of its probe. Exemplary types of identifiers, including digital barcodes and analog codes, are described *infra.*

In some aspects, the methods and kits of the present disclosure use digital barcode identifiers. For example, in some embodiments, encoded microcarriers comprise: a first photopolymer layer; a second photopolymer layer; and an intermediate layer between the first layer and the second layer. In some embodiments, the intermediate layer has an encoded pattern representing the identifier defined thereon, wherein the intermediate layer is partially substantially transmissive and partially substantially opaque to light, representing a code corresponding to the microcarrier, wherein the outermost surface of the microcarrier comprises a photoresist photopolymer, and said photoresist photopolymer is functionalized with the probe specific for the DNA mutation, and wherein said microcarrier has about the same density as water. Exemplary microcarrier descriptions may be found, *e.g.,* in U.S. Patent Nos. 7,858,307; 7,871,770; 8,148,139; 8,232,092; and 9,255,922; as well as US PG Pub. Nos. US2009/201504, 2011/0007955, and 2012/0088691.

In one embodiment, a digitally encoded microcarrier of the present disclosure comprises a body having a series of alternating light transmissive and opaque sections, with relative positions, widths and/or spacing resembling a 1D or 2D bar code image (e.g., a series of narrow slits (e.g., about 1 to 5 microns in width) representing a "0" code and wide slits (e.g., about 1 to 10 microns in width) representing a "1" code, or vice versa, to form a binary code). In one embodiment, the size of the microcarrier is sized and configured to be 150×50×10 µm, or proportionally smaller, and a slit width of about 2.5 µm. Each digital barcode on such a microcarrier can consist of up to 14 slits (or bits), allowing 16,384 unique codes. In one embodiment, the body of the coded microcarrier may be configured to have at least two orthogonal cross sections that are different in relative geometry and/or size. Further, the geometry of the cross sections may be symmetrical or non-symmetrical, and/or regular or irregular shape. In one embodiment, the longest orthogonal axis of the coded microcarrier is less than 1 mm. In one embodiment, the coded microcarrier is provided with a reflective thin film, (e.g., plating or coating the coded microcarrier with a metal thin film, or providing an intermediate layer of metal thin film) to improve contrast and optical efficiency for image recognition for decoding. One alternate embodiment may include a metal layer as a layer sandwiched between two polymeric layers, by appropriately modifying the above described process. With this embodiment, surface condition could be made the same for both exposed planar surfaces of the microcarrier, to provide similar surface coating and immobilization conditions. Another embodiment is to coat the microcarrier with polymer or functional molecules, such as a probe of the present disclosure; therefore, the whole microcarrier has the same condition for molecular immobilization.

In some aspects, the methods and kits of the present disclosure use analog code identifiers. For example, in some embodiments, the methods and kits of the present disclosure use encoded microcarriers that comprise: a substantially transparent polymer layer having a first surface and a second surface, the first and the second surfaces being parallel to each other; a substantially non-transparent polymer layer, wherein the substantially non-transparent polymer layer is affixed to the first surface of the substantially transparent polymer layer and encloses a center portion of the substantially transparent polymer layer, and wherein the substantially non-transparent polymer layer comprises a two-dimensional shape representing an analog code; and a probe of the present disclosure specific for a DNA mutation, wherein the probe is coupled to at least one of the first surface and the second surface of the substantially transparent polymer layer in at least the center portion of the substantially transparent polymer layer. The analog code represents the identifier. Thus, the microcarrier contains at least two layers: one of which is substantially transparent, and the other of which is a substantially non-transparent, two-dimensional shape that represents an analog code identifier.

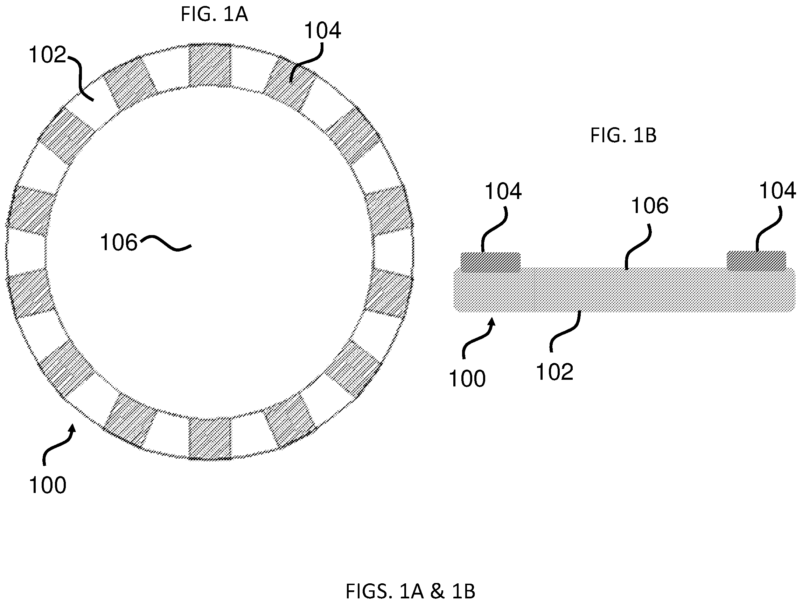

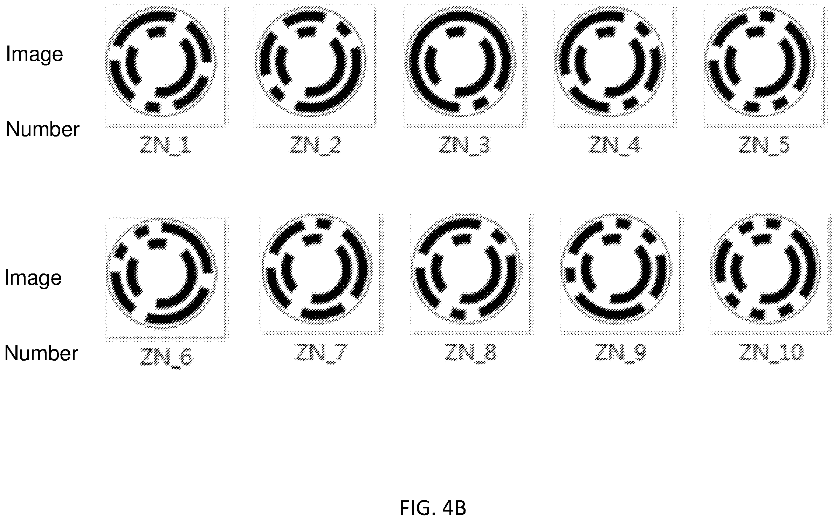

Advantageously, these microcarriers may employ a variety of two-dimensional shapes while still retaining a uniform overall form (*e.g.,* the perimeter of the substantially transparent polymer layer) for uniformity of aspects including, for example, overall dimensions, physical properties, and/or behavior in solution. This is advantageous, for example, in allowing greater uniformity between different species of microcarriers (*i.e.,* each has the same perimeter shape provided by the transparent polymer layer). Examples of this type of microcarrier and aspects thereof are illustrated in **FIGS. 1A-5B** (see, *e.g.,* **FIG. 4B**).

**FIGS. 1A & 1B** show two views of exemplary microcarrier 100. Microcarrier 100 is a circular disc of approximately 50 µm in diameter and 10 µm in thickness. **FIG. 1A** provides a view of microcarrier 100 looking at a circular face of the disc, while **FIG. 1B** shows a side view of microcarrier 100 orthogonal to the surface shown in **FIG. 1A****.** Two components of microcarrier 100 are shown. First, substantially transparent polymer layer 102 provides the body of the microcarrier. Layer 102 may be produced, *e.g*., using a polymer such as SU-8, as described herein.

Substantially non-transparent polymer layer 104 is affixed to a surface of layer 102. While the cross-section of microcarrier 100 shown in **FIG. 1B** shows a discontinuous view of layer 104, the view shown in **FIG. 1A** illustrates that layer 104 is shaped like a circular gear with a plurality of teeth. The shape, number, size, and spacing of these gear teeth constitutes a two-dimensional shape, and one or more of these aspects of the gear teeth may be modified in order to produce multiple two-dimensional shapes for analog encoding. Advantageously, the outside edge of layer 104's gear teeth fit within the perimeter of layer 102. This allows for a variety of analog codes, each representing a unique identifier for one species of microcarrier, while maintaining a uniform overall shape across multiple species of microcarrier. Stated another way, each microcarrier species within a population of multiple species may have a different two-dimensional gear shape (*i.e.,* analog code), but each microcarrier will have the same perimeter, leading to greater uniformity of physical properties (*e.g*., size, shape, behavior in solution, and the like). Layer 104 may be produced, *e.g*., using a polymer such as SU-8 mixed with a dye, or using a black matrix resist, as described herein.

Layer 104 surrounds center portion 106 of layer 102. A probe is coupled to at least center portion 106 on one or both surfaces (*i.e.,* upper/lower surfaces) of layer 102. Advantageously, this allows center portion 106 to be imaged without any potential for interference resulting from layer 104.

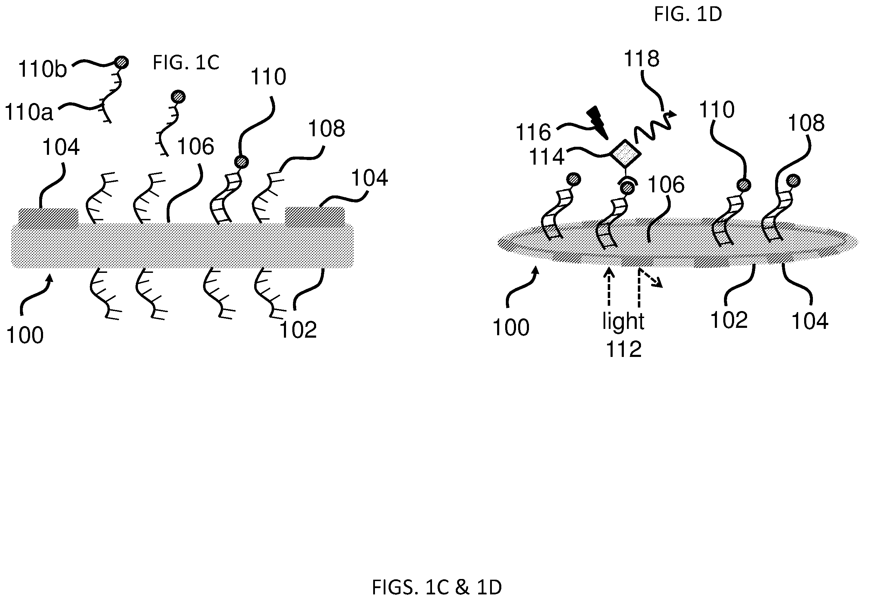

**FIGS. 1C & 1D** show an exemplary assay using microcarrier 100 for analyte detection. **FIG. 1C** shows that microcarrier 100 may include probe 108 coupled to one or more surfaces in at least center portion 106. Microcarrier 100 is contacted with a solution containing amplified DNA 110, which has been denatured prior to contacting microcarrier 100 and hybridizes to probe 108. As described above, amplified DNA 110 is coupled to a detection reagent. In this example, the detection reagent is biotin (*e.g.*, resulting from amplification of DNA using a biotin-labeled primer). Thus, amplified DNA 110 includes DNA 110a (*e.g.,* comprising the locus of a mutation described herein) and biotin 110b. **FIG. 1C** illustrates a single microcarrier species (*i.e.,* microcarrier 100), which captures amplified DNA 110, but in a multiplex assay multiple microcarrier species are used, each species having a particular probe that recognizes a specific DNA mutation.

**FIG. 1D** illustrates an exemplary process for "reading" microcarrier 100. This process includes two steps that may be accomplished simultaneously or separately. First, the hybridization of amplified DNA 110 by probe 108 is detected. In the example shown in **FIG. 1D****,** secondary detection reagent 114 (*e.g*., streptavidin conjugated to PE) binds to amplified DNA 110 via a biotin:streptavidin interaction. Amplified DNA not hybridized to a probe coupled to microcarrier 100 may have been washed off prior to detection, such that only DNA bound to microcarrier 100 is detected. The PE moiety of secondary detection reagent 114 emits light 118 (*e.g.,* a photon) when excited by light 116 at a wavelength within the excitation spectrum of PE. Light 118 may be detected by any suitable detection means, such as a fluorescence microscope, plate reader, and the like.

In addition, microcarrier 100 is read for its unique identifier. In the example shown in **FIG. 1D****,** light 112 is used to illuminate the field containing microcarrier 100 (in some embodiments, light 112 may have a different wavelength than lights 116 and 118). When light 112 illuminates the field containing microcarrier 100, it passes through substantially transparent polymer layer 102 but is blocked by substantially non-transparent polymer layer 104, as shown in **FIG. 1D****.** This generates a light pattern that can be imaged, for example, by light microscopy (*e.g.,* using differential interference contrast, or DIC, microscopy). This light pattern is based on the two-dimensional shape (*i.e.,* analog code) of microcarrier 100. Standard image recognition techniques may be used to decode the analog code represented by the image of microcarrier 100.

As described in greater detail below, the analyte detection and identifier imaging steps may occur in any order, or simultaneously. Advantageously, both detection steps shown in **FIG. 1D** may be accomplished on one imaging device. As one example, a microscope capable of both fluorescence and light (*e.g.,* bright field) microscopy may be used to quantify the amount of amplified DNA 110 bound to microcarrier 100 (*e.g.,* as detected by detection reagent 114) and image the analog code created by layers 102 and 104. This allows for a more efficient assay process with fewer equipment requirements.

In some embodiments, the microcarrier further includes a magnetic, substantially non-transparent layer affixed to a surface of the substantially transparent polymer layer that encloses the center portion of the substantially transparent polymer layer. In some embodiments, the magnetic, substantially non-transparent layer is between the substantially non-transparent polymer layer and the center portion of the substantially transparent polymer layer.

In some embodiments, the microcarrier further includes a second substantially transparent polymer layer aligned with and affixed to the first substantially transparent polymer layer. In some embodiments, the first and second substantially transparent polymer layers each have a center portion, and the center portions of both the first and second substantially transparent polymer layers are aligned. In some embodiments, the microcarrier further includes a magnetic, substantially non-transparent layer that encloses the center portions of both the first and second substantially transparent polymer layers. In some embodiments, the magnetic, substantially non-transparent layer is affixed between the first and second substantially transparent polymer layers. In some embodiments, the magnetic, substantially non-transparent layer is between the substantially non-transparent polymer layer and the center portions of both the first and second substantially transparent polymer layers.

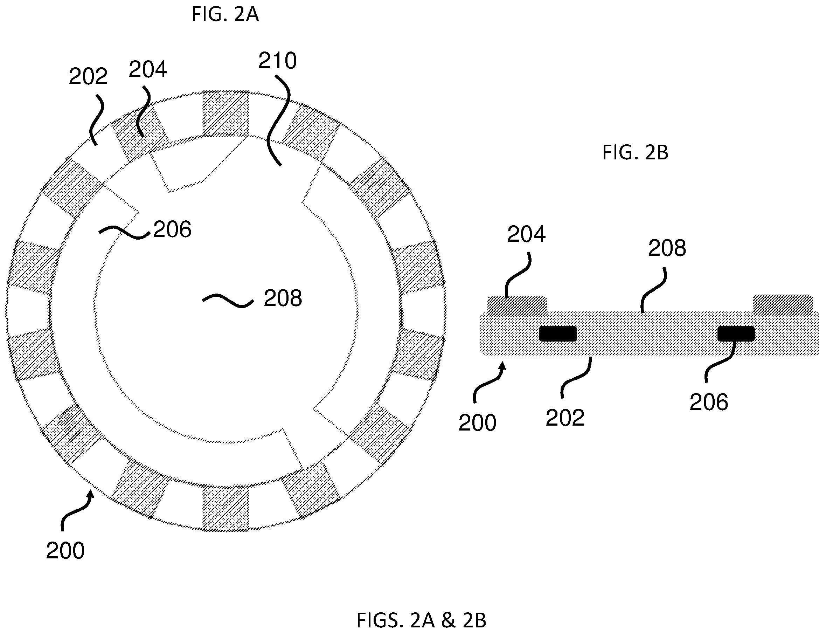

Turning now to **FIGS. 2A & 2B****,** another exemplary microcarrier 200 is shown. Like microcarrier 100, microcarrier 200 includes substantially transparent polymer layer 202 and substantially non-transparent polymer layer 204. In addition, microcarrier 200 includes magnetic layer 206. As shown in **FIG. 2A****,** magnetic layer 206 may be shaped as a ring between center portion 208 and substantially non-transparent layer 204.

**FIG. 2B** shows that magnetic layer 206 may be embedded within layer 202. Layer 202 may also include more than one layer, such that magnetic layer 206 is sandwiched between two substantially transparent polymer layers (*e.g.,* as in **FIG. 2B**). Alternatively, magnetic layer 206 may be affixed to the same surface of layer 202 as layer 204, or magnetic layer 206 may be affixed to the surface of layer 202 opposite layer 204. In some embodiments, magnetic layer 206 may include nickel.

Magnetic layer 206 bestows magnetic properties onto microcarrier 200, which advantageously may be used for many applications. For example, microcarrier 200 may be affixed to a surface by magnetic attraction during a washing step, allowing for effective washing without losing or otherwise disrupting the microcarriers.

In addition to its magnetic properties, layer 206 is also substantially non-transparent. When imaged as shown in **FIG. 1D** (*e.g.,* using light 112), layer 206 will block, either in part or in whole, transmitted light, thereby creating a pattern for imaging. As shown in **FIG. 2A****,** layer 206 is also asymmetric-in this example, it includes gap 210. This asymmetry creates an orientation indicator that can be imaged, for example, as shown in **FIG. 1D** using light 112. Advantageously, an orientation indicator may be utilized during image recognition to orient the two-dimensional shape created by imaging layer 204 in a uniform orientation for easier analog code recognition. This allows microcarriers imaged in any orientation to be decoded.

In some embodiments, the magnetic, substantially non-transparent layer is between about 50 nm and about 10 µm in thickness. In some embodiments, the thickness of the magnetic, substantially non-transparent layer is less than about any of the following thicknesses (in nm): 10000, 9500, 9000, 8500, 8000, 7500, 7000, 6500, 6000, 5500, 5000, 4500, 4000, 3500, 3000, 2500, 2000, 1500, 1000, 950, 900, 850, 800, 750, 700, 650, 600, 550, 500, 450, 400, 350, 300, 250, 200, 150, or 100. In some embodiments, the thickness of the magnetic, substantially non-transparent layer is greater than about any of the following thicknesses (in nm): 50, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, or 9500. That is, the thickness of the magnetic, substantially non-transparent layer may be any of a range of thicknesses (in nm) having an upper limit of 10000, 9500, 9000, 8500, 8000, 7500, 7000, 6500, 6000, 5500, 5000, 4500, 4000, 3500, 3000, 2500, 2000, 1500, 1000, 950, 900, 850, 800, 750, 700, 650, 600, 550, 500, 450, 400, 350, 300, 250, 200, 150, or 100 and an independently selected lower limit of 50, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, or 9500, wherein the lower limit is less than the upper limit.

In some embodiments, the magnetic, substantially non-transparent layer is about 0.1 µm in thickness. In some embodiments, the magnetic, substantially non-transparent layer is about 50 nm, about 100 nm, about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 850 nm, about 900 nm, about 950 nm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 7.5 µm, about 8 µm, about 8.5 µm, about 9 µm, about 9.5 µm, or about 10 µm in thickness. In some embodiments, the thickness of the magnetic, substantially non-transparent layer is about 0.01 µm, about 0.02 µm, about 0.03 µm, about 0.04 µm, about 0.05 µm, about 0.06 µm, about 0.07 µm, about 0.08 µm, about 0.09 µm, about 0.1 µm, about 0.11 µm, about 0.12 µm, about 0.13 µm, about 0.14 µm, about 0.15 µm, about 0.16 µm, about 0.17 µm, about 0.18 µm, about 0.19 µm, about 0.20 µm, about 0.25 µm, about 0.30 µm, about 0.35 µm, about 0.40 µm, about 0.45 µm, or about 0.50 µm.

In some embodiments, the microcarrier further includes an orientation indicator for orienting the analog code of the substantially non-transparent polymer layer. Any feature of the microcarrier that is visible and/or detectable by imaging (*e.g.,* a form of microscopic or other imaging described herein) and/or by image recognition software may serve as an orientation indicator. An orientation indicator may serve as a point of reference, *e.g.,* for an image recognition algorithm, to orient the image of an analog code in a uniform orientation (*i.e*., the shape of the substantially non-transparent polymer layer). Advantageously, this simplifies image recognition, as the algorithm would only need to compare the image of a particular analog code against a library of analog codes in the same orientation, and not against a library including all analog codes in all possible orientations. In some embodiments, the orientation indicator may be independent of the substantially non-transparent polymer layer. For example, it may be formed as a part of a magnetic layer and/or substantially transparent polymer layer. In other embodiments, the orientation indicator may be formed as part of the substantially non-transparent polymer layer. In some embodiments, the orientation indicator comprises an asymmetry of the magnetic, substantially non-transparent layer (*e.g.,* as illustrated by gap 210 in **FIG. 2A**).

In some embodiments, the microcarrier further includes one or more columns projecting from a surface of the microcarrier (*e.g*., the top and/or bottom surface of the microcarrier). As used herein, a "column" may refer to any geometric shape that projects from the microcarrier surface and does not necessarily denote any regularity in dimensions, nor any cylindrical character. For example, the outer surface of a column may or may not be parallel with the microcarrier surface. Examples of columnar shapes that may project from a microcarrier include without limitation a rectangular prism, a triangle, a pyramid, a cube, a cylinder, a sphere or half-sphere, a cone, and so forth. In some embodiments, the one or more columns are not within a center portion of the first and/or the second substantially transparent polymer layer. In some embodiments, the one or more columns may project from an outside-facing surface (*e.g.,* a surface not affixed to another layer) of one or more of the first and the second substantially transparent polymer layers. In some embodiments, the one or more columns are made from the magnetic, substantially non-transparent polymer layer. It is to be noted that any descriptions of microcarrier thickness herein do not include the one or more columns in the stated dimensions. That is to say, microcarrier thickness as described herein is independent of any optional columns projecting therefrom.

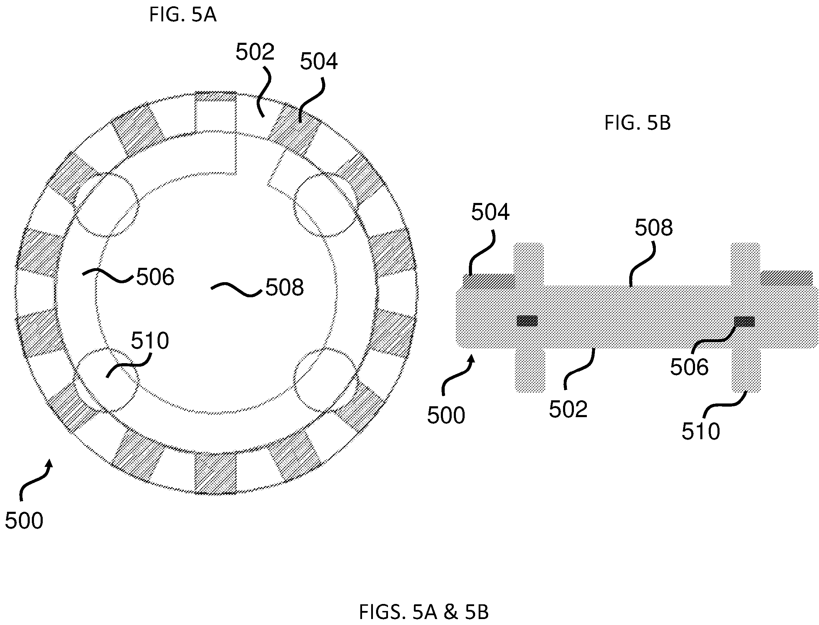

Turning now to **FIGS. 5A & 5B****,** another exemplary microcarrier 500 is shown. Like microcarrier 200, microcarrier 500 includes substantially transparent polymer layer 502, substantially non-transparent polymer layer 504, magnetic layer 506, and center portion 508. In addition, microcarrier 500 has four columns including column 510, which may be of any shape that extends from the surface of layer 502. As shown in **FIG. 5A****,** these columns may be arrayed in alignment with magnetic layer 506, preventing any potential for interfering with analyte detection in center portion 508 or with reading the two-dimensional shape (*i.e.,* the analog code) of layer 504. **FIG. 5B** shows that these columns may extend from the upper and lower surfaces of microcarrier 500. Column 510 may be made, for example, using the same substantially transparent polymer as layer 502 (exemplary methods of production are described *infra*)*.* Advantageously, one or more columns such as column 510 may be used to prevent microcarriers from sticking to each other and/or a container (*e.g.,* the side of a well in a multiwell plate), *e.g.,* through optical contact bonding.

In some embodiments, the one or more columns are between about 1 µm and about 10 µm tall. In some embodiments, the one or more columns are about 1 µm tall, about 1.5 µm tall, about 2 µm tall, about 2.5 µm tall, about 3 µm tall, about 3.5 µm tall, about 4 µm tall, about 4.5 µm tall, about 5 µm tall, about 5.5 µm tall, about 6 µm tall, about 6.5 µm tall, about 7 µm tall, about 7.5 µm tall, about 8 µm tall, about 8.5 µm tall, about 9 µm tall, about 9.5 µm tall, or about 10 µm tall. In some embodiments, the one or more columns are less than about any of the following heights (in µm): 10, 9.5, 9, 8.5, 8, 7.5, 7, 6.5, 6, 5.5, 5, 4.5, 4, 3.5, 3, 2.5, 2, or 1.5. In some embodiments, the one or more columns are greater than about any of the following heights (in µm): 1, 1.5, 2, 2.5, 3, 3.5, 4, 4.5, 5, 5.5, 6, 6.5, 7, 7.5, 8, 8.5, 9, or 9.5. That is, the one or more columns can be any of a range of heights having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit.

In some embodiments, the one or more columns may be cylindrical in shape. In some embodiments, the one or more columns have a diameter between about 1 µm and about 10 µm. In some embodiments, the one or more columns have a diameter of about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 7.5 µm, about 8 µm, about 8.5 µm, about 9 µm, about 9.5 µm, or about 10 µm. In some embodiments, the one or more columns have a diameter less than about any of the following lengths (in µm): 10, 9.5, 9, 8.5, 8, 7.5, 7, 6.5, 6, 5.5, 5, 4.5, 4, 3.5, 3, 2.5, 2, or 1.5. In some embodiments, the one or more columns have a diameter greater than about any of the following lengths (in µm): 1, 1.5, 2, 2.5, 3, 3.5, 4, 4.5, 5, 5.5, 6, 6.5, 7, 7.5, 8, 8.5, 9, or 9.5. That is, the one or more columns can have any of a range of diameters having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit. In other embodiments, the one or more columns may have roughly the same width as any diameter described *supra,* or a range of widths roughly the same as any range of diameters described *supra,* but the one or more columns may adopt the shape of an elliptical cylinder, parabolic cylinder, hyperbolic cylinder, or any other cylindrical or polyhedral shape described herein or known in the art.

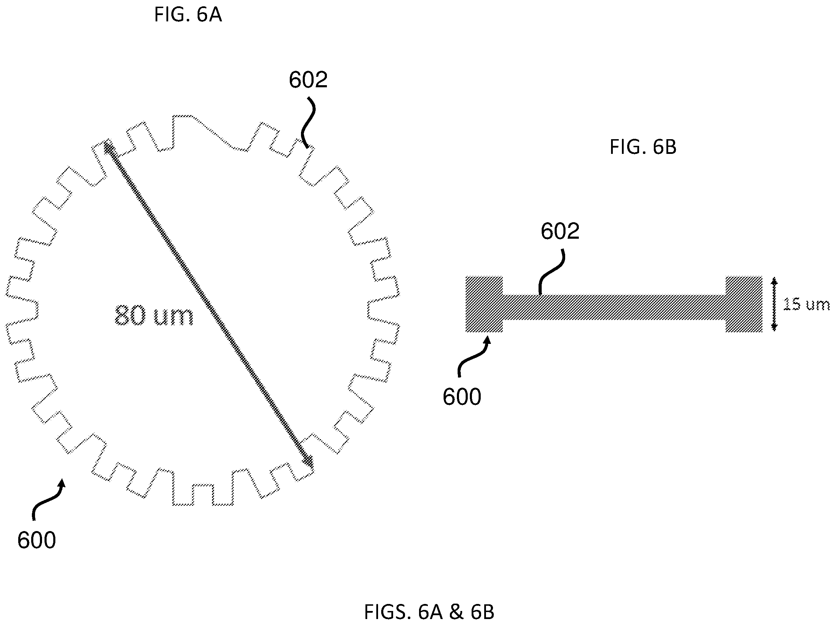

In other aspects, provided herein are encoded microcarriers that comprise: a substantially non-transparent polymer layer having a first surface and a second surface, the first and the second surfaces being parallel to each other, wherein an outline of the substantially non-transparent polymer layer comprises a two-dimensional shape that represents an analog code; and a capture agent for capturing an analyte, wherein the capture agent is coupled to at least one of the first surface and the second surface of the substantially non-transparent polymer layer in at least a center portion of the substantially non-transparent polymer layer. Thus, the microcarrier is encoded by the shape (*e.g*., outline) of the microcarrier itself: a two-dimensional shape that represents an analog code. Advantageously, these microcarriers may be manufactured efficiently and with high precision, allowing for highly accurate decoding and cost-efficient production. Examples of this type of microcarrier and aspects thereof are illustrated in **FIGS. 6A-9C****.**

**FIGS. 6A & 6B** show exemplary microcarrier 600 of this type. Microcarrier 600 is a gear-shaped disc approximately 80 µm in diameter and 15 µm in height, including optional column elements (similar to column 510 as described above). Microcarrier 600 is made of a single, non-transparent polymer layer 602, rather than separate transparent and non-transparent polymer layers. Microcarrier 600 may be imaged as shown in **FIG. 1D**, but its analog code is imaged based on the entire microcarrier shape (*e.g*., perimeter of the non-transparent polymer layer). One or both surfaces of microcarrier 600 may be used for coupling a capture agent as above, and a center portion or the entire surface may be used.

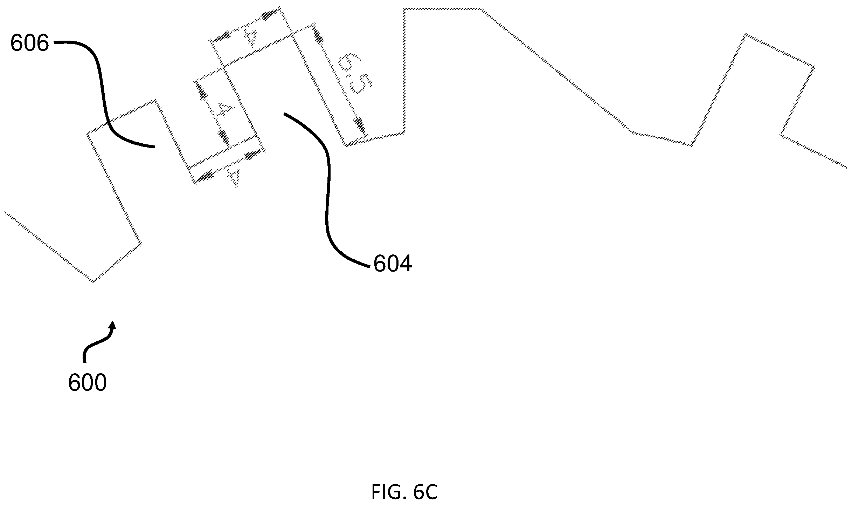

**FIG. 6C** illustrates the dimensions of gear tooth 604 of microcarrier 600. As shown, in this embodiment, gear tooth 604 is 4 µm wide and spaced 4 µm from adjacent gear tooth 606. Since the two-dimensional shape of microcarrier 600 is analog encoded, the perimeter between adjacent gear teeth may be variable, allowing for multiple gear tooth shapes. For example, gear tooth 604 extends 4 or 6.5 µm in height, relative to the adjacent perimeter segment immediately to the left or right, respectively.

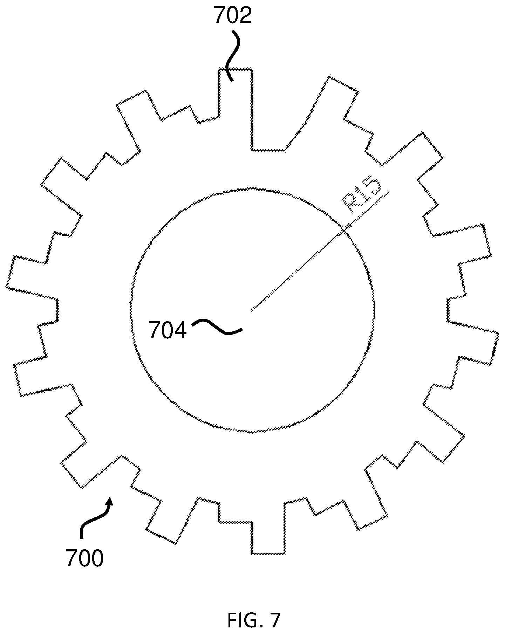

**FIG. 7** illustrates another embodiment of this type of microcarrier, microcarrier 700. Like microcarrier 600, microcarrier 700 is made from non-transparent polymer layer 702. In addition, microcarrier includes magnetic layer 704. Magnetic layer 704 may be affixed to one of the surfaces of microcarrier 700, or it may be embedded within microcarrier 700 (*e.g*., between two non-transparent polymer layers). Magnetic layer 704 may be generated, for example, by depositing nickel. As described above, a magnetic layer allows additional functionalities, such as the option for washing microcarrier 700 while magnetically attached to another surface.

In some embodiments, the microcarrier further includes one or more columns projecting from a surface of the substantially non-transparent polymer layer. As described in greater detail *supra,* a "column" may refer to any geometric shape that projects from the microcarrier surface and does not necessarily denote any regularity in columnar dimension(s). Any of the exemplary columnar shapes described above may be used.

In some embodiments, the one or more columns are between about 1 µm and about 10 µm tall. In some embodiments, the one or more columns are about 1 µm tall, about 1.5 µm tall, about 2 µm tall, about 2.5 µm tall, about 3 µm tall, about 3.5 µm tall, about 4 µm tall, about 4.5 µm tall, about 5 µm tall, about 5.5 µm tall, about 6 µm tall, about 6.5 µm tall, about 7 µm tall, about 7.5 µm tall, about 8 µm tall, about 8.5 µm tall, about 9 µm tall, about 9.5 µm tall, or about 10 µm tall. In some embodiments, the one or more columns are less than about any of the following heights (in µm): 10, 9.5, 9, 8.5, 8, 7.5, 7, 6.5, 6, 5.5, 5, 4.5, 4, 3.5, 3, 2.5, 2, or 1.5. In some embodiments, the one or more columns are greater than about any of the following heights (in µm): 1, 1.5, 2, 2.5, 3, 3.5, 4, 4.5, 5, 5.5, 6, 6.5, 7, 7.5, 8, 8.5, 9, or 9.5. That is, the one or more columns can be any of a range of heights having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit.

In some embodiments, the one or more columns may be cylindrical in shape. In some embodiments, the one or more columns have a diameter between about 1 µm and about 10 µm. In some embodiments, the one or more columns have a diameter of about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 7.5 µm, about 8 µm, about 8.5 µm, about 9 µm, about 9.5 µm, or about 10 µm. In some embodiments, the one or more columns have a diameter less than about any of the following lengths (in µm): 10, 9.5, 9, 8.5, 8, 7.5, 7, 6.5, 6, 5.5, 5, 4.5, 4, 3.5, 3, 2.5, 2, or 1.5. In some embodiments, the one or more columns have a diameter greater than about any of the following lengths (in µm): 1, 1.5, 2, 2.5, 3, 3.5, 4, 4.5, 5, 5.5, 6, 6.5, 7, 7.5, 8, 8.5, 9, or 9.5. That is, the one or more columns can have any of a range of diameters having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit. In other embodiments, the one or more columns may have roughly the same width as any diameter described *supra,* or a range of widths roughly the same as any range of diameters described *supra,* but the one or more columns may adopt the shape of an elliptical cylinder, parabolic cylinder, hyperbolic cylinder, or any other cylindrical or polyhedral shape described herein or known in the art.

In some embodiments, the microcarrier further includes a magnetic layer comprising a magnetic material affixed to a surface of the substantially non-transparent polymer layer. In some embodiments, the magnetic layer does not extend beyond the two-dimensional shape of the substantially non-transparent polymer layer. That is to say, if the outline of the substantially non-transparent polymer layer were to be imaged, the resulting image would not be altered by the presence or absence of the magnetic layer. In some embodiments, the magnetic layer may include the one or more columns described above. That is, the one or more columns described above may be made of a magnetic material described herein.

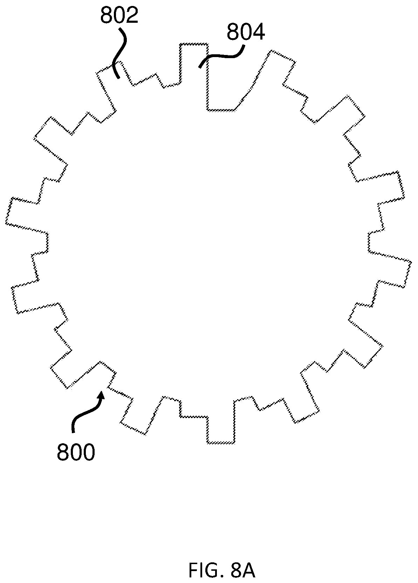

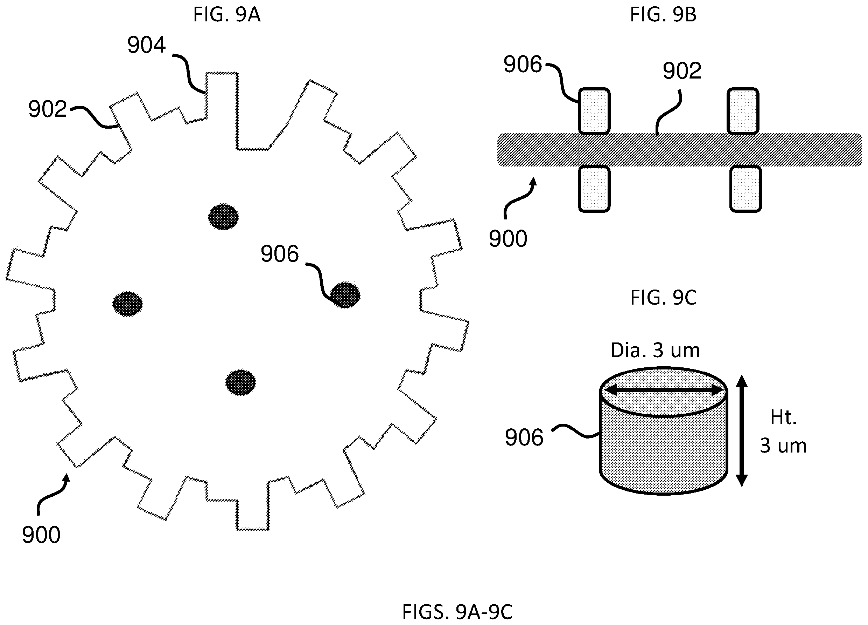

In some embodiments, the microcarrier further includes an orientation indicator for orienting the analog code of the substantially non-transparent polymer layer. Any feature of the microcarrier that is visible and/or detectable by imaging (*e.g.,* a form of microscopic or other imaging described herein) and/or by image recognition software may serve as an orientation indicator. An orientation indicator may serve as a point of reference, *e.g.,* for an image recognition algorithm, to orient the image of an analog code in a uniform orientation (*i.e*., the shape of the substantially non-transparent polymer layer). Advantageously, this simplifies image recognition, as the algorithm would only need to compare the image of a particular analog code against a library of analog codes in the same orientation, and not against a library including all analog codes in all possible orientations. In some embodiments, the orientation indicator comprises an asymmetry of the outline of the substantially non-transparent polymer layer. For example, the orientation indicator may comprise a visible feature, such as an asymmetry, of the outline of the microcarrier (*e.g*., as illustrated by start positions 804 and 904 in **FIGS. 8A** and **9A** as described *infra*)*.*

Turning now to **FIG. 8A****,** another exemplary microcarrier 800 is shown. Like microcarrier 700, microcarrier 800 includes non-transparent polymer layer 802 (and optionally, a magnetic layer such as layer 704). In addition, microcarrier 800 includes start position 804, which has a different shape than the rest of the perimeter of microcarrier 800. Start position 804 may be used as an orientation indicator for image recognition, as described above in reference to gap 210 shown in **FIG. 2A****.**

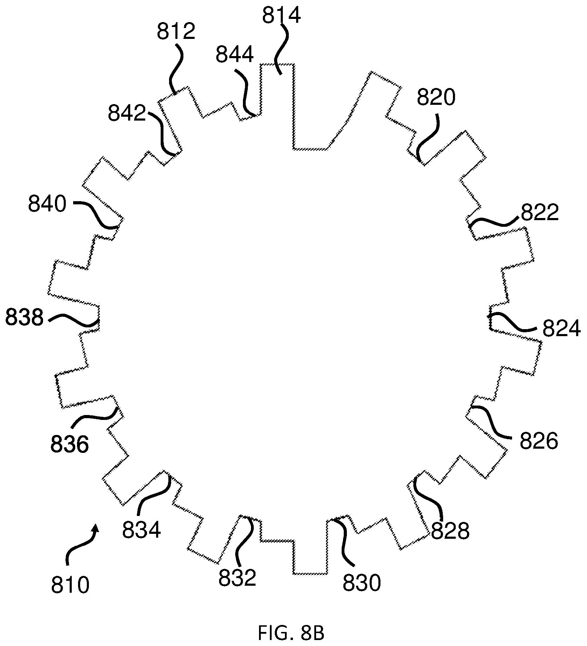

**FIG. 8B** illustrates a coding scheme that may be used. **FIG. 8B** shows microcarrier 810, which like microcarrier 800 includes non-transparent polymer layer 812 and start position 814 (and optionally, a magnetic layer such as layer 704). In this scheme, potential shape variation points around the gear are labeled, *e.g.,* at positions 820, 822, 824, 826, 828, 830, 832, 834, 836, 838, 840, 842, and 844. As shown in **FIG. 8B****,** even if only two potential shapes may be used for positions 820, 822, 824, 826, 828, 830, 832, 834, 836, 838, 840, 842, and 844, this embodiment allows up to 2¹³ unique codes. Further, as described above, the use of analog encoding greatly expands this number by allowing the use of more than two potential shapes at any or all of the indicated positions around the perimeter (*e.g.*, at each shape variation point as labeled in **FIG. 8B****).**

**FIGS. 9A-9C** illustrate yet another potential embodiment in microcarrier 900. Like microcarrier 800, microcarrier 900 is a gear-shaped microcarrier that includes non-transparent polymer layer 902 and start position 904 (and optionally, a magnetic layer such as layer 704). In addition, microcarrier 900 may have one or more columns (*e.g.*, column 906) affixed to one or both surfaces of microcarrier 900. As shown in the cross-section in **FIG. 9B****,** column 906 extends from a surface of layer 902. Advantageously, column 906 helps to reduce the potential for optical contact bonding (as described above in reference to column 510).

**FIG. 9C** illustrates the dimensions of column 906. In this example, column 906 is a cylinder 3 µm in height and 3 µm in diameter, although as described above such columns are in no way limited to a cylindrical shape. In some embodiments, column 906 is made of a magnetic material, such as nickel. This allows column 906 to function additionally as a magnetic element for magnetic manipulation of microcarrier 900, as described herein.

Any of the microcarriers described herein may include one or more of the features, elements, or aspects described below. In addition, one or more of the features, elements, or aspects described below may adopt different characteristics depending on the embodiment of the microcarrier, *e.g*., as described above.

In some embodiments, a substantially transparent polymer of the present disclosure comprises an epoxy-based polymer. Suitable epoxy-based polymers for fabrication of the compositions described herein include, but are not limited to, the EPON^{™} family of epoxy resins provided by Hexion Specialty Chemicals, Inc. (Columbus, OH) and any number of epoxy resins provided by The Dow Chemical Company (Midland, MI). Many examples of suitable polymers are commonly known in the art, including without limitation SU-8, EPON 1002F, EPON 165/154, and a poly(methyl methacrylate)/poly(acrylic acid) block copolymer (PMMA-co-PAA). For additional polymers, see, for example, Warad, IC Packaging: Package Construction Analysis in Ultra Small IC Packaging, LAP LAMBERT Academic Publishing (2010*);* The Electronic Packaging Handbook, CRC Press (Blackwell, ed.), (2000); and Pecht et al., Electronic Packaging Materials and Their Properties, CCR Press, 1st ed., (1998). These types of materials have the advantage of not swelling in aqueous environments which ensures that uniform microcarrier size and shape are maintained within the population of microcarriers. In some embodiments, the substantially transparent polymer is a photoresist polymer. In some embodiments, the epoxy-based polymer is an epoxy-based, negative-tone, near-UV photoresist. In some embodiments, the epoxy-based polymer is SU-8.

In some embodiments, the substantially non-transparent polymer is a polymer described herein (*e.g.,* SU-8) mixed with one or more non-transparent or colored dye(s). In other embodiments, the substantially non-transparent polymer is a black matrix resist. Any black matrix resist known in the art may be used; see, *e.g.,* U.S. Patent No. 8,610,848 for exemplary black matrix resists and methods related thereto. In some embodiments, the black matrix resist may be a photoresist colored with a black pigment, *e.g*., as patterned on the color filter of an LCD as part of a black matrix. Black matrix resists may include without limitation those sold by Toppan Printing Co. (Tokyo), Tokyo OHKA Kogyo (Kawasaki), and Daxin Materials Corp. (Taichung City, Taiwan).

In some embodiments, reference may be made to a center portion of one or more polymer layers. A center portion of the present disclosure may take any shape. In some embodiments, the shape of the center portion may reflect or correspond to the shape (*e.g.,* outline) of the corresponding polymer layer. In other embodiments, the shape of the center portion may be independent of the shape (*e.g*., outline) of the corresponding polymer layer. For example, a center portion of a circular microcarrier surface may be circular in some embodiments and square in other embodiments. A center portion of a square microcarrier surface may be square in some embodiments and circular in other embodiments.

In some embodiments, a center portion of a polymer layer of the present disclosure is about 5%, about 7%, about 10%, about 15%, about 20%, about 25%, about 30%, about 35%, about 40%, about 45%, about 50%, about 55%, about 60%, about 65%, about 70%, about 75%, about 80%, about 85%, or about 90% of the surface area of the polymer layer. In some embodiments, a center portion of a polymer layer of the present disclosure is less than about any of the following fractions of the substantially transparent polymer layer (in %): 90, 85, 80, 75, 70, 65, 60, 55, 50, 45, 40, 35, 30, 25, 20, 15, 10, or 7. In some embodiments, a center portion of a polymer layer of the present disclosure is greater than about any of the following fractions of the substantially transparent polymer layer (in %): 5, 7, 10, 15, 20, 25, 30, 35, 40, 45, 50, 55, 60, 65, 70, 75, 80, or 85. That is, the fraction of the polymer layer surface area included in the center portion may be any of a range of percentages having an upper limit of 90, 85, 80, 75, 70, 65, 60, 55, 50, 45, 40, 35, 30, 25, 20, 15, 10, or 7 and an independently selected lower limit of 5, 7, 10, 15, 20, 25, 30, 35, 40, 45, 50, 55, 60, 65, 70, 75, 80, or 85, wherein the lower limit is less than the upper limit. In some embodiments, the center portion of a polymer layer comprises about 25% of the surface area of the polymer layer. In some embodiments, a center portion of a microcarrier surface includes the entire surface minus an outline portion of the microcarrier.

As described above, a microcarrier of the present disclosure may further include a magnetic layer, which may adopt a variety of shapes as described herein. In some embodiments, the magnetic layer may be a substantially non-transparent layer. In some embodiments, the magnetic layer may comprise a magnetic material. A magnetic layer of the present disclosure may be made of any suitable magnetic material, such as a material with paramagnetic, ferromagnetic, or ferrimagnetic properties. Examples of magnetic materials include without limitation iron, nickel, cobalt, and some rare earth metals (*e.g*., gadolinium, dysprosium, neodymium, and so forth), as well as alloys thereof. In some embodiments, the magnetic material comprises nickel, including without limitation elemental nickel and magnetic nickel alloys such as alnico and permalloy. The inclusion of a magnetic layer in a microcarrier of the present disclosure may be advantageous, *e.g*., in facilitating magnetic separation, which may be useful for washing, collecting, and otherwise manipulating one or more microcarriers.

As described above, in some embodiments, the magnetic layer may be affixed to a surface of the substantially transparent polymer layer and enclose a center portion of the substantially transparent polymer layer. In other embodiments, as described above, the magnetic layer may include one or more columns; *i.e.,* the one or more columns described above may be made of a magnetic material described herein.

The encoded microcarriers for use in the invention may comprise: a substantially transparent polymer layer having a first surface and a second surface, the first and the second surfaces being parallel to each other; a magnetic, substantially non-transparent polymer layer that is affixed to the first surface of the substantially transparent polymer layer, encloses a center portion of the substantially transparent polymer layer; and a probe specific for a DNA mutation in the *KRAS, BRAF, CTNNB1,* or *APC* gene, wherein the probe is coupled to at least one of the first surface and the second surface of the substantially transparent polymer layer in at least the center portion of the substantially transparent polymer layer. In some embodiments, the magnetic, substantially non-transparent polymer layer comprises a two-dimensional shape representing an analog code. In some embodiments, the magnetic, substantially non-transparent polymer layer comprises at least a portion of a two-dimensional shape representing an analog code. For example, part of the two-dimensional shape could be made from the magnetic, substantially non-transparent polymer layer, and part of the two-dimensional shape could be made from a non-magnetic, substantially non-transparent polymer layer (*e.g.,* SU-8 as described herein). In other embodiments, the magnetic, substantially non-transparent polymer layer comprises all of the two-dimensional shape representing the analog code. Thus, the microcarrier contains at least two layers: one of which is substantially transparent, and the other of which is a magnetic, substantially non-transparent, two-dimensional shape that represents an analog code.

Advantageously, these microcarriers may employ a variety of two-dimensional shapes while still retaining a uniform overall form (*e.g.,* the perimeter of the substantially transparent polymer layer) for uniformity of aspects including, for example, overall dimensions, physical properties, and/or behavior in solution. Various optional aspects and elements of this type of microcarrier are illustrated in **FIGS. 1A-5B** (see, *e.g.,* **FIG. 4B**). The magnetic layer advantageously combines a two-dimensional shape for analog coding while also providing a magnetic functionality that allows for, *e.g*., facilitating magnetic separation, which may be useful for washing, collecting, and otherwise manipulating one or more microcarriers.

In some embodiments, an analog code of the present disclosure is made from a two-dimensional shape of a magnetic, substantially non-transparent polymer layer. Advantageously, this provides a microcarrier that can be produced efficiently on a large scale (*e.g*., using the techniques described herein). Moreover, generating the analog code using a magnetic, substantially non-transparent polymer layer facilitates multiplex assays by allowing for a variety of different microcarrier species (each with a unique analog code) while also providing facile manipulation via the resulting magnetic properties of the microcarrier.

In some embodiments, the magnetic, substantially non-transparent layer is between about 50 nm and about 10 µm in thickness. In some embodiments, the thickness of the magnetic, substantially non-transparent layer is less than about any of the following thicknesses (in nm): 10000, 9500, 9000, 8500, 8000, 7500, 7000, 6500, 6000, 5500, 5000, 4500, 4000, 3500, 3000, 2500, 2000, 1500, 1000, 950, 900, 850, 800, 750, 700, 650, 600, 550, 500, 450, 400, 350, 300, 250, 200, 150, or 100. In some embodiments, the thickness of the magnetic, substantially non-transparent layer is greater than about any of the following thicknesses (in nm): 50, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, or 9500. That is, the thickness of the magnetic, substantially non-transparent layer may be any of a range of thicknesses (in nm) having an upper limit of 10000, 9500, 9000, 8500, 8000, 7500, 7000, 6500, 6000, 5500, 5000, 4500, 4000, 3500, 3000, 2500, 2000, 1500, 1000, 950, 900, 850, 800, 750, 700, 650, 600, 550, 500, 450, 400, 350, 300, 250, 200, 150, or 100 and an independently selected lower limit of 50, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, 7500, 8000, 8500, 9000, or 9500, wherein the lower limit is less than the upper limit.

In some embodiments, the magnetic, substantially non-transparent layer is about 0.1 µm in thickness. In some embodiments, the magnetic, substantially non-transparent layer is about 50 nm, about 100 nm, about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 850 nm, about 900 nm, about 950 nm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 7.5 µm, about 8 µm, about 8.5 µm, about 9 µm, about 9.5 µm, or about 10 µm in thickness. In some embodiments, the thickness of the magnetic, substantially non-transparent layer is about 0.01 µm, about 0.02 µm, about 0.03 µm, about 0.04 µm, about 0.05 µm, about 0.06 µm, about 0.07 µm, about 0.08 µm, about 0.09 µm, about 0.1 µm, about 0.11 µm, about 0.12 µm, about 0.13 µm, about 0.14 µm, about 0.15 µm, about 0.16 µm, about 0.17 µm, about 0.18 µm, about 0.19 µm, about 0.20 µm, about 0.25 µm, about 0.30 µm, about 0.35 µm, about 0.40 µm, about 0.45 µm, or about 0.50 µm.

In some embodiments, a microcarrier of the present disclosure may be encoded with a substantially non-transparent layer that constitutes a two-dimensional shape. For example, as described above, the two-dimensional shape may constitute the shape of a substantially non-transparent layer that contrasts with a substantially transparent layer of the microcarrier, or it may constitute the shape of the microcarrier itself (*e.g.,* the perimeter). Any two-dimensional shape that can encompass a plurality of resolvable and distinctive varieties may be used. In some embodiments, the two-dimensional shape comprises one or more of linear, circular, elliptical, rectangular, quadrilateral, or higher polygonal aspects, elements, and/or shapes. In some embodiments, the two-dimensional shape may be produced using a magnetic, substantially non-transparent layer of the present disclosure.

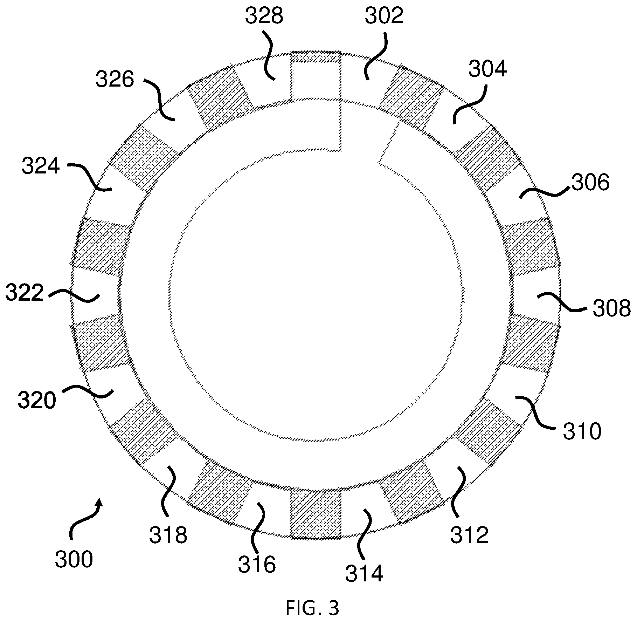

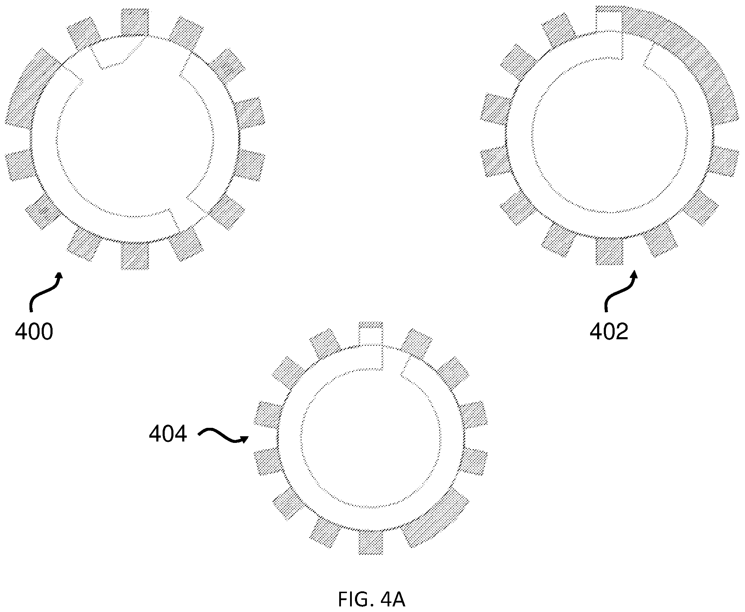

**FIG. 4A** illustrates three exemplary embodiments of the coding scheme shown in **FIG. 3****:** microcarriers 400, 402, and 404. The unique codes of microcarriers 400, 402, and 404 are generated using the simple "filled or not filled" scheme of **FIG. 3****.** Importantly, as described above, more complex encoding schemes are available using analog image recognition, thereby greatly expanding the number of potential unique codes. In some embodiments, the two-dimensional shape of the substantially non-transparent polymer layer comprises one or more rings enclosing the center portion of the substantially transparent polymer layer. In some embodiments, at least one of the one or more rings comprises a discontinuity. Exemplary and non-limiting two-dimensional shapes formed using one or more rings (*e.g*., two rings) having varying numbers and configurations of discontinuities are illustrated in **FIG. 4B. FIG. 4B** illustrates 10 exemplary embodiments of the cod, *inter alia,* in terms of number of shapes *(e.g.,* two distinct shapes in code ZN_3, as compared to seven distinct shapes in code ZN_10) and/or size of shapes (*e.g.*, large, small, and intermediate-sized shapes in code ZN_2).

In some embodiments, the two-dimensional shape of the substantially non-transparent polymer layer comprises a gear shape. A gear shape as used herein may refer to a plurality of shapes (*e.g*., gear teeth) arrayed on the perimeter of a substantially round, elliptical, or circular body, where at least two of the shapes of the plurality are spatially separated. In some embodiments, the gear shape comprises a plurality of gear teeth. In some embodiments, the analog code is represented by one or more aspects selected from the height of one or more gear teeth of the plurality, the width of one or more gear teeth of the plurality, the number of gear teeth in the plurality, and the arrangement of one or more gear teeth within the plurality. Advantageously, a gear shape encompasses multiple aspects, including the height of gear teeth, the width of gear teeth, the number of gear teeth, and the arrangement of gear teeth, that may be varied in order to generate a large diversity of potential unique two-dimensional shapes. It is to be appreciated, however, that since the gear shapes of the present disclosure are used for encoding and are not required to physically intermesh with another gear *(e.g.,* as with mechanical gears that transmit torque), gear teeth of the present disclosure are not constrained by the need for identical or intermeshing shapes, either within one gear shape or between multiple gear shapes. As such, the variety of shapes that may be considered a gear tooth of the present disclosure is significantly greater than with a mechanical gear.

**FIG. 3** shows the vast number of potential analog codes possible using the gear shape shown in **FIGS. 1A-2B****.** **FIG. 3** illustrates an exemplary coding scheme in which multiple shape variation points are labeled, *e.g.,* at positions 302, 304, 306, 308, 310, 312, 314, 316, 318, 320, 322, 324, 326, and 328 on exemplary microcarrier 300. Even if a simple "filled or not filled" scheme is used, up to 2¹⁴ unique codes are possible based on the use of 14 shape variation points. This scheme is convenient for both manufacturing and for generating two-dimensional shapes that are easily distinguishable for image recognition analysis. However, since analog encoding is used, more complex schemes using more than 2 possibilities (*e.g*., at each shape variation point as labeled in **FIG. 3****)** are possible, thereby exponentially expanding the number of unique identifiers. For example, multiple gear tooth shapes and/or multiple sizes of gear teeth are possible. A two-dimensional gear shape as shown in **FIGS. 1A-3** facilitates a wide range of unique analog codes while providing a large center portion (*e.g.*, center portions 106 and 208) for analyte detection.

In some embodiments, the plurality of gear teeth comprises one or more gear teeth that are between about 1 µm and about 10 µm wide. In some embodiments, the plurality of gear teeth comprises one or more gear teeth that are about 1 µm wide, about 1.5 µm wide, about 2 µm wide, about 2.5 µm wide, about 3 µm wide, about 3.5 µm wide, about 4 µm wide, about 4.5 µm wide, about 5 µm wide, about 5.5 µm wide, about 6 µm wide, about 6.5 µm wide, about 7 µm wide, about 7.5 µm wide, about 8 µm wide, about 8.5 µm wide, about 9 µm wide, about 9.5 µm wide, or about 10 µm wide. In some embodiments, the plurality of gear teeth comprises one or more gear teeth that are less than about any of the following widths (in µm): 10, 9.5, 9, 8.5, 8, 7.5, 7, 6.5, 6, 5.5, 5, 4.5, 4, 3.5, 3, 2.5, 2, or 1.5. In some embodiments, the plurality of gear teeth comprises one or more gear teeth that are greater than about any of the following widths (in µm): 1, 1.5, 2, 2.5, 3, 3.5, 4, 4.5, 5, 5.5, 6, 6.5, 7, 7.5, 8, 8.5, 9, or 9.5. That is, the plurality of gear teeth may comprise one or more gear teeth that can be any of a range of widths having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit.

In some embodiments, the plurality of gear teeth comprises one or more gear teeth that are between about 1 µm and about 10 µm tall. In some embodiments, the plurality of gear teeth comprises one or more gear teeth that are about 1 µm tall, about 1.5 µm tall, about 2 µm tall, about 2.5 µm tall, about 3 µm tall, about 3.5 µm tall, about 4 µm tall, about 4.5 µm tall, about 5 µm tall, about 5.5 µm tall, about 6 µm tall, about 6.5 µm tall, about 7 µm tall, about 7.5 µm tall, about 8 µm tall, about 8.5 µm tall, about 9 µm tall, about 9.5 µm tall, or about 10 µm tall. In some embodiments, the plurality of gear teeth comprises one or more gear teeth that are less than about any of the following heights (in µm): 10, 9.5, 9, 8.5, 8, 7.5, 7, 6.5, 6, 5.5, 5, 4.5, 4, 3.5, 3, 2.5, 2, or 1.5. In some embodiments, the plurality of gear teeth comprises one or more gear teeth that are greater than about any of the following heights (in µm): 1, 1.5, 2, 2.5, 3, 3.5, 4, 4.5, 5, 5.5, 6, 6.5, 7, 7.5, 8, 8.5, 9, or 9.5. That is, the plurality of gear teeth may comprise one or more gear teeth that can be any of a range of heights having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit. It is to be appreciated that a gear tooth may have different measurable heights, depending on the point of reference, if the adjacent perimeter segments from which the gear tooth extends are uneven (see, *e.g.,* gear tooth 602 in **FIG. 6C****,** which may be 4 or 6.5 µm tall, depending on the point of reference).

In some embodiments, the plurality of gear teeth comprises one or more gear teeth that are spaced between about 1 µm and about 10 µm apart. In some embodiments, the plurality of gear teeth comprises one or more gear teeth that are spaced about 1 µm apart, about 1.5 µm apart, about 2 µm apart, about 2.5 µm apart, about 3 µm apart, about 3.5 µm apart, about 4 µm apart, about 4.5 µm apart, about 5 µm apart, about 5.5 µm apart, about 6 µm apart, about 6.5 µm apart, about 7 µm apart, about 7.5 µm apart, about 8 µm apart, about 8.5 µm apart, about 9 µm apart, about 9.5 µm apart, or about 10 µm apart. In some embodiments, the plurality of gear teeth comprises one or more gear teeth that are spaced less than about any of the following widths apart (in µm): 10, 9.5, 9, 8.5, 8, 7.5, 7, 6.5, 6, 5.5, 5, 4.5, 4, 3.5, 3, 2.5, 2, or 1.5. In some embodiments, the plurality of gear teeth comprises one or more gear teeth that are spaced greater than about any of the following widths apart (in µm): 1, 1.5, 2, 2.5, 3, 3.5, 4, 4.5, 5, 5.5, 6, 6.5, 7, 7.5, 8, 8.5, 9, or 9.5. That is, the plurality of gear teeth may comprise one or more gear teeth that can be spaced any of a range of widths apart having an upper limit of 10, 9.5, 9, 8.5, 8, 7.5, 7, 6.5, 6, 5.5, 5, 4.5, 4, 3.5, 3, 2.5, 2, or 1.5 and an independently selected lower limit of 1, 1.5, 2, 2.5, 3, 3.5, 4, 4.5, 5, 5.5, 6, 6.5, 7, 7.5, 8, 8.5, 9, or 9.5, wherein the lower limit is less than the upper limit.

In some embodiments, a microcarrier of the present disclosure is a substantially circular disc. As used herein, a substantially circular shape may refer to any shape having a roughly identical distance between all of the points of the shape's perimeter and the shape's geometric center. In some embodiments, a shape is considered to be substantially circular if the variation among any of the potential radii connecting the geometric center and a given point on the perimeter exhibit 10% or lesser variation in length. As used herein, a substantially circular disc may refer to any substantially circular shape wherein the thickness of the shape is significantly less than its diameter. For example, in some embodiments, the thickness of a substantially circular disc may be less than about 50%, less than about 40%, less than about 30%, less than about 20%, less than about 15%, less than about 10%, or less than about 5% of its diameter. In certain embodiments, the thickness of the substantially circular disc may about 20% of its diameter. It is to be appreciated that the microcarriers of the present disclosure whose outline is a gear shape may also be considered substantially circular discs; for example, the shape of the microcarrier excluding the one or more gear teeth may comprise a substantially circular disc.

In some embodiments, the microcarrier is less than about 200 µm in diameter. For example, in some embodiments, the diameter of the microcarrier is less than about 200 µm, less than about 180 µm, less than about 160 µm, less than about 140 µm, less than about 120 µm, less than about 100 µm, less than about 80 µm, less than about 60 µm, less than about 40 µm, or less than about 20 µm.

In some embodiments, the diameter of the microcarrier is about 180 µm, about 160 µm, about 140 µm, about 120 µm, about 100 µm, about 90 µm, about 80 µm, about 70 µm, about 60 µm, about 50 µm, about 40 µm, about 30 µm, about 20 µm, or about 10 µm. In certain embodiments, the microcarrier is about 60 µm in diameter.

In some embodiments, the microcarrier is less than about 50 µm in thickness. For example, in some embodiments, the thickness of the microcarrier is less than about 70 µm, about 60 µm, about 50 µm, about 40 µm, about 30 µm, less than about 25 µm, less than about 20 µm, less than about 15 µm, less than about 10 µm, or less than about 5 µm. In some embodiments, the thickness of the microcarrier is less than about any of the following thicknesses (in µm): 70, 65, 60, 55, 50, 45, 40, 35, 30, 25, 20, 15, 10, 9, 8, 7, 6, 5, 4, 3, or 2. In some embodiments, the thickness of the microcarrier is greater than about any of the following thicknesses (in µm): 1, 2, 3, 4, 5, 6, 7, 8, 9, 10, 15, 20, 25, 30, 35, 40, 45, 50, 55, 60, or 65. That is, the thickness of the microcarrier may be any of a range of thicknesses (in µm) having an upper limit of 70, 65, 60, 55, 50, 45, 40, 35, 30, 25, 20, 15, 10, 9, 8, 7, 6, 5, 4, 3, or 2 and an independently selected lower limit of 1, 2, 3, 4, 5, 6, 7, 8, 9, 10, 15, 20, 25, 30, 35, 40, 45, 50, 55, 60, or 65, wherein the lower limit is less than the upper limit.

In some embodiments, the thickness of the microcarrier is about 50 µm, about 45 µm, about 40 µm, about 35 µm, about 30 µm, about 25 µm, about 20 µm, about 19 µm, about 18 µm, about 17 µm, about 16 µm, about 15 µm, about 14 µm, about 13 µm, about 12 µm, about 11 µm, about 10 µm, about 9 µm, about 8 µm, about 7 µm, about 6 µm, about 5 µm, about 4 µm, about 3 µm, about 2 µm, or about 1 µm. In certain embodiments, the microcarrier is about 10 µm in thickness.

A variety of techniques may be used to couple a probe of the present disclosure to an encoded microcarrier. In some embodiments, the probe is coupled to a surface of the microcarrier (in some embodiments, in at least a center portion of the microcarrier surface). In some embodiments, the probe may be coupled to one or both of a first or a second surface of the polymer layer. In some embodiments, the polymer comprises an epoxy-based polymer or otherwise contains an epoxide group.

In some embodiments, the probe can be chemically attached to the microcarrier. In other embodiments, the probe can be physically absorbed to the surface of the microcarrier. In some embodiments, the attachment linkage between the probe and the microcarrier surface can be a covalent bond. In other embodiments, the attachment linkage between the probe and the microcarrier surface can be a non-covalent bond including, but not limited to, a salt bridge or other ionic bond, one or more hydrogen bonds, hydrophobic interactions, Van der Waals force, London dispersion force, a mechanical bond, one or more halogen bonds, aurophilicity, intercalation, or stacking.

In some embodiments, coupling the probe involves reacting the polymer with a photoacid generator and light to generate a cross-linked polymer. In some embodiments, the light is of a wavelength that activates the photoacid generator, *e.g*., UV or near-UV light. Photoacid generators are commercially available from Sigma-Aldrich (St. Louis) and BASF (Ludwigshafen). Any suitable photoacid generator known in the art may be used, including without limitation triphenyl or triaryl sulfonium hexafluoroantimonate; triarylsulfonium hexafluorophosphate; triphenylsulfonium perfluoro-1-butanesulfonate; triphenylsulfonium triflate; Tris(4-*tert*-butylphenyl)sulfonium perfluoro-1-butanesulfonate or triflate; *Bis(4-tert-*butylphenyl)iodonium-containing photoacid generators such as Bis(4-*tert*-butylphenyl)iodonium perfluoro-1-butanesulfonate, *p*-toluenesulfonate, and triflate; Boc-methoxyphenyldiphenylsulfonium triflate; (*tert*-Butoxycarbonylmethoxynaphthyl)-diphenylsulfonium triflate; (4-*tert*-Butylphenyl)diphenylsulfonium triflate; diphenyliodonium hexafluorophosphate, nitrate, perfluoro-1-butanesulfonate, triflate, or *p*-toluenesulfonate; (4-fluorophenyl)diphenylsulfonium triflate; *N*-hydroxynaphthalimide triflate; *N*-hydroxy-5-norbornene-2,3-dicarboximide perfluoro-1-butanesulfonate; (4-iodophenyl)diphenylsulfonium triflate; (4-methoxyphenyl)diphenylsulfonium triflate; 2-(4-methoxystyryl)-4,6-bis(trichloromethyl)-1,3,5-triazine; (4-methylphenyl)diphenylsulfonium triflate; (4-methylthiophenyl)methyl phenyl sulfonium triflate; (4-phenoxyphenyl)diphenylsulfonium triflate; (4-phenylthiophenyl)diphenylsulfonium triflate; or any of the photoacid generators described in product-finder.basf.com/group/corporate/product-finder/de/literature-document:/Brand+Irgacure-Brochure--Photoacid+Generator+Selection+Guide-English.pdf. In some embodiments, the photoacid generator is a sulfonium-containing photoacid generator.

In some embodiments, coupling the probe involves reacting an epoxide of the cross-linked polymer with a functional group such as an amine, carboxyl, thiol, or the like. Alternatively, the epoxy group on the surface can be oxidized to hydroxyl group, which is subsequently used as initiation sites for graft polymerization of water soluble polymers such as poly(acrylic acid). The carboxyl groups in poly(acrylic acid) are then used to form covalent bonds with amino or hydroxyl groups in capture agents. For example, in certain embodiments, the carboxyl groups in poly(acrylic acid) are used to form covalent bonds with amino groups in the probe.

In some embodiments, coupling the probe involves reacting an epoxide of the cross-linked polymer with a compound that contains an amine and a carboxyl. In some embodiments, the amine of the compound reacts with the epoxide to form a compound-coupled, cross-linked polymer. Without wishing to be bound to theory, it is thought that the probe may be coupled to the polymer before the polymer is cross-linked; however, this may reduce the uniformity of the resulting surface. Any compound with a primary amine and a carboxyl group may be used. Compounds may include without limitation glycine, amino undecanoic acid, amino caproic acid, acrylic acid, 2-carboxyethyl acrylic acid, 4-vinylbenzoic acid, 3-acrylamido-3-methyl-1-butanoic acid, glycidyl methacrylate, and the like. In some embodiments, the carboxyl of the compound-coupled, cross-linked polymer reacts with an amine (*e.g.,* a primary amine) of the probe to couple the capture agent to the substantially transparent polymer.

### V. Kits

The invention provides a kit, comprising:
(a) at least four microcarriers, wherein each of said at least four microcarriers comprises:
   (i) a probe coupled to the microcarrier, wherein the probe is specific for a DNA mutation in the *KRAS, BRAF, CTNNB1,* or *APC* gene, wherein optionally the *KRAS, BRAF, CTNNB1,* and *APC* genes are human genes; and
   (ii) an identifier corresponding to the probe coupled thereto; wherein the kit comprises at least one microcarrier comprising a probe specific for a DNA mutation in the *KRAS* gene, at least one microcarrier comprising a probe specific for a DNA mutation in the *BRAF* gene, at least one microcarrier comprising a probe specific for a DNA mutation in the *CTNNB1* gene, and at least one microcarrier comprising a probe specific for a DNA mutation in the *APC* gene; and
(b) at least four blocking nucleic acids, wherein each of said at least four blocking nucleic acids hybridizes with a wild-type DNA locus corresponding with one of the DNA mutations in the KRAS, BRAF, CTNNB1, or APC genes.

These kits may find use, *inter alia,* in conducting a multiplex assay, such as the exemplary multiplex assays described herein (see, *e.g.,* section III above). Any of the microcarriers described herein (see, *e.g.,* section IV above) may find use in a kit of the present disclosure

A kit of the present disclosure comprises at least four encoded microcarriers. Each of the four encoded microcarriers comprises (i) a probe, specific for a DNA mutation in the *KRAS, BRAF, CTNNB1,* or *APC* gene, coupled to the microcarrier; and (ii) an identifier corresponding to the probe. The kit comprises at least one microcarrier comprising a probe specific for a DNA mutation in the *KRAS* gene, at least one microcarrier comprising a probe specific for a DNA mutation in the *BRAF* gene, at least one microcarrier comprising a probe specific for a DNA mutation in the *CTNNB1* gene, and at least one microcarrier comprising a probe specific for a DNA mutation in the *APC* gene. That is to say, each of the *KRAS, BRAF, CTNNB1,* and *APC* genes is represented in the kit by a microcarrier with a probe specific for a mutation in the gene. Exemplary *KRAS, BRAF, CTNNB1,* and *APC* genes and mutations are described *supra.* In some embodiments, the *KRAS, BRAF, CTNNB1,* and *APC* genes are human genes. In some embodiments, the kit comprises microcarriers with probes suitable for detecting each of the following mutations (*e.g*., with at least one microcarrier + probe species for each mutation in the kit): DNA mutations encoding G12D, G12V, G12S, and G13D mutated KRAS proteins; DNA mutation(s) encoding a V600E mutated BRAF protein; DNA mutations encoding T41A and T41I mutated CTNNB1 proteins; DNA mutations encoding S45F and S45P mutated CTNNB1 proteins; and DNA mutations encoding Q1367*, R1450*, E1309 frameshift, S1465 frameshift, and T1556 frameshift mutated APC proteins.

The kit further comprises at least four blocking nucleic acids. Said at least four blocking nucleic acids hybridize with wild-type DNA loci corresponding with DNA mutations in the each of the *KRAS, BRAF, CTNNB1, and APC* genes. Exemplary descriptions of blocking nucleic acids are provided in section III. In some embodiments, the kit comprises a blocking nucleic acid comprising the sequence TA*C*G*CC*A*CC*A*G*CT(invdT)*ₙ*, wherein n is 1, 2, or 3 (SEQ ID NO:3), TT*GG*A*G*CT*GG*T*GG*CGTAinvdTinvdTinvdT (SEQ ID NO: 142), GCT*GG*T*GG*C*G*TA*G*GCAinvdTinvdTinvdT (SEQ ID NO: 143), *GCTGGTGGCGTA*GGCinvdTinvdTinvdT (SEQ ID NO: 144), or TT*GG*A*G*CT*GG*T*GG*C*G*TinvdTinvdTinvdT (SEQ ID NO: 145); a blocking nucleic acid comprising the sequence G*AGAT*T*TCAC*T*GTAGC*(invdT)*ₙ,* wherein *n is* 1, 2, or 3 (SEQ ID NO:10), GA*G*AT*TTCACTGT*AGCinvdTinvdTinvdT (SEQ ID NO: 146), G*AGA*TT*TC*AC*TGTAGC* invdTinvdTinvdT (SEQ ID NO: 147), *GAGAT*T*TCACTGTAGCinvdTinvdTinvdT* (SEQ ID NO:148), or GAGAT*TTCACT*G*T*A*GC*invdTinvdTinvdT (SEQ ID NO:149); a blocking nucleic acid comprising the sequence GC*CACTACCACAG*CT(invdT)*ₙ,* wherein *n is* 1, 2, or 3 (SEQ ID NO:15), T*GCCA*CT*ACCACAG*invdTinvdTinvdT (SEQ ID NO:150), C*ACTACCACAGC*T*CC*invdTinvdTinvdT (SEQ ID NO:151), G*CCACT*A*CCA*C*AGC*TinvdTinvdTinvdT (SEQ ID NO: 152), or *GC*C*ACTA*CCA*CAG*CTinvdTinvdTinvdT (SEQ ID NO: 153), and/or a blocking nucleic acid comprising the sequence GC*TCCTTCTCTG*AGTinvdTinvdTinvdT (SEQ ID NO:20), T*CC*T*TCTC*TGA*G*T*G*GinvdTinvdTinvdT (SEQ ID NO: 174), G*C*T*CC*T*TC*TC*TGA*GTinvdTinvdTinvdT (SEQ ID NO: 175), T*CC*TT*C*TCT*GAG*T*G*GinvdTinvdTinvdT (SEQ ID NO: 176), or G*C*T*CC*TT*CT*C*TGAG*TinvdTinvdTinvdT (SEQ ID NO: 177); and one or more of: a blocking nucleic acid comprising the sequence GTG*CTCA*G*AC*ACCinvdTinvdTinvdT (SEQ ID NO:33), G*TGC*TC*A*G*A*C*ACC*invdTinvdTinvdT (SEQ ID NO: 158), AGTGGTG*CTCAGAC*ACCCAinvdTinvdTinvdT (SEQ ID NO:159), A*GTG*GT*GCTC*AGACACCCAinvdTinvdTinvdT (SEQ ID NO: 160), or A*G*T*G*GTG*CTCAGAC*A*C*C*C*AinvdTinvdTinvdT (SEQ ID NO: 161), a blocking nucleic acid comprising the sequence C*TTC*T*CGCT*T*GGT*TinvdTinvdTinvdT (SEQ ID NO:37), G*TAC*T*TCTCG*CT*TGG*TinvdTinvdTinvdT (SEQ ID NO: 162), C*TTC*T*CGCT*T*GGT*TinvdTinvdTinvdT (SEQ ID NO:163), GT*ACT*T*CTCGC*T*TGG*TinvdTinvdTinvdT (SEQ ID NO: 164), or GT*ACTTCTCGC*TT*GG*TinvdTinvdTinvdT (SEQ ID NO:165), a blocking nucleic acid comprising the sequence *CTTTTCTTTTATTTCTGCinvdTinvdTinvdT* (SEQ ID NO:29), C*TTTT*CT*TTTA*TIT*C*TG*C*invdTinvdTinvdT (SEQ ID NO:154), C*T*TT*TC*T*T*T*TATTTCTGC*invdTinvdTinvdT (SEQ ID NO:155), C*TTT*TCT*TTT*A*T*T*TC*T*GC*invdTinvdTinvdT (SEQ ID NO:156), or C*I*TT*TCTTTTATTTC*TG*C*invdTinvdTinvdT (SEQ ID NO:157), a blocking nucleic acid comprising the sequence *CC*AC*TC*TCTCT*CTT*T*CAGC*invdTinvdTinvdT (SEQ ID NO:25), T*AGG*T*CC*ACTCTCTCTC*TT*T*C*A*GC*AinvdTinvdTinvdT (SEQ ID NO:166), T*AGG*T*CCAC*T*CTC*T*CT*CTT*T*TC*AG*CA invdTinvdTinvdT (SEQ ID NO:167), *CCACTCTCTCTCTTTTC*A*GC* invdTinvdTinvdT (SEQ ID NO:168), or TA*G*GT*CC*A*CT*CT*C*TCT*C*T*T*TCA*GC*A invdTinvdTinvdT (SEQ ID NO:169), and a blocking nucleic acid comprising the sequence CAATAGTTTTITCTGCCinvdTinvdTinvdT (SEQ ID NO:41), *G*A*A*T*CAATAG*TTTTTT*CTGCCTC* invdTinvdTinvdT (SEQ ID NO:170), T*CAG*A*ATC*A*ATAG*TTTTT*TCTG* invdTinvdTinvdT (SEQ ID NO:171), *G*A*ATCAATAG*ATTTTA*CTGCCTC* invdTinvdTinvdT (SEQ ID NO:172), or *AA*T*CAATAG*TTTTTT*CTGCCT*C invdTinvdTinvdT (SEQ ID NO:173), (italicized nucleic acids representing locked nucleic acids).

In some embodiments, the kit further comprises one or more primer pairs, *e.g.,* for amplifying the locus of a DNA mutation of interest. In some embodiments, the kit comprises a primer pair specific for the locus of one or more DNA mutations in each of the *KRAS, BRAF, CTNNB1,* and *APC* genes, *e.g.,* at least four primer pairs. In some embodiments, the kit comprises a primer pair comprising the sequences GTACTGGTGGAGTATTTGATAGTG (SEQ ID NO:1) and ATCGTCAAGGCACTCTTGCCTAC (SEQ ID NO:2) for amplification of a locus comprising a *KRAS* mutation (*e.g.,* one or more *KRAS* mutations encoding G12D, G12V, G12S, and G13D mutated KRAS proteins); a primer pair comprising the sequences GGACCCACTCCATCGAGATTT (SEQ ID NO:8) and CAGATATATTTCTTCATGAAGACCTCACAGTAA (SEQ ID NO:9) for amplification of a locus comprising a *BRAF* mutation *(e.g.,* one, two, or more *BRAF* mutations encoding a V600E mutated BRAF protein); a primer pair comprising the sequences GGAATCCATTCTGGTGCCACT (SEQ ID NO: 13) and AGAAAATCCCTGTTCCCACTCATA (SEQ ID NO:14) and/or a primer pair comprising the sequences GGTGCCACTACCACAGCTCCT (SEQ ID NO:18) and TCTCAAAACTGCATTCTGACTTTCA (SEQ ID NO:19) for amplification of a locus comprising a *CTNNB1* mutation (*e.g.,* one or more *CTNNB1* mutations encoding T41A, T41I, S45F, or S45P mutated CTNNB1 proteins); and one or more of: a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40) for amplification of a locus comprising *an APC* mutation (*e.g.,* one or more *APC* mutations encoding Q1367*, R1450*, E1309 frameshift, S1465 frameshift, or T1556 frameshift mutated APC proteins).

Any of the probes described herein (*e.g.,* in section III) may be included in the kit, *e.g.,* coupled to an encoded microcarrier. For example, suitable probes for detecting a DNA mutation in the *KRAS* gene can include the sequences GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO:180), GCTGATGGCGTA (SEQ ID NO:181), GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO:184), TGGAGCTGTaGGTGG (SEQ ID NO: 185), TGGAGCTAGTGG (SEQ ID NO:6), TTGGAGCTAGTGGCGTA (SEQ ID NO:186), GCTAGTGGCGTAGGC (SEQ ID NO:187), AGCTAGTGGCGT (SEQ ID NO: 188), GTTGGAGCTAGTGG (SEQ ID NO: 189), GGAGCTAGTGG (SEQ ID NO: 190), TGGAGCTGGTGACGT (SEQ ID NO:7), GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), GGAGCTGGTGACGT (SEQ ID NO: 195), TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), TTTTTTTTTTTTTGCTGATGGCGTA (SEQ ID NO:77), TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), TTTTTTTTTATGGAGCTGTAGGTGG (SEQ ID NO:81), TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), TTTTTTTTTTTTAAGGAGCTAGTGG (SEQ ID NO:86), TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), and TTTTTTTTTAAGGAGCTGGTGACGT (SEQ ID NO:91). In some embodiments, a probe of the present disclosure can comprise eight or more nucleotides (e.g., adenines or thymines) at its 5' end. Suitable probes for detecting a DNA mutation in the *BRAF* gene can include the sequences TCTAGCTACAGAGAAAT (SEQ ID NO:11), GTCTAGCTACAGAAAAAT (SEQ ID NO:12), TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), TCTAGCTACAGAG (SEQ ID NO:200), TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), TTTTTTTTTTAATTTCTAGCTACAGAG (SEQ ID NO:96), TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and/or TTTTTTTTATTCTAGCTACAGAAAAATC (SEQ ID NO:100). Suitable probes for detecting a DNA mutation in the *CTNNB1* gene can include the sequences AGGAGCTGTGGCAG (SEQ ID NO:16), GGAGCTGTGATA (SEQ ID NO:17), TTTACCACTCAGAAAAG (SEQ ID NO:21), TACCACTCAGAGGAG (SEQ ID NO:22), TTTTTTTTTTTAGGAGCTGTGGCAG (SEQ ID NO:53), TTTTTTTTTTTAGGAGCTGTGGCAGTG (SEQ ID NO: 101), TTTTTTTTTTTAGCTGTGGCAGTGGC (SEQ ID NO:102), TTTTTTTTTTTGCTGTGGCAGTGGCA (SEQ ID NO:103), TTTTTTTTTTAAGGAGCTGTGGCAG (SEQ ID NO:104), TTTTTTTTTTTTTGGAGCTGTGATA (SEQ ID NO:54), TTTTTTTTTGGAGCTGTGATAGTGG (SEQ ID NO:105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), TTTTTTTTTTTTTTGGAGCTGTGAT (SEQ ID NO:109), TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), TTTTTTTTTCAGAAAAGGAGCTGTG (SEQ ID NO:114), TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and/or TTTTTTTTTATTACCAATCAGAGGAGG (SEQ ID NO:119). Suitable probes for detecting a DNA mutation in the *APC* gene can include the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), AGAGGCAGAAAAAAACT (SEQ ID NO:42), TTTTTTTTACTGCTGAAAAGAGAGAGT (SEQ ID NO:57), TTTTTTTTTTGCACCTACTGCTGAA (SEQ ID NO:134), TTTTTTTTTACCTACTGCTGAAAAG (SEQ ID NO:135), TTTTTTTTTGCTGAAAAGAGAGAGT (SEQ ID NO:136), and TTTTTTTTTCCTACTGCTGAAAAGAGA (SEQ ID NO:137), TTTTTTTTTTTTTGAAATAAAAGATTGG (SEQ ID NO:58), TTTTTTTTTTAAATAGCAGAAATAAAAG (SEQ ID NO:120), TTTTTTTTTTTGAAATAAAAGATTGGAA (SEQ ID NO:121), TTTTTTTTTTTTTAGAAATAAAAGATTG (SEQ ID NO:122), TTTTTTTTTTTTTGAAATAAATGAATGG (SEQ ID NO:123), TTTTTTTTTTTTTCAGAAATAAAAGATT (SEQ ID NO:124), TTTTTTTTTTTTTGGGTGTCTAAG (SEQ ID NO:59), TTTTTTTTTATTTGGGTGTCTAAG (SEQ ID NO: 125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128), TTTTTTTTTGGTGTCTAAGCACCA (SEQ ID NO: 129), TTTTTTTTTACAAACCAAGTGAGAA (SEQ ID NO:60), TTTTTTTTAAGTGAGAAGTACCTAA (SEQ ID NO:130), TTTTTTTTTTTTCAAACCAAGTGAG (SEQ ID NO:131), TTTTTTTTTTACCAAGTGAGAAGTA (SEQ ID NO: 132), TTTTTTTTAGCTCAAACCAAGTGAG (SEQ ID NO: 133), TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO: 139), TTTTTTTAGAAAGAGGCAGAAAAAAACT (SEQ ID NO: 140), and/or TTTTTTTTTTTGAGGCAGAAAAAAACTA (SEQ ID NO: 141) (probes comprising these sequences exclusive of the 5' adenine and/or thymines are also contemplated). In some embodiments, each probe is coupled to a microcarrier of the present disclosure with a unique identifier.

A kit of the present disclosure suitable for detecting mutations in a *KRAS* gene (*e.g., KRAS* mutations encoding G12D, G12V, G12S, and G13D mutated KRAS proteins) can optionally include: four probes of the present disclosure specific for mutation(s) in a *KRAS* gene, wherein each of the four probes is coupled to a microcarrier with a different identifier; a primer pair comprising the sequences GTACTGGTGGAGTATTTGATAGTG (SEQ ID NO:1) and ATCGTCAAGGCACTCTTGCCTAC (SEQ ID NO:2); and/or a blocking nucleic acid comprising the sequence of TA*C*G*CC*A*CC*A*G*CT(invdT)*ₙ*, wherein n is 1, 2, or 3 (SEQ ID NO:3), TTGGAGCTGGTGGCGTAinvdTinvdTinvdT (SEQ ID NO: 142), GCTGGTGGCGTAGGCAinvdTinvdTinvdT (SEQ ID NO: 143), GCTGGTGGCGTAGGCinvdTinvdTinvdT (SEQ ID NO: 144), or TTGGAGCTGGTGGCGTinvdTinvdTinvdT (SEQ ID NO: 145), with italicized nucleic acids representing locked nucleic acids. In some embodiments, the kit comprises: four probes comprising the sequences GGAGCTGATGG (SEQ ID NO:4), GGAGCTGTTGG (SEQ ID NO:5), TGGAGCTAGTGG (SEQ ID NO:6), and TGGAGCTGGTGACGT (SEQ ID NO:7), respectively; four probes comprising: a first probe comprising a sequence selected from GGAGCTGATGG (SEQ ID NO:4), AGCTGATGGCGTA (SEQ ID NO:178), TGGAGCTGATGGCG (SEQ ID NO: 179), TGGAGCTGATGG (SEQ ID NO: 180), or GCTGATGGCGTA (SEQ ID NO:181); a second probe comprising a sequence selected from GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO:184), or TGGAGCTGTaGGTGG (SEQ ID NO: 185); a third probe comprising a sequence selected from TTGGAGCTAGTGGCGTA (SEQ ID NO:186), GCTAGTGGCGTAGGC (SEQ ID NO:187), AGCTAGTGGCGT (SEQ ID NO: 188), GTTGGAGCTAGTGG (SEQ ID NO: 189), or GGAGCTAGTGG (SEQ ID NO:190); and a fourth probe comprising a sequence selected from GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), or GGAGCTGGTGACGT (SEQ ID NO: 195); respectively; or four probes comprising: a first probe comprising a sequence selected from TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), and TTTTTTTTTTTTTGCTGATGGCGTA (SEQ ID NO:77); a second probe comprising a sequence selected from TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), and TTTTTTTTTATGGAGCTGTAGGTGG (SEQ ID NO:81); a third probe comprising a sequence selected from TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), and TTTTTTTTTTTTAAGGAGCTAGTGG (SEQ ID NO:86); and TTTTTTTTTATGGAGCTGGTGACGT (SEQ ID NO:50), respectively (probes comprising these sequences exclusive of the 5' adenine and/or thymines are also contemplated). The kit may optionally include any of the elements described *infra* for detecting mutations in *a BRAF, CTNNB1,* and/or *APC* gene. In some embodiments, each probe is coupled to a microcarrier of the present disclosure with a unique identifier.

A kit of the present disclosure suitable for detecting mutations in a *BRAF* gene *(e.g.,* two or more *BRAF* mutations encoding a V600E mutated BRAF protein) can optionally include: two probes of the present disclosure specific for mutation(s) in a *BRAF* gene, wherein each of the two probes is coupled to a microcarrier with a different identifier; a primer pair comprising the sequences GGACCCACTCCATCGAGATTT (SEQ ID NO:8) and CAGATATATTTCTTCATGAAGACCTCACAGTAA (SEQ ID NO:9); and/or a blocking nucleic acid comprising the sequence of G*AGAT*T*TCAC*T*GTAGC*(invdT)*ₙ,* wherein *n is* 1, 2, or 3 (SEQ ID NO:10), GAGAT*TTCACTGT*AGCinvdTinvdTinvdT (SEQ ID NO:146), *GAGATTTC*AC*TGTAG*C invdTinvdTinvdT (SEQ ID NO: 147), *GAGAT*T*TCACT*G*TAGCinvdTinvdTinvdT* (SEQ ID NO:148), or G*AGA*T*TT*C*ACT*G*T*AG*C*invdTinvdTinvdT (SEQ ID NO:149), with italicized nucleic acids representing locked nucleic acids. In some embodiments, the kit comprises: two probes comprising the sequences TCTAGCTACAGAGAAAT (SEQ ID NO:11) and GTCTAGCTACAGAAAAAT (SEQ ID NO:12), respectively; two probes comprising the sequences TTTTTTAATTTCTAGCTACAGAGAAAT (SEQ ID NO:51) and TTTTTTTATGTCTAGCTACAGAAAAAT (SEQ ID NO:52), respectively; (1) a first probe comprising a sequence selected from TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), and TCTAGCTACAGAG (SEQ ID NO:200); and (2) a second probe comprising a sequence selected from GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204), respectively; or (1) a first probe comprising a sequence selected from TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), and TTTTTTTTTTAATTTCTAGCTACAGAG (SEQ ID NO:96); and (2) a second probe comprising a sequence selected from TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and TTTTTTTTATTCTAGCTACAGAAAAATC (SEQ ID NO:100), respectively (probes comprising these sequences exclusive of the 5' adenine and/or thymines are also contemplated). The kit may optionally include any of the elements described *supra* for detecting mutations in a *KRAS* gene, and/or any of the elements described *infra* for detecting mutations in a *CTNNB1* and/or *APC* gene. In some embodiments, each probe is coupled to a microcarrier of the present disclosure with a unique identifier.

A kit of the present disclosure suitable for detecting mutations in a *CTNNB1* gene (*e.g., CTNNB1* mutations encoding T41A, T41I, S45F, and S45P mutated CTNNB1 proteins) can optionally include: four probes of the present disclosure specific for mutation(s) in a *CTNNB1* gene, wherein each of the four probes is coupled to a microcarrier with a different identifier; a first primer pair comprising the sequences GGAATCCATTCTGGTGCCACT (SEQ ID NO:13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO: 19); and/or a first blocking nucleic acid comprising the sequence of GC*CACTACCACAG*CT(invdT)*ₙ,* wherein *n is* 1, 2, or 3 (SEQ ID NO:15), T*GC*C*A*CT*ACCA*C*AG*invdTinvdTinvdT (SEQ ID NO:150), C*AC*T*ACCACAGC*T*CC*invdTinvdTinvdT (SEQ ID NO:151), G*CC*A*CT*A*CCA*C*AG*CTinvdTinvdTinvdT (SEQ ID NO: 152), or *GC*C*ACTA*CCA*CAG*CTinvdTinvdTinvdT (SEQ ID NO: 153), and a second blocking nucleic acid comprising the sequence of GCTCCTTCTCTGAGTinvdTinvdTinvdT (SEQ ID NO:20), T*CC*T*TCTC*T*G*A*G*T*G*GinvdTinvdTinvdT (SEQ ID NO:174), G*C*T*CC*T*TC*TC*TGAG*TinvdTinvdTinvdT (SEQ ID NO: 175), T*CC*TT*CTC*T*GAG*TG*G*invdTinvdTinvdT (SEQ ID NO: 176), or G*CTCC*TT*CTCTGAG*TinvdTinvdTinvdT (SEQ ID NO: 177), with italicized nucleic acids representing locked nucleic acids. In some embodiments, the kit comprises: four probes comprising the sequences AGGAGCTGTGGCAG (SEQ ID NO:16), GGAGCTGTGATA (SEQ ID NO:17), TTTACCACTCAGAAAAG (SEQ ID NO:21), and TACCACTCAGAGGAG (SEQ ID NO:22), respectively; four probes comprising: a first probe comprising a sequence selected from AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), and AAGGAGCTGTGGCAG (SEQ ID NO:209); a second probe comprising a sequence selected from GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), and GGAGCTGTGAT (SEQ ID NO:214); a third probe comprising a sequence selected from ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), and CAGAAAAGGAGCTGTG (SEQ ID NO:219); and (4) a fourth probe comprising a sequence selected from ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), and TTACCAATCAGAGGAGG (SEQ ID NO:224); or four probes comprising: a first probe comprising a sequence selected from TTTTTTTTTTTAGGAGCTGTGGCAG (SEQ ID NO:53), TTTTTTTTTTTAGGAGCTGTGGCAGTG (SEQ ID NO:101), TTTTTTTTTTTAGCTGTGGCAGTGGC (SEQ ID NO:102), TTTTTTTTTTTGCTGTGGCAGTGGCA (SEQ ID NO:103), and TTTTTTTTTTAAGGAGCTGTGGCAG (SEQ ID NO:104); a second probe comprising a sequence selected from TTTTTTTTTTTTTGGAGCTGTGATA (SEQ ID NO:54), TTTTTTTTTGGAGCTGTGATAGTGG (SEQ ID NO:105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), and TTTTTTTTTTTTTTGGAGCTGTGAT (SEQ ID NO:109); a third probe comprising a sequence selected from the group consisting of TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), and TTTTTTTTTCAGAAAAGGAGCTGTG (SEQ ID NO:114); and (4) a fourth probe comprising a sequence selected from the group consisting of TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and TTTTTTTTTATTACCAATCAGAGGAGG (SEQ ID NO:119), wherein each of the four probes is coupled to a microcarrier with a different identifier (probes comprising these sequences exclusive of the 5' adenine and/or thymines are also contemplated). The kit may optionally include any of the elements described *supra* for detecting mutations in a *KRAS* and/or *BRAF* gene, and/or any of the elements described *infra* for detecting mutations in an *APC* gene. In some embodiments, each probe is coupled to a microcarrier of the present disclosure with a unique identifier.

A kit of the present disclosure suitable for detecting mutations in an *APC* gene *(e.g., APC* mutations encoding Q1367*, R1450*, E1309 frameshift, S1465 frameshift, and T1556 frameshift mutated APC proteins) can optionally include: five probes of the present disclosure specific for mutation(s) in a *APC* gene, wherein each of the five probes is coupled to a microcarrier with a different identifier; a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40); and/or a first blocking nucleic acid comprising the sequence of *CCACTC*TCTCTCT*TT*T*CAGC*invdTinvdTinvdT (SEQ ID NO:25), T*AGG*T*CC*ACTCTCTCTCT*TT*TCA*GC*AinvdTinvdTinvdT (SEQ ID NO:166), T*AGG*T*CCAC*T*CTCT*C*T*CTT*T*TC*AG*CA invdTinvdTinvdT (SEQ ID NO:167), *CCACTCTCTCTC*TTTT*C*AGC invdTinvdTinvdT (SEQ ID NO:168), or TA*G*GT*CC*AC*T*CT*C*TCT*C*T*T*TT*C*A*GC*A invdTinvdTinvdT (SEQ ID NO:169), a second blocking nucleic acid comprising the sequence of *CTTTTCTTTTATTTCTGCinvdTinvdTinvdT* (SEQ ID NO:29), C*TTTTC*T*TTTA*T*T*T*C*TG*C*invdTinvdTinvdT (SEQ ID NO:154), C*T*TT*TC*T*I*T*TATTTCTGC*invdTinvdTinvdT (SEQ ID NO:155), C*TTT*TCT*TTT*A*T*T*TC*T*GC*invdTinvdTinvdT (SEQ ID NO:156), or C*T*TT*TCTTTTATTTC*TG*C*invdTinvdTinvdT (SEQ ID NO:157), a third blocking nucleic acid comprising the sequence of GTG*CTCA*G*ACAC*CinvdTinvdTinvdT (SEQ ID NO:33), G*TGC*T*CA*G*A*C*ACC*invdTinvdTinvdT (SEQ ID NO:158), AGTGGTG*CTCAGACA*CCCAinvdTinvdTinvdT (SEQ ID NO:159), A*GTG*GT*GCTCA*G*ACACCCA*invdTinvdTinvdT (SEQ ID NO:160), or A*GTG*GTGC*TCA*G*AC*A*CCC*AinvdTinvdTinvdT (SEQ ID NO:161), a fourth blocking nucleic acid comprising the sequence of CTTCTCGCTTGGTTinvdTinvdTinvdT (SEQ ID NO:37), G*TAC*T*TCTCG*CT*TGG*TinvdTinvdTinvdT (SEQ ID NO:162), C*TTC*T*CGCT*T*GGT*TinvdTinvdTinvdT (SEQ ID NO:163), GT*ACT*T*CTCG*CT*TGG*TinvdTinvdTinvdT (SEQ ID NO:164), or GT*ACTTCTCGC*TT*GG*TinvdTinvdTinvdT (SEQ ID NO:165), and a fifth blocking nucleic acid comprising the sequence of *C*A*ATA*G*TTTT*IT*CTGC*CinvdTinvdTinvdT (SEQ ID NO:41), *GAA*T*CAATAG*TTTTTT*CTGCCTC* invdTinvdTinvdT (SEQ ID NO:170), *TCAG*A*ATCAATAG*TTTTTT*CTG* invdTinvdTinvdT (SEQ ID NO:171), *GAA*T*CAATAGA*TTTT*ACTGCCT*C invdTinvdTinvdT (SEQ ID NO:172), or A*ATCAATAG*TTTTTT*CTGCCTC* invdTinvdTinvdT (SEQ ID NO:173), with italicized nucleic acids representing locked nucleic acids. In some embodiments, the kit comprises: five probes comprising the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), and AGAGGCAGAAAAAAACT (SEQ ID NO:42), respectively; five probes comprising: (1) a first probe comprising a sequence selected from AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (2) a second probe comprising a sequence selected from TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (3) a third probe comprising a sequence selected from AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (4) a fourth probe comprising a sequence selected from GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (5) a fifth probe comprising a sequence selected from GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246), respectively; or five probes comprising: (1) a first probe comprising a sequence selected from the group consisting of TTTTTTTTTTTTTGAAATAAAAGATTGG (SEQ ID NO:58), TTTTTTTTTTAAATAGCAGAAATAAAAG (SEQ ID NO:120), TTTTTTTTTTTGAAATAAAAGATTGGAA (SEQ ID NO:121), TTTTTTTTTTTTTAGAAATAAAAGATTG (SEQ ID NO:122), TTTTTTTTTTTTTGAAATAAATGAATGG (SEQ ID NO:123), and TTTTTTTTTTTTTCAGAAATAAAAGATT (SEQ ID NO:124); (2) a second probe comprising a sequence selected from the group consisting of TTTTTTTTTTTTTGGGTGTCTAAG (SEQ ID NO:59), TTTTTTTTTATTTGGGTGTCTAAG (SEQ ID NO: 125), TTTTTTGGGTGTCTAAGCACCACT (SEQ ID NO:126), TTTTTTTTTCTAAGCACCACTTTT (SEQ ID NO:127), TTTTTTTTTTTTTTGGGTGTCTAA (SEQ ID NO:128),and TTTTTTTTTGGTGTCTAAGCACCA (SEQ ID NO:129); (3) a third probe comprising a sequence selected from the group consisting of TTTTTTTTTACAAACCAAGTGAGAA (SEQ ID NO:60), TTTTTTTTAAGTGAGAAGTACCTAA (SEQ ID NO:130), TTTTTTTTTTTTCAAACCAAGTGAG (SEQ ID NO:131), TTTTTTTTTTACCAAGTGAGAAGTA (SEQ ID NO:132), and TTTTTTTTAGCTCAAACCAAGTGAG (SEQ ID NO:133); (4) a fourth probe comprising a sequence selected from the group consisting of TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO:136), and TTTTTTTTTCCTACTGCTGAAAAGAGA (SEQ ID NO:137); and (5) a fifth probe comprising a sequence selected from the group consisting of TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO:139), TTTTTTTAGAAAGAGGCAGAAAAAAACT (SEQ ID NO:140), and TTTTTTTTTTTGAGGCAGAAAAAAACTA (SEQ ID NO:141), respectively; wherein each of the five probes is coupled to a microcarrier with a different identifier (probes comprising these sequences exclusive of the 5' adenine and/or thymines are also contemplated). The kit may optionally include any of the elements described *supra* for detecting mutations in a *KRAS, BRAF,* and/or *CTNNB1* gene. In some embodiments, each probe is coupled to a microcarrier of the present disclosure with a unique identifier. In some embodiments of any of the above embodiments, combinations of primers, probes, and/or blocking nucleic acids for 1, 2, 3, 4, 5, 6, 7, 8, 9, 10, 11, 12, 13, 14, or 15 mutations described herein can be combined in any order or combination.

In some embodiments, the kit comprises a microcarrier with an identifier corresponding to a positive control and to which a probe specific for a positive control gene sequence is coupled, and a primer pair specific for the positive control DNA sequence. For example, in some embodiments, the positive control DNA sequence comprises a sequence of a human leukocyte antigen (HLA) gene, the primer pair specific for the positive control DNA sequence comprises the sequences TGAGTGTTACTTCTTCCCACACTC (SEQ ID NO:43) and ATTGCTTTTGCGCAATCCCT (SEQ ID NO:44), and/or the probe specific for the positive control gene sequence comprises the sequence TTTTTTTTTTTTGGAGACGGTCTG (SEQ ID NO:45). In some embodiments, the positive control DNA sequence comprises a sequence of a human glyceraldehyde 3-phosphate dehydrogenase (GAPDH) gene, the primer pair specific for the positive control DNA sequence comprises the sequences AATCCCATCACCATCTTCCA (SEQ ID NO:71) and TGGACTCCACGACGTACTCA (SEQ ID NO:72), and/or the probe specific for the positive control gene sequence comprises the sequence CTGTCTTCCACTCACTCC (SEQ ID NO:73). In some embodiments, each probe is coupled to a microcarrier of the present disclosure with a unique identifier.

In some embodiments, the kit comprises a microcarrier of the present disclosure with an identifier corresponding to a negative control, *e.g*., with a probe that does not hybridize with the amplified DNA. In some embodiments, the microcarrier with the identifier corresponding to the negative control comprises a probe comprising the sequence AATATAATATATTAT (SEQ ID NO:46).

In some embodiments, the kit comprises a primer pair, with one or both primers of the pair labeled with a detection reagent, *e.g.,* as described *supra.* In some embodiments, the detection reagent comprises a fluorescent detection reagent. In some embodiments, the detection reagent comprises biotin, and the kit comprises streptavidin conjugated to a signal-emitting entity (*e.g*., streptavidin conjugated to phycoerythrin).

In some embodiments, the kits may further include one or more detection reagents of the present disclosure for detecting an amount of the first analyte bound to the first microcarrier and an amount of the second analyte bound to the second microcarrier. In some embodiments, the detection reagent for the first analyte may be the same as the detection reagent for the second analyte. In other embodiments, the detection reagent for the first analyte may be different from the detection reagent for the second analyte.

In some embodiments, the kits may further include instructions for using the kit to detect one or more DNA mutations of the present disclosure. These instructions may be for using the kit, *e.g.,* in any of the methods described herein.

In some embodiments, the kits may further include one or more detection reagents *(e.g.,* as described above, such as streptavidin conjugated to PE), along with any instructions or reagents suitable for coupling a detection reagent to one or more analytes, or for coupling a detection reagent to one or more macromolecules that recognize an analyte. The kits may further include any additional components for using the microcarriers in an assay *(e.g.,* a multiplex assay), including without limitation a plate *(e.g.,* a 96-well or other similar microplate), dish, microscope slide, or other suitable assay container; a non-transitory computer-readable storage medium (*e.g*., containing software and/or other instructions for analog shape or code recognition); washing agents; buffers; plate sealers; mixing containers; diluents or storage solutions; and the like.

### VI. Methods of Making Encoded Microcarriers

Certain aspects of the present disclosure relate to methods for making an encoded microcarrier, *e.g*., a microcarrier described herein. The methods for making an encoded microcarrier may include one or more of the microcarrier features or aspects described herein, e.g., in section IV above.

In some embodiments, the methods include depositing a substantially transparent polymer layer, where the substantially transparent polymer layer has a first surface and a second surface, the first and the second surfaces being parallel to each other. In some embodiments, the first and the second surfaces that are parallel to each other may be the top and bottom surface of a single layer. Any suitable substantially transparent polymer known in the art or described herein may be used. In some embodiments, the substantially transparent polymer layer is deposited using spin coating.

In some embodiments, the substantially transparent polymer layer may be deposited on a substrate. Suitable substrates may include substrates used in standard semiconductor and/or micro-electro-mechanical systems (MEMS) fabrication techniques. In some embodiments, the substrate may comprise glass, silicon, quartz, plastic, polyethylene terephthalate (PET), an indium tin oxide (ITO) coating, or the like.

In some embodiments, a sacrificial layer may be deposited on the substrate, e.g., a substrate as described above. In some embodiments, the sacrificial layer may be made of a polymer, including without limitation polyvinyl alcohol (PVA) or OmniCoat^{™} (MicroChem; Newton, MA). Sacrificial layers may be applied, used, and dissolved or stripped, *e.g*., according to manufacturer's instructions.

In some embodiments, a substantially transparent polymer layer of the present disclosure is deposited on a sacrificial layer. To generate a planar microcarrier surface using a substantially transparent polymer layer, the substantially transparent polymer layer may be deposited onto a planar sacrificial layer. To generate a microcarrier surface with one or more columns projecting therefrom, a sacrificial layer (*e.g.,* one deposited onto a substrate) may be patterned with one or more column-shaped holes or void areas, for example by using a standard lithographic process. In some embodiments, a substantially transparent polymer layer may be deposited over the sacrificial layer and optional substrate such that the layer is deposited in the one or more column-shaped holes or void areas. In some embodiments, another substantially transparent polymer layer may then be deposited over the sacrificial layer and the one or more column-shaped holes or void areas filled with the first substantially transparent polymer layer. In other embodiments (*e.g.*, as illustrated at block 1540 of **FIG. 15B),** a second substantially transparent polymer layer is deposited or baked onto the first substantially transparent polymer layer, then patterned to generate one or more columns projecting from the first substantially transparent polymer layer.

In some embodiments, a magnetic, substantially non-transparent layer of the present disclosure is deposited on the first surface of the substantially transparent polymer layer. In some embodiments, the magnetic, substantially non-transparent layer is deposited by sputtering. The magnetic, substantially non-transparent layer may be made of, *e.g.,* any of the magnetic materials described herein. For example, in some embodiments, the magnetic, substantially non-transparent layer comprises nickel (*e.g.*, elemental nickel, or an alloy thereof).

In some embodiments, the magnetic, substantially non-transparent layer may be etched to remove a portion of the magnetic, substantially non-transparent layer that is deposited over a center portion of the substantially transparent polymer layer. The magnetic, substantially non-transparent layer may be etched by any means known in the art. For example, in some embodiments, the magnetic, substantially non-transparent layer is etched by conventional wet etching. Exemplary dimensions, shapes, and optional asymmetries for a magnetic, substantially non-transparent layer are provided *supra.* In some embodiments, the magnetic, substantially non-transparent layer is patterned into a ring shape of the present disclosure (*e.g*., one or more rings, with at least one having one or more discontinuities, enclosing the center portion of the substantially transparent polymer layer, as shown in **FIG. 4B**). In some embodiments, the magnetic, substantially non-transparent layer is patterned into a gear shape of the present disclosure. In some embodiments, the substantially non-transparent polymer layer is deposited over the second substantially transparent polymer layer and etched (*e.g.*, using a standard lithographic or photolithographic process) into the desired two-dimensional shape.

In some embodiments, a second substantially transparent polymer layer of the present disclosure is deposited over the magnetic, substantially non-transparent layer. In some embodiments, the second substantially transparent polymer layer has a first surface and a second surface that are parallel to each other (*e.g.,* the top and bottom surface of a single layer). In some embodiments, the second surface is affixed to the magnetic, substantially non-transparent layer. In some embodiments, the second substantially transparent polymer layer is aligned with the first substantially transparent polymer layer and has a center portion that is aligned with the center portion of the substantially transparent polymer layer. Exemplary dimensions for the center portion of a substantially transparent polymer layer are provided *supra.*

In some embodiments, a substantially non-transparent polymer layer of the present disclosure is deposited on the first surface of the second substantially transparent polymer layer. In some embodiments, the substantially non-transparent polymer layer encloses the center portions of the first and the second substantially transparent polymer layers. In some embodiments, the substantially non-transparent polymer layer comprises a two-dimensional shape representing an analog code. Any of the two-dimensional shapes described or exemplified herein may be used, *e.g.,* a gear shape of the present disclosure. In some embodiments, the substantially non-transparent polymer layer is deposited over the second substantially transparent polymer layer and etched (*e.g*., using a standard lithographic process) into the desired two-dimensional shape.

In some embodiments, one or more columns may be deposited on the substantially transparent polymer, *e.g.,* on the first surface of the second substantially transparent polymer layer at a portion not covered by the substantially non-transparent polymer layer. The one or more columns may be deposited as described herein, *e.g*., using a standard lithographic process.

In some embodiments that employ an optional sacrificial layer and/or substrate of the present disclosure, the sacrificial layer may be dissolved or stripped, and/or the substrate may be removed, using a solvent. A variety of solvents useful for fabrication (*e.g*., in standard semiconductor or MEMS fabrication processes, such as photoresist removal) are known in the art. In some embodiments, the solvent is a photoresist stripper solvent, such as a DMSO- or 1-methyl-2-pyrrolidon (NMP)-based solvent. In some embodiments, the solvent is an AZ^{®} photoresist stripper, such as AZ^{®} 300T (AZ Electronic Materials; Somerville, NJ).

In some embodiments, the methods include depositing a sacrificial layer of the present disclosure on a substrate of the present disclosure. Sacrificial layers, substrates, and suitable deposition methods are described, *e.g*., as above.

In some embodiments, a substantially non-transparent polymer layer of the present disclosure is deposited on the sacrificial layer. In some embodiments, the substantially non-transparent polymer layer has a first and a second surface that are parallel to each other *(e.g.,* the top and bottom surface of a single layer). In some embodiments, the second surface is affixed to the sacrificial layer.

In some embodiments, the outline of the substantially non-transparent polymer layer is shaped into a two-dimensional shape representing an analog code, *e.g.,* as described herein. The substantially non-transparent polymer layer may be shaped by any method known in the art or described herein, *e.g*., using a standard lithographic process including but not limited to spin coating, soft baking, UV exposure, etching, and hard baking.

In some embodiments, the sacrificial layer may be dissolved or stripped, and/or the substrate may be removed, using a solvent, *e.g.,* as described above.

In other embodiments, a magnetic layer comprising a magnetic material of the present disclosure is deposited on the sacrificial layer. Exemplary magnetic materials, magnetic layer shapes/dimensions, and deposition methods related thereto are provided *supra.* For example, in some embodiments, the magnetic layer may be shaped into one or more columns, *e.g.*, as illustrated by column 906. In other embodiments, the magnetic layer may be between two non-transparent polymer layers, *e.g*., embedded as illustrated by magnetic layer 704. The magnetic material may contain, *e.g*., any of the magnetic materials described herein. For example, in some embodiments, the magnetic material comprises nickel (*e.g*., elemental nickel, or an alloy thereof).

In some embodiments, a substantially non-transparent polymer layer of the present disclosure is deposited on the magnetic layer. In some embodiments, the substantially non-transparent polymer layer has a first and a second surface that are parallel to each other *(e.g.,* the top and bottom surface of a single layer). In some embodiments, a surface *(e.g.,* the second surface) of the substantially non-transparent polymer layer is affixed to the magnetic layer.

In some embodiments, the outline of the substantially non-transparent polymer layer is shaped into a two-dimensional shape representing an analog code, *e.g.,* as described above.

In some embodiments, the sacrificial layer may be dissolved or stripped, and/or the substrate may be removed, using a solvent, *e.g.,* as described above.

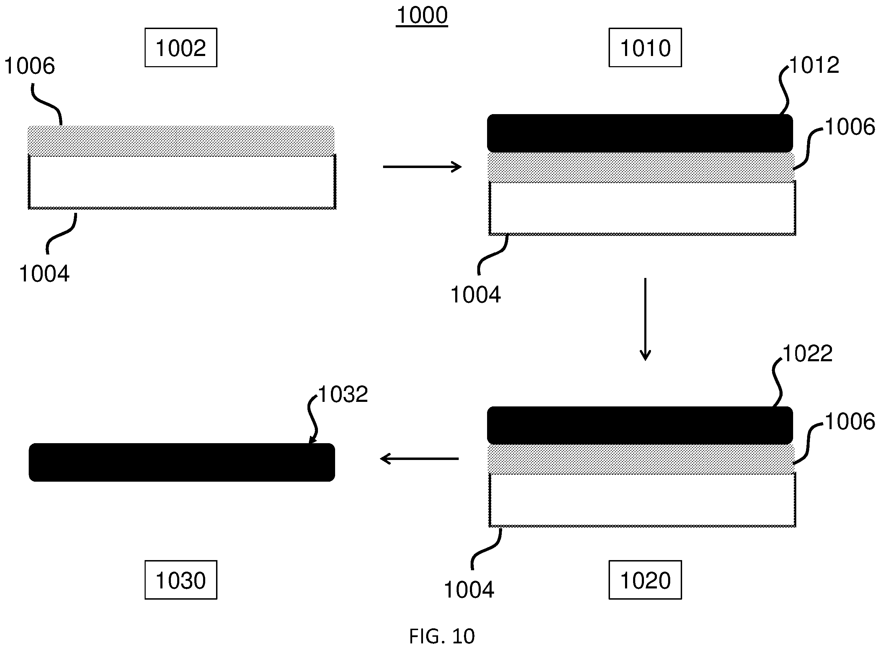

Exemplary microcarrier shapes, dimensions, and optional features suitable for the methods described above are provided throughout the present disclosure. Exemplary processes 1000, 1100, 1200, and 1300 for making a variety of the microcarriers of the present disclosure are described below in reference to **FIGS. 10-13C****.**

Process 1000 shown in **FIG. 10** illustrates an exemplary workflow for manufacturing a single layer microcarrier, such as those described above. At block 1002, sacrificial layer 1006 is constructed on substrate 1004. In some embodiments, substrate 1004 may be a glass substrate. At block 1010, layer 1012 is deposited on sacrificial layer 1006. In some embodiments, layer 1012 is a non-transparent polymer layer. At block 1020, the perimeter of layer 1012 is shaped into a gear shape (as described above) using lithography to generate gear-shaped layer 1022. At block 1030, the entire structure (*i.e.,* layer 1022, sacrificial layer 1006, and substrate 1004) is immersed in a solvent. This solvent treatment dissolves sacrificial layer 1006 and releases gear-shaped layer 1022 from substrate 1004, thereby generating microcarrier 1032. In some embodiments, microcarrier 1032 may be further modified, for example, by coupling a capture agent to one or both surfaces.

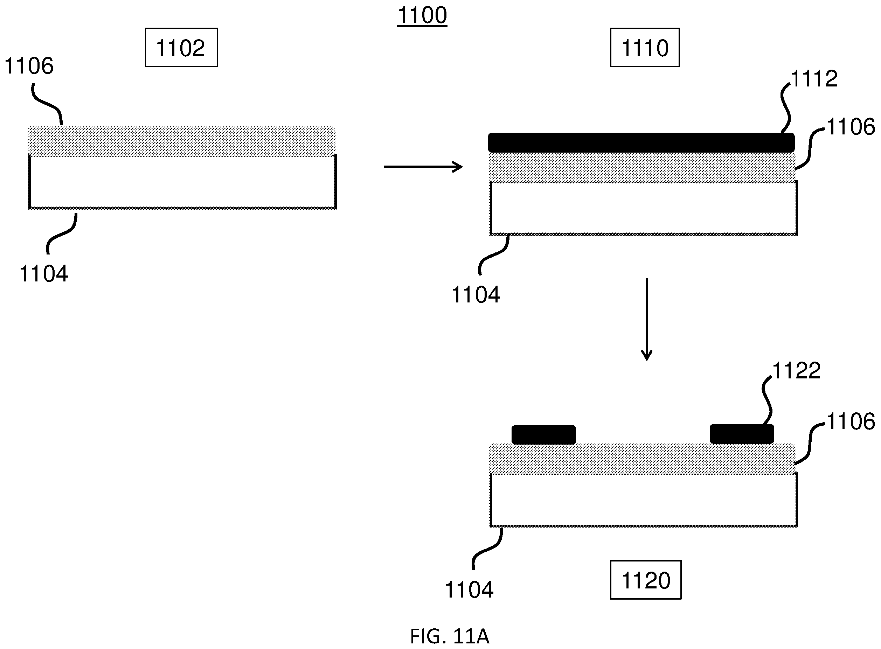

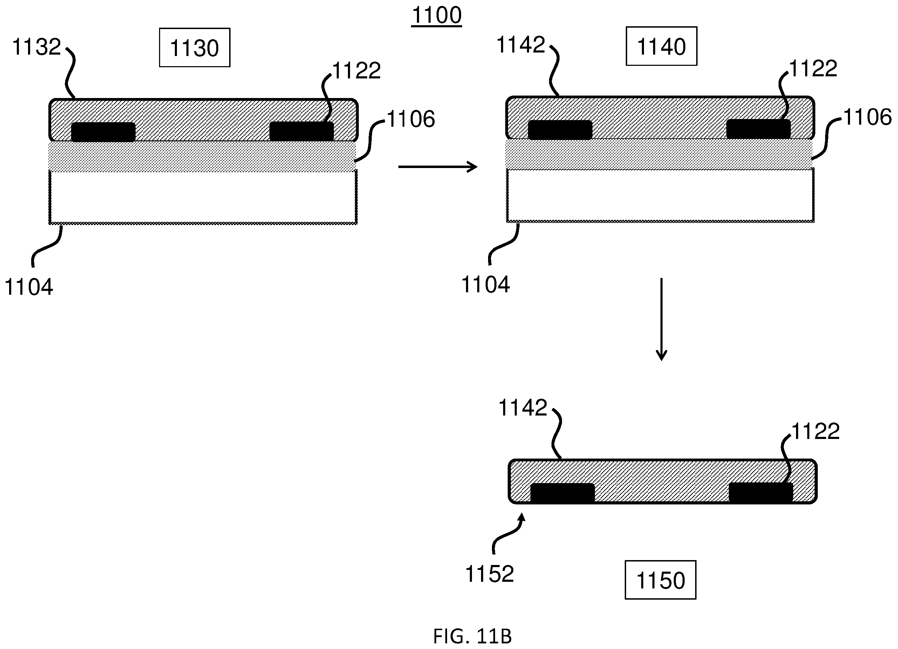

As described above, gear-shaped microcarriers may include optional elements such as magnetic components (*e.g.*, columns and/or magnetic layers). Process 1100 shown in **FIGS. 11A** **&** **11B** illustrates an exemplary workflow for manufacturing gear-shaped microcarriers with one or more magnetic components.

As shown in **FIG. 11A****,** at block 1102, sacrificial layer 1106 is constructed on substrate 1104. In some embodiments, substrate 1104 may be a glass substrate. At block 1110, magnetic layer 1112 is deposited on sacrificial layer 1106. In some embodiments, magnetic layer 1112 includes nickel. At block 1120, magnetic layer 1112 is shaped by lithography into shaped magnetic layer 1122. Shaped magnetic layer 1122 may take any desired shape, *e.g.,* it may be shaped into one or more columns, as illustrated in **FIG. 9A** with column 906.

As shown in **FIG. 11B****,** at block 1130, substantially non-transparent polymer layer 1132 is deposited over shaped magnetic layer 1122 and sacrificial layer 1106. At block 1140, the perimeter of layer 1132 is shaped by lithography into gear-shaped substantially non-transparent layer 1142 (such as one of the gear shapes illustrated in **FIGS. 6A-9A****).** At block 1150, the entire structure (*i.e.,* layer 1142, shaped magnetic layer 1122, sacrificial layer 1106, and substrate 1104) is immersed in a solvent. This solvent treatment dissolves sacrificial layer 1106 and releases gear-shaped layer 1142 and shaped magnetic layer 1122 from substrate 1104, thereby generating microcarrier 1152. In some embodiments, microcarrier 1152 may be further modified, for example, by coupling a capture agent to one or both surfaces.

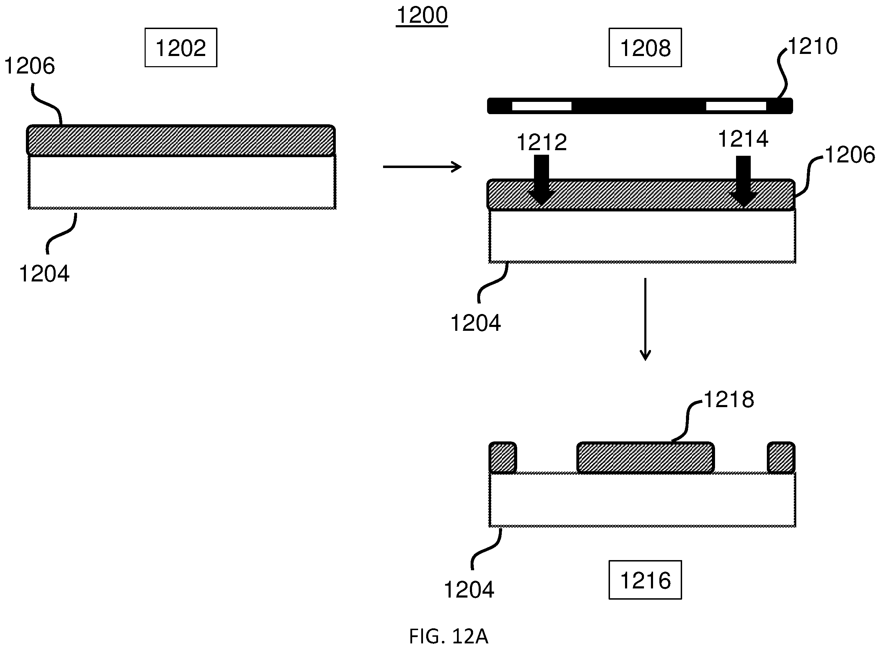

**FIGS. 12A-12E** illustrate process 1200, an exemplary workflow for manufacturing microcarriers with a substantially transparent polymer layer, a substantially non-transparent polymer layer (whose two-dimensional shape constitutes an analog code), and one or more columns.

Beginning with **FIG. 12A****,** at block 1202, sacrificial layer 1206 is deposited (*e.g*., by spin-coating) onto substrate 1204. In some embodiments, substrate 1204 may be a glass substrate. At block 1208, mask 1210 is applied, and sacrificial layer 1206 is exposed with UV light. UV light is applied through mask 1210, allowing UV light segments 1212 and 1214 to pass through and treat sacrificial layer 1206. At block 1216, after development of the structure through standard lithographic development, sacrificial layer 1206 is shaped into shaped sacrificial layer 1218 as a result of the masking of the UV treatment.

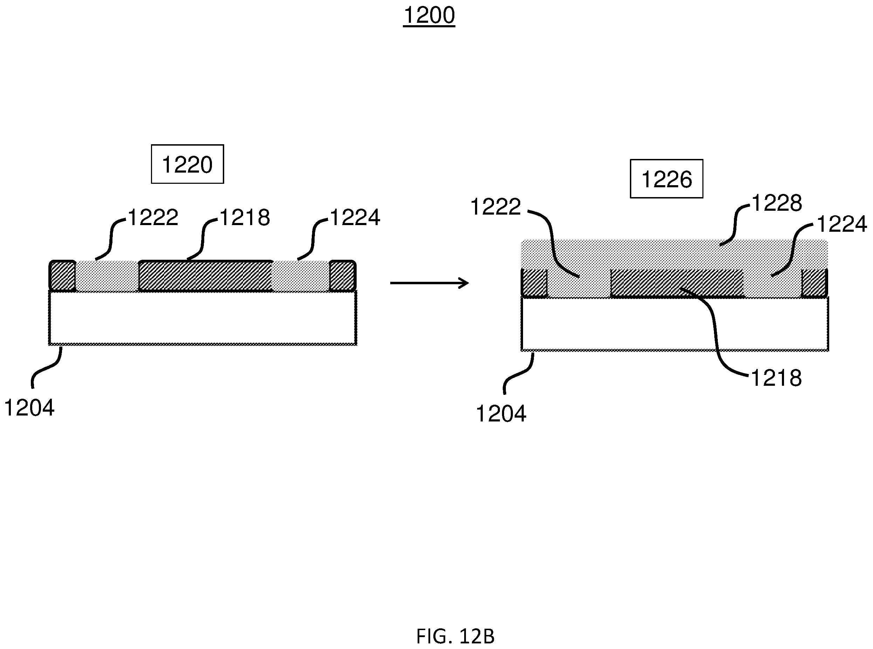

Process 1200 continues at block 1220 **(****FIG. 12B****),** where the masked holes in shaped sacrificial layer 1218 are filled with a substantially transparent polymer, creating columns 1222 and 1224. At block 1226, substantially transparent polymer layer 1228 is deposited over columns 1222 and 1224, as well as shaped sacrificial layer 1218.

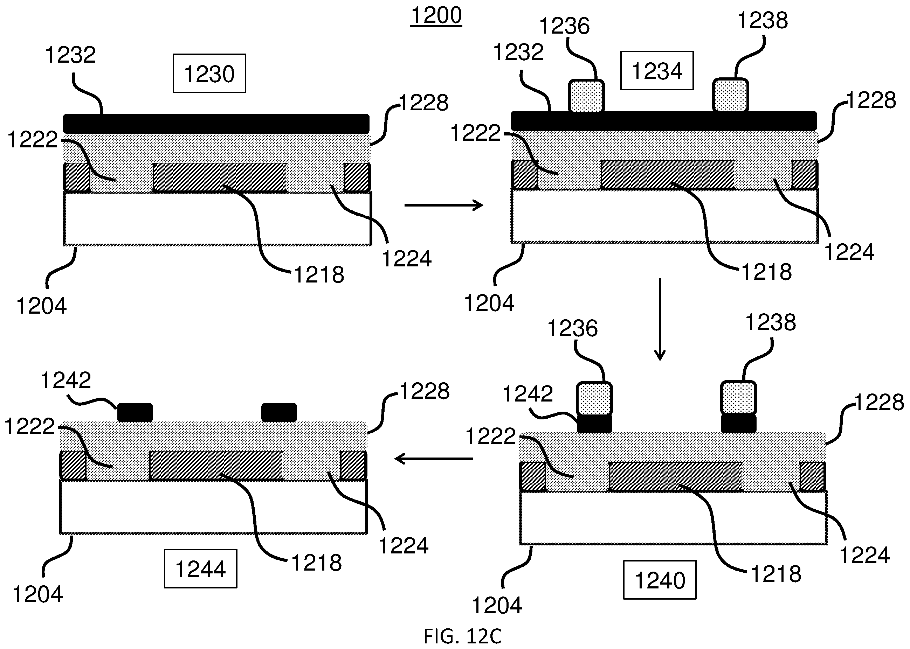

Process 1200 continues at block 1230 **(****FIG. 12C****),** where magnetic layer 1232 is deposited over layer 1228. In some embodiments, magnetic layer 1232 includes nickel. In some embodiments, magnetic layer 1232 is deposited by sputtering. At block 1234, an etch-block layer is deposited over magnetic layer 1232, as represented by etch-blocks 1236 and 1238. At block 1240, the unblocked segments of magnetic layer 1232 are etched out, generated shaped magnetic layer 1242. In some embodiments, shaped magnetic layer 1242 may be shaped into a ring shape (with optional asymmetry for indication of orientation) surrounding a center portion of layer 1228 (see, *e.g.,* layer 206 in **FIG. 2A****).** At block 1244, the etch-block layer (as represented by etch-blocks 1236 and 1238) is removed.

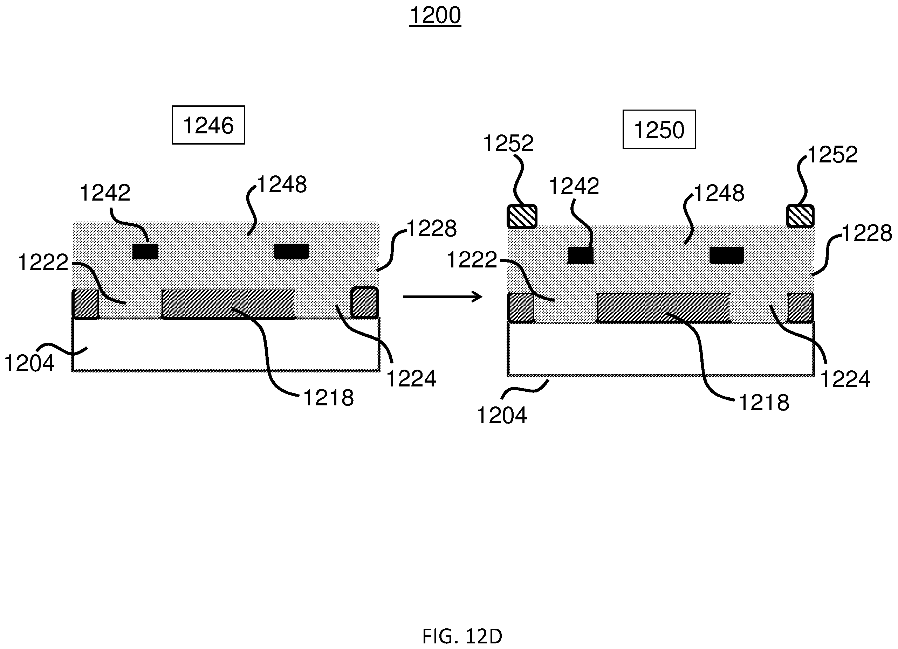

Process 1200 continues at block 1246 **(****FIG. 12D****),** where substantially transparent polymer layer 1248 is deposited over layers 1228 and 1242 (filling in any holes in layer 1242 created by etch-blocking). At block 1250, substantially non-transparent layer 1252 is deposited and shaped by lithography on top of layer 1248. In some embodiments, layer 1252 is shaped with one or more gear teeth in a ring surrounding magnetic layer 1242 (see, *e.g.,* layer 204 in relation to layers 202 and 206 and center portion 208 of **FIG. 2A****).**

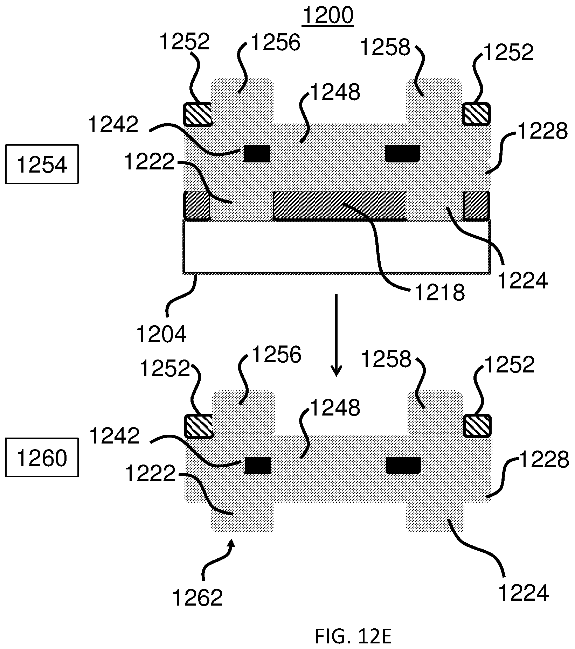

Process 1200 continues at block 1254 **(****FIG. 12E****),** where columns 1256 and 1258 are shaped by lithography on top of layer 1248. In some embodiments, columns 1256 and 1258 are made of a substantially transparent polymer. In some embodiments, the columns are positioned as shown in **FIGS. 5A & 5B****.** At block 1260, substrate 1204 is cut into one or more microcarriers of the same shape (*i.e*., although for simplicity of explanation only one microcarrier is depicted in **FIGS. 12A-12E****,** more than 1 microcarrier may be constructed on substrate 1204 in process 1200). Also at block 1260, the entire structure (*i.e.,* including 1204, 1218, 1222, 1224, 1228, 1242, 1248, 1252, 1256, and 1258) is immersed in a solvent. This solvent treatment dissolves sacrificial layer 1218 and releases microcarrier 1262 from substrate 1204. In some embodiments, microcarrier 1262 may be further modified, for example, by coupling a capture agent to one or both surfaces.

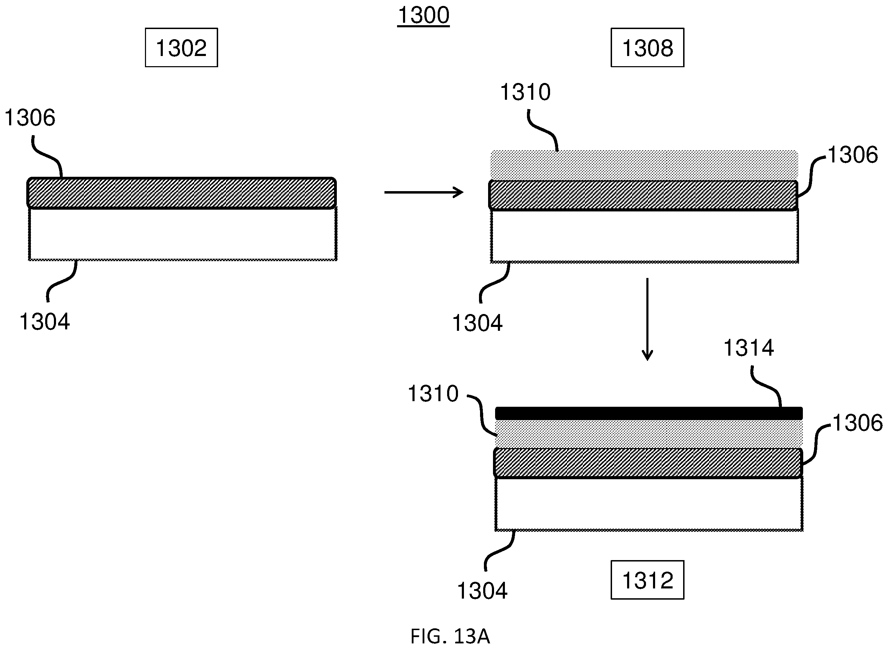

**FIGS. 13A-13C** illustrate process 1300, an exemplary workflow for generating a different type of multi-layer microcarrier. Beginning with **FIG. 13A****,** at block 1302, sacrificial layer 1306 is deposited on substrate layer 1304. In some embodiments, substrate 1304 is a glass substrate. At block 1308, substantially transparent layer 1310 is deposited over sacrificial layer 1306. At block 1312, magnetic layer 1314 is deposited over layer 1310. In some embodiments, magnetic layer 1314 includes nickel.

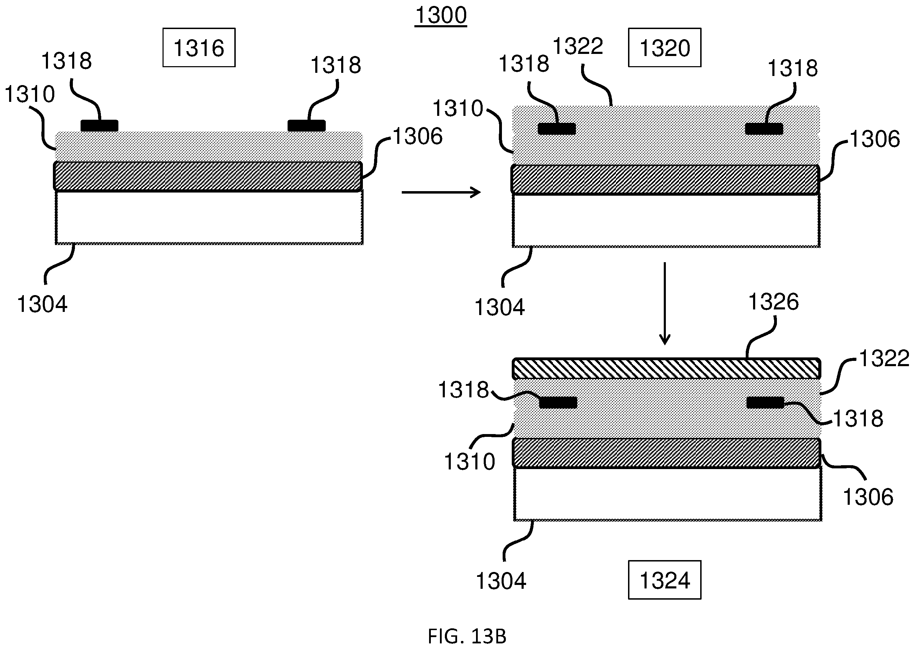

Process 1300 continues at block 1316 **(****FIG. 13B****),** where magnetic layer 1314 is defined into shaped magnetic layer 1318. In some embodiments, shaped magnetic layer 1318 is defined into a ring shape (with optional asymmetry for indication of orientation) surrounding a center portion of layer 1310 (see, *e.g.,* layer 206 in **FIG. 2A****).** At block 1320, substantially transparent layer 1322 is deposited over layers 1318 and 1310, filling in any holes created by defining shaped layer 1318. At block 1324, substantially non-transparent polymer layer 1326 is deposited over layer 1322.

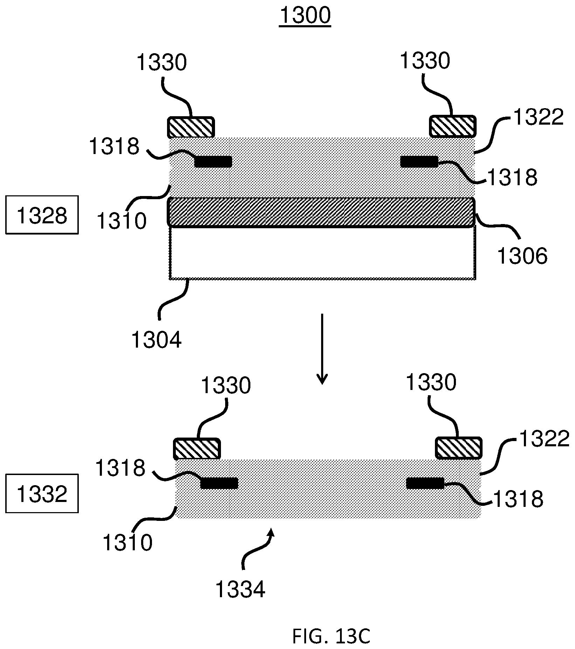

Process 1300 continues at block 1328 **(****FIG. 13C****),** where substantially non-transparent polymer layer 1326 is shaped by lithography into gear-shaped substantially non-transparent polymer layer 1330. In some embodiments, layer 1330 is shaped with one or more gear teeth in a ring surrounding shaped magnetic layer 1318 (see, *e.g.,* layer 204 in relation to layers 202 and 206 and center portion 208 of **FIG. 2A****).** At block 1332, the entire structure (*i.e.,* including 1304, 1306, 1310, 1318, 1322, and 1330) is immersed in a solvent. This solvent treatment dissolves sacrificial layer 1306 and releases microcarrier 1334 from substrate 1304. In some embodiments, microcarrier 1334 may be further modified, for example, by coupling a capture agent to one or both surfaces.

As described *supra,* in some embodiments, a microcarrier of the present disclosure comprises a magnetic, substantially non-transparent polymer layer that is affixed to the first surface of the substantially transparent polymer layer and can be used, *e.g.,* to generate an analog code of the present disclosure. Exemplary methods for producing such a microcarrier are described in greater detail *infra.*

In some embodiments, the methods include depositing a substantially transparent polymer layer, wherein the substantially transparent polymer layer has a first surface and a second surface, the first and the second surfaces being parallel to each other; patterning the substantially transparent polymer layer into a microcarrier shape; depositing a magnetic, substantially non-transparent polymer layer on the first surface of the substantially transparent polymer layer; and patterning the magnetic, substantially non-transparent polymer layer to generate a two-dimensional shape representing an analog code.

In some embodiments, a substantially transparent polymer layer of the present disclosure is deposited using spin or spray coating. In some embodiments, a magnetic, substantially non-transparent polymer layer of the present disclosure is deposited using spin or spray coating. A variety of deposition techniques are described herein and known in the art. Any suitable means known in the art for depositing a polymer such as SU-8 may be used.

In some embodiments, the methods include depositing a magnetic, substantially non-transparent polymer layer; patterning the magnetic, substantially non-transparent polymer layer to generate a two-dimensional shape representing an analog code; depositing a substantially transparent polymer layer onto the patterned magnetic, substantially non-transparent polymer layer, wherein the substantially transparent polymer layer has a first surface and a second surface, the first and the second surfaces being parallel to each other; and patterning the substantially transparent polymer layer into a microcarrier shape.

A probe of the present disclosure is coupled to a microcarrier of the present disclosure, *e.g*., a microcarrier described herein and/or a microcarrier produced by any of the methods described herein. Any of the probes described herein may find use in the methods and/or microcarriers of the present disclosure. Exemplary and non-limiting descriptions of techniques for coupling a probe to a microcarrier are provided in section IV.

### EXAMPLES

### Example 1: Multiplex detection of colorectal cancer-associated mutations using encoded microcarriers

As described above, multiplex screening for cancer-associated DNA mutations represents an attractive technique for early cancer detection. In particular, multiplex screening for colorectal cancer-associated DNA mutations using DNA isolated from a stool sample represents a non-invasive method for early detection. The following Example describes the validation of a microcarrier-based approach for multiplex screening to identify a variety of important DNA mutations associated with colorectal cancer.

### Materials and Methods

### DNA sample preparation

A flowchart of the method used to test the detection of mutations using encoded microcarriers is provided in **FIG. 14****.** DNA was isolated from the plasmids described in Table C or K562 cells as described in Table B using standard techniques. DNA was quantified using a NanoDrop^{™} UV-Vis spectrophotometer (Thermo Scientific) or Qubit^{®} Fluorometer (Thermo Scientific) according to manufacturer's instructions. Concentration of extracted DNA used for all experiments was ≥ 12.5ng/µL.

The mutations detected in these experiments are provided in Table A below.

**Table A. Mutations detected.**

| ***Gene*** | ***Exon*** | ***Mutation*** | ***Amino Acid Change*** |
|---|---|---|---|
| *KRAS* | 2 | c.35G>A | G12D |
| | | c.35G>T | G12V |
| | | c.34G>A | G12S |
| | | c.38G>A | G13D |
| *BRAF* | 15 | c.1799T> A | V600E† |
| | | c.1799_1800TG>AA ( Complex ) | V600E‡ |
| *APC* | 15 | 3927-3931delAAAGA | E1309fs*4 |
| | | 4099C>T | Q1367* |
| | | 4348C>T | R1450* |
| | | 4385-4386delAG | S1465fs*3 |
| | | 4666-4667insA | T1556fs*3 |
| *CTNNB1* | 3 | 121A>G | T41A |
| | | 122C>T | T41I |
| | | 133T>C | S45P |
| | | 134C>T | S45F |

| | | | |
|---|---|---|---|
| † Referred to as V600E1 herein. ‡ Referred to as V600E2 herein. | | | |

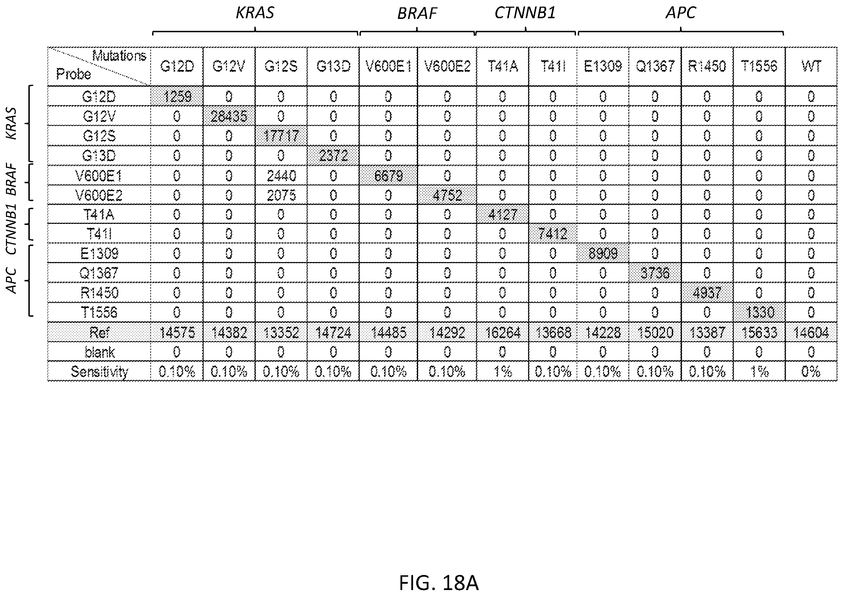

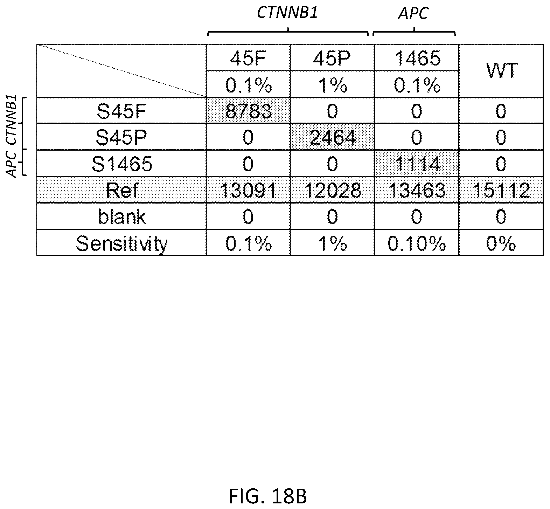

For the limit of detection (LOD) testing (results shown in **FIGS. 18A** **&** **18B****),** DNA was extracted from K562 cells for use as "wild type" DNA. DNA with one of various mutations of interest was obtained using plasmids bearing mutated *KRAS, BRAF, CTNNB1,* or *APC* sequences. Wild type and mutated DNA were mixed and diluted to achieve a total DNA concentration of 12.5ng/µL with a ratio of 1% mutated DNA to 99% wild type DNA. Concentration of each DNA sample used for the experiments is shown in Tables B and C below.

**Table B. Wild-type DNA.**

| WT | WT DNA Stock | | WT DNA 500ng/µl | | | WT DNA 100ng/µl | | |
|---|---|---|---|---|---|---|---|---|
| Cell line | mutation % | conc (ng/µl) | stock volume | final volume | Tris | 100ng/µl volume | final volume | Tris |
| K562 | - | 1208 | 207.0 | 500 | 293.0 | 100 | 1000 | 900 |

**Table C. Mutated DNA.**

| | Mutations/WT | 10% 25ng | | | 1% 25ng | | | 1% 12.5ng | | |
|---|---|---|---|---|---|---|---|---|---|---|
| Mutated Gene | Plasmid | 10000 copies stock | TE Buffer | WT DNA (100ng/µl) | 10% 25ng Stock | WT DNA (25 ng/µl) | final v (1% 25ng/µl) | 1% 25ng Stock | H2O | final v (1% 12.5ng/µl) |
| *KRAS* | G12D | 7.25 | 67.75 | 25 | 10 | 90 | 100 | 50 | 50 | 100 |
| | G12V | 7.25 | 67.75 | 25 | 10 | 90 | 100 | 50 | 50 | 100 |
| | G12S | 7.25 | 67.75 | 25 | 10 | 90 | 100 | 50 | 50 | 100 |
| | G13D | 7.25 | 67.75 | 25 | 10 | 90 | 100 | 50 | 50 | 100 |
| *BRAF* | V600E1 | 7.25 | 67.75 | 25 | 10 | 90 | 100 | 50 | 50 | 100 |
| | V600E2 | 7.25 | 67.75 | 25 | 10 | 90 | 100 | 50 | 50 | 100 |
| *CTNNB1* | T41A | 7.25 | 67.75 | 25 | 10 | 90 | 100 | 50 | 50 | 100 |
| | T41I | 7.25 | 67.75 | 25 | 10 | 90 | 100 | 50 | 50 | 100 |
| *APC* | E1309 | 7.25 | 67.75 | 25 | 10 | 90 | 100 | 50 | 50 | 100 |
| | Q1367 | 7.25 | 67.75 | 25 | 10 | 90 | 100 | 50 | 50 | 100 |
| | R1450 | 7.25 | 67.75 | 25 | 10 | 90 | 100 | 50 | 50 | 100 |
| | T1556 | 7.25 | 67.75 | 25 | 10 | 90 | 100 | 50 | 50 | 100 |
| *CTNNB1* | S45F | 7.25 | 67.75 | 25 | 10 | 90 | 100 | 50 | 50 | 100 |
| | S45P | 7.25 | 67.75 | 25 | 10 | 90 | 100 | 50 | 50 | 100 |
| *APC* | S1465 | 7.25 | 67.75 | 25 | 10 | 90 | 100 | 50 | 50 | 100 |

### PCR

Mutation-enriching PCR was used to selectively amplify polynucleotides having a mutation of interest from the DNA samples prepared above. Locked nucleic acids (LNAs) with 3' inverted dT nucleotides were used to block the amplification of wild-type DNA sequences, thereby enriching for mutations of interest. Briefly, a blocking nucleic acid was included in the PCR reaction to block amplification of the wild-type locus. The blocking nucleic acid hybridized to the sense strand of the wild-type locus and prevented primer extension to amplify from the sense strand, as shown in **FIG. 15****.** LNAs were used because of more stable hybridization, as compared to oligonucleotides with standard nucleic acids. Any copies of the mutant locus present in the sample could be amplified by PCR with no interference from the blocking nucleic acid, since it did not hybridize with the mutant sequence **(****FIG. 16****).** The following primer pairs were used: SEQ ID NOs: 1 and 2, 8 and 9, 13 and 14, 18 and 19, 23 and 24, 27 and 28, 31 and 32, 35 and 36, 39 and 40. The following blocking nucleic acids were used: SEQ ID NOs:3, 10, 15, 20, 29, 33, 37, 25, and 41.

Each sample was vortex-mixed, then spun down and kept on ice. PCR reactions were carried out as follows. For each sample, two PCR reactions were performed according to the amounts listed in Tables D and E. Each PCR reaction included 4µL of 12.5ng/µL extracted DNA, for a total of 50ng DNA. Once PCR reaction mixes were generated in PCR tubes, the tubes were mixed by tapping, spun down briefly, and placed in a thermocycler. PCR thermocycle conditions were as described in Table F below. The ramp rate was 1°C/second. PCR reaction 1 included the reagents for detecting the following mutations: *KRAS* G12D, *KRAS* G12V, *KRAS* G12S, *KRAS* G13D, *BRAF* V600E1, BRAF V600E2, *CTNNB1* T41A, *CTNNB1* T41I, *APC* E1309, *APC* Q1367, *APC* R1450, and *APC* T1556. PCR reaction 2 included the reagents for detecting the following mutations: *CTNNB1* S45F, *CTNNB1* S45P, and *APC* S1465.

**Table D. PCR reaction 1.**

| ***PCR Reaction 1*** | |
|---|---|
| Material | Vol. (µL) per reaction |
| Reaction Mix 1 | 11 |
| Primer Mix 1 | 5 |
| Extracted DNA/PC/NC | 4 |
| Total | 20 |

**Table E. PCR reaction 2.**

| ***PCR Reaction 2*** | |
|---|---|
| Material | Vol. (µL) per reaction |
| Reaction Mix 2 | 11 |
| Primer Mix 2 | 5 |
| Extracted DNA/PC/NC | 4 |
| Total | 20 |

**Table F. PCR cycling conditions.**

| ***Temp. (°C)*** | ***Time*** | ***Cycles*** |
|---|---|---|
| 95 | 10 min | 1 |
| 95 | 20 sec | 40 |
| 70 | 20 sec | |
| 60 | 60 | |
| 4 | Hold | 1 |

| | | |
|---|---|---|
| *Note: ramp rate was 1°C/sec. | | |

### Hybridization

PCR amplicons were hybridized to the capture agent probe sequences of the microcarriers. The probes were designed to hybridize with the mutant sequence and not the wild-type sequence, thereby allowing the specific detection of the mutant DNA (cf. **FIGS. 15** **&** **16****).** Combined with the use of blocking nucleic acids that bind the wild-type sequence, this strategy enables the assay to detect mutant DNA even when present at a much lower copy number than the corresponding wild-type locus. The following probes were used: SEQ ID NOs:47-61.

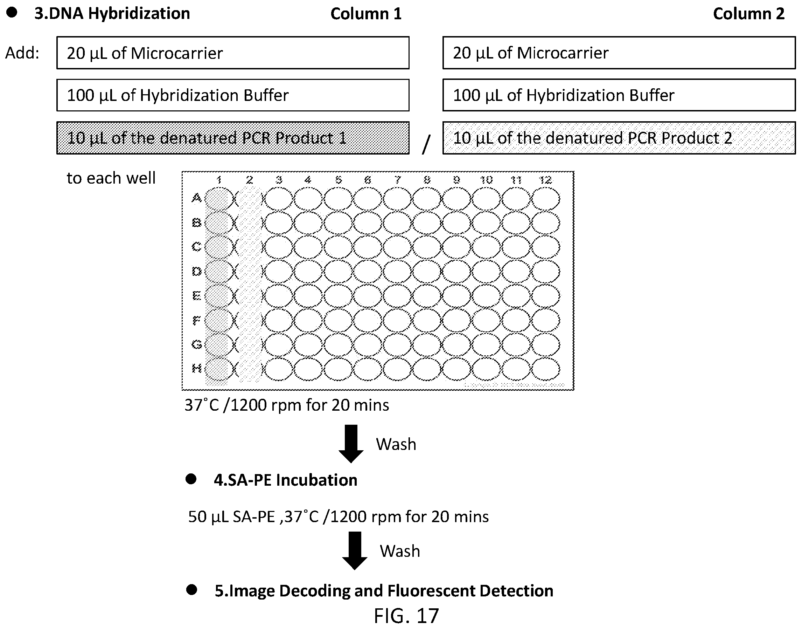

Briefly, encoded microcarriers, each individually specific for a mutation shown in Table A, were pooled into a single well of a 96-well plate. The microcarrier solution was mixed by vortexing for 10 seconds, then 20µL of microcarrier solution was added to each well of a 96-well plate, as shown in **FIG. 17****.** The stock microcarrier solution was re-vortexed every 4 wells to ensure a homogeneous suspension. 100µL hybridization buffer (5X SSPE buffer) was added to each well. PCR products from PCR reactions 1 and 2 (*see* Tables D and E) were spun down and denatured by heating to 95°C for 5 minutes, then cooled down to 4°C. 10µL of denatured PCR product 1 or 10µL of denatured PCR product 2 was added to each well. The assay plate was mixed by shaking at 1200rpm for 20 minutes at 37°C. Wells were then washed with 1X wash buffer.

For detection, 50µL of streptavidin-conjugated phycoerythrin (SA-PE) solution was added to each well, and plates were again shaken at 1200rpm for 10 minutes at 37°C. Wells were then washed again. Each well was then subject to analog image decoding and fluorescent detection.

### Results

Microcarriers with probes specific for each of the mutations listed in Table A were used to detect the presence of mutated DNA in the LOD testing assays described above. The results of these experiments are shown in **FIGS. 18A** (primer mix 1) and **18B** (primer mix 2). The microcarrier-based assay was validated for each mutation in a pairwise fashion, using one probe and one mutated DNA sequence per assay. The columns indicate which mutated DNA sequence was present in the DNA sample. The rows indicate which probe was coupled to the microcarriers for these assays. For a negative control, "blank" microcarriers with no probe were used. For a positive control, human *HLA* DNA was amplified and detected using a probe specific for the amplified sequence.

**FIGS. 18A** **&** **18B** report the fluorescence signal (in arbitrary units, AU) obtained for each experiment. As shown, in nearly all cases in which the mutated DNA sequence and the probe were mismatched, no fluorescence was detected, indicating a lack of hybridization between the probe and DNA (*KRAS* G12S DNA weakly cross-reacted with both *BRAF* probes, but yielded much lower fluorescence signal than each respective *BRAF* DNA sample). In contrast, when each mutated DNA sequence was mixed with the appropriate microcarrier-coupled probe, hybridization was detected by a strong fluorescence signal.

"Sensitivity" as shown in **FIGS. 18A** **&** **18B** refers to the LOD tested for each mutation. For example, 0.10% sensitivity indicates that 0.1ng mutated DNA was mixed with 100ng of wild-type DNA. No signal other than the positive control was detected using wild-type DNA, indicating that the LNA oligonucleotides were effective in blocking wild-type DNA amplification and/or the probe sequences were effective in eliminating hybridization of wild-type DNA.

Detection of *APC* mutation S1465 was further tested at sensitivity levels of 1.0% and 0.1% **(****FIG. 18B****).** Some cross-reactivity was observed among *CTNNB1* S45F and S45P mutations and *APC* mutation S1465. However, when all genes are tested together, the detection of signal for S1465, whether in the presence of absence of signal for S45F and S45P, can indicate the presence of S1465 mutation, given that strong S45F or S45P signal was not observed when using the S1465 probe.

These results demonstrate the sensitive and accurate detection of individual mutations of interest even with wild-type DNA present in amounts greater by orders of magnitude than the mutated sequences of interest. Advantageously, multiplex detection of several mutations in tandem leads to greater confidence and fidelity, since detection of each gene acts as an internal control for all of the other genes. These results suggest a rapid, multiplexed strategy using encoded microcarriers with oligonucleotide probes for the identification of important cancer-associated mutations from human samples.

### Example 2: Multiplex detection of colorectal cancer-associated mutations from stool samples using encoded microcarriers

The preceding Example demonstrated the use of encoded microcarriers with oligonucleotide probes for multiplex detection of cancer-associated mutations based on isolated DNA samples. The following Example demonstrates that the efficacy of this approach using DNA extracted from stool samples.

### Materials and Methods

For extraction of DNA from stool specimen, 200mg of each specimen was placed in a tube on ice, and DNA was extracted using the QIAamp^{®} Fast DNA Stool Mini Kit. Briefly, 2mL InhibitEx^{®} buffer (Qiagen) was added to each sample, then samples were vortexed for 1 minutes or until homogenization. 2mL of homogenate was pipetted into a microcentrifuge tube and centrifuged at full speed of a microcentrifuge for 1 minute. 600µL supernatant was added into a new microcentrifuge tube with 25µL Proteinase K, then 600µL buffer AL was added and tube was vortexed for 15 seconds. The tubes were then incubated for 10 minutes at 70°C. 600µL ethanol was added to lysate, then mixed by vortexing. 600µL lysate was added to a spin column and centrifuged at full speed for 1 minute. Next, 500µL buffer AW1 was added, and tubes were centrifuged at full speed for 1 minute. 500µL buffer AW2 was then added, and tubes were centrifuged at full speed for 1 minute. Spin column was then inserted into a new microcentrifuge tube, 200µL TE buffer was added to elute DNA following 1 minute incubation at room temperature and centrifugation at full speed for 1 minute.

PCR reactions were generated using the DNA isolated above according to the following proportions. Primer mixes were generated as described in Example 1.

**Table G. PCR reaction 1.**

| ***PCR Reaction 1*** | |
|---|---|
| Material | Vol. (µL) per reaction |
| Reaction Mix 1 | 10 |
| Primer Mix 1 | 10 |
| Extracted DNA/PC/NC | 20 |
| Total | 40 |

**Table H. PCR reaction 2.**

| ***PCR Reaction 2*** | |
|---|---|
| Material | Vol. (µL) per reaction |
| Reaction Mix 2 | 10 |
| Primer Mix 2 | 10 |
| Extracted DNA/PC/NC | 20 |
| Total | 40 |

Other PCR conditions (*e.g*., thermocycling), hybridization conditions, and detection were as described in Example 1. The following primer pairs were used: SEQ ID NOs: 1 and 2, 8 and 9, 13 and 14, 18 and 19, 23 and 24, 27 and 28, 31 and 32, 35 and 36, 39 and 40. The following blocking nucleic acids were used: SEQ ID NOs:3, 10, 15, 20, 29, 33, 37, 25, and 41. The following probes were used: SEQ ID NOs:47, 48, 86, 91, 95, 99, 104, 109, 113, 119, 122, 126, 60, 57, and 61.

### Results

Stool specimens from 7 patients were collected before colon cancer surgery. Five individual stool samples (2g each) representing different regions of the specimen were taken from each patient's specimen (except 4 were taken from patient #5). Each individual stool sample was homogenized separately in 7mL stabilization buffer (100mM EDTA, pH 8.0). DNA was extracted as described above. From each sample, 10ng DNA was used for the assay. Mutation status was detected from each sample, as shown in Table I. Note that no mutations were detected from stools of about 20 healthy patients.

**Table I. Results of mutation screening from stool samples.**

| Sample No. | Results of Detection |
|---|---|
| 01-001 | Wild Type |
| 01-002 | Wild Type |
| 01-003 | Wild Type |
| 01-004 | Wild Type |
| 01-005 | Wild Type |
| 02-001 | CTNNB1 T41A |
| 02-002 | Wild Type |
| 02-003 | Wild Type |
| 02-004 | Wild Type |
| 02-005 | Wild Type |
| 03-001 | KRAS G12V |
| 03-002 | KRAS G12V |
| 03-003 | KRAS G12V |
| 03-004 | Wild Type |
| 03-005 | BRAF V600E1 & APC E1309 |
| 04-001 | Wild Type |
| 04-002 | KRAS G13D |
| 04-003 | KRAS G13D |
| 04-004 | KRAS G13D |
| 04-005 | KRAS G13D |
| 05-001 | BRAF V600E2 & APC R1450 |
| 05-003 | APC R1450 |
| 05-004 | APC R1450 |
| 05-005 | BRAF V600E2 & APC E1309 |
| 06-001 | BRAF V600E2 & APC E1309 |
| 06-002 | BRAF V600E2 |
| 06-003 | BRAF V600E2 |
| 06-004 | Wild Type |
| 06-005 | Wild Type |
| 07-001 | Wild Type |
| 07-002 | BRAF V600E2 & APC E1309 |
| 07-003 | Wild Type |
| 07-004 | Wild Type |
| 07-005 | APC E1309 |

These results demonstrate the successful, multiplex detection of multiple cancer-associated mutations from patient stool samples using the methods described herein. These results further confirm the known heterogeneity of stool specimens, demonstrating that different samples obtained from a single specimen can yield different DNA. Also, as compared with Example 1, the PCR conditions used in Example 2 can be more readily adapted to samples with low DNA concentration. In Example 1, each PCR reaction included 4µL of 12.5ng/µL extracted DNA, for a total of 50ng DNA. In Example 2, each PCR reaction can include 20µL of 2.5ng/µL extracted DNA to achieve a total of 50ng DNA (or 0.5 ng/µL DNA Stock for a total of 10 ng DNA), thereby lessening the starting concentration.

## Claims

1. A method for detecting the presence of DNA mutations in the *KRAS, BRAF, CTNNB1, and APC* genes, the method comprising:
(a) isolating DNA from a sample;
(b) amplifying the isolated DNA by polymerase chain reaction (PCR) using primer pairs specific for the loci of one or more DNA mutations in each of the *KRAS, BRAF, CTNNB1,* and *APC* genes, wherein the isolated DNA is amplified in the presence of at least four blocking nucleic acids, wherein each of said at least four blocking nucleic acids hybridizes with a wild-type DNA locus corresponding with one of the DNA mutations in the KRAS, BRAF, CTNNB1, or APC genes and prevents amplification of the wild-type DNA locus, wherein optionally the *KRAS, BRAF, CTNNB1, and APC* genes are human genes;
(c) hybridizing the amplified DNA with at least four probes, said at least four probes comprising one or more probes specific for a DNA mutation in each of the *KRAS, BRAF, CTNNB1,* and *APC* genes, wherein each of said at least four probes is coupled to a microcarrier, and wherein each of the microcarriers comprises an identifier corresponding to the probe coupled thereto;
(d) detecting presence or absence of hybridization of the amplified DNA with said at least four probes, wherein hybridization between the amplified DNA and one of the probes indicates the presence of the DNA mutation corresponding to the probe;
(e) detecting the identifiers of the microcarriers; and
(f) correlating the detected identifiers of the microcarriers with the detected presence or absence of hybridization of the amplified DNA to the corresponding probes of the microcarriers.

2. The method of claim 1, wherein each of said at least four blocking nucleic acids comprises a single-stranded oligonucleotide that hybridizes with the corresponding wild-type DNA locus, and a 3' terminal moiety that blocks extension from the single-stranded oligonucleotide;
wherein optionally the 3' terminal moiety comprises one or more inverted deoxythymidines; and wherein optionally each of said at least four blocking nucleic acids comprises one or more modified nucleotides selected from the group consisting of locked nucleic acids (LNAs), peptide nucleic acids (PNAs), hexose nucleic acids (HNAs), threose nucleic acids (TNAs), glycol nucleic acids (GNAs), and cyclohexenyl nucleic acids (CeNAs).

3. The method of claim 1 or claim 2, wherein the one or more DNA mutations in the *KRAS* gene comprise *KRAS* mutations encoding G12D, G12V, G12S, and G13D mutated KRAS proteins;
wherein optionally the probes specific for one or more DNA mutations in the *KRAS* gene comprise four probes comprising the sequences GGAGCTGATGG (SEQ ID NO:4), GGAGCTGTTGG (SEQ ID NO:5), TGGAGCTAGTGG (SEQ ID NO:6), and TGGAGCTGGTGACGT (SEQ ID NO:7), and wherein each of the four probes is coupled to a microcarrier with a different identifier;
wherein optionally each of the four probes further comprises eight nucleotides at the 5' end,
wherein the eight nucleotides at the 5' end are adenine or thymine nucleotides, and wherein each of the four probes comprises at least 24 total nucleotides;
wherein optionally the probes comprise: (1) a first probe comprising a sequence selected from the group consisting of GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO:180), and GCTGATGGCGTA (SEQ ID NO:181); (2) a second probe comprising a sequence selected from the group consisting of GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO: 184), and TGGAGCTGTAGGTGG (SEQ ID NO: 185); (3) a third probe comprising a sequence selected from the group consisting of TTGGAGCTAGTGGCGTA (SEQ ID NO:186), GCTAGTGGCGTAGGC (SEQ ID NO:187), AGCTAGTGGCGT (SEQ ID NO:188), GTTGGAGCTAGTGG (SEQ ID NO:189), and GGAGCTAGTGG (SEQ ID NO:190); and (4) a fourth probe comprising a sequence selected from the group consisting of GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), and GGAGCTGGTGACGT (SEQ ID NO: 195); and wherein each of the four probes is coupled to a microcarrier with a different identifier;
wherein optionally the probes comprise: (1) a first probe comprising a sequence selected from the group consisting of TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), and TTTTTTTTTTTTTGCTGATGGCGTA (SEQ ID NO:77); (2) a second probe comprising a sequence selected from the group consisting of TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), and TTTTTTTTTATGGAGCTGTAGGTGG (SEQ ID NO:81); (3) a third probe comprising a sequence selected from the group consisting of TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), and TTTTTTTTTTTTAAGGAGCTAGTGG (SEQ ID NO:86); and (4) a fourth probe comprising a sequence selected from the group consisting of TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), and TTTTTTTTTAAGGAGCTGGTGACGT (SEQ ID NO:91); and wherein each of the four probes is coupled to a microcarrier with a different identifier;
wherein optionally step (b) comprises amplifying the isolated DNA by PCR using a primer pair comprising the sequences GTACTGGTGGAGTATTTGATAGTG (SEQ ID NO:1) and ATCGTCAAGGCACTCTTGCCTAC (SEQ ID NO:2); and
wherein optionally step (b) comprises amplifying the isolated DNA by PCR in the presence of a blocking nucleic acid comprising the sequence of TA*C*G*CC*A*CC*A*G*CT(invdT)*ₙ*,
wherein *n is* 1, 2, or 3 (SEQ ID NO:3), TT*GG*A*G*CT*GGTGGC*GTA(invdT)*ₙ*, wherein *n is* 1, 2, or 3 (SEQ ID NO: 142), GCT*GG*T*GG*C*G*TA*G*G*C*A(invdT)*ₙ*, wherein *n* is 1, 2, or 3 (SEQ ID NO: 143), *GCTGGTGGCGTAG*GC(invdT)*ₙ,* wherein *n is* 1, 2, or 3 (SEQ ID NO: 144), or TT*GG*A*G*CT*GG*T*GG*C*G*T(invdT)*ₙ*, wherein *n* is 1, 2, or 3 (SEQ ID NO: 145), with italicized nucleic acids representing locked nucleic acids.

4. The method of any one of claims 1-3, wherein the one or more DNA mutations in the *BRAF* gene comprise two or more *BRAF* mutations encoding a V600E mutated BRAF protein;
wherein optionally the probes specific for one or more DNA mutations in the *BRAF* gene comprise two probes comprising the sequences TCTAGCTACAGAGAAAT (SEQ ID NO:11) and GTCTAGCTACAGAAAAAT (SEQ ID NO:12), and wherein each of the two probes is coupled to a microcarrier with a different identifier;
wherein optionally each of the two probes further comprises eight nucleotides at the 5' end, wherein the eight nucleotides at the 5' end are adenine or thymine nucleotides, and wherein each of the two probes comprises at least 24 total nucleotides;
wherein optionally the probes comprise: (1) a first probe comprising a sequence selected from the group consisting of TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), and TCTAGCTACAGAG (SEQ ID NO:200); and (2) a second probe comprising a sequence selected from the group consisting of GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204); and wherein each of the two probes is coupled to a microcarrier with a different identifier;
wherein optionally the probes comprise (1) a first probe comprising a sequence selected from the group consisting of TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), and TTTTTTTTTTAATTTCTAGCTACAGAG (SEQ ID NO:96); and (2) a second probe comprising a sequence selected from the group consisting of TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and TTTTTTTTATTCTAGCTACAGAAAAATC (SEQ ID NO:100); and wherein each of the two probes is coupled to a microcarrier with a different identifier;
wherein optionally step (b) comprises amplifying the isolated DNA by PCR using a primer pair comprising the sequences GGACCCACTCCATCGAGATTT (SEQ ID NO:8) and CAGATATATTTCTTCATGAAGACCTCACAGTAA (SEQ ID NO:9); and
wherein optionally step (b) comprises amplifying the isolated DNA by PCR in the presence of a blocking nucleic acid comprising the sequence of G*AGAT*T*TCAC*T*GTAGC*(invdT)*ₙ,* wherein *n is* 1, 2, or 3 (SEQ ID NO: 10), G*AGAT*T*TCAC*T*GTAGC*(invdT)*ₙ,* wherein *n is* 1, 2, or 3 (SEQ ID NO: 146), G*AGAT*T*TCAC*T*GTAGC*(invdT)*ₙ,* wherein *n is* 1, 2, or 3 (SEQ ID NO: 147), *GAGAT*T*TCACT*G*TAGC*(invdT)*ₙ,* wherein *n is* 1, 2, or 3 (SEQ ID NO: 148), or G*AGAT*T*TCAC*T*GT*A*GC*(invdT)*ₙ,* wherein *n is* 1, 2, or 3 (SEQ ID NO: 149), with italicized nucleic acids representing locked nucleic acids.

5. The method of any one of claims 1-4, wherein the one or more DNA mutations in the *CTNNB1* gene comprise *CTNNB1* mutations encoding T41A, T41I, S45F, and S45P mutated CTNNB 1 proteins;
wherein optionally the probes specific for one or more DNA mutations in the *CTNNB1* gene comprise four probes comprising the sequences AGGAGCTGTGGCAG (SEQ ID NO: 16), GGAGCTGTGATA (SEQ ID NO: 17), TTTACCACTCAGAAAAG (SEQ ID NO:21), and TACCACTCAGAGGAG (SEQ ID NO:22), and wherein each of the four probes is coupled to a microcarrier with a different identifier;
wherein optionally each of the four probes further comprises eight nucleotides at the 5' end,
wherein the eight nucleotides at the 5' end are adenine or thymine nucleotides, and wherein each of the four probes comprises at least 24 total nucleotides;
wherein optionally the probes comprise: (1) a first probe comprising a sequence selected from the group consisting of AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), and AAGGAGCTGTGGCAG (SEQ ID NO:209); (2) a second probe comprising a sequence selected from the group consisting of GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), and GGAGCTGTGAT (SEQ ID NO:214); (3) a third probe comprising a sequence selected from the group consisting of ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), and CAGAAAAGGAGCTGTG (SEQ ID NO:219); and (4) a fourth probe comprising a sequence selected from the group consisting of ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), and TTACCAATCAGAGGAGG (SEQ ID NO:224); and wherein each of the four probes is coupled to a microcarrier with a different identifier;
wherein optionally the probes comprise: (1) a first probe comprising a sequence selected from the group consisting of TTTTTTTTTTTAGGAGCTGTGGCAG (SEQ ID NO:53), TTTTTTTTTTTAGGAGCTGTGGCAGTG (SEQ ID NO:101), TTTTTTTTTTTAGCTGTGGCAGTGGC (SEQ ID NO:102), TTTTTTTTTTTGCTGTGGCAGTGGCA (SEQ ID NO:103), and TTTTTTTTTTAAGGAGCTGTGGCAG (SEQ ID NO:104); (2) a second probe comprising a sequence selected from the group consisting of TTTTTTTTTTTTTGGAGCTGTGATA (SEQ ID NO:54), TTTTTTTTTGGAGCTGTGATAGTGG (SEQ ID NO: 105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), and TTTTTTTTTTTTTTGGAGCTGTGAT (SEQ ID NO:109); (3) a third probe comprising a sequence selected from the group consisting of TTTTTTTTTTTACCACTCAGAAAAG (SEQ ID NO:55), TTTAATTTTACTCAGAAAAGGAGCT (SEQ ID NO: 110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), and TTTTTTTTTCAGAAAAGGAGCTGTG (SEQ ID NO:114); and (4) a fourth probe comprising a sequence selected from the group consisting of TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and TTTTTTTTTATTACCAATCAGAGGAGG (SEQ ID NO:119); and wherein each of the four probes is coupled to a microcarrier with a different identifier;
wherein optionally step (b) comprises amplifying the isolated DNA by PCR using a first primer pair comprising the sequences GGAATCCATTCTGGTGCCACT (SEQ ID NO: 13) and AGAAAATCCCTGTTCCCACTCATA (SEQ ID NO:14), and a second primer pair comprising the sequences GGTGCCACTACCACAGCTCCT (SEQ ID NO: 18) and TCTCAAAACTGCATTCTGACTTTCA (SEQ ID NO:19); and
wherein optionally step (b) comprises amplifying the isolated DNA by PCR in the presence of a first blocking nucleic acid comprising the sequence of GC*CACTACCACAG*CT(invdT)*ₙ,* wherein *n is* 1, 2, or 3 (SEQ ID NO: 15), T*G*C*CA*CT*ACCA*C*AG*(invdT)*ₙ,* wherein *n is* 1, 2, or 3 (SEQ ID NO:150), C*AC*T*ACCA*C*AGC*T*C*C(invdT)*ₙ,* wherein *n is* 1, 2, or 3 (SEQ ID NO:151), GC*CACTACCACAG*C*T*(invdT)*ₙ,* wherein *n is* 1, 2, or 3 (SEQ ID NO:152), or GC*CACTACCACAG*CT(invdT)*ₙ,* wherein *n* is 1, 2, or 3 (SEQ ID NO: 153), and a second blocking nucleic acid comprising the sequence of GC*TCCTTCTCTG*AGT(invdT)*ₙ,* wherein *n* is 1, 2, or 3 (SEQ ID NO:20), T*CC*T*TCTC*T*G*A*G*T*G*G(invdT)*ₙ*, wherein *n* is 1, 2, or 3 (SEQ ID NO: 174), GCTCCTTCTCTGAGT(invdT)n, wherein *n* is 1, 2, or 3 (SEQ ID NO: 175), T*CC*TT*CT*CT*GAGTG*G(invdT)n, wherein *n* is 1, 2, or 3 (SEQ ID NO: 176), or G*C*T*CC*TT*CT*C*TGAG*T(invdT)n, wherein *n* is 1, 2, or 3 (SEQ ID NO: 177), with italicized nucleic acids representing locked nucleic acids.

6. The method of any one of claims 1-5, wherein the one or more DNA mutations in the *APC* gene comprise APC mutations encoding Q1367*, R1450*, E1309 frameshift, S1465 frameshift, and T1556 frameshift mutated APC proteins;
wherein optionally the probes specific for one or more DNA mutations in the *APC* gene comprise five probes comprising the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), and AGAGGCAGAAAAAAACT (SEQ ID NO:42), and wherein each of the five probes is coupled to a microcarrier with a different identifier;
wherein optionally each of the five probes further comprises eight nucleotides at the 5' end, wherein the eight nucleotides at the 5' end are adenine or thymine nucleotides, and wherein each of the five probes comprises at least 24 total nucleotides;
wherein optionally the probes comprise: (1) a first probe comprising a sequence selected from the group consisting of AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (2) a second probe comprising a sequence selected from the group consisting of TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (3) a third probe comprising a sequence selected from the group consisting of AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (4) a fourth probe comprising a sequence selected from the group consisting of GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (5) a fifth probe comprising a sequence selected from the group consisting of GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246); and wherein each of the five probes is coupled to a microcarrier with a different identifier;
wherein optionally the probes comprise: (1) a first probe comprising a sequence selected from the group consisting of TTTTTTTTTTTTTGAAATAAAAGATTGG (SEQ ID NO:58), TTTTTTTTTTAAATAGCAGAAATAAAAG (SEQ ID NO: 120), TTTTTTTTTTTGAAATAAAAGATTGGAA (SEQ ID NO:121), TTTTTTTTTTTTTAGAAATAAAAGATTG (SEQ ID NO: 122), TTTTTTTTTTTTTGAAATAAATGAATGG (SEQ ID NO: 123), and TTTTTTTTTTTTTCAGAAATAAAAGATT (SEQ ID NO: 124); (2) a second probe comprising a sequence selected from the group consisting of TTTTTTTTTTTTTGGGTGTCTAAG (SEQ ID NO:59), TTTTTTTTTATTTGGGTGTCTAAG (SEQ ID NO:125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128),and TTTTTTTTTGGTGTCTAAGCACCA (SEQ ID NO: 129); (3) a third probe comprising a sequence selected from the group consisting of TTTTTTTTTACAAACCAAGTGAGAA (SEQ ID NO:60), TTTTTTTTAAGTGAGAAGTACCTAA (SEQ ID NO: 130), TTTTTTTTTTTTCAAACCAAGTGAG (SEQ ID NO:131), TTTTTTTTTTACCAAGTGAGAAGTA (SEQ ID NO: 132), and TTTTTTTTAGCTCAAACCAAGTGAG (SEQ ID NO:133); (4) a fourth probe comprising a sequence selected from the group consisting of TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO:136), and TTTTTTTTTCCTACTGCTGAAAAGAGA (SEQ ID NO: 137); and (5) a fifth probe comprising a sequence selected from the group consisting of TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO:139), TTTTTTTAGAAAGAGGCAGAAAAAAACT (SEQ ID NO:140), and TTTTTTTTTTTGAGGCAGAAAAAAACTA (SEQ ID NO:141); and wherein each of the five probes is coupled to a microcarrier with a different identifier;
wherein optionally step (b) comprises amplifying the isolated DNA by PCR using a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40); and
wherein optionally step (b) comprises amplifying the isolated DNA by PCR in the presence of a first blocking nucleic acid comprising the sequence of *CCA*C*TC*TCTCT*C*T*TT*TC*AGC*(invdT)*ₙ,* wherein *n is* 1, 2, or 3 (SEQ ID NO:25), TA*GG*T*CC*ACTCTCTCTCT*TT*T*C*A*GC*A(invdT)n, wherein n is 1, 2, or 3 (SEQ ID NO:166), T*AGG*T*CCAC*T*CTCT*C*T*CTT*T*TC*AG*CA (invdT)n, wherein n is 1, 2, or 3 (SEQ ID NO:167), *CCACTCTCTCTCTTTTCAGC* (invdT)n, wherein n is 1, 2, or 3 (SEQ ID NO:168), or TAGGTCCACTCTCTCTCTITTCAGCA (invdT)n, wherein n is 1, 2, or 3 (SEQ ID NO:169), a second blocking nucleic acid comprising the sequence of *CTTTTCTTTTAT*TT*C*T*GC*(invdT)*ₙ,* wherein *n is* 1, 2, or 3 (SEQ ID NO:29), *CTTTTC*T*TTTA*T*T*T*C*TG*C*(invdT)n*,* wherein n is 1, 2, or 3 (SEQ ID NO:154), C*T*TT*TC*T*T*T*TATTTCTGC*(invdT)n, wherein n is 1, 2, or 3 (SEQ ID NO:155), C*TTT*TCT*TTT*A*TT*T*C*T*GC*(in*v*dT)n*,* wherein n is 1, 2, or 3 (SEQ ID NO: 156), or C*T*TT*TCTTTTATTTC*TG*C*(invdT)n, wherein n is 1, 2, or 3 (SEQ ID NO:157), a third blocking nucleic acid comprising the sequence of GTG*CTCA*G*ACAC*C(invdT)*ₙ*, wherein n is 1, 2, or 3 (SEQ ID NO:33), GTGCTCAGACACC(invdT)n, wherein n is 1, 2, or 3 (SEQ ID NO:158), *AGTGGTGCTCAGACACCCA(invdT)n,* wherein n is 1, 2, or 3 (SEQ ID NO:159), AGTGGTGCTCAGACACCCA(invdT)n, wherein n is 1, 2, or 3 (SEQ ID NO:160), or A*G*T*G*GTG*CTCA*G*AC*A*C*C*C*A(invdT)n, wherein n is 1, 2, or 3 (SEQ ID NO:161), a fourth blocking nucleic acid comprising the sequence of C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO:162), C*TTC*T*CGCT*T*GGT*T(invdT)n, wherein n is 1, 2, or 3 (SEQ ID NO:163), GT*ACT*T*CTCG*CT*TGG*T(invdT)n, wherein n is 1, 2, or 3 (SEQ ID NO:164), or GTACTTCTCGCTTGGT(invdT)n, wherein n is 1, 2, or 3 (SEQ ID NO:165), and a fifth blocking nucleic acid comprising the sequence of *C*A*ATAG*T*TTTT*T*CTGC*C(invdT)*ₙ,*
wherein *n is* 1, 2, or 3 (SEQ ID NO:41), *GAATCAATAGTTTTTTCTGCCTC* (invdT)n,
wherein n is 1, 2, or 3 (SEQ ID NO:170), *TCAGAATCAATAGTTTTTTCTG* (invdT)n,
wherein n is 1, 2, or 3 (SEQ ID NO:171), *GAATCAATAGATTTTACTGCCTC* (invdT)n,
wherein n is 1, 2, or 3 (SEQ ID NO:172), or A*A*T*CAATAG*TTTTTTC*TGCCT*C (invdT)n,
wherein n is 1, 2, or 3 (SEQ ID NO:173), with italicized nucleic acids representing locked nucleic acids.

7. The method of any one of claims 1-6, wherein the method further comprises:
amplifying a positive control DNA sequence using a primer pair specific for the positive control DNA sequence;
hybridizing the amplified positive control gene sequence with a probe specific for the positive control gene sequence, wherein the probe specific for the positive control gene sequence is coupled to a microcarrier with an identifier corresponding to a positive control;
detecting presence or absence of hybridization of the amplified positive control DNA sequence with the probe specific for the positive control gene sequence; and
detecting the identifier corresponding to the positive control
wherein optionally the positive control DNA sequence comprises a sequence of a human leukocyte antigen gene or a human glyceraldehyde 3-phosphate dehydrogenase (GAPDH) gene;
wherein optionally the primer pair specific for the positive control DNA sequence comprises the sequences TGAGTGTTACTTCTTCCCACACTC (SEQ ID NO:43) and ATTGCTTTTGCGCAATCCCT (SEQ ID NO:44) or AATCCCATCACCATCTTCCA (SEQ ID NO:71) and TGGACTCCACGACGTACTCA (SEQ ID NO:72); and
wherein optionally the probe specific for the positive control gene sequence comprises the sequence TTTTTTTTTTTTGGAGACGGTCTG (SEQ ID NO:45) or CTGTCTTCCACTCACTCC (SEQ ID NO:73).

8. The method of any one of claims 1-7, wherein the method further comprises:
detecting absence of hybridization of the amplified DNA with a microcarrier having an identifier corresponding to a negative control, wherein the microcarrier with the identifier corresponding to the negative control comprises a probe that does not hybridize with the amplified DNA; and
detecting the identifier corresponding to the negative control;
wherein optionally the microcarrier with the identifier corresponding to the negative control comprises a probe comprising the sequence AATATAATATATTAT (SEQ ID NO:46).

9. The method of any one of claims 1-8, wherein the identifiers of the micocarriers comprise (a) digital barcodes, or (b) analog codes;
wherein optionally each of the microcarriers comprises:
(i) a first photopolymer layer; (ii) a second photopolymer layer; and (iii) an intermediate layer between the first layer and the second layer, the intermediate layer having an encoded pattern representing the identifier defined thereon, wherein the intermediate layer is partially substantially transmissive and partially substantially opaque to light, representing a code corresponding to the microcarrier, wherein the outermost surface of the microcarrier comprises a photoresist photopolymer, and said photoresist photopolymer is functionalized with the probe specific for the DNA mutation, and wherein said microcarrier has about the same density as water; or
(i) a substantially transparent polymer layer having a first surface and a second surface, the first and the second surfaces being parallel to each other; (ii) a substantially non-transparent polymer layer, wherein the substantially non-transparent polymer layer is affixed to the first surface of the substantially transparent polymer layer and encloses a center portion of the substantially transparent polymer layer, and wherein the substantially non-transparent polymer layer comprises a two-dimensional shape representing an analog code, wherein the analog code represents the identifier; and (iii) the probe specific for the DNA mutation, wherein the probe is coupled to at least one of the first surface and the second surface of the substantially transparent polymer layer in at least the center portion of the substantially transparent polymer layer, wherein optionally each of the microcarriers further comprises an orientation indicator for orienting the analog code of the substantially non-transparent polymer layer, wherein optionally the substantially transparent polymer of the first or the second substantially transparent polymer layer comprises an epoxy-based polymer, and wherein optionally the epoxy-based polymer is SU-8.

10. The method of any one of claims 1-9, wherein the sample is a stool sample.

11. The method of any one of claims 1-10, wherein the method is used for detecting colon cancer, rectal cancer, colorectal cancer, colon adenoma, rectal adenoma, or colorectal adenoma.

12. A kit, comprising:
(a) at least four microcarriers, wherein each of said at least four microcarriers comprises:
(i) a probe coupled to the microcarrier, wherein the probe is specific for a DNA mutation in the *KRAS, BRAF, CTNNB1,* or *APC* gene, wherein optionally the *KRAS, BRAF, CTNNB1,* and *APC* genes are human genes; and
(ii) an identifier corresponding to the probe coupled thereto;
wherein the kit comprises at least one microcarrier comprising a probe specific for a DNA mutation in the *KRAS* gene, at least one microcarrier comprising a probe specific for a DNA mutation in the *BRAF* gene, at least one microcarrier comprising a probe specific for a DNA mutation in the *CTNNB1* gene, and at least one microcarrier comprising a probe specific for a DNA mutation in the *APC* gene; and
(b) at least four blocking nucleic acids, wherein each of said at least four blocking nucleic acids hybridizes with a wild-type DNA locus corresponding with one of the DNA mutations in the KRAS, BRAF, CTNNB1, or APC genes.

13. The kit of claim 12, wherein each of said at least four blocking nucleic acids comprises a single-stranded oligonucleotide that hybridizes with the corresponding wild-type DNA locus; and a 3' terminal moiety that blocks extension from the single-stranded oligonucleotide;
wherein optionally the 3' terminal moiety comprises one or more inverted deoxythymidines; and wherein optionally each of said at least four blocking nucleic acids comprises one or more modified nucleotides selected from the group consisting of locked nucleic acids (LNAs), peptide nucleic acids (PNAs), hexose nucleic acids (HNAs), threose nucleic acids (TNAs), glycol nucleic acids (GNAs), and cyclohexenyl nucleic acids (CeNAs).

14. The kit of claim 12 or claim 13, wherein the DNA mutation in the *KRAS* gene comprises *KRAS* mutations encoding G12D, G12V, G12S, and G13D mutated KRAS proteins;
wherein optionally the kit comprises four probes comprising the sequences GGAGCTGATGG (SEQ ID NO:4), GGAGCTGTTGG (SEQ ID NO:5), TGGAGCTAGTGG (SEQ ID NO:6), and TGGAGCTGGTGACGT (SEQ ID NO:7), and wherein each of the four probes is coupled to a microcarrier with a different identifier; wherein optionally each of the four probes further comprises eight nucleotides at the 5' end, wherein the eight nucleotides at the 5' end are adenine or thymine nucleotides, and wherein each of the four probes comprises at least 24 total nucleotides;
wherein optionally the kit comprises (1) a first probe comprising a sequence selected from the group consisting of GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO:180), and GCTGATGGCGTA (SEQ ID NO:181); (2) a second probe comprising a sequence selected from the group consisting of GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO: 184), and TGGAGCTGTAGGTGG (SEQ ID NO: 185); (3) a third probe comprising a sequence selected from the group consisting of TTGGAGCTAGTGGCGTA (SEQ ID NO:186), GCTAGTGGCGTAGGC (SEQ ID NO:187), AGCTAGTGGCGT (SEQ ID NO:188), GTTGGAGCTAGTGG (SEQ ID NO:189), and GGAGCTAGTGG (SEQ ID NO:190); and (4) a fourth probe comprising a sequence selected from the group consisting of a fourth probe comprising a sequence selected from the group consisting of GGTGACGTAGGCAA (SEQ ID NO:191), TGACGTAGGCAAGAG (SEQ ID NO: 192), GCTGGTGACGTAGG (SEQ ID NO: 193), AGCTGGTGACGTAG (SEQ ID NO: 194), and GGAGCTGGTGACGT (SEQ ID NO: 195); and wherein each of the four probes is coupled to a microcarrier with a different identifier; wherein optionally the kit comprises (1) a first probe comprising a sequence selected from the group consisting of TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), and TTTTTTTTTTTTTGCTGATGGCGTA (SEQ ID NO:77); (2) a second probe comprising a sequence selected from the group consisting of TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), and TTTTTTTTTATGGAGCTGTAGGTGG (SEQ ID NO:81); (3) a third probe comprising a sequence selected from the group consisting of TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), and TTTTTTTTTTTTAAGGAGCTAGTGG (SEQ ID NO:86); and (4) a fourth probe comprising a sequence selected from the group consisting of TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90), and TTTTTTTTTAAGGAGCTGGTGACGT (SEQ ID NO:91); and wherein each of the four probes is coupled to a microcarrier with a different identifier;
wherein optionally the kit further comprises a primer pair comprising the sequences GTACTGGTGGAGTATTTGATAGTG (SEQ ID NO:1) and ATCGTCAAGGCACTCTTGCCTAC (SEQ ID NO:2); and
wherein optionally the kit further comprises a blocking nucleic acid comprising the sequence of TA*C*G*CC*A*CC*A*G*CT(invdT)*ₙ*, wherein n is 1, 2, or 3 (SEQ ID NO:3), TT*GG*A*G*CT*GGTGGC*GTA(invdT)*ₙ*, wherein n is 1, 2, or 3 (SEQ ID NO:142), GCT*GG*T*GG*C*G*TA*G*G*C*A(invdT)*ₙ*, wherein n is 1, 2, or 3 (SEQ ID NO:143), *GCTGGTGGCGTA*GGC(invdT)*ₙ,* wherein *n is* 1, 2, or 3 (SEQ ID NO:144), or TT*GG*A*G*CT*GG*T*GG*C*G*T(invdT)*ₙ*, wherein n is 1, 2, or 3 (SEQ ID NO:145), with italicized nucleic acids representing locked nucleic acids.

15. The kit of any one of claims 12-14, wherein the DNA mutations in the *BRAF* gene comprises two or more *BRAF* mutations encoding a V600E mutated BRAF protein;
wherein optionally the kit comprises two probes comprising the sequences TCTAGCTACAGAGAAAT (SEQ ID NO:11) and GTCTAGCTACAGAAAAAT (SEQ ID NO:12), and wherein each of the two probes is coupled to a microcarrier with a different identifier;
wherein optionally each of the two probes further comprises eight nucleotides at the 5' end, wherein the eight nucleotides at the 5' end are adenine or thymine nucleotides, and wherein each of the two probes comprises at least 24 total nucleotides;
wherein optionally the kit comprises (1) a first probe comprising a sequence selected from the group consisting of TACAGAGAAATCTCGAT (SEQ ID NO:196), TACAGAGAAATCTC (SEQ ID NO:197), CTAGCTACAGAGAAAT (SEQ ID NO:198), CTAGCTACAGAGAAA (SEQ ID NO:199), and TCTAGCTACAGAG (SEQ ID NO:200); and (2) a second probe comprising a sequence selected from the group consisting of GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203), and TCTAGCTACAGAAAAATC (SEQ ID NO:204); and wherein each of the two probes is coupled to a microcarrier with a different identifier;
wherein optionally the kit comprises (1) a first probe comprising a sequence selected from the group consisting of TTTTTTAATTTCTAGCTACAGAGAAAT (SEQ ID NO:51), TTTTTTTTTATACAGAGAAATCTCGAT (SEQ ID NO:92), TTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95), and TTTTTTTTTTAATTTCTAGCTACAGAG (SEQ ID NO:96); and (2) a second probe comprising a sequence selected from the group consisting of TTTTTTTATGTCTAGCTACAGAAAAAT (SEQ ID NO:52), TTTTATGTCTAGCTACAGAAAAATC (SEQ ID NO:97), TTTTTTTTATTTTTAGCTACAGAAAAA (SEQ ID NO:98), TTTTTTTATTTCTAGCTACAGAAAAAT (SEQ ID NO:99), and TTTTTTTTATTCTAGCTACAGAAAAATC (SEQ ID NO:100); and wherein each of the two probes is coupled to a microcarrier with a different identifier;
wherein optionally the kit further comprises a primer pair comprising the sequences GGACCCACTCCATCGAGATTT (SEQ ID NO:8) and CAGATATATTTCTTCATGAAGACCTCACAGTAA (SEQ ID NO:9); and
wherein optionally the kit further comprises a blocking nucleic acid comprising the sequence of G*AGAT*T*TCAC*T*GTAGC*(invdT)*ₙ,* wherein *n is* 1, 2, or 3 (SEQ ID NO:10), GA*G*AT*TTCACTGT*AGC(invdT)*ₙ*, wherein n is 1, 2, or 3 (SEQ ID NO: 146), GA*G*AT*TTCACTGT*AGC(invdT)*ₙ*, wherein *n is* 1, 2, or 3 (SEQ ID NO: 147), *GAGAT*T*TCACT*G*TAGC*(invdT)*ₙ,* wherein *n is* 1, 2, or 3 (SEQ ID NO: 148), or GA*G*AT*TTCACTGT*AGC(invdT)*ₙ*, wherein *n is* 1, 2, or 3 (SEQ ID NO: 149), with italicized nucleic acids representing locked nucleic acids.

16. The kit of any one of claims 12-15, wherein the DNA mutation in the *CTNNB1* gene comprises *CTNNB1* mutations encoding T41A, T41I, S45F, and S45P mutated CTNNB1 proteins;
wherein optionally the kit comprises four probes comprising the sequences AGGAGCTGTGGCAG (SEQ ID NO:16), GGAGCTGTGATA (SEQ ID NO: 17), TTTACCACTCAGAAAAG (SEQ ID NO:21), and TACCACTCAGAGGAG (SEQ ID NO:22), and wherein each of the four probes is coupled to a microcarrier with a different identifier;
wherein optionally each of the four probes further comprises eight nucleotides at the 5' end,
wherein the eight nucleotides at the 5' end are adenine or thymine nucleotides, and wherein
each of the four probes comprises at least 24 total nucleotides;
wherein optionally the kit comprises (1) a first probe comprising a sequence selected from the group consisting of AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), and AAGGAGCTGTGGCAG (SEQ ID NO:209); (2) a second probe comprising a sequence selected from the group consisting of GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), and GGAGCTGTGAT (SEQ ID NO:214); (3) a third probe comprising a sequence selected from the group consisting of ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), and CAGAAAAGGAGCTGTG (SEQ ID NO:219); and (4) a fourth probe comprising a sequence selected from the group consisting of ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), and TTACCAATCAGAGGAGG (SEQ ID NO:224); wherein each of the four probes is coupled to a microcarrier with a different identifier;
wherein optionally the kit comprises (1) a first probe comprising a sequence selected from the group consisting of TTTTTTTTTTTAGGAGCTGTGGCAG (SEQ ID NO:53), TTTTTTTTTTTAGGAGCTGTGGCAGTG (SEQ ID NO:101), TTTTTTTTTTTAGCTGTGGCAGTGGC (SEQ ID NO: 102), TTTTTTTTTTTGCTGTGGCAGTGGCA (SEQ ID NO:103), and TTTTTTTTTTAAGGAGCTGTGGCAG (SEQ ID NO:104); (2) a second probe comprising a sequence selected from the group consisting of TTTTTTTTTTTTTGGAGCTGTGATA (SEQ ID NO:54), TTTTTTTTTGGAGCTGTGATAGTGG (SEQ ID NO: 105), TTTTTTTTTGAGCTGTGATAGTGGC (SEQ ID NO:106), TTTTTTTTTAGCTGTGATAGTGGCA (SEQ ID NO:107), TTTTTTTTTAGAAGGAGCTGTGATA (SEQ ID NO:108), and TTTTTTTTTTTTTTGGAGCTGTGAT (SEQ ID NO:109); (3) a third probe comprising a sequence selected from the group consisting of TTTTTTTTTTTACCACTCAGAAAAG (SEQ ID NO:55), TTTAATTTTACTCAGAAAAGGAGCT (SEQ ID NO: 110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113), and TTTTTTTTTCAGAAAAGGAGCTGTG (SEQ ID NO:114); and (4) a fourth probe comprising a sequence selected from the group consisting of TTTTTTTTTAATACCACTCAGAGGAG (SEQ ID NO:56), TTTTTTTTTAAAACTCAGAGGAGGAGC (SEQ ID NO:115), TTTTTTTTTTTATTACCACTCAGAGGA (SEQ ID NO:116), TTTTTTTTTATTACCACTCAGAGGAGG (SEQ ID NO:117), TTTTTTTTTTATTAACACTCAGAGGAG (SEQ ID NO:118), and TTTTTTTTTATTACCAATCAGAGGAGG (SEQ ID NO:119); wherein each of the four probes is coupled to a microcarrier with a different identifier;
wherein optionally the kit further comprises a first primer pair comprising the sequences GGAATCCATTCTGGTGCCACT (SEQ ID NO: 13) and AGAAAATCCCTGTTCCCACTCATA (SEQ ID NO:14), and a second primer pair comprising the sequences GGTGCCACTACCACAGCTCCT (SEQ ID NO:18) and TCTCAAAACTGCATTCTGACTTTCA (SEQ ID NO:19); and
wherein optionally the kit further comprises a first blocking nucleic acid comprising the sequence of GC*CACTACCACAG*CT(invdT)*ₙ,* wherein *n is* 1, 2, or 3 (SEQ ID NO:15), T*G*C*CA*CT*ACCA*C*AG*(invdT)*ₙ,* wherein *n is* 1, 2, or 3 (SEQ ID NO:150), C*AC*T*ACCA*C*AGC*T*C*C(invdT)*ₙ,* wherein *n is* 1, 2, or 3 (SEQ ID NO:151), GC*CACTACCACAG*CT(invdT)*ₙ,* wherein *n is* 1, 2, or 3 (SEQ ID NO:152), or GC*CACTACCACAG*CT(invdT)*ₙ,* wherein n is 1, 2, or 3 (SEQ ID NO: 153), and a second blocking nucleic acid comprising the sequence of GC*TCCTTCTCTG*AGT(invdT)*ₙ,* wherein *n* is 1, 2, or 3 (SEQ ID NO:20), T*CC*T*TCTC*T*GAG*T*G*G(invdT)*ₙ,* wherein n is 1, 2, or 3 (SEQ ID NO:174), GCTCCTTCTCTGAGT(invdT)n, wherein n is 1, 2, or 3 (SEQ ID NO:175), TCCTTCTCTGAGTGG(invdT)n, wherein n is 1, 2, or 3 (SEQ ID NO:176), or GCTCCTTCTCTGAGT(invdT)n, wherein n is 1, 2, or 3 (SEQ ID NO:177), with italicized nucleic acids representing locked nucleic acids.

17. The kit of any one of claims 12-16, wherein the DNA mutation in the *APC* gene comprises APC mutations encoding Q1367*, R1450*, E1309 frameshift, S1465 frameshift, and T1556 frameshift mutated APC proteins;
wherein optionally the kit comprises five probes comprising the sequences ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), and AGAGGCAGAAAAAAACT (SEQ ID NO:42), and wherein each of the five probes is coupled to a microcarrier with a different identifier;
wherein optionally each of the five probes further comprises eight nucleotides at the 5' end, wherein the eight nucleotides at the 5' end are adenine or thymine nucleotides, and wherein each of the five probes comprises at least 24 total nucleotides;
wherein optionally the kit comprises (1) a first probe comprising a sequence selected from the group consisting of AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228), and CAGAAATAAAAGATT (SEQ ID NO:229); (2) a second probe comprising a sequence selected from the group consisting of TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), and GGTGTCTAAGCACCA (SEQ ID NO:234); (3) a third probe comprising a sequence selected from the group consisting of AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237), and AGCTCAAACCAAGTGAG (SEQ ID NO:238); (4) a fourth probe comprising a sequence selected from the group consisting of GCACCTACTGCTGAA (SEQ ID NO:239), ACCTACTGCTGAAAAG (SEQ ID NO:240), TGCTGAAAAGAGAGAGT (SEQ ID NO:241), ACTGCTGAAAAGAGAGAGT (SEQ ID NO:26), and CCTACTGCTGAAAAGAGA (SEQ ID NO:242); and (5) a fifth probe comprising a sequence selected from the group consisting of GCAGAAAAAAACTATTG (SEQ ID NO:243), AGAGGCAGAAAAAAACT (SEQ ID NO:42), CAGAAAAAAACTATTGATT (SEQ ID NO:244), AGAAAGAGGCAGAAAAAAACT (SEQ ID NO:245), and GAGGCAGAAAAAAACTA (SEQ ID NO:246); and wherein each of the five probes is coupled to a microcarrier with a different identifier;
wherein optionally the kit comprises: (1) a first probe comprising a sequence selected from the group consisting of TTTTTTTTTTTTTGAAATAAAAGATTGG (SEQ ID NO:58), TTTTTTTTTTAAATAGCAGAAATAAAAG (SEQ ID NO: 120), TTTTTTTTTTTGAAATAAAAGATTGGAA (SEQ ID NO:121), TTTTTTTTTTTTTAGAAATAAAAGATTG (SEQ ID NO: 122), TTTTTTTTTTTTTGAAATAAATGAATGG (SEQ ID NO: 123), and TTTTTTTTTTTTTCAGAAATAAAAGATT (SEQ ID NO: 124); (2) a second probe comprising a sequence selected from the group consisting of TTTTTTTTTTTTTGGGTGTCTAAG (SEQ ID NO:59), TTTTTTTTTATTTGGGTGTCTAAG (SEQ ID NO:125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128),and TTTTTTTTTGGTGTCTAAGCACCA (SEQ ID NO: 129); (3) a third probe comprising a sequence selected from the group consisting of TTTTTTTTTACAAACCAAGTGAGAA (SEQ ID NO:60), TTTTTTTTAAGTGAGAAGTACCTAA (SEQ ID NO: 130), TTTTTTTTTTTTCAAACCAAGTGAG (SEQ ID NO:131), TTTTTTTTTTACCAAGTGAGAAGTA (SEQ ID NO: 132), and TTTTTTTTAGCTCAAACCAAGTGAG (SEQ ID NO:133); (4) a fourth probe comprising a sequence selected from the group consisting of TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO:135), TTTTTTTTTGCTGAAAAGAGAGAGT (SEQ ID NO: 136), and TTTTTTTTTCCTACTGCTGAAAAGAGA (SEQ ID NO: 137); and (5) a fifth probe comprising a sequence selected from the group consisting of TTTTTTTTTTAGAGGCAGAAAAAAACT (SEQ ID NO:61), TTTTTTTTTTGCAGAAAAAAACTATTG (SEQ ID NO:138), TTTTTTTTTTTCAGAAAAAAACTATTGATT (SEQ ID NO: 139), TTTTTTTAGAAAGAGGCAGAAAAAAACT (SEQ ID NO: 140), and TTTTTTTTTTTGAGGCAGAAAAAAACTA (SEQ ID NO:141); and wherein each of the five probes is coupled to a microcarrier with a different identifier;
wherein optionally the kit further comprises a first primer pair comprising the sequences TAAAAATAAAGCACCTACTGCTGAAA (SEQ ID NO:23) and AGCTTGCTTAGGTCCACTCTCTCT (SEQ ID NO:24); a second primer pair comprising the sequences TAGGATGTAATCAGACGACACAGGA (SEQ ID NO:27) and CAGCTGACCTAGTTCCAATCTTTTA (SEQ ID NO:28); a third primer pair comprising the sequences TCTCCCTCCAAAAGTGGTGCT (SEQ ID NO:31) and TGGCAATCGAACGACTCTCAA (SEQ ID NO:32); a fourth primer pair comprising the sequences GCAGAAGTAAAACACCTCCACCA (SEQ ID NO:35) and GGTGCTTTATTTTTAGGTACTTC (SEQ ID NO:36), with italicized nucleic acids representing locked nucleic acids; and a fifth primer pair comprising the sequences CAGGAAAATGACAATGGGAATG (SEQ ID NO:39) and ATCTAATAGGTCCTTTTCAGAATCAATAG (SEQ ID NO:40); and
wherein optionally the kit further comprises a first blocking nucleic acid comprising the sequence of *CCA*C*TC*TCTCT*C*T*TT*T*CAGC*(invdT)*ₙ,* wherein *n is* 1, 2, or 3 (SEQ ID NO:25), TA*GG*T*CC*ACTCTCTCTCT*TT*T*C*A*GC*A(invdT)n, wherein n is 1, 2, or 3 (SEQ ID NO:166), T*AGG*T*CCAC*T*CTCT*C*T*CTT*T*TC*AG*CA (invdT)n, wherein n is 1, 2, or 3 (SEQ ID NO:167), *CCACTCTCTCTCTTTTCAGC* (invdT)n, wherein n is 1, 2, or 3 (SEQ ID NO:168), or TAGGTCCACTCTCTCTCTITTCAGCA (invdT)n, wherein n is 1, 2, or 3 (SEQ ID NO:169), a second blocking nucleic acid comprising the sequence of C*TTTTCTTTTAT*TT*C*T*GC*(invdT)*ₙ,* wherein *n is* 1, 2, or 3 (SEQ ID NO:29), *CTTTTC*T*TTTA*T*T*T*C*TG*C*(invdT)n*,* wherein n is 1, 2, or 3 (SEQ ID NO:154), C*T*TT*TC*T*T*T*TATTTCTGC*(invdT)n, wherein n is 1, 2, or 3 (SEQ ID NO:155), C*TTT*TCT*TTT*A*T*T*TC*T*GC*(invdT)n*,* wherein n is 1, 2, or 3 (SEQ ID NO: 156), or C*T*TT*TCTTTTATTTC*TGC(invdT)n*,* wherein n is 1, 2, or 3 (SEQ ID NO:157), a third blocking nucleic acid comprising the sequence of GTG*CTCA*G*ACAC*C(invdT)*ₙ*, wherein n is 1, 2, or 3 (SEQ ID NO:33), GTGCTCAGACACC(invdT)n, wherein n is 1, 2, or 3 (SEQ ID NO:158), *AGTGGTGCTCAGACACCCA(invdT)n,* wherein n is 1, 2, or 3 (SEQ ID NO:159), AGTGGTGCTCAGACACCCA(invdT)n, wherein n is 1, 2, or 3 (SEQ ID NO:160), or A*G*T*G*GTG*CTCA*G*AC*A*C*C*C*A(invdT)n, wherein n is 1, 2, or 3 (SEQ ID NO:161), a fourth blocking nucleic acid comprising the sequence of C*TTC*T*CGCT*T*GGT*T(invdT)*ₙ,* wherein *n* is 1, 2, or 3 (SEQ ID NO:37), *GTACTTCTCGCTTGGT(invdT)n,* wherein n is 1, 2, or 3 (SEQ ID NO:162), C*TTC*T*CGCT*T*GGT*T(invdT)n*,* wherein n is 1, 2, or 3 (SEQ ID NO:163), GTA*CT*T*CTCG*CT*TGG*T(invdT)n, wherein n is 1, 2, or 3 (SEQ ID NO:164), or GTACTTCTCGCTTGGT(invdT)n, wherein n is 1, 2, or 3 (SEQ ID NO:165), and a fifth blocking nucleic acid comprising the sequence of *C*A*ATAG*T*TTTT*T*CTGC*C(invdT)*ₙ,*
wherein *n is* 1, 2, or 3 (SEQ ID NO:41), *GAATCAATAGTTTTTTCTGCCTC* (invdT)n,
wherein n is 1, 2, or 3 (SEQ ID NO:170), *TCAGAATCAATAGTTTTTTCTG* (invdT)n,
wherein n is 1, 2, or 3 (SEQ ID NO:171), *GAATCAATAGATTTTACTGCCTC* (invdT)n,
wherein n is 1, 2, or 3 (SEQ ID NO:172), or A*A*T*CAATAG*TTTTTTC*TGCCT*C (invdT)n,
wherein n is 1, 2, or 3 (SEQ ID NO:173), with italicized nucleic acids representing locked nucleic acids.

18. The kit of any one of claims 12-17, further comprising a microcarrier with an identifier corresponding to a positive control and to which a probe specific for a positive control gene sequence is coupled; and a primer pair specific for the positive control DNA sequence;
wherein optionally the positive control DNA sequence comprises a sequence of a human leukocyte antigen gene or a human glyceraldehyde 3-phosphate dehydrogenase (GAPDH) gene;
wherein optionally the primer pair specific for the positive control DNA sequence comprises the sequences TGAGTGTTACTTCTTCCCACACTC (SEQ ID NO:43) and ATTGCTTTTGCGCAATCCCT (SEQ ID NO:44) or AATCCCATCACCATCTTCCA (SEQ ID NO:71) and TGGACTCCACGACGTACTCA (SEQ ID NO:72); and
wherein optionally the probe specific for the positive control gene sequence comprises the sequence TTTTTTTTTTTTGGAGACGGTCTG (SEQ ID NO:45) or CTGTCTTCCACTCACTCC (SEQ ID NO:73);
wherein optionally the kit further comprises a microcarrier with an identifier corresponding to a negative control and to which is coupled a probe that does not hybridize with the amplified DNA, and wherein optionally the microcarrier with the identifier corresponding to the negative control comprises a probe comprising the sequence AATATAATATATTAT (SEQ ID NO:46).

19. The kit of any one of claims 12-18, wherein the identifiers of the micocarriers comprise (a) digital barcodes or (b) analog codes;
wherein optionally each of the microcarriers comprises:
(i) a first photopolymer layer; (ii) a second photopolymer layer; and (iii) an intermediate layer between the first layer and the second layer, the intermediate layer having an encoded pattern representing the identifier defined thereon, wherein the intermediate layer is partially substantially transmissive and partially substantially opaque to light, representing a code corresponding to the microcarrier, wherein the outermost surface of the microcarrier comprises a photoresist photopolymer, and said photoresist photopolymer is functionalized with the probe specific for the DNA mutation, and wherein said microcarrier has about the same density as water; or
(i) a substantially transparent polymer layer having a first surface and a second surface, the first and the second surfaces being parallel to each other; (ii) a substantially non-transparent polymer layer, wherein the substantially non-transparent polymer layer is affixed to the first surface of the substantially transparent polymer layer and encloses a center portion of the substantially transparent polymer layer, and wherein the substantially non-transparent polymer layer comprises a two-dimensional shape representing an analog code, wherein the analog code represents the identifier; and (iii) the probe specific for the DNA mutation, wherein the probe is coupled to at least one of the first surface and the second surface of the substantially transparent polymer layer in at least the center portion of the substantially transparent polymer layer, wherein optionally each of the microcarriers further comprises an orientation indicator for orienting the analog code of the substantially non-transparent polymer layer, wherein optionally the substantially transparent polymer of the first or the second substantially transparent polymer layer comprises an epoxy-based polymer, and wherein optionally the epoxy-based polymer is SU-8.

## Patentansprüche

1. Verfahren zum Nachweisen des Vorhandenseins von DNA-Mutationen in den *KRAS-, BRAF-, CTNNBI-* und AFC-Genen, wobei das Verfahren umfasst:
(a) Isolieren von DNA aus einer Probe;
(b) Amplifizieren der isolierten DNA durch Polymerase-Kettenreaktion (PCR) unter Verwendung von Primer-Paaren, die für die Loci einer oder mehrerer DNA-Mutationen in jedem der *KRAS-, BRAF-, CTNNBI-* und AFC-Gene spezifisch sind, wobei die isolierte DNA in der Gegenwart von wenigstens vier blockierenden Nukleinsäuren amplifiziert wird, wobei jede der wenigstens vier blockierenden Nukleinsäuren mit einem Wildtyp-DNA-Locus hybridisiert, der einer der DNA-Mutationen in den KRAS-, BRAF-, CTNNB1- oder APC-Genen entspricht, und die Amplifikation des Wildtyp-DNA-Locus verhindert, wobei die *KRAS-, BRAF-, CTNNBI-* und AFC-Genen optional menschliche Gene sind;
(c) Hybridisieren der amplifizierten DNA mit wenigstens vier Sonden, wobei die wenigstens vier Sonden eine oder mehrere Sonden umfassen, die für eine DNA-Mutation in jedem der *KRAS-, BRAF-, CTNNBI-* und AFC-Gene spezifisch sind, wobei jede der wenigstens vier Sonden an einen Mikroträger gekoppelt ist, und wobei jeder der Mikroträger einen Identifikator umfasst, der der daran gekoppelten Sonde entspricht;
(d) Nachweisen des Vorhandenseins oder der Abwesenheit einer Hybridisierung der amplifizierten DNA mit den wenigstens vier Sonden, wobei die Hybridisierung zwischen der amplifizierten DNA und einer der Sonden das Vorhandensein der der Sonde entsprechenden DNA-Mutation anzeigt;
(e) Nachweisen der Identifikatoren der Mikroträger; und
(f) Korrelieren der nachgewiesenen Identifikatoren der Mikroträger mit dem nachgewiesenen Vorhandensein oder der Abwesenheit der Hybridisierung der amplifizierten DNA mit den entsprechenden Sonden der Mikroträger.

2. Verfahren nach Anspruch 1, wobei jede der wenigstens vier blockierenden Nukleinsäuren ein einzelsträngiges Oligonukleotid umfasst, das mit dem entsprechenden Wildtyp-DNA-Locus hybridisiert, und einen 3'-terminalen Teil, der die Verlängerung des einzelsträngigen Oligonukleotids blockiert;
wobei optional der 3'-terminale Teil ein oder mehrere invertierte Desoxythymidine umfasst; und wobei optional jede der wenigstens vier blockierenden Nukleinsäuren ein oder mehrere modifizierte Nukleotide umfasst, die aus der Gruppe ausgewählt sind, die aus blockierten Nukleinsäuren (LNAs), Peptidnukleinsäuren (PNAs), Hexosenukleinsäuren (HNAs), Threosenukleinsäuren (TNAs), Glykolnukleinsäuren (GNAs) und Cyclohexenylnukleinsäuren (CeNAs) besteht.

3. Verfahren nach Anspruch 1 oder 2, wobei die eine oder die mehreren DNA-Mutationen in dem *KRAS*-Gen *KRAS*-Mutationen umfassen, die für G12D-, G12V-, G12S- und G13D-mutierte KRAS-Proteine kodieren;
wobei optional die für eine oder mehrere DNA-Mutationen in dem *KRAS-Gen* spezifischen Sonden vier Sonden umfassen, die die Sequenzen GGAGCTGATGG (SEQ ID NO:4), GGAGCTGTTGG (SEQ ID NO:5), TGGAGCTAGTGG (SEQ ID NO:6) und TGGAGCTGGTGACGT (SEQ ID NO:7) umfassen, und wobei jede der vier Sonden an einen Mikroträger mit einem anderen Identifikator gekoppelt ist;
wobei optional jede der vier Sonden ferner acht Nukleotide an dem 5'-Ende umfasst, wobei die acht Nukleotide an dem 5'-Ende Adenin- oder Thyminnukleotide sind, und wobei jede der vier Sonden wenigstens 24 Gesamtnukleotide umfasst;
wobei die Sonden optional umfassen: (1) eine erste Sonde, umfassend eine Sequenz, ausgewählt aus der Gruppe bestehend aus GGAGCTGATGG (SEQ ID NO:4), AGCTGATGGCGTA (SEQ ID NO: 178), TGGAGCTGATGGCG (SEQ ID NO: 179), TGGAGCTGATGG (SEQ ID NO: 180) und GCTGATGGCGTA (SEQ ID NO:181); (2) eine zweite Sonde, umfassend eine Sequenz, ausgewählt aus der Gruppe bestehend aus GGAGCTGTTGG (SEQ ID NO:5), TGGAGCTGTTGGTGGC (SEQ ID NO:182), GGAGCTGTTGGTG (SEQ ID NO: 183), TGGAGCTGTTGGT (SEQ ID NO: 184) und TGGAGCTGTAGGTGG (SEQ ID NO:185); (3) eine dritte Sonde, umfassend eine Sequenz, ausgewählt aus der Gruppe, bestehend aus TTGGAGCTAGTGGCGTA (SEQ ID NO: 186), GCTAGTGGCGTAGGC (SEQ ID NO: 187), AGCTAGTGGCGT (SEQ ID NO: 188), GTTGGAGCTAGTGG (SEQ ID NO: 189) und GGAGCTAGTGG (SEQ ID NO:190); und (4) eine vierte Sonde, umfassend eine Sequenz, ausgewählt aus der Gruppe, bestehend aus GGTGACGTAGGCAA (SEQ ID NO:191), TGACGTAGGGCAAGAG (SEQ ID NO: 192), GCTGGTGACGTAGG (SEQ ID NO: 193), AGCTGGTGACGTAG (SEQ ID NO: 194) und GGAGCTGGTGACGT (SEQ ID NO:195); und wobei jede der vier Sonden an einen Mikroträger mit einem anderen Identifikator gekoppelt ist;
wobei die Sonden optional umfassen: (1) eine erste Sonde, die eine Sequenz umfasst, die aus der Gruppe ausgewählt ist, bestehend aus TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), und TTTTTTTTTTTTTGCTGATGGCGTA (SEQ ID NO:77); (2) eine zweite Sonde, die eine Sequenz umfasst, die aus der Gruppe ausgewählt ist, bestehend aus TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), und TTTTTTTTTATGGAGCTGTAGGTGG (SEQ ID NO:81); (3) eine dritte Sonde, umfassend eine Sequenz, die aus der Gruppe ausgewählt ist, bestehend aus TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), und TTTTTTTTTTTTAAGGAGCTAGTGG (SEQ ID NO:86); und (4) eine vierte Sonde, umfassend eine Sequenz, die aus der Gruppe ausgewählt ist, bestehend aus TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90) und TTTTTTTTTAAGGAGCTGGTGACGT (SEQ ID NO:91); und wobei jede der vier Sonden an einen Mikroträger mit einem anderen Identifikator gekoppelt ist;
wobei optional Schritt (b) das Amplifizieren der isolierten DNA durch PCR unter Verwendung eines Primer-Paars umfasst, das die Sequenzen GTACTGGTGGAGTATTTGATAGTG (SEQ ID NO:1) und ATCGTCAAGGCACTCTTGCCTAC (SEQ ID NO:2) umfasst; und
wobei optional Schritt (b) das Amplifizieren der isolierten DNA durch PCR in der Gegenwart einer blockierenden Nukleinsäure umfasst, die die Sequenz TA*C*G*CC*A*CC*A*G*CT(invdT)*ₙ*, wobei *n* 1, 2 oder 3 ist (SEQ ID NO:3), TT*GG*A*G*CT*GGTGGC*GTA(invdT)*ₙ*, wobei *n 1,* 2 oder 3 ist (SEQ ID NO:142), GCT*GG*T*GG*C*G*TA*G*G*C*A(invdT)*ₙ*, wobei *n* 1, 2 oder 3 ist (SEQ ID NO:143), *GCTGGTGGCGTA*GGC(invdT)*ₙ,* wobei *n* 1, 2 oder 3 ist (SEQ ID NO: 144), oder TT*GG*A*G*CT*GG*T*GG*C*G*T(invdT)*ₙ*, wobei *n* 1, 2 oder 3 ist (SEQ ID NO:145),
wobei kursiv gedruckte Nukleinsäuren geschlossene Nukleinsäuren darstellen.

4. Verfahren nach einem der Ansprüche 1-3, wobei die eine oder die mehreren DNA-Mutationen in dem *BRAF-Gen* zwei oder mehr BRAF-Mutationen umfassen, die ein V600E-mutiertes BRAF-Protein kodieren;
wobei optional die für eine oder mehrere DNA-Mutationen in dem *BRAF-Gen* spezifischen Sonden zwei Sonden umfassen, die die Sequenzen TCTAGCTACAGAGAAAT (SEQ ID NO: 11) und GTCTAGCTACAGAAAAAT (SEQ ID NO: 12) umfassen, und wobei jede der beiden Sonden an einen Mikroträger mit einem anderen Identifikator gekoppelt ist;
wobei optional jede der zwei Sonden ferner acht Nukleotide an dem 5'-Ende umfasst, wobei die acht Nukleotide an dem 5'-Ende Adenin- oder Thyminnukleotide sind, und wobei jede der zwei Sonden wenigstens 24 Gesamtnukleotide umfasst;
wobei die Sonden optional umfassen: (1) eine erste Sonde, umfassend eine Sequenz, die aus der Gruppe ausgewählt ist, bestehend aus TACAGAGAAATCTCGAT (SEQ ID NO: 196), TACAGAGAAATCTC (SEQ ID NO: 197), CTAGCTACAGAGAAAT (SEQ ID NO:198), CTAGCTACAGAGAAA (SEQ ID NO: 199) und TCTAGCTACAGAG (SEQ ID NO:200); und (2) eine zweite Sonde, umfassend eine Sequenz, die aus der Gruppe ausgewählt ist, bestehend aus GTCTAGCTACAGAAAAATC (SEQ ID NO:201), GTCTAGCTACAGAAAAAT (SEQ ID NO: 12), TAGCTACAGAAAAA (SEQ ID NO:202), TCTAGCTACAGAAAAAT (SEQ ID NO:203) und TCTAGCTACAGAAAAATC (SEQ ID NO:204); und wobei jede der zwei Sonden an einen Mikroträger mit einem anderen Identifikator gekoppelt ist;
wobei die Sonden optional umfassen: (1) eine erste Sonde, die eine Sequenz umfasst, die aus der Gruppe ausgewählt ist, bestehend aus TTTTTTTAATTTCTAGCTACAGAGAAAT (SEQ ID NO:51), TTTTTTTTTATACAGAGAAATCTCGAT (SEQ ID NO:92), TTTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95) und TTTTTTTTTTAATTTCTAGCTACAGAGAG (SEQ ID NO:96); und (2) eine zweite Sonde, die eine Sequenz umfasst, die aus der Gruppe ausgewählt ist, bestehend aus TTTTTTTATGTCTAGCTACAGAAAAAT (SEQ ID NO:52), TTTTTTTCTAGCTACAGAAATC (SEQ ID NO:97), TTTTTTTATTTTTTTAGCTACAGAAAAA (SEQ ID NO:98), TTTTTTTATTTCTAGCTACAGAAAAAT (SEQ ID NO:99) und TTTTTTTTATTCTAGCTACAGAAAAATC (SEQ ID NO:100), und wobei jede der zwei Sonden an einen Mikroträger mit einem anderen Identifikator gekoppelt ist;
wobei optional Schritt (b) das Amplifizieren der isolierten DNA durch PCR unter Verwendung eines Primer-Paars umfasst, das die Sequenzen GGACCCACTCCATCGAGATTT (SEQ ID NO:8) und CAGATATATTTCTTCATGAAGACCTCACAGTAA (SEQ ID NO:9) umfasst; und
wobei optional Schritt (b) das Amplifizieren der isolierten DNA durch PCR in der Gegenwart einer blockierenden Nukleinsäure umfasst, die die Sequenz GA*G*AT*TTCACTGT*AGC(invdT)*ₙ*, wobei *n* 1, 2 oder 3 ist (SEQ ID NO: 10), GA*G*AT*TTCACTGT*AGC(invdT)*ₙ*, wobei *n* 1, 2 oder 3 ist (SEQ ID NO: 146), GA*G*AT*TTCACTGT*AGC(invdT)*ₙ*, wobei *n* 1, 2 oder 3 ist (SEQ ID NO: 147), GA*G*AT*TTCACTGT*AGC(invdT)*ₙ*, wobei *n* 1, 2 oder 3 ist (SEQ ID NO:148), oder GA*G*AT*TTCACTGT*AGC(invdT)*ₙ*, wobei *n* 1, 2 oder 3 ist (SEQ ID NO: 149), wobei kursiv gedruckte Nukleinsäuren geschlossene Nukleinsäuren darstellen.

5. Verfahren nach einem der Ansprüche 1-4, wobei die eine oder die mehreren DNA-Mutationen im *CTNNB1-*Gen CTNNBI-Mutationen umfassen, die für T41A-, T41I-, S45F- und S45P-mutierte CTNNB1-Proteine kodieren;
wobei optional die für eine oder mehrere DNA-Mutationen in dem *CTNNBI-Gen* spezifischen Sonden vier Sonden umfassen, die die Sequenzen AGGAGCTGTGGCAG (SEQ ID NO: 16), GGAGCTGTGATA (SEQ ID NO: 17), TTTACCACTCAGAAAAG (SEQ ID NO:21) und TACCACTCAGAGGAG (SEQ ID NO:22) umfassen, und wobei jede der vier Sonden an einen Mikroträger mit einem anderen Identifikator gekoppelt ist;
wobei optional jede der vier Sonden ferner acht Nukleotide an dem 5'-Ende umfasst, wobei die acht Nukleotide an dem 5'-Ende Adenin- oder Thyminnukleotide sind, und wobei jede der vier Sonden wenigstens 24 Gesamtnukleotide umfasst;
wobei die Sonden optional umfassen: (1) eine erste Sonde, umfassend eine Sequenz, ausgewählt aus der Gruppe bestehend aus AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), und AAGGAGCTGTGGCAG (SEQ ID NO:209); (2) eine zweite Sonde, umfassend eine Sequenz, ausgewählt aus der Gruppe bestehend aus GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), und GGAGCTGTGAT (SEQ ID NO:214); (3) eine dritte Sonde, umfassend eine Sequenz, ausgewählt aus der Gruppe, bestehend aus ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), und CAGAAAAGGAGCTGTG (SEQ ID NO:219); und (4) eine vierte Sonde, umfassend eine Sequenz, ausgewählt aus der Gruppe, bestehend aus ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), und TTACCAATCAGAGGAGG (SEQ ID NO:224); und wobei jede der vier Sonden an einen Mikroträger mit einem anderen Identifikator gekoppelt ist;
wobei die Sonden optional umfassen: (1) eine erste Sonde, die eine Sequenz umfasst, die aus der Gruppe ausgewählt ist, bestehend TTTTTTTTTTTTAGGAGCTGTGGCAG (SEQ ID NO:53), TTTTTTTTTTTAGGAGCTGTGGCAGTGTG (SEQ ID NO:101), TTTTTTTTTTTAGCTGTGGCAGTGGGC (SEQ ID NO:102), TTTTTTTTTGCTGTGGCAGTGGCA (SEQ ID NO:103) und TTTTTTTTTTAAGGAGCTGTGGCAG (SEQ ID NO:104); (2) eine zweite Sonde, umfassend eine Sequenz, die aus der Gruppe ausgewählt ist, bestehend aus TTTTTTTTTTTTTTGGAGCTGTGATA (SEQ ID NO:54), TTTTTTTTTGGAGCTGTGATAGTGG (SEQ ID NO:105), TTTTTTTTGAGCTGTGATAGTGGC (SEQ ID NO:106), TTTTTTTTTAGCTGTGATAGTGGCA (SEQ ID NO: 107), TTTTTTTTAGAAGGAGCTGTGATA (SEQ ID NO: 108), und TTTTTTTTTTTTTGGAGCTGTGAT (SEQ ID NO:109); (3) eine dritte Sonde, umfassend eine Sequenz, die aus der Gruppe ausgewählt ist, bestehend aus TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO:111), TTTTTTTTACCACTCAGAAAAGGAG (SEQ ID NO:112), TTTTTTTTATTACCACTCAGAAAAG (SEQ ID NO:113) und TTTTTTTTTCAGAAAAGGAGCTGTG (SEQ ID NO:114); und (4) eine vierte Sonde, umfassend eine Sequenz, die aus der Gruppe ausgewählt ist, bestehend aus TTTTTTTTTAATACCACTCAGAGGAG (SEQ ID NO:56), TTTTTTTTTAAAACTCAGAGGAGC (SEQ ID NO:115), TTTTTTTTTTATTACCACTCAGAGGA (SEQ ID NO: 116), TTTTTTTTTATTACCACTCAGAGGAGG (SEQ ID NO:117), TTTTTTTTTTATTAACACTCAGAGGAG (SEQ ID NO:118), und TTTTTTTTTATTACCAATCAGAGGAGG (SEQ ID NO: 119) umfassen; und wobei jede der vier Sonden an einen Mikroträger mit einem anderen Identifikator gekoppelt ist;
wobei optional Schritt (b) das Amplifizieren der isolierten DNA durch PCR unter Verwendung eines ersten Primer-Paars, das die Sequenzen GGAATCCATTCTGGTGCCACT (SEQ ID NO: 13) und AGAAAATCCCTGTTCCCACTCATA (SEQ ID NO: 14) umfasst, und eines zweiten Primer-Paars umfasst, das die Sequenzen GGTGCCACTACCACAGCTCCT (SEQ ID NO: 18) und TCTCAAAACTGCATTCTGACTTTCA (SEQ ID NO: 19) umfasst; und
wobei optional Schritt (b) das Amplifizieren der isolierten DNA durch PCR in der Gegenwart einer ersten blockierenden Nukleinsäure, die die Sequenz GC*CACTACCACAG*CT(invdT)*ₙ,* wobei *n* 1, 2 oder 3 ist (SEQ ID NO:15), T*G*C*CA*CT*ACCA*C*AG*(invdT)*ₙ,* wobei *n* 1, 2 oder 3 ist (SEQ ID NO:150), C*AC*T*ACCA*C*AGC*T*C*C(invdT)*ₙ,* wobei *n* 1, 2 oder 3 ist (SEQ ID NO:151), GC*CACTACCACAG*CT(invdT)*ₙ,* wobei *n* 1, 2 oder 3 ist (SEQ ID NO:152), oder GC*CACTACCACAG*CT(invdT)*ₙ,* wobei *n* 1, 2 oder 3 ist (SEQ ID NO: 153) umfasst, und einer zweiten blockierenden Nukleinsäure umfasst, die die Sequenz GC*TCCTTCTCTG*AGT(invdT)*ₙ,* wobei *n* 1, 2 oder 3 ist (SEQ ID NO:20), T*CC*T*TCTC*T*GAG*T*G*G(invdT)*ₙ,* wobei *n* 1, 2 oder 3 ist (SEQ ID NO: 174), GC*TCCTTCTCTG*AGT(invdT)*ₙ,* wobei n 1, 2 oder 3 ist (SEQ ID NO: 175), T*CC*T*TCTC*T*GAG*T*G*G(invdT)*ₙ,* wobei n 1, 2 oder 3 ist (SEQ ID NO: 176), oder GC*TCCTTCTCTG*AGT(invdT)*ₙ,* wobei n 1, 2 oder 3 ist (SEQ ID NO: 177), wobei kursiv gedruckte Nukleinsäuren geschlossene Nukleinsäuren darstellen.

6. Verfahren nach einem der Ansprüche 1-5, wobei die eine oder die mehreren DNA-Mutationen in dem AFC-Gen AFC-Mutationen umfassen, die für die mutierten APC-Proteine Q1367*, R1450*, E1309 Frameshift, S1465 Frameshift und T1556 Frameshift kodieren; wobei optional die für eine oder mehrere DNA-Mutationen in dem *AFC-Gen* spezifischen Sonden fünf Sonden umfassen, die die Sequenzen ACTGCTGAAAAGAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38), und AGAGGCAGAAAAAAACT (SEQ ID NO:42) umfassen, und wobei jede der fünf Sonden an einen Mikroträger mit einem anderen Identifikator gekoppelt ist;
wobei optional jede der fünf Sonden ferner acht Nukleotide an dem 5'-Ende umfasst, wobei die acht Nukleotide an dem 5'-Ende Adenin- oder Thyminnukleotide sind, und wobei jede der fünf Sonden wenigstens 24 Gesamtnukleotide umfasst;
wobei die Sonden optional umfassen: (1) eine erste Sonde, umfassend eine Sequenz, die aus der Gruppe ausgewählt ist, bestehend aus AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228) und CAGAAATAAAAGATT (SEQ ID NO:229); (2) eine zweite Sonde, umfassend eine Sequenz, die aus der Gruppe ausgewählt ist, bestehend aus TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), und GGTGTCTAAGCACCA (SEQ ID NO:234); (3) eine dritte Sonde, umfassend eine Sequenz, die aus der Gruppe ausgewählt ist, bestehend aus AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237) und AGCTCAAACCAAGTGAG (SEQ ID NO:238); (4) eine vierte Sonde, umfassend eine Sequenz, die aus der Gruppe ausgewählt ist, bestehend aus GCACCTACTGCTGAA (SEQ ID NO:239), ACCTACTGCTGAAAAG (SEQ ID NO:240), TGCTGAAAAGAGAGT (SEQ ID NO:241), ACTGCTGAAAAGAGAGT (SEQ ID NO:26), und CCTACTGCTGAAAAGAGA (SEQ ID NO:242); und (5) eine fünfte Sonde, umfassend eine Sequenz, die aus der Gruppe ausgewählt ist, bestehend aus GCAGAAAAAAACTATTG (SEQ ID NO: 243), AGAGGCAGAAAAACT (SEQ ID NO: 42), CAGAAAAAAACTATTGATT (SEQ ID NO:244), AGAAAGAGGCAGAAAAACT (SEQ ID NO:245) und GAGGCAGAAAAAAACTA (SEQ ID NO:246); und wobei jede der fünf Sonden an einen Mikroträger mit einem anderen Identifikator gekoppelt ist;
wobei die Sonden optional umfassen: (1) eine erste Sonde, umfassend eine Sequenz, die aus der Gruppe ausgewählt ist, bestehend aus TTTTTTTTTTTTTGAAATAAAAGATTGG (SEQ ID NO:58), TTTTTTTTTTAAATAGCAGAAATAAAAG (SEQ ID NO:120), TTTTTTTTTTTGAAATAAAAGATTGGAA (SEQ ID NO: 121), TTTTTTTTTTTTTAGAAATAAAAGATTG (SEQ ID NO:122), TTTTTTTTTTTTTGAAATAAATGAATGG (SEQ ID NO:123), und TTTTTTTTTTTTTCAGAAATAAAAGATT (SEQ ID NO:124); (2) eine zweite Sonde, umfassend eine Sequenz, die aus der Gruppe ausgewählt ist, bestehend aus TTTTTTTTTTTTTGGGTGTCTAAG (SEQ ID NO:59), TTTTTTTTTATTTGGGTGTCTAAG (SEQ ID NO:125), TTTTTTGGGTGTCTAAGCACCACT (SEQ ID NO: 126), TTTTTTTTTCTAAGCACCACTTTT (SEQ ID NO: 127), TTTTTTTTTTTTTTGGGTGTCTAA (SEQ ID NO: 128), und TTTTTTTTTGGTGTCTAAGCACCA (SEQ ID NO:129); (3) eine dritte Sonde, umfassend eine Sequenz, die aus der Gruppe ausgewählt ist, bestehend aus TTTTTTTTTACAAACCAAGTGAGAA (SEQ ID NO:60), TTTTTTTTAAGTGAGAAGTACCTAA (SEQ ID NO: 130), TTTTTTTTTTTTCAAACCAAGTGAG (SEQ ID NO: 131), TTTTTTTTTTACCAAGTGAGAAGTA (SEQ ID NO: 132) und TTTTTTTTAGCTCAAACCAAGTGAG (SEQ ID NO: 133); (4) eine vierte Sonde, umfassend eine Sequenz, ausgewählt aus der Gruppe bestehend aus TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO: 135), TTTTTTTTTGCTGAAAAGAGAGAGT (SEQ ID NO: 136), und TTTTTTTTTCCTACTGCTGAAAAGAGA (SEQ ID NO: 137); und (5) eine fünfte Sonde, umfassend eine Sequenz, die aus der Gruppe ausgewählt ist, bestehend aus TTTTTTTTTTAGAGGCAGAAAAAAACT (SEQ ID NO:61), TTTTTTTTTTGCAGAAAAAAACTATTG (SEQ ID NO: 138), TTTTTTTTTTTCAGAAAAAAACTATTGATT (SEQ ID NO: 139), TTTTTTTAGAAAGAGGCAGAAAAAAACT (SEQ ID NO: 140), und TTTTTTTTTTTGAGGCAGAAAAAAACTA (SEQ ID NO: 141); und wobei jede der fünf Sonden an einen Mikroträger mit einem anderen Identifikator gekoppelt ist;
wobei optional Schritt (b) das Amplifizieren der isolierten DNA durch PCR unter Verwendung eines ersten Primer-Paars, das die Sequenzen TAAAAATAAAGCACCTACTGCTGAAA (SEQ ID NO:23) und AGCTTGCTTAGGTCCACTCTCTCT (SEQ ID NO:24); eines zweiten Primer-Paars, umfassend TAGGATGTAATCAGACGACACAGGA (SEQ ID NO:27) und CAGCTGACCTAGTTCCAATCTTTTA (SEQ ID NO:28); eines dritten Primer-Paars, umfassend die Sequenzen TCTCCCTCCAAAAGTGGTGCT (SEQ ID NO:31) und TGGCAATCGAACGACTCTCAA (SEQ ID NO:32); eines vierten Primer-Paars, umfassend GCAGAAGTAAAACACCTCCACCA (SEQ ID NO:35) und GGTGCTTTATTTTTAGGTACTTC (SEQ ID NO:36), wobei die kursiv gedruckten Nukleinsäuren geschlossene Nukleinsäuren darstellen; und eines fünften Primer-Paars, umfassend die Sequenzen CAGGAAAATGACAATGGGAATG (SEQ ID NO:39) und ATCTAATAGGTCCTTTCAGAATCAATAG (SEQ ID NO:40); und wobei optional Schritt (b) das Amplifizieren der isolierten DNA durch PCR in der Gegenwart einer ersten blockierenden Nukleinsäure, die die Sequenz *CCA*C*TC*TCT*C*TCT*TT*T*CAGC*(invdT)*ₙ,* wobei *n* 1, 2 oder 3 ist (SEQ ID NO:25), TA*GG*T*CC*ACTCTCTCTCT*TT*T*C*A*GC*A(invdT)n, wobei *n* 1, 2 oder 3 ist (SEQ ID NO:166), *TAGGTCCACTCTCTCTCTTTTCAGCA* (invdT)*ₙ*, wobei *n* 1, 2 oder 3 ist (SEQ ID NO:167), C*CA*C*T*C*T*C*T*C*TC*TTTT*C*AG*C*(invdT)*ₙ*, wobei *n 1,* 2 oder 3 ist (SEQ ID NO: 168), oder T*AGG*T*CCAC*T*CTCT*C*T*CTT*T*TC*AG*CA (invdT)*ₙ*, wobei *n* 1, 2 oder 3 ist (SEQ ID NO: 169), umfasst, einer zweiten blockierenden Nukleinsäure, die die Sequenz C*T*TT*TC*T*T*T*TATTTCTGC*(invdT)n, wobei *n* 1, 2 oder 3 ist (SEQ ID NO:29), *CTTTTC*T*TTTA*T*T*T*C*TG*C*(invdT)n*,* wobei *n* 1, 2 oder 3 ist (SEQ ID NO:154), C*T*TT*TC*T*T*T*TATTTCTGC*(invdT)*ₙ,* wobei *n* 1, 2 oder 3 ist (SEQ ID NO:155), C*T*TT*TC*T*T*T*TATTTCTGC*(invdT)n, wobei *n* 1, 2 oder 3 ist (SEQ ID NO: 156), oder C*T*TT*TC*T*T*T*TATTTCTGC*(invdT)n, wobei *n* 1, 2 oder 3 ist (SEQ ID NO:157), umfasst, einer dritten blockierenden Nukleinsäure, die die Sequenz GTG*CTCA*G*ACAC*C(invdT)*ₙ*, wobei *n* 1, 2 oder 3 ist (SEQ ID NO:33), GTG*CTCA*G*ACAC*C(invdT)*ₙ*, wobei *n* 1, 2 oder 3 ist (SEQ ID NO:158), A*G*T*G*GTG*CTCA*G*AC*A*C*C*C*A(invdT)n, wobei n 1, 2 oder 3 ist (SEQ ID NO:159), A*G*T*G*GTG*CTCA*G*AC*A*C*C*C*A(invdT)n, wobei *n* 1, 2 oder 3 ist (SEQ ID NO:160), oder A*G*T*G*GTG*CTCA*G*AC*A*C*C*C*A(invdT)n, wobei n 1, 2 oder 3 ist (SEQ ID NO:161), umfasst, einer vierten blockierenden Nukleinsäure, die die Sequenz C*TTC*T*CGCT*T*GGT*T(invdT)*ₙ,* wobei *n* 1, 2 oder 3 ist (SEQ ID NO:37), GTA*CT*T*CTCG*CT*TGG*T(invdT)n, wobei *n* 1, 2 oder 3 ist (SEQ ID NO: 162), C*TTC*T*CGCT*T*GGT*T(invdT)*ₙ,* wobei *n* 1, 2 oder 3 ist (SEQ ID NO: 163), GTA*CT*T*CTCG*CT*TGG*T(invdT)n, wobei *n* 1, 2 oder 3 ist (SEQ ID NO: 164), oder GTA*CT*T*CTCG*CT*TGG*T(invdT)n, wobei *n* 1, 2 oder 3 ist (SEQ ID NO: 165), umfasst, und einer fünften blockierenden Nukleinsäure, die die Sequenz CA*ATAG*T*TTTT*T*CTGC*C(invdT)*ₙ,* wobei *n* 1, 2 oder 3 ist (SEQ ID NO:41), GA*A*T*CAATAG*TTTTTT*CTGCCT*C(invdT)*ₙ,* wobei *n* 1, 2 oder 3 ist (SEQ ID NO: 170), *TCAGAATCAATAGTTTTTTCTG* (invdT)*ₙ*, wobei *n* 1, 2 oder 3 ist (SEQ ID NO: 171), *GAATCAATAGATTTTACTGCCTC* (invdT)*ₙ*, wobei *n* 1, 2 oder 3 ist (SEQ ID NO: 172), oder *AATCAATAGTTTTTTCTGCCTC* (invdT)*ₙ*, wobei *n* 1, 2 oder 3 ist (SEQ ID NO: 173), umfasst, wobei kursiv gedruckte Nukleinsäuren geschlossene Nukleinsäuren darstellen.

7. Verfahren nach einem der Ansprüche 1-6, wobei das Verfahren ferner umfasst:
Amplifizieren einer positiven Kontroll-DNA-Sequenz unter Verwendung eines für die positive Kontroll-DNA-Sequenz spezifischen Primer-Paares;
Hybridisieren der amplifizierten positiven Kontrollgensequenz mit einer für die positiven Kontrollgensequenz spezifischen Sonde, wobei die für die positive Kontrollgensequenz spezifische Sonde an einen Mikroträger mit einem einer positiven Kontrolle entsprechenden Identifikator gekoppelt ist;
Nachweisen des Vorhandenseins oder der Abwesenheit einer Hybridisierung der amplifizierten positiven Kontroll-DNA-Sequenz mit der für die positive Kontrollgensequenz spezifischen Sonde; und
Nachweisen des Identifikators, der der positiven Kontrolle entspricht wobei die positive Kontroll-DNA-Sequenz optional eine Sequenz eines Gens eines menschlichen Leukozyten-Antigens oder eines menschlichen Glyceraldehyd-3-Phosphat-Dehydrogenase(GAPDH)-Gens umfasst;
wobei optional das für die positive Kontroll-DNA-Sequenz spezifische Primer-Paar die Sequenzen TGAGTGTTACTTCTTCCCACACTC (SEQ ID NO:43) und ATTGCTTTGCGCAATCCCT (SEQ ID NO:44) oder AATCCCATCACCATCTTCCA (SEQ ID NO:71) und TGGACTCCACGACGTACTCA (SEQ ID NO:72) umfasst; und
wobei optional die für die positive Kontrollgensequenz spezifische Sonde die Sequenz TTTTTTTTTTTTGGAGACGGTCTG (SEQ ID NO:45) oder CTGTCTTCCACTCACTCC (SEQ ID NO:73) umfasst.

8. Verfahren nach einem der Ansprüche 1-7, wobei das Verfahren ferner umfasst:
Nachweisen der Abwesenheit der Hybridisierung der amplifizierten DNA mit einem Mikroträger, der
einen Identifikator aufweist, der einer negativen Kontrolle entspricht, wobei der Mikroträger mit dem Identifikator, der der negativen Kontrolle entspricht, eine Sonde umfasst, die nicht mit der amplifizierten DNA hybridisiert; und
Nachweisen des Identifikators, der der negativen Kontrolle entspricht;
wobei optional der Mikroträger mit dem Identifikator, der der negativen Kontrolle entspricht, eine Sonde umfasst, die die Sequenz AATATAATATATATTAT (SEQ ID NO:46) umfasst.

9. Verfahren nach einem der Ansprüche 1-8, wobei die Identifikatoren der Mikroträger (a) digitale Strichcodes oder (b) analoge Codes umfassen;
wobei optional jeder der Mikroträger umfasst:
(i) eine erste Photopolymerschicht; (ii) eine zweite Photopolymerschicht; und (iii) eine Zwischenschicht zwischen der ersten Schicht und der zweiten Schicht, wobei die Zwischenschicht ein kodiertes Muster aufweist, das den darauf definierten Identifikator darstellt, wobei die Zwischenschicht teilweise im Wesentlichen lichtdurchlässig und teilweise im Wesentlichen lichtundurchlässig ist und einen Code darstellt, der dem Mikroträger entspricht, wobei die äußerste Oberfläche des Mikroträgers ein Photoresist-Photopolymer umfasst und das Photoresist-Photopolymer mit der für die DNA-Mutation spezifischen Sonde funktionalisiert ist und wobei der Mikroträger etwa die gleiche Dichte wie Wasser aufweist; oder
(i) eine im Wesentlichen transparente Polymerschicht mit einer ersten Oberfläche und einer zweiten Oberfläche, wobei die erste und die zweite Oberfläche parallel zueinander sind; (ii) eine im Wesentlichen nichttransparente Polymerschicht, wobei die im Wesentlichen nichttransparente Polymerschicht an der ersten Oberfläche der im Wesentlichen transparenten Polymerschicht befestigt ist und einen mittleren Abschnitt der im Wesentlichen transparenten Polymerschicht umschließt, und wobei die im Wesentlichen nichttransparente Polymerschicht eine zweidimensionale Form umfasst, die einen analogen Code darstellt, wobei der analoge Code den Identifikator darstellt; und (iii) die für die DNA-Mutation spezifische Sonde, wobei die Sonde an wenigstens eine von der ersten Oberfläche und der zweiten Oberfläche der im Wesentlichen transparenten Polymerschicht in wenigstens dem mittleren Abschnitt der im Wesentlichen transparenten Polymerschicht gekoppelt ist, wobei optional jeder der Mikroträger ferner einen Orientierungsindikator zum Orientieren des analogen Codes der im Wesentlichen nichttransparenten Polymerschicht umfasst, wobei optional das im Wesentlichen transparente Polymer der ersten oder der zweiten im Wesentlichen transparenten Polymerschicht ein Polymer auf Epoxidharzbasis umfasst und wobei optional das Polymer auf Epoxidharzbasis SU-8 ist.

10. Verfahren nach einem der Ansprüche 1-9, wobei die Probe eine Stuhlprobe ist.

11. Verfahren nach einem der Ansprüche 1-10, wobei das Verfahren zum Nachweisen von Dickdarmkrebs, Enddarmkrebs, kolorektalem Krebs, Dickdarm-Adenom, Enddarm-Adenom oder kolorektalem Adenom verwendet wird.

12. Kit, umfassend:
(a) wenigstens vier Mikroträger, wobei jeder der wenigstens vier Mikroträger umfasst:
(i) eine an den Mikroträger gekoppelte Sonde, wobei die Sonde für eine DNA-Mutation in dem *KRAS-, BRAF-, CTNNBI-* oder *AFC-Gen* spezifisch ist, wobei die *KRAS-, BRAF-, CTNNBI-* und AFC-Gene optional menschliche Gene sind; und
(ii) einen Identifikator, der der daran gekoppelten Sonde entspricht;
wobei der Kit wenigstens einen Mikroträger, der eine für eine DNA-Mutation in dem *KRAS-*Gen spezifische Sonde umfasst, wenigstens einen Mikroträger, der eine für eine DNA-Mutation in dem *BRAF-Gen* spezifische Sonde umfasst, wenigstens einen Mikroträger, der eine für eine DNA-Mutation im *CTNNB1-*Gen spezifische Sonde umfasst, und wenigstens einen Mikroträger umfasst, der eine für eine DNA-Mutation in dem *AFC-Gen* spezifische Sonde umfasst; und
(b) wenigstens vier blockierenden Nukleinsäuren, wobei jede der wenigstens vier blockierenden Nukleinsäuren mit einem Wildtyp-DNA-Locus hybridisiert, der einer der DNA-Mutationen in den KRAS-, BRAF-, CTNNB1- oder APC-Genen entspricht.

13. Kit nach Anspruch 12, wobei jede der wenigstens vier blockierenden Nukleinsäuren ein einzelsträngiges Oligonukleotid umfasst, das mit dem entsprechenden Wildtyp-DNA-Locus hybridisiert; und einen 3'-terminalen Teil, der die Verlängerung des einzelsträngigen Oligonukleotids blockiert;
wobei optional der 3'-terminale Teil ein oder mehrere invertierte Desoxythymidine umfasst; und wobei optional jede der wenigstens vier blockierenden Nukleinsäuren ein oder mehrere modifizierte Nukleotide umfasst, die aus der Gruppe ausgewählt sind, die aus blockierten Nukleinsäuren (LNAs), Peptidnukleinsäuren (PNAs), Hexosenukleinsäuren (HNAs), Threosenukleinsäuren (TNAs), Glykolnukleinsäuren (GNAs) und Cyclohexenylnukleinsäuren (CeNAs) besteht.

14. Kit nach Anspruch 12 oder 13, wobei die DNA-Mutation in dem *KRAS-Gen KRAS-*Mutationen umfasst, die für G12D-, G12V-, G12S- und G13D-mutierte KRAS-Proteine kodieren;
wobei der Kit optional vier Sonden umfasst, die die Sequenzen GGAGCTGATGG (SEQ ID NO:4), GGAGCTGTTGG (SEQ ID NO:5), TGGAGCTAGTGG (SEQ ID NO:6) und TGGAGCTGGTGACGT (SEQ ID NO:7) umfassen, und
wobei jede der vier Sonden mit einem Mikroträger mit einem unterschiedlichen Identifikator gekoppelt ist; wobei optional jede der vier Sonden ferner acht Nukleotide an dem 5'-Ende umfasst, wobei die acht Nukleotide an dem 5'-Ende Adenin- oder Thyminnukleotide sind, und wobei jede der vier Sonden wenigstens 24 Gesamtnukleotide umfasst;
wobei der Kit optional (1) eine erste Sonde, die eine Sequenz umfasst, die aus der Gruppe ausgewählt ist, bestehend aus GGAGCTGATGG (SEQ ID NO:4), AGCTGATGGGCGTA (SEQ ID NO:178), TGGAGCTGATGGCG (SEQ ID NO:179), TGGAGCTGATGG (SEQ ID NO: 180) und GCTGATGGGCGTA (SEQ ID NO:181); (2) eine zweite Sonde, die eine Sequenz umfasst, die aus der Gruppe ausgewählt ist, bestehend aus GGAGCTGTTGG (SEQ ID NO:5), TGGAGCTGTTGGTGGC (SEQ ID NO:182), GGAGCTGTTGGTG (SEQ ID NO:183), TGGAGCTGTTGGT (SEQ ID NO:184), und TGGAGCTGTAGGTGG (SEQ ID NO:185); (3) eine dritte Sonde, die eine Sequenz umfasst, die aus der Gruppe ausgewählt ist, bestehend aus TTGGAGCTAGTGGCGTA (SEQ ID NO:186), GCTAGTGGCGTAGGC (SEQ ID NO:187), AGCTAGTGGCGT (SEQ ID NO:188), GTTGGAGCTAGTGG (SEQ ID NO:189) und GGAGCTAGTGG (SEQ ID NO:190); und (4) eine vierte Sonde umfasst, die eine Sequenz umfasst, die aus der Gruppe ausgewählt ist, die aus einer vierten Sonde besteht, die eine Sequenz umfasst, die aus der Gruppe ausgewählt ist, die aus GGTGACGTAGGGCAA (SEQ ID NO:191), TGACGTAGGCAAGAG (SEQ ID NO: 192), GCTGGTGACGTAGG (SEQ ID NO:193), AGCTGGTGACGTAG (SEQ ID NO:194) und GGAGCTGGTGACGT (SEQ ID NO: 195) besteht; und wobei jede der vier Sonden mit einem Mikroträger mit einem unterschiedlichen Identifikator gekoppelt ist;
wobei optional der Kit (1) eine erste Sonde, die eine Sequenz umfasst, die aus der Gruppe ausgewählt ist, bestehend aus TTTTTTTTTTTTAAGGAGCTGATGG (SEQ ID NO:47), TTTTTTTTTTTTAGCTGATGGCGTA (SEQ ID NO:74), TTTTTTTTTTATGGAGCTGATGGCG (SEQ ID NO:75), TTTTTTTTTTTTATGGAGCTGATGG (SEQ ID NO:76), und TTTTTTTTTTTTTGCTGATGGCGTA (SEQ ID NO:77); (2) eine zweite Sonde, die eine Sequenz umfasst, die aus der Gruppe ausgewählt ist, bestehend aus TTTTTTTTTTTTAAGGAGCTGTTGG (SEQ ID NO:48), TTTTTTTTATGGAGCTGTTGGTGGC (SEQ ID NO:78), TTTTTTTTTTAAGGAGCTGTTGGTG (SEQ ID NO:79), TTTTTTTTTTTATGGAGCTGTTGGT (SEQ ID NO:80), und TTTTTTTTTATGGAGCTGTAGGTGG (SEQ ID NO:81); (3) eine dritte Sonde, umfassend eine Sequenz, die aus der Gruppe ausgewählt ist, bestehend aus TTTTTTTTTTTATGGAGCTAGTGG (SEQ ID NO:49), TTTTTTTTTTGGAGCTAGTGGCGTA (SEQ ID NO:82), TTTTTAATTTGCTAGTGGCGTAGGC (SEQ ID NO:83), TTTTTTTTTATTTAGCTAGTGGCGT (SEQ ID NO:84), TTTTTTTTTTTGTTGGAGCTAGTGG (SEQ ID NO:85), und TTTTTTTTTTTTAAGGAGCTAGTGG (SEQ ID NO:86); und (4) eine vierte Sonde, umfassend eine Sequenz, die aus der Gruppe ausgewählt ist, bestehend aus TTTTTTTTTATGGAGCTGGTGACGT (SEQ ID NO:50), TTTTTTTTAAAGGTGACGTAGGCAA (SEQ ID NO:87), TTTTTTTTTATGACGTAGGCAAGAG (SEQ ID NO:88), TTTTTTTTTTTGCTGGTGACGTAGG (SEQ ID NO:89), TTTTTTTTTTAAGCTGGTGACGTAG (SEQ ID NO:90) und TTTTTTTTTAAGGAGCTGGTGACGT (SEQ ID NO:91); und wobei jede der vier Sonden an einen Mikroträger mit einem anderen Identifikator gekoppelt ist;
wobei optional der Kit ferner ein Primer-Paar umfasst, das die Sequenzen GTACTGGTGGAGTATTTGATAGTG (SEQ ID NO:1) und ATCGTCAAGGCACTCTTGCCTAC (SEQ ID NO:2) umfasst; und
wobei optional der Kit ferner eine blockierende Nukleinsäure umfasst, die die Sequenz TA*C*G*CC*A*CC*A*G*CT(invdT)*ₙ*, wobei n 1, 2 oder 3 ist (SEQ ID NO:3), TT*GG*A*G*CT*GGTGGC*GTA(invdT)*ₙ*, wobei n 1, 2 oder 3 ist (SEQ ID NO: 142), GCT*GG*T*GG*C*G*TA*G*G*C*A(invdT)*ₙ*, wobei n 1, 2 oder 3 ist (SEQ ID NO:143), *GCTGGTGGCGTA*GGC(invdT)*ₙ,* wobei *n* 1, 2 oder 3 ist (SEQ ID NO: 144), oder TT*GG*A*G*CT*GG*T*GG*C*G*T(invdT)*ₙ*, wobei n 1, 2 oder 3 ist (SEQ ID NO:145), wobei kursiv gedruckte Nukleinsäuren geschlossene Nukleinsäuren darstellen.

15. Kit nach einem der Ansprüche 12-14, wobei die DNA-Mutationen in dem *BRAF-Gen* zwei oder mehr BRAF-Mutationen umfassen, die für ein V600E-mutiertes BRAF-Protein kodieren; wobei das Kit optional zwei Sonden umfasst, die die Sequenzen TCTAGCTACAGAGAAAT (SEQ ID NO: 11) und GTCTAGCTACAGAAAAAT (SEQ ID NO: 12) umfassen, und wobei jede der zwei Sonden an einen Mikroträger mit einem anderen Identifikator gekoppelt ist;
wobei optional jede der zwei Sonden ferner acht Nukleotide an dem 5'-Ende umfasst, wobei die acht Nukleotide an dem 5'-Ende Adenin- oder Thyminnukleotide sind, und wobei jede der zwei Sonden wenigstens 24 Gesamtnukleotide umfasst;
wobei optional der Kit (1) eine erste Sonde, umfassend eine Sequenz, die aus der Gruppe ausgewählt ist, bestehend aus TACAGAGAAATCTCGAT (SEQ ID NO: 196), TACAGAGAAATCTC (SEQ ID NO:197), CTAGCTACAGAGAAAT (SEQ ID NO:198), CTAGCTACAGAGAAA (SEQ ID NO: 199) und TCTAGCTACAGAG (SEQ ID NO:200); und (2) eine zweite Sonde umfasst, umfassend eine Sequenz, die aus der Gruppe ausgewählt ist, bestehend aus GTCTAGCTACAGAAAAATC (SEQ ID NO:201), GTCTAGCTACAGAAAAAT (SEQ ID NO:12), TAGCTACAGAAAAA (SEQ ID NO:202), TCTAGCTACAGAAAAAT (SEQ ID NO:203) und TCTAGCTACAGAAAAATC (SEQ ID NO:204); und wobei jede der zwei Sonden an einen Mikroträger mit einem anderen Identifikator gekoppelt ist;
wobei der Kit optional (1) eine erste Sonde, die eine Sequenz umfasst, die aus der Gruppe ausgewählt ist, bestehend aus TTTTTTTAATTTCTAGCTACAGAGAAAT (SEQ ID NO:51), TTTTTTTTTATACAGAGAAATCTCGAT (SEQ ID NO:92), TTTTTTTTTTAATTTACAGAGAAATCTC (SEQ ID NO:93), TTTTTTAATTACTAGCTACAGAGAAAT (SEQ ID NO:94), TTTTTTTAATTACTAGCTACAGAGAAA (SEQ ID NO:95) und TTTTTTTTTTAATTTCTAGCTACAGAGAG (SEQ ID NO:96); und (2) eine zweite Sonde, die eine Sequenz umfasst, die aus der Gruppe ausgewählt ist, bestehend aus TTTTTTTATGTCTAGCTACAGAAAAAT (SEQ ID NO:52), TTTTTTTCTAGCTACAGAAATC (SEQ ID NO:97), TTTTTTTATTTTTTTAGCTACAGAAAAA (SEQ ID NO:98), TTTTTTTATTTCTAGCTACAGAAAAAT (SEQ ID NO:99) und TTTTTTTTATTCTAGCTACAGAAAAATC (SEQ ID NO: 100); und wobei jede der zwei Sonden an einen Mikroträger mit einem anderen Identifikator gekoppelt ist;
wobei optional der Kit ferner ein Primer-Paar umfasst, das die Sequenzen GGACCCACTCCATCGAGATTT (SEQ ID NO:8) and CAGATATATTTCTTCATGAAGACCTCACAGTAA (SEQ ID NO:9) umfasst; und
wobei optional der Kit ferner eine blockierende Nukleinsäure umfasst, die die Sequenz GA*G*AT*TTCACTGT*AGC(invdT)*ₙ*, wobei *n* 1, 2 oder 3 ist (SEQ ID NO: 10), GA*G*AT*TTCACTGT*AGC(invdT*)ₙ,* wobei *n* 1, 2 oder 3 ist (SEQ ID NO:146), GA*G*AT*TTCACTGT*AGC(invdT)*ₙ*, wobei *n* 1, 2 oder 3 ist (SEQ ID NO:147), GA*G*AT*TTCACTGT*AGC(invdT)*ₙ*, wobei n 1, 2 oder 3 ist (SEQ ID NO:148), oder GA*G*AT*TTCACTGT*AGC(invdT)*ₙ*, wobei *n* 1, 2 oder 3 ist (SEQ ID NO:149), wobei kursiv gedruckte Nukleinsäuren geschlossene Nukleinsäuren darstellen.

16. Kit nach einem der Ansprüche 12-15, wobei die DNA-Mutation in dem *CTNNBI-Gen* CTNNBI-Mutationen umfasst, die für T41A-, T411-, S45F- und S45P-mutierte CTNNB1-Proteine kodieren;
wobei der Kit optional vier Sonden umfasst, die die Sequenzen AGGAGCTGTGGCAG (SEQ ID NO: 16), GGAGCTGTGATA (SEQ ID NO: 17), TTTACCACTCAGAAAAG (SEQ ID NO:21) und TACCACTCAGAGGAG (SEQ ID NO:22) umfassen, und wobei jede der vier Sonden an einen Mikroträger mit einem anderen Identifikator gekoppelt ist;
wobei optional jede der vier Sonden ferner acht Nukleotide an dem 5'-Ende umfasst, wobei die acht Nukleotide an dem 5'-Ende Adenin- oder Thyminnukleotide sind, und wobei jede der vier Sonden wenigstens 24 Gesamtnukleotide umfasst;
wobei optional der Kit (1) eine erste Sonde, umfassend eine Sequenz, ausgewählt aus der Gruppe bestehend aus AGGAGCTGTGGCAGT (SEQ ID NO:205), AGGAGCTGTGGCAGTG (SEQ ID NO:206), GCTGTGGCAGTGGC (SEQ ID NO:207), GCTGTGGCAGTGGCA (SEQ ID NO:208), und AAGGAGCTGTGGCAG (SEQ ID NO:209); (2) eine zweite Sonde, umfassend eine Sequenz, ausgewählt aus der Gruppe bestehend aus GGAGCTGTGATAGTGG (SEQ ID NO:210), GAGCTGTGATAGTGGC (SEQ ID NO:211), AGCTGTGATAGTGGCA (SEQ ID NO:212), AGAAGGAGCTGTGATA (SEQ ID NO:213), und GGAGCTGTGAT (SEQ ID NO:214); (3) eine dritte Sonde, umfassend eine Sequenz, ausgewählt aus der Gruppe, bestehend aus ACTCAGAAAAGGAGCT (SEQ ID NO:215), TACCACTCAGAAAAGGA (SEQ ID NO:216), TTTACCACTCAGAAAAGGAG (SEQ ID NO:217), TTACCACTCAGAAAAG (SEQ ID NO:218), und CAGAAAAGGAGCTGTG (SEQ ID NO:219); und (4) eine vierte Sonde umfasst, umfassend eine Sequenz, ausgewählt aus der Gruppe, bestehend aus ACTCAGAGGAGGAGC (SEQ ID NO:220), TTACCACTCAGAGGA (SEQ ID NO:221), TTACCACTCAGAGGAGG (SEQ ID NO:222), TTAACACTCAGAGGAG (SEQ ID NO:223), und TTACCAATCAGAGGAGG (SEQ ID NO:224); wobei jede der vier Sonden an einen Mikroträger mit einem anderen Identifikator gekoppelt ist;
wobei optional der Kit (1) eine erste Sonde, umfassend eine Sequenz, ausgewählt aus der Gruppe bestehend aus TTTTTTTTTTTAGGAGCTGTGGCAG (SEQ ID NO:53), TTTTTTTTTTTAGGAGCTGTGGCAGTG (SEQ ID NO: 101), TTTTTTTTTTTAGCTGTGGCAGTGGC (SEQ ID NO: 102), TTTTTTTTTTTGCTGTGGCAGTGGCA (SEQ ID NO: 103), und TTTTTTTTTTAAGGAGCTGTGGCAG (SEQ ID NO: 104); (2) eine zweite Sonde, umfassend eine Sequenz, ausgewählt aus der Gruppe bestehend aus TTTTTTTTTTTTTGGAGCTGTGATA (SEQ ID NO:54), TTTTTTTTTGGAGCTGTGATAGTGG (SEQ ID NO: 105), TTTTTTTTTGAGCTGTGATAGTGGC (SEQ ID NO: 106), TTTTTTTTTAGCTGTGATAGTGGCA (SEQ ID NO: 107), TTTTTTTTTAGAAGGAGCTGTGATA (SEQ ID NO: 108), und TTTTTTTTTTTTTTGGAGCTGTGAT (SEQ ID NO:109), (3) eine dritte Sonde, umfassend eine Sequenz, ausgewählt aus der Gruppe, bestehend aus TTTTTTTTTTTACCACTCAGAAAAG (SEQ ID NO:55), TTTAATTTTACTCAGAAAAGGAGCT (SEQ ID NO:110), TTTTTTAATACCACTCAGAAAAGGA (SEQ ID NO: 111), TTTTTTTTACCACTCAGAAAAGGAG (SEQ ID NO: 112), TTTTTTTTATTACCACTCAGAAAAG (SEQ ID NO: 113), und TTTTTTTTTCAGAAAAGGAGCTGTG (SEQ ID NO:114); und (4) eine vierte Sonde umfasst, umfassend eine Sequenz, ausgewählt aus der Gruppe, bestehend aus TTTTTTTTTAATACCACTCAGAGGAG (SEQ ID NO:56), TTTTTTTTTAAAACTCAGAGGAGGAGC (SEQ ID NO: 115), TTTTTTTTTTTATTACCACTCAGAGGA (SEQ ID NO: 116), TTTTTTTTTATTACCACTCAGAGGAGG (SEQ ID NO:117), TTTTTTTTTTATTAACACTCAGAGGAG (SEQ ID NO:118), und TTTTTTTTTATTACCAATCAGAGGAGG (SEQ ID NO:119); wobei jede der vier Sonden an einen Mikroträger mit einem anderen Identifikator gekoppelt ist;
wobei optional der Kit ein erstes Primer-Paar, das die Sequenzen GGAATCCATTCTGGTGCCACT (SEQ ID NO: 13) und AGAAAATCCCTGTTCCCACTCATA (SEQ ID NO: 14) umfasst, und eines zweiten Primer-Paars umfasst, das die Sequenzen GGTGCCACTACCACAGCTCCT (SEQ ID NO:18) und TCTCAAAACTGCATTCTGACTTTCA (SEQ ID NO: 19) umfasst; und
wobei optional der Kit ferner eine erste blockierende Nukleinsäure, die die Sequenz GC*CACTACCACAG*CT(invdT)*ₙ,* wobei *n* 1, 2 oder 3 ist (SEQ ID NO: 15), T*G*C*CA*CT*ACCA*C*AG*(invdT)*ₙ,* wobei *n* 1, 2 oder 3 ist (SEQ ID NO:150), C*AC*T*ACCA*C*AGC*T*C*C(invdT)*ₙ,* wobei *n* 1, 2 oder 3 ist (SEQ ID NO:151), GC*CACTACCACAG*CT(invdT)*ₙ,* wobei *n* 1, 2 oder 3 ist (SEQ ID NO:152), oder GC*CACTACCACAG*CT(invdT)*ₙ,* wobei *n* 1, 2 oder 3 ist (SEQ ID NO: 153) umfasst, und einer zweiten blockierenden Nukleinsäure umfasst, die die Sequenz GC*TCCTTCTCTG*AGT(invdT)*ₙ,* wobei *n* 1, 2 oder 3 ist (SEQ ID NO:20), T*CC*T*TCTC*T*GAG*T*G*G(invdT)*ₙ,* wobei *n* 1, 2 oder 3 ist (SEQ ID NO: 174), GC*TCCTTCTCTG*AGT(invdT)*ₙ,* wobei n 1, 2 oder 3 ist (SEQ ID NO: 175), T*CC*T*TCTC*T*GAG*T*G*G(invdT)*ₙ,* wobei n 1, 2 oder 3 ist (SEQ ID NO: 176), oder GC*TCCTTCTCTG*AGT(invdT)*ₙ,* wobei n 1, 2 oder 3 ist (SEQ ID NO: 177), wobei kursiv gedruckte Nukleinsäuren geschlossene Nukleinsäuren darstellen.

17. Kit nach einem der Ansprüche 12-16, wobei die DNA-Mutation in dem *AFC-Gen* AFC-Mutationen umfasst, die für die mutierten APC-Proteine Q1367*, R1450*, E1309 Frameshift, S1465 Frameshift und T1556 Frameshift kodieren;
wobei der Kit optional fünf Sonden umfasst, die die Sequenzen ACTGCTGAAAAGAGAGT (SEQ ID NO:26), GAAATAAAAGATTGG (SEQ ID NO:30), TTTTGGGTGTCTAAG (SEQ ID NO:34), CAAACCAAGTGAGAA (SEQ ID NO:38) und AGAGGCAGAAAAACT (SEQ ID NO:42) umfassen, und wobei jede der fünf Sonden an einen Mikroträger mit einem anderen Identifikator gekoppelt ist;
wobei optional jede der fünf Sonden ferner acht Nukleotide an dem 5'-Ende umfasst, wobei die acht Nukleotide an dem 5'-Ende Adenin- oder Thyminnukleotide sind, und wobei jede der fünf Sonden wenigstens 24 Gesamtnukleotide umfasst;
wobei optional der Kit (1) eine erste Sonde, umfassend eine Sequenz, die aus der Gruppe ausgewählt ist, bestehend aus AAATAGCAGAAATAAAAG (SEQ ID NO:225), GAAATAAAAGATTGGAA (SEQ ID NO:226), AGAAATAAAAGATTG (SEQ ID NO:227), GAAATAAATGAATGG (SEQ ID NO:228) und CAGAAATAAAAGATT (SEQ ID NO:229); (2) eine zweite Sonde, umfassend eine Sequenz, die aus der Gruppe ausgewählt ist, bestehend aus TTTGGGTGTCTAAG (SEQ ID NO:230), GGGTGTCTAAGCACCACT (SEQ ID NO:231), CTAAGCACCACTTTT (SEQ ID NO:232), TTTTGGGTGTCTAA (SEQ ID NO:233), und GGTGTCTAAGCACCA (SEQ ID NO:234); (3) eine dritte Sonde, umfassend eine Sequenz, die aus der Gruppe ausgewählt ist, bestehend aus AAGTGAGAAGTACCTAA (SEQ ID NO:235), CAAACCAAGTGAGAA (SEQ ID NO:38), TCAAACCAAGTGAG (SEQ ID NO:236), ACCAAGTGAGAAGTA (SEQ ID NO:237) und AGCTCAAACCAAGTGAG (SEQ ID NO:238); (4) eine vierte Sonde, umfassend eine Sequenz, die aus der Gruppe ausgewählt ist, bestehend aus GCACCTACTGCTGAA (SEQ ID NO:239), ACCTACTGCTGAAAAG (SEQ ID NO:240), TGCTGAAAAGAGAGT (SEQ ID NO:241), ACTGCTGAAAAGAGAGT (SEQ ID NO:26), und CCTACTGCTGAAAAGAGA (SEQ ID NO:242); und (5) eine fünfte Sonde, umfassend eine Sequenz, die aus der Gruppe ausgewählt ist, bestehend aus GCAGAAAAAAACTATTG (SEQ ID NO: 243), AGAGGCAGAAAAACT (SEQ ID NO: 42), CAGAAAAAAACTATTGATT (SEQ ID NO:244), AGAAAGAGGCAGAAAAACT (SEQ ID NO:245) und GAGGCAGAAAAAAACTA (SEQ ID NO:246); und wobei jede der fünf Sonden an einen Mikroträger mit einem anderen Identifikator gekoppelt ist;
wobei der Kit optional umfasst: (1) eine erste Sone, umfassend eine Sequenz, die aus der Gruppe ausgewählt ist, bestehend aus TTTTTTTTTTTTTGAAATAAAAGATTGG (SEQ ID NO:58), TTTTTTTTTTAAATAGCAGAAATAAAAG (SEQ ID NO:120), TTTTTTTTTTTGAAATAAAAGATTGGAA (SEQ ID NO: 121), TTTTTTTTTTTTTAGAAATAAAAGATTG (SEQ ID NO:122), TTTTTTTTTTTTTGAAATAAATGAATGG (SEQ ID NO:123), und TTTTTTTTTTTTTCAGAAATAAAAGATT (SEQ ID NO:124); (2) eine zweite Sonde, umfassend eine Sequenz, die aus der Gruppe ausgewählt ist, bestehend aus TTTTTTTTTTTTTGGGTGTCTAAG (SEQ ID NO:59), TTTTTTTTTATTTGGGTGTCTAAG (SEQ ID NO:125), TTTTTTGGGTGTCTAAGCACCACT (SEQ ID NO:126), TTTTTTTTTCTAAGCACCACTTTT (SEQ ID NO:127), TTTTTTTTTTTTTTGGGTGTCTAA (SEQ ID NO:128), und TTTTTTTTTGGTGTCTAAGCACCA (SEQ ID NO:129); (3) eine dritte Sonde, umfassend eine Sequenz, die aus der Gruppe ausgewählt ist, bestehend aus TTTTTTTTTACAAACCAAGTGAGAA (SEQ ID NO:60), TTTTTTTTAAGTGAGAAGTACCTAA (SEQ ID NO:130), TTTTTTTTTTTTCAAACCAAGTGAG (SEQ ID NO: 131), TTTTTTTTTTACCAAGTGAGAAGTA (SEQ ID NO:132) und TTTTTTTTAGCTCAAACCAAGTGAG (SEQ ID NO:133); (4) eine vierte Sonde, umfassend eine Sequenz, ausgewählt aus der Gruppe bestehend aus TTTTTTTTACTGCTGAAAAGAGAGAGT (SEQ ID NO:57), TTTTTTTTTTGCACCTACTGCTGAA (SEQ ID NO: 134), TTTTTTTTTACCTACTGCTGAAAAG (SEQ ID NO: 135), TTTTTTTTTGCTGAAAAGAGAGAGT (SEQ ID NO:136), und TTTTTTTTTCCTACTGCTGAAAAGAGA (SEQ ID NO:137); und (5) eine fünfte Sonde, umfassend eine Sequenz, die aus der Gruppe ausgewählt ist, bestehend aus TTTTTTTTTTAGAGGCAGAAAAAAACT (SEQ ID NO:61), TTTTTTTTTTGCAGAAAAAAACTATTG (SEQ ID NO: 138), TTTTTTTTTTTCAGAAAAAAACTATTGATT (SEQ ID NO:139), TTTTTTTAGAAAGAGGCAGAAAAAAACT (SEQ ID NO: 140), und TTTTTTTTTTTGAGGCAGAAAAAAACTA (SEQ ID NO: 141); und wobei jede der fünf Sonden an einen Mikroträger mit einem anderen Identifikator gekoppelt ist;
wobei der Kit optional ferner ein erstes Primer-Paar, das die Sequenzen TAAAAATAAAGCACCTACTGCTGAAA (SEQ ID NO:23) und AGCTTGCTTAGGTCCACTCTCT (SEQ ID NO:24) umfasst; ein zweites Primer-Paar, das die Sequenzen TAGGATGTAATCAGACGACACAGGA (SEQ ID NO:27) und CAGCTGACCTAGTTCCAATCTTTTA (SEQ ID NO:28) umfasst; ein drittes Primer-Paar, das die Sequenzen TCTCCCTCCAAAAGTGGTGCT (SEQ ID NO:31) und TGGCAATCGAACGACTCTCAA (SEQ ID NO:32) umfasst; ein viertes Primer-Paar, das die Sequenzen GCAGAAGTAAAACACCTCCACCA (SEQ ID NO:35) und GGTGCTTTATTTTTAGGTACTTC (SEQ ID NO:36) umfasst, wobei die kursiv gedruckten Nukleinsäuren verschlossene Nukleinsäuren darstellen; und ein fünftes Primer-Paar umfasst, das die Sequenzen CAGGAAAATGACAATGGGAATG (SEQ ID NO:39) und ATCTAATAGGTCCTTTTCAGAATCAATAG (SEQ ID NO:40) umfasst; und
wobei optional der Kit ferner eine erste blockierende Nukleinsäure, die die Sequenz *CCA*C*TC*TCT*C*TCT*TT*T*CAGC*(invdT)*ₙ,* wobei *n* 1, 2 oder 3 ist (SEQ ID NO:25), TA*GG*T*CC*ACTCTCTCTCT*TT*T*C*A*GC*A(invdT)n, wobei n 1, 2 oder 3 ist (SEQ ID NO: 166), T*AGG*T*CCAC*T*CTCT*C*T*CTT*T*TC*AG*CA (invdT)*ₙ*, wobei *n* 1, 2 oder 3 ist (SEQ ID NO:167), C*CA*C*T*C*T*C*T*C*TC*TTTT*C*AG*C*(invdT)*ₙ*, wobei n 1, 2 oder 3 ist (SEQ ID NO:168), oder T*AGG*T*CCAC*T*CTCT*C*T*CTT*T*TC*AG*CA (invdT)*ₙ*, wobei n 1, 2 oder 3 ist (SEQ ID NO: 169), umfasst, eine zweite blockierende Nukleinsäure, die die Sequenz C*T*TT*TC*T*T*T*TATTTCTGC*(invdT)n, wobei *n* 1, 2 oder 3 ist (SEQ ID NO:29), C*T*TT*TC*T*T*T*TATTTCTGC*(invdT)n, wobei *n* 1, 2 oder 3 ist (SEQ ID NO:154), C*T*TT*TC*T*T*T*TATTTCTGC*(invdT)n, wobei *n* 1, 2 oder 3 ist (SEQ ID NO:155), C*T*TT*TC*T*T*T*TATTTCTGC*(invdT)n, wobei *n* 1, 2 oder 3 ist (SEQ ID NO: 156), oder C*T*TT*TC*T*T*T*TATTTCTGC*(invdT)n, wobei *n* 1, 2 oder 3 ist (SEQ ID NO:157), umfasst, eine dritte blockierende Nukleinsäure, die die Sequenz GTG*CTCA*G*ACAC*C(invdT)*ₙ*, wobei *n 1,* 2 oder 3 ist (SEQ ID NO:33), GTG*CTCA*G*ACAC*C(invdT)*ₙ*, wobei *n* 1, 2 oder 3 ist (SEQ ID NO:158), A*G*T*G*GTG*CTCA*G*AC*A*C*C*C*A(invdT)n, wobei n 1, 2 oder 3 ist (SEQ ID NO:159), A*G*T*G*GTG*CTCA*G*AC*A*C*C*C*A(invdT)n, wobei *n* 1, 2 oder 3 ist (SEQ ID NO:160), oder A*G*T*G*GTGC*TCA*G*AC*A*C*C*C*A(invdT)*ₙ*, wobei n 1, 2 oder 3 ist (SEQ ID NO: 161), umfasst, eine vierte blockierende Nukleinsäure, die die Sequenz C*TTC*T*CGCT*T*GGT*T(invdT)n*,* wobei *n* 1, 2 oder 3 ist (SEQ ID NO:37), GTA*CT*T*CTCG*CT*TGG*T(invdT)n, wobei *n* 1, 2 oder 3 ist (SEQ ID NO: 162), C*TTC*T*CGCT*T*GGT*T(invdT)*ₙ,* wobei *n* 1, 2 oder 3 ist (SEQ ID NO: 163), GT*ACT*T*CTCG*CT*TGG*T(invdT)*ₙ,* wobei *n* 1, 2 oder 3 ist (SEQ ID NO: 164), oder GTA*CT*T*CTCG*CT*TGG*T(invdT)n, wobei *n* 1, 2 oder 3 ist (SEQ ID NO: 165), umfasst, und eine fünfte blockierende Nukleinsäure umfasst, die die Sequenz CA*ATAG*T*TTTT*T*CTGC*C(invdT)*ₙ,* wobei *n* 1, 2 oder 3 ist (SEQ ID NO:41), GA*A*T*CAATAG*TTTTTT*CTGCCT*C(invdT)*ₙ,* wobei *n* 1, 2 oder 3 ist (SEQ ID NO: 170), *TCAGAATCAATAGTTTTTTCTG* (invdT)*ₙ*, wobei *n* 1, 2 oder 3 ist (SEQ ID NO: 171), *G*A*A*T*CAATAG*ATTTTA*CTGCCTC* (invdT)*ₙ*, wobei *n* 1, 2 oder 3 ist (SEQ ID NO: 172), oder A*A*T*CAATAG*TTTTTTC*TGCCTC* (invdT)*ₙ*, wobei *n* 1, 2 oder 3 ist (SEQ ID NO: 173), umfasst, wobei kursiv gedruckte Nukleinsäuren geschlossene Nukleinsäuren darstellen.

18. Kit nach einem der Ansprüche 12-17, ferner umfassend einen Mikroträger mit einem Identifikator, der einer positiven Kontrolle entspricht und an den eine für eine positive Kontroll-Gensequenz spezifische Sonde gekoppelt ist, und ein für die positive Kontroll-DNA-Sequenz spezifisches Primer-Paar;
wobei die positive Kontroll-DNA-Sequenz optional eine Sequenz eines Gens eines menschlichen Leukozyten-Antigens oder eines menschlichen Glyceraldehyd-3-Phosphat-Dehydrogenase(GAPDH)-Gens umfasst;
wobei optional das für die positive Kontroll-DNA-Sequenz spezifische Primer-Paar die Sequenzen TGAGTGTTACTTCTTCCCACACTC (SEQ ID NO:43) und ATTGCTTTGCGCAATCCCT (SEQ ID NO:44) oder AATCCCATCACCATCTTCCA (SEQ ID NO:71) und TGGACTCCACGACGTACTCA (SEQ ID NO:72) umfasst; und
wobei optional die für die positive Kontrollgensequenz spezifische Sonde die Sequenz TTTTTTTTTTTTGGAGACGGTCTG (SEQ ID NO:45) oder CTGTCTTCCACTCACTCC (SEQ ID NO:73) umfasst;
wobei optional der Kit ferner einen Mikroträger mit einem Identifikator umfasst, der einer negativen Kontrolle entspricht und an die eine Sonde gekoppelt ist, die nicht mit der amplifizierten DNA hybridisiert, und wobei optional der Mikroträger mit dem Identifikator, der der negativen Kontrolle entspricht, eine Sonde umfasst, die die Sequenz AATATAATATATATTAT (SEQ ID NO:46) umfasst.

19. Kit nach einem der Ansprüche 12-18, wobei die Identifikatoren der Mikroträger (a) digitale Strichcodes oder (b) analoge Codes umfassen;
wobei optional jeder der Mikroträger umfasst:
(i) eine erste Photopolymerschicht; (ii) eine zweite Photopolymerschicht; und (iii) eine Zwischenschicht zwischen der ersten Schicht und der zweiten Schicht, wobei die Zwischenschicht ein kodiertes Muster aufweist, das den darauf definierten Identifikator darstellt, wobei die Zwischenschicht teilweise im Wesentlichen lichtdurchlässig und teilweise im Wesentlichen lichtundurchlässig ist und einen Code darstellt, der dem Mikroträger entspricht, wobei die äußerste Oberfläche des Mikroträgers ein Photoresist-Photopolymer umfasst und das Photoresist-Photopolymer mit der für die DNA-Mutation spezifischen Sonde funktionalisiert ist und wobei der Mikroträger etwa die gleiche Dichte wie Wasser aufweist; oder
(i) eine im Wesentlichen transparente Polymerschicht mit einer ersten Oberfläche und einer zweiten Oberfläche, wobei die erste und die zweite Oberfläche parallel zueinander sind; (ii) eine im Wesentlichen nichttransparente Polymerschicht, wobei die im Wesentlichen nichttransparente Polymerschicht an der ersten Oberfläche der im Wesentlichen transparenten Polymerschicht befestigt ist und einen mittleren Abschnitt der im Wesentlichen transparenten Polymerschicht umschließt, und wobei die im Wesentlichen nichttransparente Polymerschicht eine zweidimensionale Form umfasst, die einen analogen Code darstellt, wobei der analoge Code den Identifikator darstellt; und (iii) die für die DNA-Mutation spezifische Sonde, wobei die Sonde an wenigstens eine von der ersten Oberfläche und der zweiten Oberfläche der im Wesentlichen transparenten Polymerschicht in wenigstens dem mittleren Abschnitt der im Wesentlichen transparenten Polymerschicht gekoppelt ist, wobei optional jeder der Mikroträger ferner einen Orientierungsindikator zum Orientieren des analogen Codes der im Wesentlichen nichttransparenten Polymerschicht umfasst, wobei optional das im Wesentlichen transparente Polymer der ersten oder der zweiten im Wesentlichen transparenten Polymerschicht ein Polymer auf Epoxidharzbasis umfasst und wobei optional das Polymer auf Epoxidharzbasis SU-8 ist.

## Revendications

1. Procédé de détection de la présence de mutations d'ADN dans les gènes *KRAS, BRAF, CTNNB1,* et *APC,* le procédé comprenant :
(a) l'isolement d'ADN à partir d'un échantillon ;
(b) l'amplification de l'ADN isolé par réaction en chaîne par polymérase (PCR) à l'aide de paires d'amorces spécifiques pour les loci d'une ou de plusieurs mutations d'ADN dans chacun des gènes *KRAS, BRAF, CTNNB1, et APC,* dans lequel l'ADN isolé est amplifié en présence d'au moins quatre acides nucléiques de blocage, dans lequel chacun desdits au moins quatre acides nucléiques de blocage s'hybride avec un locus d'ADN de type sauvage correspondant à l'une des mutations d'ADN dans les gènes KRAS, BRAF, CTNNB1, ou APC et empêche l'amplification du locus d'ADN de type sauvage, dans lequel éventuellement les gènes *KRAS, BRAF, CTNNB1, et APC* sont des gènes humains ;
(c) l'hybridation de l'ADN amplifié avec au moins quatre sondes, lesdites au moins quatre sondes comprenant une ou plusieurs sondes spécifiques pour une mutation d'ADN dans chacun des gènes *KRAS, BRAF, CTNNB1,* et *APC,* dans lequel chacune desdites au moins quatre sondes est couplée à un microsupport, et dans lequel chacun des microsupports comprend en identifiant correspondant à la sonde qui lui est couplée ;
(d) la détection de la présence ou de l'absence d'hybridation de l'ADN amplifié avec lesdites au moins quatre sondes, dans lequel l'hybridation entre l'ADN amplifié et l'une des sondes indique la présence de la mutation d'ADN correspondant à la sonde ;
(e) la détection des identifiants des microsupports ; et
(f) la corrélation des identifiants détectés avec la présence ou l'absence détectée d'hybridation de l'AND amplifié aux sondes correspondantes des microsupports.

2. Procédé selon la revendication 1, dans lequel chacun desdits au moins quatre acides nucléiques de blocage comprend un oligonucléotide simple brin qui s'hybride avec le locus d'ADN de type sauvage correspondant, et une fraction 3'-terminale qui bloque l'extension à partir de l'oligonucléotide simple brin ;
dans lequel éventuellement la fraction 3'-terminale comprend une ou plusieurs désoxythymidines inversées ; et dans lequel éventuellement chacun desdits au moins quatre acides nucléiques de blocage comprend un ou plusieurs nucléotides modifiés choisis dans le groupe constitué par les acides nucléiques bloqués (LNA), les acides nucléiques peptidiques (PNA), les acides nucléiques hexosiques (HNA), les acides nucléiques thréosiques (TNA), les acides nucléiques glycoliques (GNA), et les acides nucléiques cyclohéxényliques (CeNA).

3. Procédé selon la revendication 1 ou la revendication 2, dans lequel les une ou plusieurs mutations d'ADN dans le gène *KRAS* comprennent des mutations de *KRAS* codant pour des protéines KRAS mutées G12D, G12V, G12S, et G13D ;
dans lequel éventuellement les sondes spécifiques pour une ou plusieurs mutations d'ADN dans le gène *KRAS* comprennent quatre sondes comprenant les séquences GGAGCTGATGG (SEQ ID NO : 4), GGAGCTGTTGG (SEQ ID NO : 5), TGGAGCTAGTGG (SEQ ID NO : 6), et TGGAGCTGGTGACGT (SEQ ID NO : 7), et dans lequel chacune des quatre sondes est couplée à un microsupport avec un identifiant différent ;
dans lequel éventuellement chacune des quatre sondes comprend en outre huit nucléotides à l'extrémité 5', dans lequel les huit nucléotides à l'extrémité 5' sont des nucléotides d'adénine ou de thymine, et dans lequel chacune des quatre sondes comprend au moins 24 nucléotides au total ;
dans lequel éventuellement les sondes comprennent : (1) une première sonde comprenant une séquence choisie dans le groupe constitué par GGAGCTGATGG (SEQ ID NO : 4), AGCTGATGGCGTA (SEQ ID NO : 178), TGGAGCTGATGGCG (SEQ ID NO : 179), TGGAGCTGATGG (SEQ ID NO : 180), et GCTGATGGCGTA (SEQ ID NO: 181) ; (2) une deuxième sonde comprenant une séquence choisie dans le groupe constitué par GGAGCTGTTGG (SEQ ID NO : 5), TGGAGCTGTTGGTGGC (SEQ ID NO : 182), GGAGCTGTTGGTG (SEQ ID NO : 183), TGGAGCTGTTGGT (SEQ ID NO : 184), et TGGAGCTGTAGGTGG (SEQ ID NO : 185) ; (3) une troisième sonde comprenant une séquence choisie dans le groupe constitué par TTGGAGCTAGTGGCGTA (SEQ ID NO : 186), GCTAGTGGCGTAGGC (SEQ ID NO : 187), AGCTAGTGGCGT (SEQ ID NO : 188), GTTGGAGCTAGTGG (SEQ ID NO : 189), et GGAGCTAGTGG (SEQ ID NO : 190) ; et (4) une quatrième sonde comprenant une séquence choisie dans le groupe constitué par GGTGACGTAGGCAA (SEQ ID NO : 191), TGACGTAGGCAAGAG (SEQ ID NO : 192), GCTGGTGACGTAGG (SEQ ID NO : 193), AGCTGGTGACGTAG (SEQ ID NO : 194), et GGAGCTGGTGACGT (SEQ ID NO : 195) ; et dans lequel chacune des quatre sondes est couplée à un microsupport avec un identifiant différent ;
dans lequel éventuellement les sondes comprennent : (1) une première sonde comprenant une séquence choisie dans le groupe constitué par TTTTTTTTTTTTAAGGAGCTGATGG (SEQ ID NO : 47), TTTTTTTTTTTTAGCTGATGGCGTA (SEQ ID NO : 74), TTTTTTTTTTATGGAGCTGATGGCG (SEQ ID NO : 75), TTTTTTTTTTTTATGGAGCTGATGG (SEQ ID NO : 76), et TTTTTTTTTTTTTGCTGATGGCGTA (SEQ ID NO : 77) ; (2) une deuxième sonde comprenant une séquence choisie dans le groupe constitué par TTTTTTTTTTTTAAGGAGCTGTTGG (SEQ ID NO : 48), TTTTTTTTATGGAGCTGTTGGTGGC (SEQ ID NO : 78), TTTTTTTTTTAAGGAGCTGTTGGTG (SEQ ID NO : 79), TTTTTTTTTTTATGGAGCTGTTGGT (SEQ ID NO : 80), et TTTTTTTTTATGGAGCTGTAGGTGG (SEQ ID NO : 81) ; (3) une troisième sonde comprenant une séquence choisie dans le groupe constitué par TTTTTTTTTTTATGGAGCTAGTGG (SEQ ID NO : 49), TTTTTTTTTTGGAGCTAGTGGCGTA (SEQ ID NO : 82), TTTTTAATTTGCTAGTGGCGTAGGC (SEQ ID NO : 83), TTTTTTTTTATTTAGCTAGTGGCGT (SEQ ID NO : 84), TTTTTTTTTTTGTTGGAGCTAGTGG (SEQ ID NO : 85), et TTTTTTTTTTTTAAGGAGCTAGTGG (SEQ ID NO : 86) ; et (4) une quatrième sonde comprenant une séquence choisie dans le groupe constitué par TTTTTTTTTATGGAGCTGGTGACGT (SEQ ID NO : 50), TTTTTTTTAAAGGTGACGTAGGCAA (SEQ ID NO : 87), TTTTTTTTTATGACGTAGGCAAGAG (SEQ ID NO : 88), TTTTTTTTTTTGCTGGTGACGTAGG (SEQ ID NO : 89), TTTTTTTTTTAAGCTGGTGACGTAG (SEQ ID NO : 90), et TTTTTTTTTAAGGAGCTGGTGACGT (SEQ ID NO : 91) ; et dans lequel chacune des quatre sondes est couplée à un microsupport avec un identifiant différent ;
dans lequel éventuellement l'étape (b) comprend l'amplification de l'ADN isolé par PCR à l'aide d'une paire d'amorces comprenant les séquences GTACTGGTGGAGTATTTGATAGTG (SEQ ID NO : 1) et ATCGTCAAGGCACTCTTGCCTAC (SEQ ID NO : 2) ; et
dans lequel éventuellement l'étape (b) comprend l'amplification de l'AND isolé par PCR en présence d'un acide nucléique de blocage comprenant la séquence de TA*C*G*CC*A*CC*A*G*CT(invdT)*ₙ*, où *n* vaut 1, 2, ou 3 (SEQ ID NO : 3), TT*GG*A*G*CT*GGTGGC*GTA(invdT)*ₙ*, où *n* vaut 1, 2, ou 3 (SEQ ID NO : 142), GCT*GG*T*GG*C*G*TA*G*G*C*A(invdT)*ₙ*, où *n* vaut 1, 2, ou 3 (SEQ ID NO : 143), *GCTGGTGGCGTA*GGC(invdT)*ₙ,* où *n* vaut 1, 2, ou 3 (SEQ ID NO : 144), ou TT*GG*A*G*CT*GG*T*GG*C*G*T(invdT)*ₙ*, où *n* vaut 1, 2, ou 3 (SEQ ID NO : 145), les acides nucléiques en italique représentant des acides nucléiques bloqués.

4. Procédé selon l'une quelconque des revendications 1 à 3, dans lequel les une ou plusieurs mutations d'ADN dans le gène *BRAF* comprennent au moins deux mutations de *BRAF* codant pour une protéine BRAF mutée V600E ;
dans lequel éventuellement les sondes spécifiques pour une ou plusieurs mutations d'AND dans le gène *BRAF* comprennent deux sondes comprenant les séquences TCTAGCTACAGAGAAAT (SEQ ID NO : 11) et GTCTAGCTACAGAAAAAT (SEQ ID NO : 12), et dans lequel chacune des deux sondes est couplée à un microsupport avec un identifiant différent ;
dans lequel éventuellement chacune des deux sondes comprend en outre huit nucléotides à l'extrémité 5', dans lequel les huit nucléotides à l'extrémité 5' sont des nucléotides d'adénine ou de thymine, et dans lequel chacune des deux sondes comprend au moins 24 nucléotides au total ;
dans lequel éventuellement les sondes comprennent : (1) une première sonde comprenant une séquence choisie dans le groupe constitué par TACAGAGAAATCTCGAT (SEQ ID NO : 196), TACAGAGAAATCTC (SEQ ID NO : 197), CTAGCTACAGAGAAAT (SEQ ID NO : 198), CTAGCTACAGAGAAA (SEQ ID NO : 199), et TCTAGCTACAGAG (SEQ ID NO : 200) ; et (2) une deuxième sonde comprenant une séquence choisie dans le groupe constitué par GTCTAGCTACAGAAAAATC (SEQ ID NO : 201), GTCTAGCTACAGAAAAAT (SEQ ID NO : 12), TAGCTACAGAAAAA (SEQ ID NO: 202), TCTAGCTACAGAAAAAT (SEQ ID NO : 203), et TCTAGCTACAGAAAAATC (SEQ ID NO : 204) ; et dans lequel chacune des deux sondes est couplée à un microsupport avec un identifiant différent ;
dans lequel éventuellement les sondes comprennent (1) une première sonde comprenant une séquence choisie dans le groupe constitué par TTTTTTAATTTCTAGCTACAGAGAAAT (SEQ ID NO : 51), TTTTTTTTTATACAGAGAAATCTCGAT (SEQ ID NO : 92), TTTTTTTTTAATTTACAGAGAAATCTC (SEQ ID NO : 93), TTTTTTAATTACTAGCTACAGAGAAAT (SEQ ID NO 94), TTTTTTTAATTACTAGCTACAGAGAAA (SEQ ID NO : 95), et TTTTTTTTTTAATTTCTAGCTACAGAG (SEQ ID NO : 96) ; et (2) une deuxième sonde comprenant une séquence choisie dans le groupe constitué par TTTTTTTATGTCTAGCTACAGAAAAAT (SEQ ID NO : 52), TTTTATGTCTAGCTACAGAAAAATC (SEQ ID NO : 97), TTTTTTTTATTTTTAGCTACAGAAAAA (SEQ ID NO : 98), TTTTTTTATTTCTAGCTACAGAAAAAT (SEQ ID NO : 99), et TTTTTTTTATTCTAGCTACAGAAAAATC (SEQ ID NO : 100) ; et dans lequel chacune des deux sonde est couplée à un microsupport avec un identifiant différent ;
dans lequel éventuellement l'étape (b) comprend l'amplification de l'ADN isolé par PCR à l'aide d'une paire d'amorces comprenant les séquences GGACCCACTCCATCGAGATTT (SEQ ID NO : 8) et CAGATATATTTCTTCATGAAGACCTCACAGTAA (SEQ ID NO : 9) ; et dans lequel éventuellement l'étape (b) comprend l'amplification de l'ADN isolé par PCR en présence d'un acide nucléique de blocage comprenant la séquence de G*AGAT*T*TCAC*T*GTAGC*(invdT)*ₙ,* où *n* vaut 1, 2, ou 3 (SEQ ID NO : 10), GA*G*AT*TTCACTGT*AGC(invdT)*ₙ*, où *n* vaut 1, 2, ou 3 (SEQ ID NO : 146), *GAGATTTCACTGTAGC* (invdT)*ₙ*, où *n* vaut 1, 2, ou 3 (SEQ ID NO : 147), *GAGATT TCACT*G*TAGC*(invdT)*ₙ,* où *n* vaut 1, 2, ou 3 (SEQ ID NO : 148), ou GA*G*AT*TTCACTGT*AGC(invdT)*ₙ*, où *n* vaut 1, 2, ou 3 (SEQ ID NO : 149), les acides nucléiques en italique représentant des acides nucléiques bloqués.

5. Procédé selon l'une quelconque des revendications 1 à 4, dans lequel les une ou plusieurs mutations d'ADN dans le gène *CTNNB1* comprennent des mutations de *CTNNB1* codant pour des protéines CTNNB1 mutées T41A, T41I, S45F, et S45P ;
dans lequel éventuellement les sondes spécifiques pour les une ou plusieurs mutations d'ADN dans le gène *CTNNB1* comprennent quatre sondes comprenant les séquences AGGAGCTGTGGCAG (SEQ ID NO : 16), GGAGCTGTGATA (SEQ ID NO : 17), TTTACCACTCAGAAAAG (SEQ ID NO : 21), et TACCACTCAGAGGAG (SEQ ID NO : 22), et dans lequel chacune des quatre sondes est couplée à un microsupport avec un identifiant différent ;
dans lequel éventuellement chacune des quatre sondes comprend en outre huit nucléotides à l'extrémité 5', dans lequel les huit nucléotides à l'extrémité 5' sont des nucléotides d'adénine ou de thymine, et dans lequel chacune des quatre sondes comprend au moins 24 nucléotides au total ;
dans lequel éventuellement les sondes comprennent : (1) une première sonde comprenant une séquence choisie dans le groupe constitué par AGGAGCTGTGGCAGT (SEQ ID NO : 205), AGGAGCTGTGGCAGTG (SEQ ID NO : 206), GCTGTGGCAGTGGC (SEQ ID NO : 207), GCTGTGGCAGTGGCA (SEQ ID NO : 208), et AAGGAGCTGTGGCAG (SEQ ID NO : 209) ; (2) une deuxième sonde comprenant une séquence choisie dans le groupe constitué par GGAGCTGTGATAGTGG (SEQ ID NO : 210), GAGCTGTGATAGIGGC (SEQ ID NO : 211), AGCTGTGATAGTGGCA (SEQ ID NO : 212), AGAAGGAGCTGTGATA (SEQ ID NO : 213), et GGAGCTGTGAT (SEQ ID NO : 214) ; (3) une troisième sonde comprenant une séquence choisie dans le groupe constitué par ACTCAGAAAAGGAGCT (SEQ ID NO : 215), TACCACTCAGAAAAGGA (SEQ ID NO : 216), TTTACCACTCAGAAAAGGAG (SEQ ID NO : 217), TTACCACTCAGAAAAG (SEQ ID NO : 218), et CAGAAAAGGAGCTGTG (SEQ ID NO : 219) ; et (4) une quatrième sonde comprenant une séquence choisie dans le groupe constitué par ACTCAGAGGAGGAGC (SEQ ID NO : 220), TTACCACTCAGAGGA (SEQ ID NO : 221), TTACCACTCAGAGGAGG (SEQ ID NO : 222), TTAACACTCAGAGGAG (SEQ ID NO : 223), et TTACCAATCAGAGGAGG (SEQ ID NO : 224) ; et dans lequel chacune des quatre sondes est couplée à un microsupport avec un identifiant différent ;
dans lequel éventuellement les sondes comprennent : (1) une première sonde comprenant une séquence choisie dans le groupe constitué par TTTTTTTTTTTAGGAGCTGTGGCAG (SEQ ID NO : 53), TTTTTTTTTTTAGGAGCTGTGGCAGTG (SEQ ID NO:101), TTTTTTTTTTTAGCTGTGGCAGTGGC (SEQ ID NO : 102), TTTTTTTTTTTGCTGTGGCAGTGGCA (SEQ ID NO : 103), et TTTTTTTTTTAAGGAGCTGTGGCAG (SEQ ID NO : 104); (2) une deuxième sonde comprenant une séquence choisie dans le groupe constitué par TTTTTTTTTTTTTGGAGCTGTGATA (SEQ ID NO : 54), TTTTTTTTTGGAGCTGTGATAGTGG (SEQ ID NO : 105), TTTTTTTTTGAGCTGTGATAGTGGC (SEQ ID NO : 106), TTTTTTTTTAGCTGTGATAGTGGCA (SEQ ID NO : 107), TTTTTTTTTAGAAGGAGCTGTGATA (SEQ ID NO : 108), et TTTTTTTTTTTTTTGGAGCTGTGAT (SEQ ID NO : 109) ; (3) une troisième sonde comprenant une séquence choisie dans le groupe constitué par TTTTTTTTTTTACCACTCAGAAAAG (SEQ ID NO : 55), TTTAATTTTACTCAGAAAAGGAGCT (SEQ ID NO : 110), TTTTTTAATACCACTCAGAAAAGGA (SEQ ID NO : 111), TTTTTTTTACCACTCAGAAAAGGAG (SEQ ID NO : 112), TTTTTTTTATTACCACTCAGAAAAG (SEQ ID NO : 113), et TTTTTTTTTCAGAAAAGGAGCTGTG (SEQ ID NO : 114) ; et (4) une quatrième sonde comprenant une séquence choisie dans le groupe constitué par TTTTTTTTTAATACCACTCAGAGGAG (SEQ ID NO : 56), TTTTTTTTTAAAACTCAGAGGAGGAGC (SEQ ID NO : 115), TTTTTTTTTTTATTACCACTCAGAGGA (SEQ ID NO: 116), TTTTTTTTTATTACCACTCAGAGGAGG (SEQ ID NO : 117), TTTTTTTTTTATTAACACTCAGAGGAG (SEQ ID NO : 1 18), et TTTTTTTTTATTACCAATCAGAGGAGG (SEQ ID NO : 119) ; et dans lequel chacune des quatre sondes est couplée à un microsupport avec un identifiant différent ;
dans lequel éventuellement l'étape (b) comprend l'amplification de l'ADN isolé par PCR à l'aide d'une première paire d'amorces comprenant les séquences GGAATCCATTCTGGTGCCACT (SEQ ID NO : 13) et AGAAAATCCCTGTTCCCACTCATA (SEQ ID NO : 14), et d'une deuxième paire d'amorces comprenant les séquences GGTGCCACTACCACAGCTCCT (SEQ ID NO : 18) et TCTCAAAACTGCATTCTGACTTTCA (SEQ ID NO : 19) ; et
dans lequel éventuellement l'étape (b) comprend l'amplification de l'ADN isolé par PCR en présence d'un premier acide nucléique de blocage comprenant la séquence de GC*CACTACCACAG*CT(invdT)*ₙ,* où *n* vaut 1, 2, ou 3 (SEQ ID NO : 15), T*G*C*CA*CT*ACCA*C*AG*(invdT)*ₙ,* où *n* vaut 1, 2, ou 3 (SEQ ID NO : 150), C*AC*T*ACCA*C*AGC*T*C*C(invdT)*ₙ,* où *n* vaut 1, 2, ou 3 (SEQ ID NO : 151), GC*CACTACCACAG*CT(invdT)*ₙ,* où *n* vaut 1, 2, ou 3 (SEQ ID NO : 152), ou GC*CACTACCACAG*CT(invdT)*ₙ,* où *n* vaut 1, 2, ou 3 (SEQ ID NO : 153), et d'un deuxième acide nucléique de blocage comprenant la séquence de GC*TCCTTCTCTG*AGT(invdT)*ₙ,* où *n* vaut 1, 2, ou 3 (SEQ ID NO : 20), T*CC*T*TCTC*T*GAG*T*G*G(invdT)*ₙ,* où *n* vaut 1, 2, ou 3 (SEQ ID NO : 174), GC*TCCTTCTCTG*AGT(invdT)*ₙ,* où *n* vaut 1, 2, ou 3 (SEQ ID NO : 175), T*CC*T*TCTC*T*GAG*T*G*G(invdT)*ₙ,* où *n* vaut 1, 2, ou 3 (SEQ ID NO : 176), ou G*C*T*CC*TT*CT*C*TGAG*(invdT)*ₙ,* où *n* vaut 1, 2, ou 3 (SEQ ID NO : 177), les acides nucléiques en italique représentant des acides nucléiques bloqués.

6. Procédé selon l'une quelconque des revendications 1 à 5, dans lequel les une ou plusieurs mutations d'ADN dans le gène *APC* comprennent des mutations *d'APC* codant pour des protéines APC mutées Q1367*, R1450*, décalante E1309, décalante S1465, et décalante T1556 ;
dans lequel éventuellement les sondes spécifiques pour une ou plusieurs mutations d'ADN dans le gène *APC* comprennent cinq sondes comprenant les séquences ACTGCTGAAAAGAGAGAGT (SEQ ID NO : 26), GAAATAAAAGATTGG (SEQ ID NO : 30), TTTTGGGTGTCTAAG (SEQ ID NO : 34), CAAACCAAGTGAGAA (SEQ ID NO : 38), et AGAGGCAGAAAAAAACT (SEQ ID NO : 42), et dans lequel chacune des cinq sondes est couplée à un microsupport avec un identifiant différent ;
dans lequel éventuellement chacune des cinq sondes comprend en outre huit nucléotides à l'extrémité 5', dans lequel les huit nucléotides à l'extrémité 5' sont des nucléotides d'adénine ou de thymine, et dans lequel chacune des cinq sondes comprend au moins 24 nucléotides au total ;
dans lequel éventuellement les sondes comprennent : (1) une première sonde comprenant une séquence choisie dans le groupe constitué par AAATAGCAGAAATAAAAG (SEQ ID NO : 225), GAAATAAAAGATTGGAA (SEQ ID NO : 226), AGAAATAAAAGATTG (SEQ ID NO : 227), GAAATAAATGAATGG (SEQ ID NO : 228), et CAGAAATAAAAGATT (SEQ ID NO : 229) ; (2) une deuxième sonde comprenant une séquence choisie dans le groupe constitué par TTTGGGTGTCTAAG (SEQ ID NO : 230), GGGTGTCTAAGCACCACT (SEQ ID NO : 231), CTAAGCACCACTTTT (SEQ ID NO : 232), TTTTGGGTGTCTAA (SEQ ID NO : 233), et GGTGTCTAAGCACCA (SEQ ID NO : 234) ; (3) une troisième sonde comprenant une séquence choisie dans le groupe constitué par AAGTGAGAAGTACCTAA (SEQ ID NO : 235), CAAACCAAGTGAGAA (SEQ ID NO : 38), TCAAACCAAGTGAG (SEQ ID NO : 236), ACCAAGTGAGAAGTA (SEQ ID NO : 237), et AGCTCAAACCAAGTGAG (SEQ ID NO : 238) ; (4) une quatrième sonde comprenant une séquence choisie dans le groupe constitué par GCACCTACTGCTGAA (SEQ ID NO : 239), ACCTACTGCTGAAAAG (SEQ ID NO : 240), TGCTGAAAAGAGAGAGT (SEQ ID NO : 241), ACTGCTGAAAAGAGAGAGT (SEQ ID NO : 26), et CCTACTGCTGAAAAGAGA (SEQ ID NO : 242) ; et (5) une cinquième sonde comprenant une séquence choisie dans le groupe constitué par GCAGAAAAAAACTATTG (SEQ ID NO : 243), AGAGGCAGAAAAAAACT (SEQ ID NO : 42), CAGAAAAAAACTATTGATT (SEQ ID NO : 244), AGAAAGAGGCAGAAAAAAACT (SEQ ID NO : 245), et GAGGCAGAAAAAAACTA (SEQ ID NO : 246) ; et dans lequel chacune des cinq sondes est couplée à un microsupport avec un identifiant différent ;
dans lequel éventuellement les sondes comprennent : (1) une première sonde comprenant une séquence choisie dans le groupe constitué par TTTTTTTTTTTTTGAAATAAAAGATTGG (SEQ ID NO : 58), TTTTTTTTTTAAATAGCAGAAATAAAAG (SEQ ID NO : 120), TTTTTTTTTTTGAAATAAAAGATTGGAA (SEQ ID NO : 121), TTTTTTTTTTTTTAGAAATAAAAGATTG (SEQ ID NO: 122), TTTTTTTTTTTTTGAAATAAATGAATGG (SEQ ID NO : 123), et TTTTTTTTTTTTTCAGAAATAAAAGATT (SEQ ID NO : 124) ; (2) une deuxième sonde comprenant une séquence choisie dans le groupe constitué par TTTTTTTTTTTTTGGGTGTCTAAG (SEQ ID NO : 59), TTTTTTTTTATTTGGGTGTCTAAG (SEQ ID NO: 125), TTTTTTGGGTGTCTAAGCACCACT (SEQ ID NO : 126), TTTTTTTTTCTAAGCACCACTTTT (SEQ ID NO : 127), TTTTTTTTTTTTTTGGGTGTCTAA (SEQ ID NO : 128), et TTTTTTTTTGGTGTCTAAGCACCA (SEQ ID NO : 129) ; (3) une troisième sonde comprenant une séquence choisie dans le groupe constitué par TTTTTTTTTACAAACCAAGTGAGAA (SEQ ID NO : 60), TTTTTTTTAAGTGAGAAGTACCTAA (SEQ ID NO : 130), TTTTTTTTTTTTCAAACCAAGTGAG (SEQ ID NO : 131), TTTTTTTTTTACCAAGTGAGAAGTA (SEQ ID NO : 132), et TTTTTTTTAGCTCAAACCAAGTGAG (SEQ ID NO : 133) ; (4) une quatrième sonde comprenant une séquence choisie dans le groupe constitué par TTTTTTTTACTGCTGAAAAGAGAGAGT (SEQ ID NO : 57), TTTTTTTTTTGCACCTACTGCTGAA (SEQ ID NO : 134), TTTTTTTTTACCTACTGCTGAAAAG (SEQ ID NO : 135), TTTTTTTTTGCTGAAAAGAGAGAGT (SEQ ID NO : 136), et TTTTTTTTTCCTACTGCTGAAAAGAGA (SEQ ID NO : 137) ; et (5) une cinquième sonde comprenant une séquence choisie dans le groupe constitué par TTTTTTTTTTAGAGGCAGAAAAAAACT (SEQ ID NO : 61), TTTTTTTTTTGCAGAAAAAAACTATTG (SEQ ID NO : 138), TTTTTTTTTTTCAGAAAAAAACTATTGATT (SEQ ID NO : 139), TTTTTTTAGAAAGAGGCAGAAAAAAACT (SEQ ID NO : 140), et TTTTTTTTTTTGAGGCAGAAAAAAACTA (SEQ ID NO : 141) ; et dans lequel chacune des cinq sondes est couplée à un microsupport avec un identifiant différent ;
dans lequel éventuellement l'étape (b) comprend l'amplification de l'ADN isolé par PCR à l'aide d'une première paire d'amorces comprenant les séquences TAAAAATAAAGCACCTACTGCTGAAA (SEQ ID NO : 23) et AGCTTGCTTAGGTCCACTCTCTCT (SEQ ID NO : 24) ; d'une deuxième paire d'amorces comprenant les séquences TAGGATGTAATCAGACGACACAGGA (SEQ ID NO : 27) et CAGCTGACCTAGTTCCAATCTTTTA (SEQ ID NO : 28) ; d'une troisième paire d'amorces comprenant les séquences TCTCCCTCCAAAAGTGGTGCT (SEQ ID NO : 31) et TGGCAATCGAACGACTCTCAA (SEQ ID NO : 32) ; d'une quatrième paire d'amorces comprenant les séquences GCAGAAGTAAAACACCTCCACCA (SEQ ID NO : 35) et GGTGCTTTATTTTTAGGTACTTC (SEQ ID NO : 36), les acides nucléiques en italique représentant des acides nucléiques bloqués ; et d'une cinquième paire d'amorces comprenant les séquences CAGGAAAATGACAATGGGAATG (SEQ ID NO : 39) et ATCTAATAGGTCCTTTTCAGAATCAATAG (SEQ ID NO : 40) ; et
dans lequel éventuellement l'étape (b) comprend l'amplification de l'ADN isolé par PCR en présence d'un premier acide nucléique de blocage comprenant la séquence de *CCA*C*TC*TCTCTCT*TT*T*CAGC*(invdT)*ₙ,* où *n* vaut 1, 2, ou 3 (SEQ ID NO 25), TA*GG*T*CC*ACTCTCTCTCT*TT*T*CAGC*A(invdT)*ₙ*, où *n* vaut 1, 2, ou 3 (SEQ ID NO 166), T*AGG*T*CCAC*T*CTCTCT*CTT*T*TC*AG*CA(invdT)*ₙ,* où *n* vaut 1, 2, ou 3 (SEQ ID NO : 167), C*CA*C*T*C*T*C*T*C*TC*TTTT*C*AG*C*(invdT)*ₙ*, où *n* vaut 1, 2, ou 3 (SEQ ID NO : 168), ou TA*G*GT*CC*AC*T*CT*C*TCT*C*T*T*TT*C*A*GC*A(invdT)*ₙ*, où *n* vaut 1, 2, ou 3 (SEQ ID NO 169), d'un deuxième acide nucléique de blocage comprenant la séquence de C*TTTTCTTTTAT*TT*C*T*GC*(invdT)*ₙ,* où *n* vaut 1, 2, ou 3 (SEQ ID NO 29), C*TTTTC*T*TTTA*T*TC*TG*C*(invdT)*ₙ,* où *n* vaut 1, 2, ou 3 (SEQ ID NO : 154), C*T*TT*TC*T*T*T*TATTTCTGC*(invdT)n, où *n* vaut 1, 2, ou 3 (SEQ ID NO : 155), C*T*TT*TC*T*T*T*TATTTCTGC*(invdT)n, où *n* vaut 1, 2, ou 3 (SEQ ID NO 156), ou C*T*TT*TCTTTTATTTC*TG*C*(invdT)*ₙ,* où *n* vaut 1, 2, ou 3 (SEQ ID NO : 157), d'un troisième acide nucléique de blocage comprenant la séquence de GTG*CTCA*G*ACAC*C(invdT)*ₙ*, où *n* vaut 1, 2, ou 3 (SEQ ID NO : 33), GTG*CTCA*G*ACAC*C(invdT)*ₙ*, où *n* vaut 1, 2, ou 3 (SEQ ID NO 158), A*G*T*G*GTGC*TCA*G*AC*A*C*C*C*A(invdT)*ₙ*, où *n* vaut 1, 2, ou 3 (SEQ ID NO 159), A*G*T*G*GTGC*TCA*G*AC*A*C*C*C*A(invdT)*ₙ*, où *n* vaut 1, 2, ou 3 (SEQ ID NO : 160), ou A*G*T*G*GTGC*TCA*G*AC*A*C*C*C*A(invdT)*ₙ*, où *n* vaut 1, 2, ou 3 (SEQ ID NO : 161), d'un quatrième acide nucléique de blocage comprenant la séquence de C*TTC*T*CGCT*T*GGT*T(invdT)n*,* où *n* vaut 1, 2, ou 3 (SEQ ID NO 37), G*TAC*T*T*C*TCG*CT*TGG*T(invdT)*ₙ,* où *n* vaut 1, 2, ou 3 (SEQ ID NO : 162), C*TTC*T*CGCT*T*GGT*T(invdT)*ₙ,* où *n* vaut 1, 2, ou 3 (SEQ ID NO : 163), GT*ACT*T*CTCG*CT*TGG*T(invdT)*ₙ,* où *n* vaut 1, 2, ou 3 (SEQ ID NO 164), ou GTA*CT*T*CTCG*CT*TGG*T(invdT)n, où *n* vaut 1, 2, ou 3 (SEQ ID NO : 165), et d'un cinquième acide nucléique de blocage comprenant la séquence de *C*A*ATAG*T*TTTT*T*CTGC*C(invdT)*ₙ,* où *n* vaut 1, 2, ou 3 (SEQ ID NO : 41), GA*A*T*CAATAG*TTTTTT*CTGCCT*C(invdT)*ₙ,* où *n* vaut 1, 2, ou 3 (SEQ ID NO : 170), T*CAG*A*ATC*A*ATAG*TTTTT*TCTG*(invdT)*ₙ,* où *n* vaut 1, 2, ou 3 (SEQ ID NO : 171), *G*A*A*T*CAATA*GATTTTAC*TGCCT*C(invdT)*ₙ,* où *n* vaut 1, 2, ou 3 (SEQ ID NO : 172), ou A*A*T*CAATAG*TTTTTTC*TGCCT*C(invdT)*ₙ,* où *n* vaut 1, 2, ou 3 (SEQ ID NO : 173), les acides nucléiques en italique représentant des acides nucléiques bloqués.

7. Procédé selon l'une quelconque des revendications 1 à 6, dans lequel le procédé comprend en outre :
l'amplification d'une séquence d'AND témoin positif à l'aide d'une paire d'amorces spécifique pour la séquence d'ADN témoin positif ;
l'hybridation de la séquence de gène témoin positif amplifiée avec une sonde spécifique pour la séquence de gène témoin positif, dans lequel la sonde spécifique pour la séquence de gène témoin positif est couplée à un microsupport avec un identifiant correspondant à un témoin positif ;
la détection de la présence ou de l'absence d'hybridation de la séquence d'ADN témoin positif amplifiée avec la sonde spécifique pour la séquence de gène témoin positif ; et
la détection de l'identifiant correspondant au témoin positif
dans lequel éventuellement la séquence d'ADN positif comprend une séquence d'un gène d'antigène leucocytaire humain ou d'un gène de glyceraldéhyde-3-phosphate déshydrogénase (GAPDH) humain ;
dans lequel éventuellement la paire d'amorces spécifique pour la séquence d'ADN témoin positif comprend les séquences TGAGTGTTACTTCTTCCCACACTC (SEQ ID NO : 43) et ATTGCTTTTGCGCAATCCCT (SEQ ID NO : 44) ou AATCCCATCACCATCTTCCA (SEQ ID NO : 71) et TGGACTCCACGACGTACTCA (SEQ ID NO : 72) ; et
dans lequel éventuellement la sonde spécifique pour la séquence de gène témoin positif comprend la séquence TTTTTTTTTTTTGGAGACGGTCTG (SEQ ID NO : 45) ou CTGTCTTCCACTCACTCC (SEQ ID NO : 73).

8. Procédé selon l'une quelconque des revendications 1 à 7, dans lequel le procédé comprend en outre :
la détection de l'absence d'hybridation de l'AND amplifié avec un microsupport ayant un identifiant correspondant à un témoin négatif, dans lequel le microsupport avec l'identifiant correspondant au témoin négatif comprend une sonde qui ne s'hybride pas avec l'ADN amplifié ; et
la détection de l'identifiant correspondant au témoin négatif ;
dans lequel éventuellement le microsupport avec l'identifiant correspondant au témoin négatif comprend une sonde comprenant la séquence AATATAATATATTAT (SEQ ID NO : 46).

9. Procédé selon l'une quelconque des revendications 1 à 8, dans lequel les identifiants des microsupports comprennent (a) des codes à barres numériques, ou (b) des codes à barres analogiques ;
dans lequel éventuellement chacun des microsupports comprend :
(i) une première couche de photopolymère ; (ii) une deuxième couche de photopolymère ; et (iii) une couche intermédiaire entre la première couche et la deuxième couche, la couche intermédiaire ayant un motif codé représentant l'identifiant défini sur celle-ci, dans lequel la couche intermédiaire est partiellement sensiblement transmissive et partiellement sensiblement opaque à la lumière, représentant un code correspondant au microsupport, dans lequel la surface la plus externe du microsupport comprend un photopolymère en résine photosensible, et ledit photopolymère en résine photosensible est fonctionnalisé avec la sonde spécifique pour la mutation d'ADN, et dans lequel ledit microsupport a environ la même densité que l'eau ; ou
(i) une couche de polymère sensiblement transparent ayant une première surface et une deuxième surface, les première et deuxième surfaces étant parallèles l'une à l'autre ; (ii) une couche de polymère sensiblement non transparent, dans lequel la couche de polymère sensiblement non transparent est apposée sur la première surface de la couche de polymère sensiblement transparent et renferme une portion centrale de la couche de polymère sensiblement transparent, et dans lequel la couche de polymère sensiblement non transparent comprend une forme bidimensionnelle représentant un code analogique, dans lequel le code analogique représente l'identifiant ; et (iii) la sonde spécifique pour la mutation d'ADN, dans lequel la sonde est couplée à au moins l'une parmi la première surface et la deuxième surface de la couche de polymère sensiblement transparent dans au moins la portion centrale de la couche de polymère sensiblement transparent, dans lequel éventuellement chacun des microsupports comprend en outre un indicateur d'orientation destiné à orienter le code analogique de la couche de polymère sensiblement non transparent, dans lequel éventuellement le polymère sensiblement transparent de la première ou de la deuxième couche de polymère sensiblement transparent comprend un polymère à base d'époxy, et dans lequel éventuellement le polymère à base d'époxy est SU-8.

10. Procédé selon l'une quelconque des revendications 1 à 9, dans lequel l'échantillon est un échantillon de selles.

11. Procédé selon l'une quelconque des revendications 1 à 10, dans lequel le procédé est utilisé pour détecter un cancer du côlon, un cancer du rectum, un cancer colorectal, un adénome du côlon, un adénome du rectum, ou un adénome colorectal.

12. Kit, comprenant :
(a) au moins quatre microsupports, dans lequel chacun desdits au moins quatre microsupports comprend :
(i) une sonde couplée au microsupport, dans lequel la sonde est spécifique pour une mutation d'ADN dans le gène *KRAS, BRAF, CTNNB1,* ou *APC,* dans lequel éventuellement les gènes *KRAS, BRAF, CTNNB1,* et *APC* sont des gènes humains ; et
(ii) un identifiant correspondant à la sonde qui lui est couplée ;
dans lequel le kit comprend au moins un microsupport comprenant une sonde spécifique pour une mutation d'ADN dans le gène *KRAS,* au moins un microsupport comprenant une sonde spécifique pour une mutation d'ADN dans le gène *BRAF,* au moins un microsupport comprenant une sonde spécifique pour une mutation d'ADN dans le gène *CTNNB1,* et au moins un microsupport comprenant une sonde spécifique pour une mutation d'ADN dans le gène *APC* ; et
(b) au moins quatre acides nucléiques de blocage, dans lequel chacun desdits au moins quatre acides nucléiques de blocage s'hybride avec un locus d'ADN de type sauvage correspondant à l'une des mutations d'ADN dans les gènes KRAS, BRAF, CTNNB1, ou APC.

13. Kit selon la revendication 12, dans lequel chacun desdits au moins quatre acides nucléiques de blocage comprend un oligonucléotide simple brin qui s'hybride avec le locus d'ADN de type sauvage correspondant ; et une fraction 3'-terminale qui bloque l'extension à partir de l'oligonucléotide simple brin ;
dans lequel éventuellement la fraction 3'-terminale comprend une ou plusieurs désoxythymidines inversées ; et dans lequel éventuellement chacun desdits au moins quatre acides nucléiques de blocage comprend un ou plusieurs nucléotides modifiés choisis dans le groupe constitué par les acides nucléiques bloqués (LNA), les acides nucléiques peptidiques (PNA), les acides nucléiques hexosiques (HNA), les acides nucléiques thréosiques (TNA), les acides nucléiques glycoliques (GNA), et les acides nucléiques cyclohéxényliques (CeNA).

14. Kit selon la revendication 12 ou la revendication 13, dans lequel la mutation d'ADN dans le gène *KRAS* comprend des mutations de *KRAS* codant pour des protéines KRAS mutées G12D, G12V, G12S, et G13D ;
dans lequel éventuellement le kit comprend quatre sondes comprenant les séquences GGAGCTGATGG (SEQ ID NO : 4), GGAGCTGTTGG (SEQ ID NO : 5), TGGAGCTAGTGG (SEQ ID NO : 6), et TGGAGCTGGTGACGT (SEQ ID NO : 7), et dans lequel chacune des quatre sondes est couplée à un microsupport avec un identifiant différent ; dans lequel éventuellement chacune des quatre sondes comprend en outre huit nucléotides à l'extrémité 5, dans lequel les huit nucléotides à l'extrémité 5' sont des nucléotides d'adénine ou de thymine, et dans lequel chacune des quatre sondes comprend au moins 24 nucléotides au total ;
dans lequel éventuellement le kit comprend (1) une première sonde comprenant une séquence choisie dans le groupe constitué par GGAGCTGATGG (SEQ ID NO : 4), AGCTGATGGCGTA (SEQ ID NO : 178), TGGAGCTGATGGCG (SEQ ID NO : 179), TGGAGCTGATGG (SEQ ID NO : 180), et GCTGATGGCGTA (SEQ ID NO : 181) ; (2) une deuxième sonde comprenant une séquence choisie dans le groupe constitué par GGAGCTGTTGG (SEQ ID NO : 5), TGGAGCTGTTGGTGGC (SEQ ID NO : 182), GGAGCTGTTGGTG (SEQ ID NO : 183), TGGAGCTGTTGGT (SEQ ID NO : 184), et TGGAGCTGTAGGTGG (SEQ ID NO : 185) ; (3) une troisième sonde comprenant une séquence choisie dans le groupe constitué par TTGGAGCTAGTGGCGTA (SEQ ID NO : 186), GCTAGTGGCGTAGGC (SEQ ID NO : 187), AGCTAGTGGCGT (SEQ ID NO: 188), GTTGGAGCTAGTGG (SEQ ID NO : 189), et GGAGCTAGTGG (SEQ ID NO : 190) ; et (4) une quatrième sonde comprenant une séquence choisie dans le groupe constitué par GGTGACGTAGGCAA (SEQ ID NO : 191), TGACGTAGGCAAGAG (SEQ ID NO : 192), GCTGGTGACGTAGG (SEQ ID NO : 193), AGCTGGTGACGTAG (SEQ ID NO : 194), et GGAGCTGGTGACGT (SEQ ID NO : 195) ; et dans lequel chacune des quatre sondes est couplée à un microsupport avec un identifiant différent ;
dans lequel éventuellement le kit comprend (1) une première sonde comprenant une séquence choisie dans le groupe constitué par TTTTTTTTTTTTAAGGAGCTGATGG (SEQ ID NO : 47), TTTTTTTTTTTTAGCTGATGGCGTA (SEQ ID NO : 74), TTTTTTTTTTATGGAGCTGATGGCG (SEQ ID NO : 75), TTTTTTTTTTTTATGGAGCTGATGG (SEQ ID NO : 76), et TTTTTTTTTTTTTGCTGATGGCGTA (SEQ ID NO : 77) ; (2) une deuxième sonde comprenant une séquence choisie dans le groupe constitué par TTTTTTTTTTTTAAGGAGCTGTTGG (SEQ ID NO : 48), TTTTTTTTATGGAGCTGTTGGTGGC (SEQ ID NO : 78), TTTTTTTTTTAAGGAGCTGTTGGTG (SEQ ID NO : 79), TTTTTTTTTTTATGGAGCTGTTGGT (SEQ ID NO : 80), et TTTTTTTTTATGGAGCTGTAGGTGG (SEQ ID NO : 81) ; (3) une troisième sonde comprenant une séquence choisie dans le groupe constitué par TTTTTTTTTTTATGGAGCTAGTGG (SEQ ID NO : 49), TTTTTTTTTTGGAGCTAGTGGCGTA (SEQ ID NO : 82), TTTTTAATTTGCTAGTGGCGTAGGC (SEQ ID NO : 83), TTTTTTTTTATTTAGCTAGTGGCGT (SEQ ID NO : 84), TTTTTTTTTTTGTTGGAGCTAGTGG (SEQ ID NO : 85), et TTTTTTTTTTTTAAGGAGCTAGTGG (SEQ ID NO : 86) ; et (4) une quatrième sonde comprenant une séquence choisie dans le groupe constitué par TTTTTTTTTATGGAGCTGGTGACGT (SEQ ID NO : 50), TTTTTTTTAAAGGTGACGTAGGCAA (SEQ ID NO : 87), TTTTTTTTTATGACGTAGGCAAGAG (SEQ ID NO : 88), TTTTTTTTTTTGCTGGTGACGTAGG (SEQ ID NO : 89), TTTTTTTTTTAAGCTGGTGACGTAG (SEQ ID NO : 90), et TTTTTTTTTAAGGAGCTGGTGACGT (SEQ ID NO : 91) ; et dans lequel chacune des quatre sondes est couplée à un microsupport avec un identifiant différent ;
dans lequel éventuellement le kit comprend en outre une paire d'amorces comprenant les séquences GTACTGGTGGAGTATTTGATAGTG (SEQ ID NO : l) et ATCGTCAAGGCACTCTTGCCTAC (SEQ ID NO : 2) ; et
dans lequel éventuellement le kit comprend un acide nucléique de blocage comprenant la séquence de TA*C*G*CC*A*CC*AG*C*T(invdT)*ₙ*, où *n* vaut 1, 2, ou 3 (SEQ ID NO : 3), TT*GG*A*G*CT*GG*T*GGC*GTA(invdT)*ₙ*, où *n* vaut 1, 2, ou 3 (SEQ ID NO : 142), GCT*GG*T*GG*C*G*TA*G*G*C*A(invdT)*ₙ*, où *n* vaut 1, 2, ou 3 (SEQ ID NO : 143), *GCTGGTGGCGTAGGC(invdT)ₙ,* où *n* vaut 1, 2, ou 3 (SEQ ID NO : 144), ou TT*GG*A*G*CT*GG*T*GG*C*G*T(invdT)*ₙ*, où *n* vaut 1, 2, ou 3 (SEQ ID NO : 145), les acides nucléiques en italique représentant des acides nucléiques bloqués.

15. Kit selon l'une quelconque des revendication 12 à 14, dans lequel les mutations d'ADN dans le gène *BRAF* comprennent au moins deux mutations de *BRAF* codant pour une protéine BRAF mutée V600E ;
dans lequel éventuellement le kit comprend deux sondes comprenant les séquences TCTAGCTACAGAGAAAT (SEQ ID NO : 11) et GTCTAGCTACAGAAAAAT (SEQ ID NO : 12), et dans lequel chacune des deux sondes est couplée à un microsupport avec un identifiant différent ;
dans lequel éventuellement chacune des deux sondes comprend en outre huit nucléotides à l'extrémité 5', dans lequel les huit nucléotides à l'extrémité 5' sont des nucléotides d'adénine ou de thymine, et dans lequel chacune des deux sondes comprend au moins 24 nucléotides au total ;
dans lequel éventuellement le kit comprend (1) une première sonde comprenant une séquence choisie dans le groupe constitué par TACAGAGAAATCTCGAT (SEQ ID NO : 196), TACAGAGAAATCTC (SEQ ID NO : 197), CTAGCTACAGAGAAAT (SEQ ID NO : 198), CTAGCTACAGAGAAA (SEQ ID NO : 199), et TCTAGCTACAGAG (SEQ ID NO : 200) ; et (2) une deuxième sonde comprenant une séquence choisie dans le groupe constitué par GTCTAGCTACAGAAAAATC (SEQ ID NO : 201), GTCTAGCTACAGAAAAAT (SEQ ID NO : 12), TAGCTACAGAAAAA (SEQ ID NO : 202), TCTAGCTACAGAAAAAT (SEQ ID NO : 203), et TCTAGCTACAGAAAAATC (SEQ ID NO : 204) ; et dans lequel chacune des deux sondes est couplée à un microsupport avec un identifiant différent ;
dans lequel éventuellement le kit comprend (1) une première sonde comprenant une séquence choisie dans le groupe constitué par TTTTTTAATTTCTAGCTACAGAGAAAT (SEQ ID NO : 51), TTTTTTTTTATACAGAGAAATCTCGAT (SEQ ID NO : 92), TTTTTTTTTAATTTACAGAGAAATCTC (SEQ ID NO : 93), TTTTTTAATTACTAGCTACAGAGAAAT (SEQ ID NO 94), TTTTTTTAATTACTAGCTACAGAGAAA (SEQ ID NO : 95), et TTTTTTTTTTAATTTCTAGCTACAGAG (SEQ ID NO : 96) ; et (2) une deuxième sonde comprenant une séquence choisie dans le groupe constitué par TTTTTTTATGTCTAGCTACAGAAAAAT (SEQ ID NO : 52), TTTTATGTCTAGCTACAGAAAAATC (SEQ ID NO : 97), TTTTTTTTATTTTTAGCTACAGAAAAA (SEQ ID NO : 98), TTTTTTTATTTCTAGCTACAGAAAAAT (SEQ ID NO : 99), et TTTTTTTTATTCTAGCTACAGAAAAATC (SEQ ID NO : 100) ; et dans lequel chacune des deux sonde est couplée à un microsupport avec un identifiant différent ;
dans lequel éventuellement le kit comprend en outre une paire d'amorces comprenant les séquences GGACCCACTCCATCGAGATTT (SEQ ID NO : 8) et CAGATATATTTCTTCATGAAGACCTCACAGTAA (SEQ ID NO : 9) ; et
dans lequel éventuellement le kit comprend en outre un acide nucléique de blocage comprenant la séquence de G*A*G*A*TTC*A*CTGT*A*GC(invdT)*ₙ,* où *n* vaut 1, 2, ou 3 (SEQ ID NO : 10), GA*GA*T*TTCACTGT*AGC(invdT)*ₙ,* où *n* vaut 1, 2, ou 3 (SEQ ID NO : 146), *GAGATTTCACTGTAGC* (invdT)*ₙ*, où *n* vaut 1, 2, ou 3 (SEQ ID NO : 147), *GAGAT*T*TCACT*G*TAGC*(invdT)*ₙ,* où *n* vaut 1, 2, ou 3 (SEQ ID NO : 148), ou G*AGA*T*TT*C*ACT*G*TAGC*(invdT)*ₙ*, où *n* vaut 1, 2, ou 3 (SEQ ID NO : 149), les acides nucléiques en italique représentant des acides nucléiques bloqués.

16. Kit selon l'une quelconque des revendications 12 à 15, dans lequel la mutation d'ADN dans le gène *CTNNB1* comprend des mutations de *CTNNB1* codant pour des protéines CTNNB1 mutées T41A, T41I, S45F, et S45P ;
dans lequel éventuellement le kit comprend quatre sondes comprenant les séquences AGGAGCTGTGGCAG (SEQ ID NO : 16), GGAGCTGTGATA (SEQ ID NO : 17), TTTACCACTCAGAAAAG (SEQ ID NO : 21), et TACCACTCAGAGGAG (SEQ ID NO : 22), et dans lequel chacune des quatre sondes est couplée à un microsupport avec un identifiant différent ;
dans lequel éventuellement chacune des quatre sondes comprend en outre huit nucléotides à l'extrémité 5', dans lequel les huit nucléotides à l'extrémité 5' sont des nucléotides d'adénine ou de thymine, et dans lequel chacune des quatre sondes comprend au moins 24 nucléotides au total ;
dans lequel éventuellement le kit comprend (1) une première sonde comprenant une séquence choisie dans le groupe constitué par AGGAGCTGTGGCAGT (SEQ ID NO : 205), AGGAGCTGTGGCAGTG (SEQ ID NO : 206), GCTGTGGCAGTGGC (SEQ ID NO : 207), GCTGTGGCAGTGGCA (SEQ ID NO : 208), et AAGGAGCTGTGGCAG (SEQ ID NO : 209) ; (2) une deuxième sonde comprenant une séquence choisie dans le groupe constitué par GGAGCTGTGATAGTGG (SEQ ID NO : 210), GAGCTGTGATAGIGGC (SEQ ID NO : 211), AGCTGTGATAGTGGCA (SEQ ID NO : 212), AGAAGGAGCTGTGATA (SEQ ID NO : 213), et GGAGCTGTGAT (SEQ ID NO : 214) ; (3) une troisième sonde comprenant une séquence choisie dans le groupe constitué par ACTCAGAAAAGGAGCT (SEQ ID NO : 215), TACCACTCAGAAAAGGA (SEQ ID NO : 216), TTTACCACTCAGAAAAGGAG (SEQ ID NO : 217), TTACCACTCAGAAAAG (SEQ ID NO : 218), et CAGAAAAGGAGCTGTG (SEQ ID NO : 219) ; et (4) une quatrième sonde comprenant une séquence choisie dans le groupe constitué par ACTCAGAGGAGGAGC (SEQ ID NO : 220), TTACCACTCAGAGGA (SEQ ID NO : 221), TTACCACTCAGAGGAGG (SEQ ID NO : 222), TTAACACTCAGAGGAG (SEQ ID NO : 223), et TTACCAATCAGAGGAGG (SEQ ID NO : 224) ; et dans lequel chacune des quatre sondes est couplée à un microsupport avec un identifiant différent ;
dans lequel éventuellement le kit comprend (1) une première sonde comprenant une séquence choisie dans le groupe constitué par TTTTTTTTTTTAGGAGCTGTGGCAG (SEQ ID NO : 53), TTTTTTTTTTTAGGAGCTGTGGCAGTG (SEQ ID NO: 101), TTTTTTTTTTTAGCTGTGGCAGTGGC (SEQ ID NO : 102), TTTTTTTTTTTGCTGTGGCAGTGGCA (SEQ ID NO : 103), et TTTTTTTTTTAAGGAGCTGTGGCAG (SEQ ID NO : 104); (2) une deuxième sonde comprenant une séquence choisie dans le groupe constitué par TTTTTTTTTTTTTGGAGCTGTGATA (SEQ ID NO : 54), TTTTTTTTTGGAGCTGTGATAGTGG (SEQ ID NO : 105), TTTTTTTTTGAGCTGTGATAGTGGC (SEQ ID NO : 106), TTTTTTTTTAGCTGTGATAGTGGCA (SEQ ID NO : 107), TTTTTTTTTAGAAGGAGCTGTGATA (SEQ ID NO : 108), et TTTTTTTTTTTTTTGGAGCTGTGAT (SEQ ID NO : 109) ; (3) une troisième sonde comprenant une séquence choisie dans le groupe constitué par TTTTTTTTTTTACCACTCAGAAAAG (SEQ ID NO : 55), TTTAATTTTACTCAGAAAAGGAGCT (SEQ ID NO : 110), TTTTTTAATACCACTCAGAAAAGGA (SEQ ID NO : 111), TTTTTTTTACCACTCAGAAAAGGAG (SEQ ID NO : 112), TTTTTTTTATTACCACTCAGAAAAG (SEQ ID NO : 113), et TTTTTTTTTCAGAAAAGGAGCTGTG (SEQ ID NO : 114) ; et (4) une quatrième sonde comprenant une séquence choisie dans le groupe constitué par TTTTTTTTTAATACCACTCAGAGGAG (SEQ ID NO : 56), TTTTTTTTTAAAACTCAGAGGAGGAGC (SEQ ID NO : 115), TTTTTTTTTTTATTACCACTCAGAGGA (SEQ ID NO : 116), TTTTTTTTTATTACCACTCAGAGGAGG (SEQ ID NO : 117), TTTTTTTTTTATTAACACTCAGAGGAG (SEQ ID NO : 1 18), et TTTTTTTTTATTACCAATCAGAGGAGG (SEQ ID NO : 119) ; dans lequel chacune des quatre sondes est couplée à un microsupport avec un identifiant différent ;
dans lequel éventuellement le kit comprend en outre une première paire d'amorces comprenant les séquences GGAATCCATTCTGGTGCCACT (SEQ ID NO : 13) et AGAAAATCCCTGTTCCCACTCATA (SEQ ID NO : 14), et une deuxième paire d'amorces comprenant les séquences GGTGCCACTACCACAGCTCCT (SEQ ID NO : 18) et TCTCAAAACTGCATTCTGACTTTCA (SEQ ID NO : 19) ; et
dans lequel éventuellement le kit comprend en outre un premier acide nucléique de blocage comprenant la séquence de GC*CACTACCACAG*CT(invdT)*ₙ*, où *n* vaut 1, 2, ou 3 (SEQ ID NO : 15), T*G*C*CA*C*TACCA*C*A*G(invdT)*ₙ*, où *n* vaut 1, 2, ou 3 (SEQ ID NO : 150), C*AC*T*AC*C*ACAGC*T*C*C(invdT)*ₙ,* où *n* vaut 1, 2, ou 3 (SEQ ID NO : 151), G*CCACTACCACAG*CT(invdT)*ₙ*, où *n* vaut 1, 2, ou 3 (SEQ ID NO : 152), ou G*C*C*ACTA*CCA*CAG*CT(invdT)*ₙ*, où *n* vaut 1, 2, ou 3 (SEQ ID NO : 153), et un deuxième acide nucléique de blocage comprenant la séquence de GC*TCCTTCTCTG*AGT(invdT)*ₙ*, où *n* vaut 1, 2, ou 3 (SEQ ID NO : 20), T*CC*T*TCTCTG*A*G*T*G*G(invdT)*ₙ,* où *n* vaut 1, 2, ou 3 (SEQ ID NO : 174), G*C*T*CC*T*TC*T*CTGA*GT(invdT)*ₙ*, où *n* vaut 1, 2, ou 3 (SEQ ID NO : 175), T*CC*TT*CT*CT*GAG*T*G*G(invdT)*ₙ*, où *n* vaut 1, 2, ou 3 (SEQ ID NO : 176), ou G*C*T*CC*TT*CTC*T*GAG*(invdT)*ₙ*, où *n* vaut 1, 2, ou 3 (SEQ ID NO : 177), les acides nucléiques en italique représentant des acides nucléiques bloqués.

17. Kit selon l'une quelconque des revendications 12 à 16, dans lequel la mutation d'ADN dans le gène *APC* comprend des mutations d'*APC* codant pour des protéines APC mutées Q1367*, R1450*, décalante E1309, décalante S1465, et décalante T1556 ;
dans lequel éventuellement le kit comprend cinq sondes comprenant les séquences ACTGCTGAAAAGAGAGAGT (SEQ ID NO : 26), GAAATAAAAGATTGG (SEQ ID NO : 30), TTTTGGGTGTCTAAG (SEQ ID NO : 34), CAAACCAAGTGAGAA (SEQ ID NO : 38), et AGAGGCAGAAAAAAACT (SEQ ID NO : 42), et dans lequel chacune des cinq sondes est couplée à un microsupport avec un identifiant différent ;
dans lequel éventuellement chacune des cinq sondes comprend en outre huit nucléotides à l'extrémité 5', dans lequel les huit nucléotides à l'extrémité 5' sont des nucléotides d'adénine ou de thymine, et dans lequel chacune des cinq sondes comprend au moins 24 nucléotides au total ;
dans lequel éventuellement le kit comprend (1) une première sonde comprenant une séquence choisie dans le groupe constitué par AAATAGCAGAAATAAAAG (SEQ ID NO : 225), GAAATAAAAGATTGGAA (SEQ ID NO : 226), AGAAATAAAAGATTG (SEQ ID NO : 227), GAAATAAATGAATGG (SEQ ID NO : 228), et CAGAAATAAAAGATT (SEQ ID NO : 229) ; (2) une deuxième sonde comprenant une séquence choisie dans le groupe constitué par TTTGGGTGTCTAAG (SEQ ID NO : 230), GGGTGTCTAAGCACCACT (SEQ ID NO : 231), CTAAGCACCACTTTT (SEQ ID NO : 232), TTTTGGGTGTCTAA (SEQ ID NO : 233), et GGTGTCTAAGCACCA (SEQ ID NO : 234) ; (3) une troisième sonde comprenant une séquence choisie dans le groupe constitué par AAGTGAGAAGTACCTAA (SEQ ID NO : 235), CAAACCAAGTGAGAA (SEQ ID NO : 38), TCAAACCAAGTGAG (SEQ ID NO : 236), ACCAAGTGAGAAGTA (SEQ ID NO : 237), et AGCTCAAACCAAGTGAG (SEQ ID NO : 238) ; (4) une quatrième sonde comprenant une séquence choisie dans le groupe constitué par GCACCTACTGCTGAA (SEQ ID NO : 239), ACCTACTGCTGAAAAG (SEQ ID NO : 240), TGCTGAAAAGAGAGAGT (SEQ ID NO : 241), ACTGCTGAAAAGAGAGAGT (SEQ ID NO : 26), et CCTACTGCTGAAAAGAGA (SEQ ID NO : 242) ; et (5) une cinquième sonde comprenant une séquence choisie dans le groupe constitué par GCAGAAAAAAACTATTG (SEQ ID NO : 243), AGAGGCAGAAAAAAACT (SEQ ID NO : 42), CAGAAAAAAACTATTGATT (SEQ ID NO : 244), AGAAAGAGGCAGAAAAAAACT (SEQ ID NO : 245), et GAGGCAGAAAAAAACTA (SEQ ID NO : 246) ; et dans lequel chacune des cinq sondes est couplée à un microsupport avec un identifiant différent ;
dans lequel éventuellement le kit comprend : (1) une première sonde comprenant une séquence choisie dans le groupe constitué par TTTTTTTTTTTTTGAAATAAAAGATTGG (SEQ ID NO : 58), TTTTTTTTTTAAATAGCAGAAATAAAAG (SEQ ID NO : 120), TTTTTTTTTTTGAAATAAAAGATTGGAA (SEQ ID NO : 121), TTTTTTTTTTTTTAGAAATAAAAGATTG (SEQ ID NO : 122), TTTTTTTTTTTTTGAAATAAATGAATGG (SEQ ID NO : 123), et TTTTTTTTTTTTTCAGAAATAAAAGATT (SEQ ID NO : 124) ; (2) une deuxième sonde comprenant une séquence choisie dans le groupe constitué par TTTTTTTTTTTTTGGGTGTCTAAG (SEQ ID NO : 59), TTTTTTTTTATTTGGGTGTCTAAG (SEQ ID NO : 125), TTTTTTGGGTGTCTAAGCACCACT (SEQ ID NO : 126), TTTTTTTTTCTAAGCACCACTTTT (SEQ ID NO : 127), TTTTTTTTTTTTTTGGGTGTCTAA (SEQ ID NO : 128), et TTTTTTTTTGGTGTCTAAGCACCA (SEQ ID NO : 129) ; (3) une troisième sonde comprenant une séquence choisie dans le groupe constitué par TTTTTTTTTACAAACCAAGTGAGAA (SEQ ID NO : 60), TTTTTTTTAAGTGAGAAGTACCTAA (SEQ ID NO : 130), TTTTTTTTTTTTCAAACCAAGTGAG (SEQ ID NO : 131), TTTTTTTTTTACCAAGTGAGAAGTA (SEQ ID NO : 132), et TTTTTTTTAGCTCAAACCAAGTGAG (SEQ ID NO : 133) ; (4) une quatrième sonde comprenant une séquence choisie dans le groupe constitué par TTTTTTTTACTGCTGAAAAGAGAGAGT (SEQ ID NO : 57), TTTTTTTTTTGCACCTACTGCTGAA (SEQ ID NO : 134), TTTTTTTTTACCTACTGCTGAAAAG (SEQ ID NO : 135), TTTTTTTTTGCTGAAAAGAGAGAGT (SEQ ID NO : 136), et TTTTTTTTTCCTACTGCTGAAAAGAGA (SEQ ID NO : 137) ; et (5) une cinquième sonde comprenant une séquence choisie dans le groupe constitué par TTTTTTTTTTAGAGGCAGAAAAAAACT (SEQ ID NO : 61), TTTTTTTTTTGCAGAAAAAAACTATTG (SEQ ID NO : 138), TTTTTTTTTTTCAGAAAAAAACTATTGATT (SEQ ID NO : 139), TTTTTTTAGAAAGAGGCAGAAAAAAACT (SEQ ID NO : 140), et TTTTTTTTTTTGAGGCAGAAAAAAACTA (SEQ ID NO : 141) ; et dans lequel chacune des cinq sondes est couplée à un microsupport avec un identifiant différent ;
dans lequel éventuellement le kit comprend en outre une première paire d'amorces comprenant les séquences TAAAAATAAAGCACCTACTGCTGAAA (SEQ ID NO : 23) et AGCTTGCTTAGGTCCACTCTCTCT (SEQ ID NO : 24) ; une deuxième paire d'amorces comprenant les séquences TAGGATGTAATCAGACGACACAGGA (SEQ ID NO : 27) et CAGCTGACCTAGTTCCAATCTTTTA (SEQ ID NO : 28) ; une troisième paire d'amorces comprenant les séquences TCTCCCTCCAAAAGTGGTGCT (SEQ ID NO : 31) et TGGCAATCGAACGACTCTCAA (SEQ ID NO : 32) ; une quatrième paire d'amorces comprenant les séquences GCAGAAGTAAAACACCTCCACCA (SEQ ID NO : 35) et GGTGCTTTATTTTTAGGTACTTC (SEQ ID NO : 36), les acides nucléiques en italique représentant des acides nucléiques bloqués ; et une cinquième paire d'amorces comprenant les séquences CAGGAAAATGACAATGGGAATG (SEQ ID NO : 39) et ATCTAATAGGTCCTTTTCAGAATCAATAG (SEQ ID NO : 40) ; et
dans lequel éventuellement le kit comprend en outre un premier acide nucléique de blocage comprenant la séquence de *CCA*C*TC*TCTCTCT*TT*T*CAGC*(invdT)*ₙ*, où *n* vaut 1, 2, ou 3 (SEQ ID NO : 25), TA*GG*T*CC*ACTCTCTCTCT*TT*T*CAGC*A(invdT)*ₙ*, où *n* vaut 1, 2, ou 3 (SEQ ID NO : 166), T*AGG*T*CCAC*T*CTCTCTC*TT*T*T*CAGCA(invdT)ₙ,* où *n* vaut 1, 2, ou 3 (SEQ ID NO : 167), *CCA*C*T*C*T*C*T*C*T*CTTTTCA*GC*(invdT)*ₙ*, où *n* vaut 1, 2, ou 3 (SEQ ID NO : 168), ou TA*G*GT*CC*AC*TC*T*C*TCT*C*T*T*TT*C*A*GC*A(invdT)*ₙ*, où *n* vaut 1, 2, ou 3 (SEQ ID NO : 169), un deuxième acide nucléique de blocage comprenant la séquence de C*TTTTCTTTTAT*TTCT*G*C(invdT)*ₙ*, où *n* vaut 1, 2, ou 3 (SEQ ID NO : 29), C*TTTT*CT*TTTA*T*T*TCTGC(invdT)*ₙ*, où *n* vaut 1, 2, ou 3 (SEQ ID NO : 154), C*T*TT*TC*T*T*T*TATTTCTGC*(invdT)*ₙ*, où *n* vaut 1, 2, ou 3 (SEQ ID NO : 155), C*TTT*TCT*TTT*A*T*T*TC*T*GC*(invdT)*ₙ*, où *n* vaut 1, 2, ou 3 (SEQ ID NO : 156), ou C*T*TT*T*C*TTTTATTTC*TG*C*(invdT)*ₙ*, où *n* vaut 1, 2, ou 3 (SEQ ID NO : 157), un troisième acide nucléique de blocage comprenant la séquence de GTG*CTCAGACAC*C(invdT)*ₙ*, où *n* vaut 1, 2, ou 3 (SEQ ID NO : 33), G*TGC*TC*A*G*A*C*ACC*(invdT)*ₙ*, où *n* vaut 1, 2, ou 3 (SEQ ID NO : 158), AGTGGTG*CTCAGAC*ACCCA(invdT)*ₙ*, où *n* vaut 1, 2, ou 3 (SEQ ID NO : 159), A*GTG*GT*GCTC*AG*A*C*ACCCA*(invdT)*ₙ*, où *n* vaut 1, 2, ou 3 (SEQ ID NO : 160), ou A*G*T*G*GTG*CTCA*G*AC*A*C*C*CA*(invdT)*ₙ*, où *n* vaut 1, 2, ou 3 (SEQ ID NO : 161), un quatrième acide nucléique de blocage comprenant la séquence de *CTTCTCGCTTGGTT(invdT)ₙ,* où *n* vaut 1, 2, ou 3 (SEQ ID NO : 37), G*TAC*T*TCTCG*CT*TGG*T(invdT)*ₙ*, où *n* vaut 1, 2, ou 3 (SEQ ID NO : 162), C*TTC*T*CGCT*T*GGT*T(invdT)*ₙ*, où *n* vaut 1, 2, ou 3 (SEQ ID NO : 163), GT*ACT*T*CTCG*CT*TGG*T(invdT)_{*n*,} où *n* vaut 1, 2, ou 3 (SEQ ID NO : 164), ou GT*A*C*TTCTCGC*TT*GG*T(invdT)*ₙ*, où *n* vaut 1, 2, ou 3 (SEQ ID NO : 165), et un cinquième acide nucléique de blocage comprenant la séquence de *C*A*ATAG*T*TTTT*T*CTGCC*(invdT)*ₙ,* où *n* vaut 1, 2, ou 3 (SEQ ID NO : 41), *G*A*A*T*CAATAG*TTTTTT*CTGCCT*C(invdT)_{*n*,} où *n* vaut 1, 2, ou 3 (SEQ ID NO : 170), T*CAG*A*ATC*A*ATAG*TTTTT*TCTG*(invdT)ₙ, où *n* vaut 1, 2, ou 3 (SEQ ID NO : 171), *G*A*A*T*CAATA*GATTTTAC*TGCCT*C(invdT)*ₙ*, où *n* vaut 1, 2, ou 3 (SEQ ID NO : 172), ou A*A*T*CAATAG*TTTTTTC*TGCCTC*(invdT*)ₙ,* où *n* vaut 1, 2, ou 3 (SEQ ID NO : 173), les acides nucléiques en italique représentant des acides nucléiques bloqués.

18. Kit selon l'une quelconque des revendications 12 à 17, comprenant en outre un microsupport avec un identifiant correspondant à un témoin positif et auquel une sonde spécifique pour une séquence de gène témoin positif est couplée ; et une paire d'amorces spécifique pour la séquence d'ADN témoin positif ;
dans lequel éventuellement la séquence d'ADN témoin positif comprend une séquence d'un gène d'antigène leucocytaire humain ou d'un gène de glyceraldéhyde-3-phosphate déshydrogénase (GAPDH) humain ;
dans lequel éventuellement la paire d'amorces spécifique pour la séquence d'ADN témoin positif comprend les séquences TGAGTGTTACTTCTTCCCACACTC (SEQ ID NO : 43) et ATTGCTTTTGCGCAATCCCT (SEQ ID NO : 44) ou AATCCCATCACCATCTTCCA (SEQ ID NO : 71) et TGGACTCCACGACGTACTCA (SEQ ID NO : 72) ; et
dans lequel éventuellement la sonde spécifique pour la séquence de gène témoin positif comprend la séquence TTTTTTTTTTTTGGAGACGGTCTG (SEQ ID NO : 45) ou CTGTCTTCCACTCACTCC (SEQ ID NO : 73) ;
dans lequel éventuellement le kit comprend en outre un microsupport avec un identifiant correspondant à un témoin négatif et auquel est couplée une sonde qui ne s'hybride pas avec l'ADN amplifié, et dans lequel éventuellement le microsupport avec l'identifiant correspondant au témoin négatif comprend une sonde comprenant la séquence AATATAATATATTAT (SEQ ID NO : 46).

19. Kit selon l'une quelconque des revendications 12 à 18, dans lequel les identifiants des microsupports comprennent (a) des codes à barres numériques ou (b) des codes à barres analogiques ;
dans lequel éventuellement chacun des microsupports comprend :
(i) une première couche de photopolymère ; (ii) une deuxième couche de photopolymère ; et (iii) une couche intermédiaire entre la première couche et la deuxième couche, la couche intermédiaire ayant un motif codé représentant l'identifiant défini sur celle-ci, dans lequel la couche intermédiaire est partiellement sensiblement transmissive et partiellement sensiblement opaque à la lumière, représentant un code correspondant au microsupport, dans lequel la surface la plus externe du microsupport comprend un photopolymère en résine photosensible, et ledit photopolymère en résine photosensible est fonctionnalisé avec la sonde spécifique pour la mutation d'ADN, et dans lequel ledit microsupport a environ la même densité que l'eau ; ou
(i) une couche de polymère sensiblement transparent ayant une première surface et une deuxième surface, les première et deuxième surfaces étant parallèles l'une à l'autre ; (ii) une couche de polymère sensiblement non transparent, dans lequel la couche de polymère sensiblement non transparent est apposée sur la première surface de la couche de polymère sensiblement transparent et renferme une portion centrale de la couche de polymère sensiblement transparent, et dans lequel la couche de polymère sensiblement non transparent comprend une forme bidimensionnelle représentant un code analogique, dans lequel le code analogique représente l'identifiant ; et (iii) la sonde spécifique pour la mutation d'ADN, dans lequel la sonde est couplée à au moins l'une parmi la première surface et la deuxième surface de la couche de polymère sensiblement transparent dans au moins la portion centrale de la couche de polymère sensiblement transparent, dans lequel éventuellement chacun des microsupports comprend en outre un indicateur d'orientation destiné à orienter le code analogique de la couche de polymère sensiblement non transparent, dans lequel éventuellement le polymère sensiblement transparent de la première ou de la deuxième couche de polymère sensiblement transparent comprend un polymère à base d'époxy, et dans lequel éventuellement le polymère à base d'époxy est SU-8.
